(19) Europäisches Patentamt
European Patent Office
Office européen des brevets

(11) EP 4 763 281 A2

(12) EUROPEAN PATENT APPLICATION

(43) Date of publication:
**24.06.2026 Bulletin 2026/26**

(21) Application number: **26169127.3**

(22) Date of filing: **18.03.2020**

(51) International Patent Classification (IPC):
***A61P 13/10*** *(2006.01)*

(52) Cooperative Patent Classification (CPC):
**A61K 31/519; A61K 45/06; A61P 13/10**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.03.2019 US 201962820230 P**
**05.04.2019 US 201962830302 P**
**02.05.2019 US 201962842435 P**
**12.08.2019 US 201962885767 P**
**06.09.2019 US 201962897019 P**
**23.09.2019 US 201962904429 P**

(62) Document number(s) of the earlier application(s) in accordance with Art. 76 EPC:
**20716934.3 / 3 941 473**

(71) Applicant: **Sumitomo Pharma Co., Ltd.**
**Osaka-shi, Osaka 541-0045 (JP)**

(72) Inventor: **MUDD JR., Paul N.**
**Irvine, California, 92617 (US)**

(74) Representative: **Hoffmann Eitle**
**Patent- und Rechtsanwälte PartmbB**
**Arabellastraße 30**
**81925 München (DE)**

Remarks:
This application was filed on 30-03-2026 as a divisional application to the application mentioned under INID code 62.

(54) **USE OF VIBEGRON TO TREAT OVERACTIVE BLADDER**

(57) The present disclosure is directed to a method of treating overactive bladder comprising orally administering to a subject in need thereof an amount of 75 mg of vibegron per day.

EP 4 763 281 A2

## Description

BACKGROUND

Overactive bladder (OAB) is a chronic and sometimes debilitating condition of the lower urinary tract. The function of the lower urinary tract is to store and periodically release urine. This requires the orchestration of storage and micturition reflexes which involve a variety of afferent and efferent neural pathways, leading to modulation of central and peripheral neuroeffector mechanisms, and resultant coordinated regulation of sympathetic and parasympathetic components of the autonomic nervous system as well as somatic motor pathways. These proximally regulate the contractile state of bladder (detrusor) and urethral smooth muscle, and urethral sphincter striated muscle.

Overactive bladder, from a pathophysiologic perspective, has been linked with detrusor overactivity. OAB is characterized by the symptoms of urinary urgency, with or without urgency urinary incontinence, usually associated with frequency and nocturia. The prevalence of OAB in the United States and Europe has been estimated at 16 to 17% in both women and men over the age of 18 years. Overactive bladder is most often classified as idiopathic, but can also be secondary to neurological condition, bladder outlet obstruction, and other causes.

Currently, the predominant class of drugs used to treat OAB is antimuscarinics. The clinical use of antimuscarinics is limited by modest efficacy and poor tolerability due to mechanism-based side effects including dry mouth, constipation and the potential for CNS adverse effects (e.g., cognitive impairment). High discontinuation rates have been observed for both tolterodine and oxybutynin, two commonly prescribed antimuscarinics, in both clinical trials and real-world settings. In addition, recent evidence from observational studies suggested that higher cumulative anticholinergic use is associated with an increased risk of dementia. See Gray SL, et al., JAMA Intern Med 2015;175(3): 401-407.

Beta-3 adrenergic receptor ($\beta_3$-AR) activation is an effective way of relaxing the detrusor in normal and pathogenic states. Functional evidence in support of an important role for the $\beta_3$-AR in urine storage emanates from studies *in vivo.* $\beta_3$-AR agonists have demonstrated efficacy in alleviating symptoms of OAB. To date, only one $\beta_3$-AR agonist, mirabegron (Astellas Pharma Global Development, Inc), has received marketing approval in the US and Japan for the treatment of OAB. Mirabegron activates the $\beta_3$-AR in the detrusor muscle in the bladder, which leads to muscle relaxation and an increase in bladder capacity. Reductions in micturition frequency, urinary incontinence and urgency episodes, and increases in mean volume voided per micturition were observed with mirabegron

Vibegron, (6S)-N-[4-[[(2S,5R)-5-[(R)-hydroxy(phenyl)methyl]pyrrolidin-2-yl]methyl]phenyl]-4-oxo-7,8-dihydro-6H-pyrrolo[1,2-a]pyrimidine-6-carboxamide, is a potent and highly selective beta-3 adrenergic receptor ($\beta_3$-AR) agonist demonstrating >9,000 fold selectivity for activation of $\beta_3$-AR over $\beta_2$-AR and $\beta_1$-AR in cell based *in vitro* assays. *See* Edmondson et al., J. Med. Chem. 59:609-623 (2016).

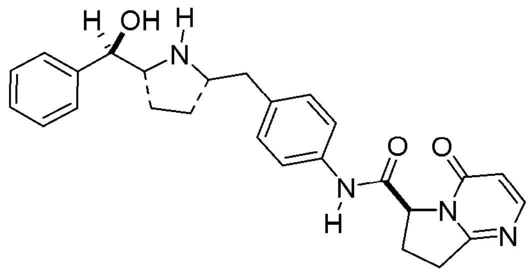

Vibegron is disclosed as a $\beta_3$-AR agonist in United States Patent Nos. 8,399,480 and 8,247,415, and WO2018/224989. Synthetic methods for preparing vibegron are disclosed in United States Publication Nos. US 2017/0145014, US 2015/0087832, US 2016/0176884 and US 2014/0242645. All of the cited publications are herein incorporated by reference in their entireties.

BRIEF DESCRIPTION OF THE DRAWINGS

Figure 1 depicts an overlay of density plots of exposure with vibegron 100 mg and 75 mg, as estimated in special populations.

Figure 2 depicts the mean (+SD) tolterodine plasma concentration vs. time profile alone and in the presence of vibegron.

Figure 3 depicts the mean (+SD) metoprolol plasma concentration vs. time profile alone and in the presence of vibegron.

Figure 4 depicts reduction in urge urinary incontinence (UUI) over time. Statistically significant onset of action was achieved at 2 weeks for the vibegron treatment.

Figure 5 depicts reduction in micturitions over time. Statistically significant onset of action was achieved at 2 weeks for the vibegron treatment.

Figure 6 depicts reduction in urgency episodes over time. Statistically significant onset of action was achieved at 2 weeks for the vibegron treatment.

Figure 7 depicts the change in urge incontinence responder rate over time. Statistically significant onset of action was achieved at 2 weeks for the vibegron treatment.

Figure 8 depicts percent of responders with 75% and 100% reductions in UUI episodes and 50% reduction in urgency episodes at week 12.

Figure 9 depicts reductions in total incontinence episodes over time. Statistically significant onset of action was achieved at 2 weeks for the vibegron treatment.

Figure 10 depicts improvement in volume voided over time. Statistically significant onset of action was achieved at 2 weeks for the vibegron treatment.

Figure 11 shows the time and events for the ABPM study.

Figure 12 shows the 90% confidence interval for treatment difference in Day 28 change from baseline for ABPM systolic blood pressure.

Figure 13 shows the 90% confidence interval for treatment difference in Day 28 change from baseline for ABPM diastolic blood pressure.

Figure 14 shows the 90% confidence interval for treatment difference in Day 28 change from baseline for ABPM heart rate.

Figure 15 shows the reduction in micturitions over 52 weeks.

Figure 16 shows the reduction in Urinary Urge Incontinence (UUI) episodes over 52 weeks.

Figure 17 shows the reduction in urgency episodes over 52 weeks.

Figure 18 shows the reduction in total incontinence episodes over 52 weeks.

Figure 19 shows the Kaplan Meier plot of time to 75% reduction in Urinary Urge Incontinence (UUI).

Figure 20 shows the Kaplan Meier plot of time to 50% reduction in urgency.

Figure 21 shows a plot of LS Mean of change from baseline urgency in the dry OAB population over time.

## SUMMARY

The present disclosure provides a method of treating overactive bladder, the method comprising orally administering to a subject in need thereof an amount of 75 mg of vibegron per day.

The present disclosure further provides a method of treating overactive bladder, the method comprising orally administering to a subject in need thereof an amount of 75 mg of vibegron per day, wherein the treatment achieves at least one of changes (1) to (5) from baseline over a treatment period:

(1) a change in average number of micturitions per 24 hours of from about -1.3 to about -2.3;

(2) a change in average number of UUI episodes per 24 hours of from about -1.5 to about -2.5 when the subject is an OAB Wet patient;

(3) a change in average number of urgency episodes per 24 hours of from about -2.2 to about -3.2;

(4) a change in average number of total incontinence episodes per 24 hours of from about -1.7 to about -2.7; and

(5) a change in average volume voided per micturition of from about 18 mL to about 30 mL.

In some embodiments, the treatment achieves a statistically significant change as compared to placebo in at least one of the following: average number of micturitions per 24 hours; average number of UUI episodes per 24 hours; average number of urgency episodes per 24 hours; average number of total incontinence episodes; and average volume voided per micturition.

The present disclosure also provides a method of treating overactive bladder in a treatment-experienced subject in need thereof, the method comprising orally administering to the subject a therapeutically effective amount of vibegron per day, wherein the therapeutically effective amount is about 75 mg, and wherein following administration of vibegron to the subject over a treatment period:

a. the decrease in the average number of micturitions in a 24-hour period in the subject is about 1.5 to about 10 more than the decrease in the average number of micturitions in a subject who receives a placebo; or

b. the average number of urge urinary incontinence (UUI) episodes per 24 hours by the subject decreases by about 1.7 to about 6 times the decrease as that of a subject treated with placebo.

The present disclosure further provides a method of improving Health-related Quality of Life (HRQL) in a subject suffering from overactive bladder symptoms, the method comprising orally administering to the subject in need thereof a therapeutically effective amount of vibegron per day over a treatment period, wherein the therapeutically effective amount is about 75 mg and wherein the improvement is compared to the HRQL of a subject receiving placebo.

The present disclosure also provides a method of treating overactive bladder in a subject in need thereof while improving Health-related Quality of Life (HRQL), the method comprising orally administering to the subject in need thereof a therapeutically effective amount of vibegron per day over a treatment period, wherein the therapeutically effective amount is about 75 mg and wherein the improvement is compared to the HRQL of a subject receiving placebo.

In some embodiments, the HRQL includes one or more subscales selected from coping, concern, sleep, social interaction, and combinations thereof.

The present disclosure further provides a method of reducing coping behaviors in a subject suffering from overactive bladder symptoms, the method comprising orally administering to the subject in need thereof a therapeutically effective amount of vibegron per day over a treatment period, wherein the therapeutically effective amount is about 75 mg and wherein the coping behaviors in the subject are reduced compared to a subject receiving placebo.

The present disclosure further provides a method of treating overactive bladder in a subject in need thereof while reducing coping behaviors, the method comprising orally administering to the subject in need thereof a therapeutically effective amount of vibegron per day over a treatment period, wherein the therapeutically effective amount is about 75 mg and wherein the coping behaviors in the subject are reduced compared to a subject receiving placebo.

The present disclosure further provides a method of maintaining ambulatory blood pressure and/or ambulatory heart rate while treating overactive bladder in a subject in need thereof, the method comprising orally administering to the subject a therapeutically effective amount of vibegron per day, wherein the therapeutically effective amount is about 75 mg.

The present disclosure also provides a method of treating overactive bladder in a subject in need thereof while maintaining ambulatory blood pressure and/or ambulatory heart rate, the method comprising orally administering to the subject a therapeutically effective amount of vibegron per day, wherein the therapeutically effective amount is about 75 mg,

## DETAILED DESCRIPTION

In order that the present disclosure can be more readily understood, certain terms are first defined. As used in this application, except as otherwise expressly provided herein, each of the following terms shall have the meaning set forth below. Additional definitions are set forth throughout the application.

In this specification and the appended embodiments, the singular forms "a," "an" and "the" include plural referents unless the context clearly dictates otherwise. The terms "a" (or "an"), as well as the terms "one or more," and "at least one" can be used interchangeably herein. In certain aspects, the term "a" or "an" means "single." In other aspects, the term "a" or "an" includes "two or more" or "multiple."

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art to which this disclosure is related.

The term "about" as used in connection with a numerical value throughout the specification and the embodiments denotes an interval of accuracy, familiar and acceptable to a person skilled in the art. In some embodiments, such interval of accuracy is $\pm$ 10 %. In other embodiments, such interval of accuracy is $\pm$ 5 %.

The term "overactive bladder" generally refers to urinary urgency, usually accompanied by frequency and nocturia, with or without urgency urinary incontinence, in the absence of urinary tract infection or other obvious pathology. The term "overactive bladder" is defined by the International Continence Society (ICS) as follows: Overactive bladder (OAB) is a symptom complex consisting of urgency with or without urge incontinence, usually with frequency and nocturia, in the absence of local pathologic or hormonal factors (Abrams P et al., Urology 2003, 61(1): 37-49; Abrams P et al., Urology 2003, 62(Supplement 5B): 28-37 and 40-42). Synonyms of overactive bladder (OAB) include "urge syndrome" and "urge frequency syndrome."

The term "urge incontinence" refers to a complaint of involuntary loss of urine.

The term "urgency urinary incontinence" (UUI) refers to a complaint of involuntary loss of urine associated with urgency and can be used interchangeably with "urge urinary incontinence" or "urge incontinence." UUI is distinguished from stress urinary incontinence, which is the involuntary loss of urine on effort or physical exertion (e.g., sporting activities), or on sneezing or coughing.

The term "impairment" as used herein means acute or chronic reduction in function. For example, renal impairment refers to a medical condition where the kidneys fail to maintain their normal function, so that waste products

and metabolites accumulate in the blood.

The term "urinary urgency" as used herein refers to a complaint of a sudden, compelling desire to void which is difficult to defer.

The term "urinary frequency" as used herein refers to a complaint by the patient who considers that he/she voids too often by day.

The term "Health-Related Quality of Life" or Health-Related QoL" (HRQL) as used herein refers to a multidomain concept that represents the patient's general perception of the effect of illness and treatment on physical, psychological, and social aspects of life. Since OAB is mainly defined by sympotoms as opposed to objective measures and has been shown to significantly affect HRQL, evaluation of treatment effectiveness usually includes an assessment of patient perception. The Overactive Bladder Questionnaire (OAB-q) was developed to assess a patient's perception of symptom bother and inpact on HRQL. The OAB-q consists of an 8-item symptom bother scale and 25 HRQL items that form 4 subscales: coping, concern, sleep, and social interaction, and provides a total HRQL score. Patients rate each item on a 6-point scale ranging from 'not at all' to 'a very great deal' for the symptom bother items. Subscales are summed and transformed into scores ranging from 0 to 100. Higher symptom bother scores indicate increasing symptom bother, while higher HRQL scores indicate better health-related quality of life. An improvement in HRQL or improving HRQL over a treatment period refers to, for example, how a subject feels or functions as a result of the targeted disease and its treatment, demonstration of a general improvement, and that no decrement was demonstrated in any domain.

The term "coping" as used herein refers to the OAB-q subscore directed toward behaviors an OAB subject uses to manage the demands of OAB. For example, the coping behaviors or tasks include, but are not limited to, planning escape routes to restrooms, carefully planning a commute, planning activities more carefully, decreasing physical activities, locating the closest restroom when arriving at a new place, adjusting travel plans, avoiding activities away from restrooms, and being uncomfortable traveling with others. Thus, for example, a reduction in coping behaviors refers to a decrease in the overall coping behaviors or a decrease in the number of instances over a treatment period in which a subject experiences any one or more of these coping behaviors, such as planning escape routes to restrooms. Similarly, an improvement in a coping domain score refers to a reduction or other change in the observed instances of a coping behavior over a treatment period.

The terms "concern" and "worry" as used herein refer to the OAB-q subscore directed toward characterizing how troubled a subject is by his/her OAB. For example, an OAB subject may be distressed, anxious about possible odor or hygiene, and/or embarrassed by his/her OAB symptoms. A reduction in the concern/worry subscore indicates, for example, a decrease in the amount of concern/worry the subject has regading his/her OAB symptoms.

The term "sleep" as used herein refers to the OAB-q subscore directed toward an OAB subject's perception of quality of sleep. For example, an OAB subject may be tired/drowsy during the day and/or not feel well-rested from waking up at night having to urinate. An improvement in sleep score indicates, for example, an increase in the subject's perception of sleep quality as it pertains to his/her OAB.

The term "social interaction" as used herein refers to the OAB-q subscore directed toward the effect of an OAB subject's symptoms on his/her socialization habits. For example, an OAB subject may feel his/her symptoms has affected relationships with family/friends, has caused problems with his/her partner, has caused him/her to stay home more often than desired, and/or has led to a decrease in social gathering participation. An improvement in social interaction score indicates, for example, a decrease in the subject's perception of the effect OAB has on his/her socializing habits.

The term "symptom bother" as used herein refers to the OAB-q subscale that includes 8 items related to frequency of urgency, nocturia, and incontinence symptoms. As explained below in Example 6, the Symptom Bother Scale items are scored from 1 (not at all) to 6 (a very great deal), with higher symptom bother scores indicating greater symptom severity. Thus, for example, a decrease in symptom bother in a subject or a decreased symptom bother score in a subject over a treatment period refers to a subject's general perception of improvement in bother associated with symptoms of OAB.

The term "free base" as used herein refers to a basic chemical compound itself, not in the form of a salt. For example, vibegron free base refers to (6S)-N-[4-[[(2S,5R)-5-[(R)-hydroxy(phenyl)methyl]pyrrolidin-2-yl]methyl]phenyl]-4-oxo-7,8-dihydro-6H-pyrrolo[1,2-a]pyrimidine-6-carboxamide.

The term "OAB wet" as used herein means overactive bladder as defined by urinary frequency and urinary urgency, with incontinence.

The term "OAB dry" as used herein means overactive bladder as defined by urinary frequency and urinary urgency, without incontinence.

The term "pharmaceutically acceptable salt" means those salts of compounds that are safe and effective for use in subjects and that possess the desired biological activity.

Pharmaceutically acceptable salts of a basic compound can be salts of organic or inorganic acids. In some embodiments, the organic and inorganic acids include but are not limited to hydrochloric acid, hydrobromic acid, sulfuric acid, phosphoric acid, citric acid, maleic acid, mandelic acid, succinic acid and methanesulfonic acid. See generally, Journal of Pharmaceutical Science, 66, 2 (1977), which is incorporated herein by reference in its entirety.

The term "Cmax" as used herein refers to the maximum plasma concentration of a drug after it is administered.

The term "Tmax" as used herein refers to the time after administration of a drug when the maximum plasma concentration is reached.

The term "AUC" as used herein refers to the area under the curve of a plot of plasma concentration versus time following administration of a drug.

The term "steady state" means that the amount of the drug reaching the system is approximately the same as the amount of the drug leaving the system. Thus, at "steady-state," the patient's body eliminates the drug at approximately the same rate that the drug becomes available to the patient's system through absorption into the blood stream.

As used herein, the terms "treated," "treating," or "treatment" or "therapy" refer to partially or completely alleviating, ameliorating, improving, relieving, delaying onset of, inhibiting progression of, reducing severity of, reducing incidence of one or more symptoms or features of disease, or any combination thereof.

In general, the term "treatment" refers to countering the effects caused as a result of the disease or pathological condition of interest in a subject including (i) inhibiting the progress of the disease or pathological condition, in other words, slowing or stopping the development or progression thereof, or one or more symptoms of such disorder or condition; (ii) relieving the disease or pathological condition, in other words, causing said disease or pathological condition, or the symptoms thereof, to regress; (iii) stabilizing the disease or pathological condition or one or more symptoms of such disorder or condition, (iv) reversing the disease or pathological condition or one or more symptoms of such disorder or condition to a normal state, (v) preventing the disease or pathological condition or one or more symptoms of such disorder or condition, and (vi) any combination thereof.

The term "treatment period" means the period of time during which the drug is administered to a subject. For example, the treatment period can be from about 2 weeks to about 2 years. In some embodiments, the treatment period can be about 2, about 4, about 6, about 8, about 10, about 12, about 14, about 16, about 18, about 20, about 24, about 52, about 76 or about 104 weeks. The efficacy of the drug can be assessed by measuring certain parameters and calculating the changes from baseline over the treatment period. The efficacy parameters includes, but are not limited to, micturitions, urge urinary incontinence episodes, total incontinence episodes, and urgency episodes. Likewise, the safety of the drug can be assessed by measuring certain parameters and calculating the changes from baseline over the treatment period. Safety parameters include, but are not limited to, systolic blood pressure, diastolic blood pressure, and heart rate.

The term "treatment-experienced" refers to a subject with OAB who has previously been treated for OAB or is currently being treated for OAB with a treatment other than vibegron. In certain aspects, the current or previous treatment is administration of an anticholinergic. Examples of antilcholinergics include, but are not limited to, atropine, belladonna alkaloids, benztropine mesylate, clidinium, cyclopentolate, darifenacin, dicylomine, fesoterodine, flavoxate, glycopyrrolate, homatropine hydrobromide, hyoscyamine, ipratropium, orphenadrine, oxybutynin, propantheline, scopolamine, methscopolamine, solifenacin, tiotropium, tolterodine, trihexyphenidyl, trospium, and salts thereof. In some embodiments, the subject was previously treated with an anticholinergic which is tolterodine. In some aspects, the current or previous treatment is administration of a beta-3 agonist. Examples of beta-3 agonists include, but are not limited to, mirabegron, and solabegron. In some embodiments, the subject was previously treated with a beta-3 agonist which is mirabegron.

Additional definitions related to urological conditions can be found, e.g., in Chapple et al. (2018) "Terminology report from the International Continence Society (ICS) Working Group on Underactive Bladder (UAB)" Neurology and Urodynamics 37:2928-2931.

Additional discussion related to HRQL and the OAB-q can be found, e.g., in Coyne et al. (2005) ) "The responsiveness of the Overactive Bladder Questionnaire (OAB-q)," Quality of Life Research 14: 849-855; and Coyne et al. (2002) "Psychometric validation of an overactive bladder symptom and health-related quality of life questionnaire: The OAB-q," Quality of Life Research 11: 563-574, each of which are which is incorporated by reference in its entirety.

<u>Methods of Treatment</u>

The present disclosure relates to a method of treating overactive bladder comprising orally administering to a subject in need thereof a dosage of vibegron such that the desired efficacy is maintained while the undesirable side effects are minimized.

The present disclosure provides a method of treating overactive bladder, the method comprising orally administering to a subject in need thereof an amount of 75 mg of vibegron per day.

The present disclosure provides a method of treating overactive bladder, the method comprising orally administering to a subject in need thereof an amount of 75 mg of vibegron per day, wherein the treatment achieves at least one of changes (1) to (5) from baseline over a treatment period:

(1) a change in average number of micturitions per 24 hours of from about -1.3 to about -2.3;
(2) a change in average number of UUI episodes per 24 hours of from about -1.5 to about -2.5 when the subject is an

OAB Wet patient;

(3) a change in average number of urgency episodes per 24 hours of from about -2.2 to about -3.2;

(4) a change in average number of total incontinence episodes per 24 hours of from about -1.7 to about -2.7; and

(5) a change in average volume voided per micturition of from about 18 mL to about 30 mL.

In some embodiments, the treatment achieves at least two, at least three, at least four, or all five of changes (1) to (5) from baseline over the treatment period.

In some embodiments, the treatment achieves changes (1) and (2) from baseline over the treatment period, i.e., (1) a change in average number of micturitions per 24 hours of from about -1.3 to about -2.3; and (2) a change in average number of UUI episodes per 24 hours of from about -1.5 to about -2.5 when the subject is an OAB Wet patient.

In some embodiments, the changes from baseline are placebo adjusted. When placebo adjusted, the changes over a treatment period can be at least one of:

(1) a change in average number of micturitions per 24 hours of from about -0.1 to about -1.0;

(2) a change in average number of UUI episodes per 24 hours of from about -0.1 to about -1.1 when the subject is an OAB Wet patient;

(3) a change in average number of urgency episodes per 24 hours of from about -0.2 to about -1.2;

(4) a change in average number of total incontinence episodes per 24 hours of from about -0.2 to about -1.2; and

(5) a change in average volume voided per micturition of from about 15 mL to about 26 mL.

In some embodiments, the treatment achieves changes (1) and (2) when placebo adjusted over the treatment period, i.e., (1) a change in average number of micturitions per 24 hours of from about -0.1 to about -1.0; and (2) a change in average number of UUI episodes per 24 hours of from about -0.1 to about -1.1 when the subject is an OAB Wet patient.

In some embodiments, the present disclosure provides a method of maintaining daytime ambulatory blood pressure while treating overactive bladder in a subject in need thereof, the method comprising orally administering to the subject a therapeutically effective amount of vibegron per day, wherein the therapeutically effective amount is about 75 mg, In some embodiments, the present disclosure provides a method of treating overactive bladder in a subject in need thereof while maintaining daytime ambulatory blood pressure, the method comprising orally administering to the subject a therapeutically effective amount of vibegron per day, wherein the therapeutically effective amount is about 75 mg. In some embodiments, the subject experiences a mean change of daytime ambulatory blood pressure from baseline over a treatment period of less than about 2.0 mm Hg. For example, Tables 101 and 102 show the mean increases in ambulatory systolic blood pressure from baseline over the 4-week treatment period are 0.89 mmHg and 0.19 mmHg (full analysis set and per protocol set, respectively), while Table 103 shows the mean increase in ambulatory diastolic blood pressure from baseline over the 4-week treatment period is 0.5 mmHg (full analysis set). In some embodiments, the subject experiences a mean change of daytime ambulatory systolic blood pressure from baseline over a treatment period, wherein the mean change from that of a subject taking a placebo has an upper bound of a 90% confidence interval less than about 3.5 mm Hg. In some embodiments, the upper bound of a 90% confidence interval is less than about 2.5 mm Hg. In some embodiments, the upper bound of a 90% confidence interval is about 2.0 mm Hg. For example, Tables 101 and 102 show that the mean daytime ambulatory systolic upper bounds of the 90% confidence interval are 2.49 and 2.00 (full analysis set and per protocol set, respectively), while Table 103 shows that the mean daytime ambulatory systolic upper bound of the 90% confidence interval is 1.11 (full analysis set).

In some embodiments, the subject experiences a mean change of daytime ambulatory systolic blood pressure from baseline over a treatment period, wherein the mean change is less than about 1.0 mm Hg from that of a subject taking a placebo, for example, about 0.1 mm Hg, 0.2 mm Hg, 0.3 mm Hg, 0.4 mm Hg, 0.5 mm Hg, 0.6, mm Hg 0.7 mm Hg, 0.8 mm Hg, 0.9 mm Hg, or a range between any two of the preceding values. In some embodiments, the subject experiences a mean change of daytime ambulatory systolic blood pressure from baseline over a treatment period, wherein the mean change is less than about 0.5 mm Hg from that of a subject taking a placebo, for example, about 0.1 mm Hg, 0.2 mm Hg, 0.3 mm Hg, 0.4 mm Hg, or a range between any two of the preceding values. For example, as shown in Tables 101 and 102, the mean changes of daytime ambulatory systolic blood pressure from baseline over the 4-week treatment period are 0.81 mmHg and 0.41 mmHg higher than that of subjects taking a placebo (full analysis set and per protocol set, respectively).

In some embodiments, the subject experiences a mean change of daytime ambulatory systolic blood pressure from baseline over a treatment period of less than about 1.0 mm Hg, for example, about 0.1 mm Hg, 0.2 mm Hg, 0.3 mm Hg, 0.4 mm Hg, 0.5 mm Hg, 0.6, mm Hg 0.7 mm Hg, 0.8 mm Hg, 0.9 mm Hg, or a range between any two of the preceding values. In some embodiments, the subject experiences a mean change of daytime ambulatory systolic blood pressure from baseline over a treatment period of less than about 0.25 mm Hg, for example, about 0.1 mm Hg, 0.11 mm Hg, 0.12 mm Hg, 0.13 mm Hg, 0.14 mm Hg, 0.15 mm Hg, 0.16 mm Hg, 0.17 mm Hg, 0.18 mm Hg, 0.19 mm Hg, 0.20 mm Hg,0.21 mm Hg, 0.22 mm Hg, 0.23 mm Hg, 0.24 mm Hg, or a range between any two of the preceding values. As shown in Table 102, for example, subjects experienced a mean change of daytime ambulatory systolic blood pressure from baseline of about 0.19

mmHg over the 4-week treatment period.

In some embodiments, the subject does not experience a mean change of daytime ambulatory diastolic blood pressure from baseline over a treatment period greater than that of a subject taking a placebo. For example, Table 103 shows that the mean change of daytime ambulatory diastolic blood pressure from baseline over the 4-week treatment period is only 0.04 mmHg lower than that of subjects taking a placebo.

In some embodiments, the subject experiences a mean change of daytime ambulatory diastolic blood pressure from baseline over a treatment period of less than about 0.75 mm Hg, for example, about 0.1 mm Hg, 1.5 mm Hg, 0.20 mm Hg, 0.25 mm Hg, 0.30 mm Hg, 0.35 mm Hg, 0.40 mm Hg, 0.45 mm Hg, 0.50 mm Hg, 0.55 mm Hg, 0.6 mm Hg, 0.65 mm Hg, 0.7 mm Hg, or a range between any two of the preceding values. As shown in Table 103, for example, subjects experienced a mean change of daytime ambulatory diastolic blood pressure from baseline of about 0.5 mmHg over the 4-week treatment period.

In some embodiments, the present disclosure provides a method of maintaining daytime ambulatory heart rate while treating overactive bladder in a subject in need thereof, the method comprising orally administering to the subject a therapeutically effective amount of vibegron per day, wherein the therapeutically effective amount is about 75 mg. In some embodiments, the present disclosure provides a method of treating overactive bladder to a subject in need thereof while maintaining daytime ambulatory heart rate, the method comprising orally administering to the subject a therapeutically effective amount of vibegron per day, wherein the therapeutically effective amount is about 75 mg. In some embodiments, the subject experiences a mean change of daytime ambulatory heart rate from baseline over a treatment period of less than about 1.25 bpm, for example, about 1.0 bpm, 1.01 bpm, 1.02 bpm, 1.03 bpm, 1.04 bpm, 1.05 bpm, 1.06 bpm, 1.07 bpm, 1.08 bpm, 1.09 bpm, 1.10 bpm, 1.11 bpm, 1.12 bpm, 1.13 bpm, 1.14 bpm, 1.15 bpm, 1.16 bpm, 1.17 bpm, 1.18 bpm, 1.19 bpm, 1.20 bpm, 1.21 bpm, 1.22 bpm, 1.23 bpm, 1.24 bpm, or a range between any two of the preceding values. As shown in Table 103, for example, subjects experienced a mean change of daytime ambulatory heart rate from baseline of about 1.08 bpm over the 4-week treatment period.

In some embodiments, the subject experiences a mean change of daytime ambulatory heart rate from baseline over a treatment period and wherein the mean change from that of a subject taking a placebo is less than about 1.0 bpm, for example, about 0.1 bpm, 0.2 bpm, 0.3 bpm, 0.4, bpm 0.5 bpm, 0.6 bpm, 0.7 bpm, 0.8 bpm, 0.9 bpm, or a range between any two of the preceding values. For example, Table 103 shows that the mean change of daytime ambulatory heart rate from baseline over the 4-week treatment period is 0.88 bpm higher that of subjects taking a placebo.

In some embodiments, the present disclosure provides a method of maintaining 24-hour ambulatory blood pressure while treating overactive bladder in a subject in need thereof, the method comprising orally administering to the subject a therapeutically effective amount of vibegron per day. In some embodiments, the present disclosure provides a method of treating overactive bladder in a subject in need thereof while maintaining 24-hour ambulatory blood pressure, the method comprising orally administering to the subject a therapeutically effective amount of vibegron per day. In some embodiments, the subject experiences a mean change of 24-hour ambulatory blood pressure from baseline over a treatment period of less than about 2.0 mm Hg. For example, Table 103 shows the mean increase in 24-hour ambulatory systolic blood pressure from baseline over the 4-week treatment period is 0.61 mmHg and the mean increase in 24-hour ambulatory diastolic blood pressure from baseline over the 4-week treatment period is 0.51 mmHg.

In some embodiments, the subject experiences a mean change of 24-hour ambulatory systolic blood pressure from baseline over a treatment period and wherein the mean change from that of a subject taking a placebo is less than about 0.75 mm Hg, for example, about 0.10 mm Hg, 0.15 mm Hg, 0.20 mm Hg, 0.25 mm Hg, 0.30 mm Hg, 0.35 mm Hg, 0.40 mm Hg, 0.45 mm Hg, 0.50 mm Hg, 0.55 mm Hg, 0.60 mm Hg, 0.65 mm Hg, 0.70 mm Hg or a range between any two of the preceding values. For example, Table 103 shows that the mean change of 24-hour ambulatory systolic blood pressure from baseline over the 4-week treatment period is 0.57 mmHg higher than that of subjects taking a placebo.

In some embodiments, the subject experiences a mean change of 24-hour ambulatory systolic blood pressure from baseline over a treatment period of less than about 0.75 mm Hg, for example, about 0.10 mm Hg, 0.15 mm Hg, 0.20 mm Hg, 0.25 mm Hg, 0.30 mm Hg, 0.35 mm Hg, 0.40 mm Hg, 0.45 mm Hg, 0.50 mm Hg, 0.55 mm Hg, 0.60 mm Hg, 0.65 mm Hg, 0.70 mm Hg or a range between any two of the preceding values. For example, Table 103 shows the mean increase in 24-hour ambulatory systolic blood pressure from baseline over the 4-week treatment period is 0.60 mmHg.

In some embodiments, the subject does not experience a mean change of 24-hour ambulatory diastolic blood pressure from baseline over a treatment period greater than that of a subject taking a placebo. For example, Table 103 shows that the mean change of 24-hour ambulatory diastolic blood pressure from baseline over the 4-week treatment period is only 0.19 mmHg lower than that of subjects taking a placebo.

In some embodiments, the subject experiences a mean change of 24-hour ambulatory diastolic blood pressure from baseline over a treatment period of less than 0.75 mm Hg, for example, 0.10 mm Hg, 0.15 mm Hg, 0.20 mm Hg, 0.25 mm Hg, 0.30 mm Hg, 0.35 mm Hg, 0.40 mm Hg, 0.45 mm Hg, 0.50 mm Hg, 0.55 mm Hg, 0.60 mm Hg, 0.65 mm Hg, 0.70 mm Hg or a range between any two of the preceding values. For example, Table 103 shows the mean increase in 24-hour ambulatory diastolic blood pressure from baseline over the 4-week treatment period is 0.51 mmHg.

In some embodiments, the present disclosure provides a method of maintaining 24-hour ambulatory heart rate

while treating overactive bladder in a subject in need thereof, the method comprising orally administering to the subject a therapeutically effective amount of vibegron per day, wherein the therapeutically effective amount is about 75 mg. In some embodiments, the present disclosure provides a method of treating overactive bladder in a subject in need thereof while maintaining 24-hour ambulatory heart rate, the method comprising orally administering to the subject a therapeutically effective amount of vibegron per day, wherein the therapeutically effective amount is about 75 mg. In some embodiments, the subject experiences a mean change of 24-hour ambulatory heart rate from baseline over a treatment period of less than about 1.0 bpm, for example, about 0.1 bpm, 0.2 bpm, 0.3 bpm, 0.4 bpm, 0.5 bpm, 0.6 bpm, 0.7 bpm, 0.8 bpm, 0.9 bpm, or a range between any two of the preceding values. As shown in Table 103, for example, subjects experienced a mean change of 24-hour ambulatory heart rate from baseline of about 0.80 over the 4-week treatment period.

In some embodiments, the subject experiences a mean change of 24-hour ambulatory heart rate from baseline over a treatment period wherein the mean change from that of a subject taking a placebo is less than about 1.0 bpm, for example, about 0.1 bpm, 0.2 bpm, 0.3 bpm, 0.4 bpm, 0.5 bpm, 0.6 bpm, 0.7 bpm, 0.8 bpm, 0.9 bpm, or a range between any two of the preceding values. For example, Table 103 shows that the mean change of 24-hour ambulatory heart rate from baseline over the 4-week treatment period is 0.96 bpm higher than that of subjects taking a placebo.

In some embodiments, the the present disclosure provides a method of maintaining one or more of: daytime ambulatory blood pressure, daytime ambulatory systolic blood pressure, daytime ambulatory diastolic blood pressure, daytime ambulatory heart rate, 24-hour ambulatory blood pressure, 24-hour ambulatory systolic blood pressure, 24-hour diastolic ambulatory blood pressure, and/or 24-hour ambulatory heart rate while treating overactive bladder in a subject in need thereof, the method comprising orally administering to the subject a therapeutically effective amount of vibegron per day, wherein the therapeutically effective amount is about 75 mg. In some embodiments, the present disclosure provides a method of treating overactive bladder while maintaining one or more of: daytime ambulatory blood pressure, daytime ambulatory systolic blood pressure, daytime ambulatory diastolic blood pressure, daytime ambulatory heart rate, 24-hour ambulatory blood pressure, 24-hour ambulatory systolic blood pressure, 24-hour diastolic ambulatory blood pressure, and/or 24-hour ambulatory heartrate, the method comprising orally administering to the subject a therapeutically effective amount of vibegron per day, wherein the therapeutically effective amount is about 75 mg.

In some embodiments, the subject experiences a mean change of systolic blood pressure at $C_{max}$ of less than about 0.50 mm Hg, for example, about 0.05 bpm, 0.1 bpm, 0.15 bpm, 0.2 bpm, 0.25 bpm, 0.3 bpm, 0.35 bpm, 0.4 bpm, 0.45 bpm or a range between any two of the preceding values. In some embodiments, the subject experiences a mean change of 24-hour systolic blood pressure of less than about 0.50 mm Hg, for example, about 0.05 bpm, 0.1 bpm, 0.15 bpm, 0.2 bpm, 0.25 bpm, 0.3 bpm, 0.35 bpm, 0.4 bpm, 0.45 bpm or a range between any two of the preceding values.

In some embodiments, the subject experiences a maximum mean change of blood pressure from baseline over 0.5 hours to 6.5 hours post-dose of less than about 2.0 mm Hg. In some embodiments, the subject experiences a maximum mean change of systolic blood pressure from baseline over 0.5 hours to 6.5 hours post-dose and wherein the maximum mean change is less than about 1.75 mm Hg from that of a subject taking a placebo, for example, about 1.0 mm Hg, 1.05 mm Hg, 1.10 mm Hg, 1.15 mm Hg, 1.20 mm Hg, 1.25 mm Hg, 1.30 mm Hg, 1.35 mm Hg, 1.40 mm Hg, 1.45 mm Hg, 1.50 mm Hg, 1.55 mm Hg, 1.60 mm Hg, 1.65 mm Hg, 1.70 mm Hg, or a range between any two of the preceding values. In some embodiments, the subject experiences a maximum mean change of systolic blood pressure from baseline over 0.5 hours to 6.5 hours post-dose of less than about 2.0 mm Hg, for example, about 1.0 mm Hg, 1.05 mm Hg, 1.10 mm Hg, 1.15 mm Hg, 1.20 mm Hg, 1.25 mm Hg, 1.30 mm Hg, 1.35 mm Hg, 1.40 mm Hg, 1.45 mm Hg, 1.50 mm Hg, 1.55 mm Hg, 1.60 mm Hg, 1.65 mm Hg, 1.70 mm Hg, 1.75 mm Hg, 1.80 mm Hg, 1.85 mm Hg, 1.90 mm Hg, 1.95 mm Hg, or a range between any two of the preceding values. In some embodiments, the subject experiences a maximum mean change of diastolic blood pressure from baseline over 0.5 hours to 6.5 hours post-dose and wherein the maximum mean change is less than about 1.25 mm Hg from that of a subject taking a placebo, for example, about 1.0 mm Hg, 1.05 mm Hg, 1.10 mm Hg, 1.15 mm Hg, 1.20 mm Hg, or a range between any two of the preceding values. In some embodiments, the subject experiences a maximum mean change of diastolic blood pressure from baseline over 0.5 hours to 6.5 hours post-dose of less than 0.75 mm Hg, for example, about 0.10 mm Hg, 0.15 mm Hg, 0.20 mm Hg, 0.25 mm Hg, 0.30 mm Hg, 0.35 mm Hg, 0.40 mm Hg, 0.45 mm Hg, 0.50 mm Hg, 0.55 mm Hg, 0.60 mm Hg, 0.65 mm Hg, 0.70 mm Hg or a range between any two of the preceding values.

In some embodiments, the subject experiences a maximum mean change of heart rate from baseline over 0.5 hours to 6.5 hours post-dose of less than about 2.0 bpm. In some embodiments, the subject experiences a maximum mean change of heart rate from baseline over 0.5 hours to 6.5 hours post-dose, wherein the maximum mean change is less than about 1.50 bpm from that of a subject taking a placebo, for example, about 1.0 bpm, 1.05 bpm, 1.10 bpm, 1.15 bpm, 1.20 bpm, 1.25 bpm, 1.30 bpm, 1.35 bpm, 1.40 bpm, 1.45 bpm, or a range between any two of the preceding values.

In some embodiments, the present disclosure provides a method of treating overactive bladder, the method comprising orally administering to a subject in need thereof an amount of from about 75 mg to about 400 mg of vibegron per day, wherein the subject experiences a smaller maximum mean change in systolic blood pressure than a subject receiving a therapeutically effective amount of mirabegron. In some embodiments, the subject receiving vibegron experiences an increase in maximum mean change in systolic blood pressure of from about 1.5 mm Hg to about 4.0 mm Hg smaller than a

subject receiving a therapeutically effective amount of mirabegron.

In some embodiments, the present disclosure provides a method of treating overactive bladder, the method comprising orally administering to a subject in need thereof an amount of from about 75 mg to about 400 mg of vibegron per day, wherein the subject experiences a smaller mean 24-hour change in systolic blood pressure than a subject receiving a therapeutically effective amount of mirabegron. In some embodiments, the subject receiving vibegron experiences an increase in mean 24-hour change in systolic blood pressure of from about 1.0 mm Hg to about 10.0 mm Hg smaller than a subject receiving a therapeutically effective amount of mirabegron.

In some embodiments, the present disclosure provides a method of treating overactive bladder, the method comprising orally administering to a subject in need thereof an amount of from about 75 mg to about 400 mg of vibegron per day, wherein the subject experiences a smaller maximum mean increase in heart rate than a subject receiving a therapeutically effective amount of mirabegron. In some embodiments, the subject receiving vibegron experiences an increase in maximum mean increase in heart rate that is from about 2 bpm to about 14 bpm smaller than a subject receiving a therapeutically effective amount of mirabegron.

In some embodiments, the amount of vibegron per day is from about 75 mg to about 200 mg. In some embodiments, the amount of vibegron per day is less than about 400 mg or less than about 200 mg. In some embodiments, the amount of vibegron per day is more than about 75 mg.

In some embodiments, the therapeutically effective amount of mirabegron is from about 50 mg to about 200 mg. In some embodiments, the therapeutically effective amount of mirabegron is 50 mg, 100 mg, or 200 mg.

In some embodiments, the present disclosure provides a method of treating overactive bladder, the method comprising orally administering to a subject in need thereof an amount of 75 mg of vibegron per day, wherein the subject has a body weight of greater than about 65 kg and wherein the subject experiences a similar mean change of systolic blood pressure from baseline over a treatment period compared to a subject taking placebo.

In some embodiments, the present disclosure provides a method of treating overactive bladder, the method comprising orally administering to a subject in need thereof an amount of 75 mg of vibegron per day, wherein the subject is at or over the age of about 67 years and wherein the subject experiences a similar mean change of systolic blood pressure from baseline over a treatment period compared to a subject taking placebo.

In some embodiments, the present disclosure provides a method of treating overactive bladder in a subject with bladder outlet obstruction (BOO), the method comprising orally administering to a subject in need thereof an amount of 75 mg of vibegron per day. In some aspects, the subject does not experience urinary retention. In some aspects, the subject experiences urinary retention. In some embodiments, the method comprises monitoring the subject for signs and/or symptoms of urinary retention.

In various embodiments related to ambulatory blood pressure and heart rate, the subject is a human. In some embodiments, the human is a male. In some embodiments, the human is a female. In some embodiments, the human has a body weight greater than about 65 kg. In some embodiments, the human has a body weight less than about 65 kg. In some embodiments, the human is at or over the age of about 67 years. In some embodiments, the human is from the age of about 67 years to about 75 years. In some embodiments, the human is under the age of about 67 years.

In various embodiments related to ambulatory blood pressure and heart rate, the human is hypertensive or is at risk of becoming hypertensive. In other various embodiments related to ambulatory blood pressure and heart rate, the human has hypertension or is at risk of hypertension. The term "hypertensive," or "hypertension," as used herein refers to a subject having high blood pressure, or a subject taking an antihypertensive medication. High blood pressure generally means a systolic blood pressure (SBP) of about 139 mmHg or more, a diastolic blood pressure (DBP) of about 89 mmHg or more, or both.

In some embodiments, the human has chronic kidney disease. In some embodiments, the chronic kidney disease is Stage 1, Stage 2, Stage 3a, or Stage 3b. In some embodiments, the human has an estimated glomerular filtration rate (eGFR) of greater than about 30 mL/min/1.73m$^2$. In some embodiments, the eGFR is selected from about 30 to about 44 mL/min/1.73m$^2$, about 45 to about 59 mL/min/1.73m$^2$, about 60 to about 89 mL/min/1.73m$^2$, or about 90 or higher mL/min/1.73m$^2$. In some embodiments, the eGFR is greater than about 72 mL/min/1.73m$^2$. the eGFR is less than about 72 mL/min/1.73m$^2$.

The present disclosure also provides a method of reducing the average number of micturitions per 24 hours in a subject suffering from overactive bladder, the method comprising orally administering to the subject in need thereof an amount of 75 mg of vibegron per day over a treatment period.

The present disclosure also provides a method of treating overactive bladder while reducing the average number of micturitions per 24 hours in a subject in need thereof, the method comprising orally administering to the subject an amount of 75 mg of vibegron per day over a treatment period.

The present disclosure also provides a method of reducing the average number of UUI episodes per 24 hours in a subject suffering from overactive bladder, the method comprising orally administering to the subject in need thereof an amount of 75 mg of vibegron per day over a treatment period.

The present disclosure also provides a method of treating overactive bladder while reducing the average number

of UUI episodes per 24 hours in a subject in need thereof, the method comprising orally administering to the subject an amount of 75 mg of vibegron per day over a treatment period.

The present disclosure also provides a method of reducing the average number of urgency episodes per 24 hours in a subject suffering from overactive bladder, the method comprising orally administering to the subject in need thereof an amount of 75 mg of vibegron per day over a treatment period.

The present disclosure also provides a method of treating overactive bladder while reducing the average number of urgency episodes per 24 hours in a subject in need thereof, the method comprising orally administering to the subject an amount of 75 mg of vibegron per day over a treatment period.

The present disclosure also provides a method of reducing the average number of total incontinence episodes per 24 hours in a subject suffering from overactive bladder, the method comprising orally administering to the subject in need thereof an amount of 75 mg of vibegron per day over a treatment period.

The present disclosure also provides a method of treating overactive bladder while reducing the average number of total incontinence episodes per 24 hours in a subject in need thereof, the method comprising orally administering to the subject an amount of 75 mg of vibegron per day over a treatment period.

The present disclosure also provides a method of increasing the average volume voided per micturition in a subject suffering from overactive bladder, the method comprising orally administering to the subject in need thereof an amount of 75 mg of vibegron per day over a treatment period.

The present disclosure also provides a method of treating overactive bladder while increasing the average volume voided per micturition in a subject in need thereof, the method comprising orally administering to the subject an amount of 75 mg of vibegron per day over a treatment period.

The present disclosure provides a method of treating overactive bladder, the method comprising orally administering to a subject in need thereof an amount of 75 mg of vibegron per day, wherein the treatment achieves at least one of changes (1) to (5) over a 12-week treatment period:

(1) a reduction in average number of micturitions per 24 hours that is greater than or equivalent to that achieved with tolterodine extended release (ER) 4mg;
(2) a reduction in average number of UUI episodes per 24 hours that is greater than or equivalent to that achieved with tolterodine ER 4mg, when the subject is an OAB Wet patient;
(3) a reduction in average number of urgency episodes per 24 hours that is greater than or equivalent to that achieved with tolterodine ER 4mg;
(4) a reduction in average number of total incontinence episodes per 24 hours that is greater than or equivalent to that achieved with tolterodine ER 4mg; and
(5) an increase in average volume voided per micturition that is greater than or equivalent to that achieved with tolterodine ER 4mg.

In some embodiments, the treatment achieves in at least one of changes (1) to (5) a change that is greater than that achieved with tolterodine ER 4mg. In some embodiments, the treatment achieves in at least one of changes (1) to (5) a change that is equivalent to that achieved with tolterodine ER 4mg.

In some embodiments, the treatment achieves in changes (2) and (4) a change that is greater than that achieved with tolterodine ER 4mg and a change that is greater than that achieved with 50 mg or 100 mg of vibegron. In some embodiments, the treatment achieves in change (1) a change that is greater than that achieved with tolterodine ER 4mg but less than that achieved with 50 mg or 100 mg of vibegron. In some embodiments, the treatment achieves in change (5) a change that is greater than that achieved with tolterodine ER 4mg but less than that achieved with 50 mg or 100 mg of vibegron.

In some embodiments the treatment achieves at least one of changes (1) to (5) over a 12-week treatment period:

(1) a reduction in average number of micturitions per 24 hours that is greater than that achieved with an equivalent dose of mirabegron;
(2) a reduction in average number of UUI episodes per 24 hours that is greater than that achieved with an equivalent dose of mirabegron, when the subject is an OAB Wet patient;
(3) a reduction in average number of urgency episodes per 24 hours that is greater than that achieved with an equivalent dose of mirabegron;
(4) a reduction in average number of total incontinence episodes per 24 hours that is greater than that achieved with an equivalent dose of mirabegron; and
(5) an increase in average volume voided per micturition that is greater than that achieved with an equivalent dose of mirabegron.

In some embodiments the treatment achieves at least one of changes (1) to (5) over a 12-week treatment period:

(1) a reduction in average number of micturitions per 24 hours that is greater than or equivalent to that achieved with 50 mg or 100 mg of vibegron;

(2) a reduction in average number of UUI episodes per 24 hours that is greater than or equivalent to that achieved with 50 mg or 100 mg of vibegron, when the subject is an OAB Wet patient;

(3) a reduction in average number of urgency episodes per 24 hours that is greater than or equivalent to that achieved with 50 mg or 100 mg of vibegron;

(4) a reduction in average number of total incontinence episodes per 24 hours that is greater than or equivalent to that achieved with 50 mg or 100 mg of vibegron; and

(5) an increase in average volume voided per micturition that is greater than or equivalent to that achieved with 50 mg or 100 mg of vibegron.

In some embodiments, the treatment achieves in changes (2) and (4) a change that is greater than that achieved with 50 mg or 100 mg of vibegron.

In some embodiments the treatment achieves at least one of changes (1) or (5) over a 12-week treatment period:

(1) a reduction in average number of micturitions per 24 hours that is less than that achieved with 50 mg or 100 mg of vibegron; or

(5) an increase in average volume voided per micturition that is less than that achieved with 50 mg or 100 mg of vibegron.

The present disclosure also provides a method of treating overactive bladder while reducing the average number of micturitions per 24 hours in a subject in need thereof, the method comprising orally administering to the subject an amount of 75 mg of vibegron per day over a 12-week treatment period, wherein the reduction is greater than or equivalent to that achieved with tolterodine extended release (ER) 4mg.

The present disclosure also provides a method of reducing the average number of micturitions per 24 hours in a subject suffering from overactive bladder, the method comprising orally administering to the subject in need thereof an amount of 75 mg of vibegron per day over a 12-week treatment period, wherein the reduction is greater than or equivalent to that achieved with tolterodine extended release (ER) 4mg.

In some embodiments, the subject is a human at or over the age of 65 years. In some embodiments, the reduction or decrease in average number of micturitions in a 24-hour period for said subject is between about 1.0 and about 2.5 times greater than that achieved with a placebo, for example, about 1.0, 1.1, 1.2, 1.3, 1.4, 1.5, 1.6, 1.7, 1.8, 1.9, 2.0, 2.1, 2.2, 2.3, 2.4, 2.5, or a range between any two of the preceding values. In some embodiments, the reduction or decrease in average number of micturitions in a 24-hour period is between about 1.5 and about 2.0 times greater than that achieved with a placebo. In some embodiments, the reduction or decrease in average number of micturitions in a 24-hour period is between about 0.5 and about 2.0 times greater than that achieved with tolterodine extended release (ER) 4mg, for example, about 0.5, 0.6, 0.7, 0.8, 0.9, 1.0, 1.1, 1.2. 1.3, 1.4, 1.5, 1.6, 1.7, 1.8, 1.9, 2.0, or a range between any two of the preceding values. In some embodiments, the reduction or decrease in average number of micturitions in a 24-hour period is between about 1.0 and about 1.5 times greater than that achieved with tolterodine extended release (ER) 4mg.

In some embodiments, the present disclosure provides a method of reducing the average number of urgency episodes per 24 hours by about -2.2 to about -3.5 in a subject at or over the age of 65 in need thereof, the method comprising orally administering to the subject a therapeutically effective amount of vibegron or a pharmaceutically acceptable salt thereof, wherein the therapeutically effective amount is about 75 mg per day. In some embodiments, the present disclosure provides a method of treating overactive bladder while reducing the average number of urgency episodes per 24 hours by about -2.2 to about -3.5 in a subject at or over the age of 65 in need thereof, the method comprising orally administering to the subject a therapeutically effective amount of vibegron or a pharmaceutically acceptable salt thereof, wherein the therapeutically effective amount is about 75 mg per day. In some embodiments, the subject achieves a change in average number of micturitions per 24 hours of from about -1.3 to about -2.5; and/or a change in average number of UUI episodes per 24 hours of from about -1.5 to about -2.5 when the subject is an OAB Wet patient. In some embodiments, the human is over the age of 75 years.

In some embodiments, the subject received an anticholinergic up to 12 months before vibegron administration. In some embodiments, the reduction or decrease in average number of micturitions in a 24-hour period is between about 1.0 and about 2.5 times greater than that achieved with a placebo, for example, about 1.0, 1.1, 1.2, 1.3, 1.4, 1.5, 1.6, 1.7, 1.8, 1.9, 2.0, 2.1, 2.2, 2.3, 2.4, 2.5, or a range between any two of the preceding values. In some embodiments, the reduction or decrease in average number of micturitions in a 24-hour period is between about 1.5 and about 2.0 times greater than that achieved with a placebo. In some embodiments, the reduction or decrease in average number of micturitions in a 24-hour period is between about 0.5 and about 2.0 times greater than that achieved with tolterodine extended release (ER) 4mg, for example, about 0.5, 0.6, 0.7, 0.8, 0.9, 1.0, 1.1, 1.2. 1.3, 1.4, 1.5, 1.6, 1.7, 1.8, 1.9, 2.0, or a range between any two of the preceding values. In some embodiments, the reduction or decrease in average number of micturitions in a 24-hour period

is between about 1.0 and about 1.5 times greater than that achieved with tolterodine extended release (ER) 4mg.

In some embodiments, the subject received a beta-3 agonist other than vibegron up to 12 months before vibegron administration. In some embodiments, the reduction or decrease in the average number of micturitions in a 24-hour period is between about 1 and about 10 fewer than average number of micturitions in a subject who receives a placebo, for example, about 1, 2, 3, 4, 5, 6, 7, 8, 9, 10, or a range between any two of the preceding values. In some embodiments, the reduction or decrease in average number of micturitions in a 24-hour period is between about 1 and about 4 fewer than the average number of micturitions in a subject who receives a placebo. In some embodiments, the reduction or decrease in average number of micturitions in a 24-hour period is between about 1.0 and about 3.0 times greater than that achieved with tolterodine extended release (ER) 4mg for example, about 1.0, 1.1, 1.2, 1.3, 1.4, 1.5, 1.6, 1.7, 1.8, 1.9 , 2.0, 2.1, 2.2, 2.3, 2.4, 2.5, 2.6, 2.7, 2.8, 2.9, 3.0, or a range between any two of the preceding values. In some embodiments, the reduction or decrease in average number of micturitions in a 24-hour period is between 2.0 and 2.5 times greater than that achieved with tolterodine extended release (ER) 4mg

The present disclosure also provides a method of reducing the average number of UUI episodes per 24 hours in a subject suffering from overactive bladder, the method comprising orally administering to the subject in need thereof an amount of 75 mg of vibegron per day over a 12-week treatment period, wherein the reduction is greater than or equivalent to that achieved with tolterodine extended release (ER) 4mg.

In some embodiments, the subject is a human at or over the age of 65 years. In some embodiments, the reduction or decrease in average number of UUI episodes in a 24-hour period for said subject is between about 0.5 and about 2.0 times greater than that achieved with a placebo, for example, about 0.5, 0.6, 0.7, 0.8, 0.9, 1.0, 1.1, 1.2, 1.3, 1.4, 1.5, 1.6, 1.7, 1.8, 1.9, 2.0, or a range between any two of the preceding values. In some embodiments, the reduction or decrease in average number of UUI episodes in a 24-hour period for said subject is between about 1.25 and about 1.75 times greater than that achieved with a placebo.

In some embodiments, the subject received an anticholinergic up to 12 months before vibegron administration. In some embodiments, the reduction or decrease in average number of UUI episodes in a 24-hour period is between about 1.0 and about 2.5 times greater than that achieved with a placebo, for example, about 1.0, 1.1, 1.2, 1.3, 1.4, 1.5, 1.6, 1.7, 1.8, 1.9, 2.0, 2.1, 2.2, 2.3, 2.4, 2.5, or a range between any two of the preceding values. In some embodiments, the reduction or decrease in average number of UUI episodes in a 24-hour period is between about 1.5 and about 2.0 times greater than that achieved with a placebo. In some embodiments, the reduction or decrease in average number of UUI episodes in a 24-hour period is between about 0.5 and about 2.0 times greater than that achieved with tolterodine extended release (ER) 4mg for example, about 0.5, 0.6, 0.7, 0.8, 0.9, 1.0, 1.1, 1.2, 1.3, 1.4, 1.5, 1.6, 1.7, 1.8, 1.9, 2.0, or a range between any two of the preceding values. In some embodiments, the reduction or decrease in average number of UUI episodes in a 24-hour period is between about 1.25 and about 1.75 times greater than that achieved with tolterodine extended release (ER) 4mg.

In some embodiments, the subject received a beta-3 agonist other than vibegron up to 12 months before vibegron administration. In some embodiments, the reduction or decrease in the average number of UUI episodes in a 24-hour period is between about 2 and about 10 times greater than that achieved with a placebo, for example, about 2, 3, 4, 5, 6, 7, 8, 9, 10, or a range between any two of the preceding values. In some embodiments, the reduction or decrease in the average number of UUI episodes in a 24-hour period is between about 4 and about 6 times greater than that achieved with a placebo. In some embodiments, the reduction or decrease in average number of UUI episodes in a 24-hour period is between about 0.5 and about 3.5 times greater than that achieved with tolterodine extended release (ER) 4mg, for example, about 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5,2.6, 2.7, 2.8, 2.9, 3.0, 3.1, 3.2, 3.3, 3.4, 3.5, or a range between any two of the preceding values. In some embodiments, the reduction or decrease in average number of UUI episodes in a 24-hour period is between about 1.5 and about 3.0 times greater than that achieved with tolterodine extended release (ER) 4mg.

The present disclosure also provides a method of reducing the average number of urgency episodes per 24 hours in a subject suffering from overactive bladder, or a method of treating overactive bladder in a subject in need thereof while reducing the average number of urgency episodes per 24 hours, the method comprising orally administering to the subject in need thereof an amount of 75 mg of vibegron per day over a 12-week treatment period, wherein the reduction is greater than or equivalent to that achieved with tolterodine extended release (ER) 4mg.

The present disclosure also provides a method of reducing the average number of total incontinence episodes per 24 hours in a subject suffering from overactive bladder, or a method of treating overactive bladder in a subject in need thereof while reducing the average number of total incontinence episodes per 24 hours, the method comprising orally administering to the subject in need thereof an amount of 75 mg of vibegron per day over a 12-week treatment period, wherein the reduction is greater than or equivalent to that achieved with tolterodine extended release (ER) 4mg.The present disclosure also provides a method of increasing the average volume voided per micturition in a subject suffering from overactive bladder, or a method of treating overactive bladder in a subject in need thereof while increasing the average volume voided per micturition, the method comprising orally administering to the subject in need thereof an amount of 75 mg of vibegron per day over a 12-week treatment period, wherein the increase is greater than or equivalent to

that achieved with tolterodine extended release (ER) 4mg.

In some embodiments, the reduction of average number of micturitions per 24 hours, the average number of UUI episodes per 24 hours, the average number of urgency episodes per 24 hours, or the average number of total incontinence episodes per 24 hours is a reduction that is greater than that achieved with tolterodine ER 4mg. In some embodiments the reduction of average number of micturitions per 24 hours, the average number of UUI episodes per 24 hours, the average number of urgency episodes per 24 hours, or the average number of total incontinence episodes per 24 hours is a reduction that is equivalent to that achieved with tolterodine ER 4mg.

In some embodiments the present disclosure provides a method of treating overactive bladder while reducing the average number of UUI episodes in a subject wherein the subject is female. In some embodiments the reduction in UUI episodes in a female subject is greater than that achieved with a male subject.

In some embodiments, the increase in the average volume voided per micturition is an increase that is greater than that achieved with tolterodine ER 4mg. In some embodiments the increase in the average volume voided per micturition is an increase that is equivalent to that achieved with tolterodine ER 4mg.

The present disclosure also provides a method of reducing the average number of micturitions per 24 hours in a subject suffering from overactive bladder, or a method of treating overactive bladder in a subject in need thereof while reducing the average number of micturitions per 24 hours, the method comprising orally administering to the subject in need thereof an amount of 75 mg of vibegron per day over a 12-week treatment period, wherein the reduction is greater than that achieved with an equivalent dose of mirabegron.

The present disclosure also provides a method of reducing the average number of UUI episodes per 24 hours in a subject suffering from overactive bladder, or a method of treating overactive bladder in a subject in need thereof while reducing the average number of UUI episodes per 24 hours, the method comprising orally administering to the subject in need thereof an amount of 75 mg of vibegron per day over a 12-week treatment period, wherein the reduction is greater than that achieved with an equivalent dose of mirabegron.

The present disclosure also provides a method of reducing the average number of urgency episodes per 24 hours in a subject suffering from overactive bladder, or a method of treating overactive bladder in a subject in need thereof while reducing the average number of urgency episodes per 24 hours, the method comprising orally administering to the subject in need thereof an amount of 75 mg of vibegron per day over a 12-week treatment period, wherein the reduction is greater than that achieved with an equivalent dose of mirabegron.

The present disclosure also provides a method of reducing the average number of total incontinence episodes per 24 hours in a subject suffering from overactive bladder, or a method of treating overactive bladder in a subject in need thereof while reducing the average number of total incontinence episodes per 24 hours, the method comprising orally administering to the subject in need thereof an amount of 75 mg of vibegron per day over a 12-week treatment period, wherein the reduction is greater than that achieved with an equivalent dose of mirabegron.

The present disclosure also provides a method of increasing the average volume voided per micturition in a subject suffering from overactive bladder, or a method of treating overactive bladder in a subject in need thereof while increasing the average volume voided per micturition, the method comprising orally administering to the subject in need thereof an amount of 75 mg of vibegron per day over a 12-week treatment period, wherein the increase is greater than that achieved with an equivalent dose of mirabegron.

The present disclosure also provides a method of reducing the average number of micturitions per 24 hours in a subject suffering from overactive bladder, or a method of treating overactive bladder in a subject in need thereof while reducing the average number of micturitions per 24 hours, the method comprising orally administering to the subject in need thereof an amount of 75 mg of vibegron per day over a 12-week treatment period, wherein the reduction is greater than or equivalent to that achieved with 50 mg or 100 mg of vibegron.

The present disclosure also provides a method of reducing the average number of UUI episodes per 24 hours in a subject suffering from overactive bladder, or a method of treating overactive bladder in a subject in need thereof while reducing the average number of UUI episodes per 24 hours, the method comprising orally administering to the subject in need thereof an amount of 75 mg of vibegron per day over a 12-week treatment period, wherein the reduction is greater than or equivalent to that achieved with 50 mg or 100 mg of vibegron.

The present disclosure also provides a method of reducing the average number of urgency episodes per 24 hours in a subject suffering from overactive bladder, or a method of treating overactive bladder in a subject in need thereof while reducing the average number of urgency episodes per 24 hours, the method comprising orally administering to the subject in need thereof an amount of 75 mg of vibegron per day over a 12-week treatment period, wherein the reduction is greater than or equivalent to that achieved with 50 mg or 100 mg of vibegron.

The present disclosure also provides a method of reducing the average number of total incontinence episodes per 24 hours in a subject suffering from overactive bladder, or a method of treating overactive bladder in a subject in need thereof while reducing the average number of total incontinence episodes per 24 hours, the method comprising orally administering to the subject in need thereof an amount of 75 mg of vibegron per day over a 12-week treatment period, wherein the reduction is greater than or equivalent to that achieved with 50 mg or 100 mg of vibegron.

The present disclosure also provides a method of increasing the average volume voided per micturition in a subject suffering from overactive bladder, or a method of treating overactive bladder in a subject in need thereof while increasing the average volume voided per micturition, the method comprising orally administering to the subject in need thereof an amount of 75 mg of vibegron per day over a 12-week treatment period, wherein the increase is greater than or equivalent to that achieved with 50 mg or 100 mg of vibegron.

In some embodiments, the subject is treated with vibegron for a treatment period, or the subject is treated with vibegron over a treatment period, wherein the treatment period is selected from the group consisting of about 2, 4, 6, 8, 10, 12, 14, 16, 18, 20, 22, 24, 26, 28, 30, 32, 34, 36, 38, 40, 42, 44, 46, 48, 50, and 52 weeks. In some embodiments, the treatment period is a range between any of these weeks. In some embodiments, the treatment period is selected from about 2 weeks, about 4 weeks, about 8 weeks, about 12 weeks, or about 52 weeks. In some embodiments, the treatment period is about 12 weeks. In some embodiments, the treatment period is about 52 weeks.

The present disclosure provides a method of reducing coping behaviors in a subject suffering from overactive bladder symptoms or a method of improving the coping domain score of a subject based on the OAB-q LF (OAB-q long form). The present disclosure also provides a method of treating overactive bladder in a subject in need thereof while reducing coping behaviors or while improving the coping domain score of a subject based on the OAB-q LF (OAB-q long form). The method comprises orally administering to the subject in need thereof a therapeutically effective amount of vibegron per day over a treatment period, wherein the therapeutically effective amount is about 75 mg and wherein the coping behaviors in the subject are reduced compared to a subject receiving placebo. The coping domain score of the OAB-q LF assesses certain aspects of OAB that are measurable tasks and are important to patients. In certain embodiments, the coping behavior or tasks include, but are not limited to, planning escape routes to restrooms, carefully planning your commute, planning activities more carefully, decreasing physical activities, locating the closest restroom when arriving at a new place, adjusting travel plans, avoiding activities away from restrooms, and being uncomfortable traveling with others. In some embodiments, the coping behaviors of a subject are reduced compared to before the subject initiated treatement with vibegron (i.e., baseline) or compared to coping behaviors in a subject receiving placebo, tolterodine extended release (ER) 4 mg, or another compound for treatment of OAB. In some embodiments, the coping domain score of a subject is improved compared to before the subject initiated treatment with vibegron. In some embodiments, the coping domain score of a subject is improved compared to a subject receiving placebo, tolterodine extended release (ER) 4 mg, or another compound for treatment of OAB. In certain embodiments, the present disclosure provides a method of improving coping domain score in a subject suffering from overactive bladder, the method comprising orally administering to the subject in need thereof an amount of 75 mg of vibegron per day over a treatment period. In some embodiments, the improvement in coping domain score is greater than that achieved with tolterodine extended release (ER) 4mg. In some embodiments, the improvement in coping domain score is greater than that achieved with placebo. In some embodiments, the improvement is measured from baseline. In some embodiments, the coping domain score of the subject receiving vibegron is improved by a score of at least about 3.2 compared to a subject receiving placebo. For example, as shown in Table 59, the coping doman score of subjects treated with vibegron over the 12-week treatment period experiences an improvement of 3.6 greater than subjects receiving placebo.

The present disclosure also provides a method of improving sleep in a subject suffering from overactive bladder, or a method of treating overactive bladder in a subject in need thereof while improving sleep, the method comprising orally administering to the subject in need thereof an amount of 75 mg of vibegron per day over a treatment period. In some embodiments, the improvement in sleep is greater than that achieved with tolterodine extended release (ER) 4mg. In some embodiments, the improvement in sleep is greater than that achieved with placebo. In some embodiments, the improvement is measured from baseline. In some embodiments, the sleep of the subject receiving vibegron is improved compared to a subject receiving placebo by a score of at least about 2.6. For example, as shown in Table 60, the sleep score of subjects treated with vibegron over the 12-week treatment period experiences an improvement of 4.5 greater than subjects receiving placebo.

The present disclosure also provides a method of improving Health-related Quality of Life (HRQL) in a subject suffering from overactive bladder or a method of treating overactive bladder in a subject in need thereof while improving Health-related Quality of Life (HRQL), the method comprising orally administering to the subject in need thereof an amount of 75 mg of vibegron per day over a treatment period. In some embodiments, the improvement in HRQL is greater than that achieved with tolterodine extended release (ER) 4mg. In some embodiments, the improvement in HRQL is greater than that achieved with placebo. In some embodiments, the HRQL includes one or more subscales selected from coping, concern, sleep, or social interaction. In some embodiments, the improvement of HRQL is measured from baseline. In some embodiments, the subject receiving vibegron is improved by a total score of at least about 3.8 compared to a subject receiving placebo. For example, as shown in Table 60, the HRQL Total Score of subjects treated with vibegron over the 12-week treatment period experiences a score improvement of 3.8 greater than subjects receiving placebo.

The present disclosure also provides a method of decreasing symptom bother in a subject suffering from overactive bladder or a method of treating overactive bladder in a subject in need thereof while decreasing symptom bother, the method comprising orally administering to the subject in need thereof an amount of 75 mg of vibegron per day

over a treatment period. In some embodiments, the decrease in symptom bother is greater than that achieved with tolterodine extended release (ER) 4mg. In some embodiments, the decrease in symptom bother is greater than that achieved with placebo. In some embodiments, the decrease is measured from baseline. In some embodiments, the symptom bother of the subject receiving vibegron is decreased compared to a subject receiving placebo by a score of at least about -5.0. For example, as shown in Table 60, the symptom bother score of subjects treated with vibegron over the 12-week treatment period decreases by 6.9 more than subjects receiving placebo.

In some embodiments, the subject has the symptoms of urgency urinary incontinence, urinary urgency, and urinary frequency.

In some embodiments, the subject has one or more symptoms of urgency urinary incontinence (or urge urinary incontinence), urinary urgency, urinary frequency and nocturia.

In some embodiments, the subject is a mammal. In some embodiments the subject is a human or an animal. In some embodiments, the subject is a human.

In some embodiments, the subject is over the age of 18 years. In some embodiments, the subject is under the age of about 18 years. In some embodiments, the subject is between about 6 to about 18 years, about 6 to about 12 years, or about 12 to about 18 years. In some embodiments, the subject is over the age of about 20 years. In some embodiments the subject is over the age of about 25 years. In some embodiments, the subject is over the age of about 30 years. In some embodiments, the subject is over the age of about 35 years. In some embodiments, the subject is over the age of 40 years. In some embodiments, the subject is over the age of 45 years. In some embodiments, the subject is over the age of 50 years. In some embodiments, the subject is over the age of 55 years. In some embodiments, the subject is over the age of 60 years. In some embodiments, the subject is at or over the age of 65 years. In some embodiments, the subject is over the age of 70 years. In some embodiments, the subject is over the age of 75 years.

In some embodiments, the method comprises crushing a pharmaceutical unit dose composition comprising vibegron before administration to a subject. In some embodiments, the subject is orally administered a crushed pharmaceutical unit dose comprising vibegron.

In some embodiments, the subject suffers from renal impairment or is at risk of suffering from renal impairment. In some embodiments, the subject suffers from mild renal impairment, moderate renal impairment, or severe renal impairment.

In some embodiments, the subject has received prior OAB therapy. In some embodiments, the subject has not received prior OAB therapy.

In some embodiments, vibegron is administered with a second pharmaceutical agent, including, e.g., any recited in the present application. In some embodiments, vibegron is administered concomitantly with the second pharmaceutical agent. In some embodiments, vibegron is administered sequentially with the second pharmaceutical agent. In some embodiments, vibegron is administered before and/or after the second pharmaceutical agent. The embodiments described below include such sequential administrations

In some embodiments, the subject has received, has taken, or was otherwise previously exposed to a $\beta_3$-AR agonist, such as mirabegron or solabegron.

In some embodiments, the subject has received, has taken, or was otherwise previously exposed to an anticholinergic.

In some embodiments, the present disclosure provides a method of treating overactive bladder in a treatment-experienced subject in need thereof, the method comprising orally administering to the subject a therapeutically effective amount of vibegron per day, wherein the therapeutically effective amount is about 75 mg, and wherein following administration of vibegron to the subject over a treatment period:

a. the decrease in the average number of micturitions in a 24-hour period in the subject is about 1.5 to about 10 more than the decrease in the average number of micturitions in a subject who receives a placebo; or

b. the average number of urge urinary incontinence (UUI) episodes per 24 hours by the subject decreases by about 1.7 to about 6 times the decrease as that of a subject treated with placebo.

In some embodiments, the decrease in the average number of micturitions in a 24-hour period in the subject is between about 1.5 and about 9, between about 1.5 and about 8, between about 1.5 and about 7, between about 1.5 and about 6, between about 1.5 and about 5, between about 1.5 and about 4, or between about 1.5 and about 3 more than the average number of micturitions in a 24-hour period in a subject who receives a placebo. In some embodiments, the decrease in the average number of micturitions in a 24-hour period in the subject is about 2 to about 4 more than the decrease in the average number of micturitions in a subject who receives a placebo. In some embodiments, the decrease in the average number of micturitions in a 24-hour period in the subject is about 2 to about 3 more than the decrease in the average number of micturitions in a subject who receives a placebo.

In some embodiments, the average number of urge urinary incontinence (UUI) episodes per 24 hours by the

subject decreases by about 1 to about 2, about 1.1 to about 1.9, about 1.3 to about 1.8, about 1.4 to about 1.8, or about 1.5 to about 1.8 times the decrease as that of a subject treated with placebo. In some embodiments, the average number of urge urinary incontinence (UUI) episodes per 24 hours by the subject decreases by about 1.7 times the decrease as that of a subject treated with placebo.

In some embodiments, the average number of urge urinary incontinence (UUI) episodes per 24 hours by the subject decreases by about 5 to about 6.5, about 5.1 to about 6.4, about 5.2 to about 6.3, about 5.3 to about 6.4, about 5.4 to about 6.1, or about 5.5 to about 6.2 times the decrease as that of a subject treated with placebo In some embodiments, the average number of urge urinary incontinence (UUI) episodes per 24 hours by the subject decreases by about 6 times the decrease as that of a subject treated with placebo.

In some embodiments, the average number of micturitions per 24 hours by the subject decreases by about -2.0 to about -4.0, about -1.9 to -3.9, about -1.8 to about -3.8, about -1.7 to about -3.7, about -1.6 to about -3.6, -1.5 to about -3.5, about -1.4 to about - 3.4, about -1.3 to about -3.3, about -1.2 to about -3.2, about -1.1 to about -3.1, or about - 1.0 to about -3.0. In some embodiments, the average number of micturitions per 24 hours decreases by about -1.3 to about -2.5.

In some embodiments, the average number of urge urinary incontinence (UUI) episodes per 24 hours by the subject decreases by about -2.0 to about -4.0, about -1.9 to - 3.9, about -1.8 to about -3.8, about -1.7 to about -3.7, about -1.6 to about -3.6, -1.5 to about -3.5, about -1.4 to about -3.4, about -1.3 to about -3.3, about -1.2 to about -3.2, about -1.1 to about -3.1, or about -1.0 to about -3.0.

In some embodiments, following administration of vibegron to the subject over a treatment period, the average number of micturitions in a 24-hour period by the subject decreases by about -1.0, -1.1, -1.2, -1.3, -1.4, -1.5, -1.6, -1.7, -1.8, -1.9, -2.0, -2.1, -2.2, - 2.3, -2.4, -2.5, -2.6, -2.7, -2.8, -2.9, or -3.0 and the average number of UUI episodes per 24 hours by the subject decreases by about -1.0, -1.1, -1.2, -1.3, -1.4, -1.5, -1.6, -1.7, -1.8, -1.9, -2.0, -2.1, -2.2, -2.3, -2.4, -2.5, -2.6, -2.7, -2.8, -2.9, or -3.0. In some embodiments, the average number of micturitions decreases by about -1.3 to about -2.5 and the average number of UUI episodes decreases by about -1.5 to about -2.5.

In some embodiments, following administration of vibegron to the subject over a treatment period the average number of micturitions in a 24-hour period by the subject decreases between about 1.5 and about 10 compared to the average number of micturitions in a subject who receives a placebo and the average number of UUI episodes per 24 hours by the subject decreases by about -1.5 to about -2.5.

In some embodiments, following administration of vibegron to the subject over a treatment period the average number of urgency episodes per 24 hours decreases by about -1.5 to about -4.2, about -1.6 to about -4.1, about -1.7 to about -4.0, about -1.8 to about - 3.9, about -1.9 to about -3.8, about -2.0 to about -3.7, about -2.1 to about -3.6, or about - 2.2 to about -3.5.

In some embodiments, following administration of vibegron to the subject over a treatment period, the average number of total incontinence episodes decreases by about - 1.0 to about -3.0, about -1.1 to about -2.9, about -1.2 to about -2.8, about -1.3 to about - 2.7, about -1.4 to about -2.6, about -1.5 to about -2.7, about -1.7 to about -2.7, about -1.6 to about -2.6, or about -1.5 to about -2.5. In some embodiments, the aveage number of total incontinence episodes decreases by about -1.7 to about -2.7.

In some embodiments, following administration of vibegron to the subject over a treatment period, the average amount of volume voided per micturition increases by about 10 mL to about 40 mL, about 11 mL to about 39 mL, about 12 mL to about 38 mL, about 13 mL to about 37 mL, about 14 mL to about 36 mL, about 15 mL to about 35 mL, about 16 mL to about 34 mL, about 17 mL to about 33 mL, about 18 mL to about 30 mL, about 19 mL to about 29 mL, about 20 mL to about 28 mL, or about 21 mL to about 27 mL. In some embodiments, the average amount of volume voided per micturition increases by about 18 to about 30 mL.

In some embodiments, the treatment-experienced subject has been previously treated with an anticholinergic. In some embodiments, the treatment-experienced subject has been treated with an anticholinergic within the 12 months prior to treatment with vibegron. In some embodiments, the treatment-experienced subject has been treated with an anticholinergic more than 12 months prior to treatment with vibegron. In some embodiments, the treatment-experienced subject is concomitantly being treated with an anticholinergic while being treated with vibegron.

In some embodiments, the treatment-experienced subject has been previously treated with a beta-3 agonist other than vibegron. In some embodiments, the treatment-experienced subject has been treated with a beta-3 agonist other than vibegron within the 12 months prior to treatment with vibegron. In some embodiments, the treatment-experienced subject has been treated with a beta-3 agonist other than vibegron more than 12 months prior to treatment with vibegron. In some embodiments, the treatment-experienced subject is concomitantly being treated with a beta-3 agonist other than vibegron while being treated with vibegron. In some embodiments, the beta-3 agonist is mirabegron. In some embodiments, the beta-3 agonist is solabegron.

In some embodiments, the subject is concomitantly receiving, taking or otherwise being exposed to a cytochrome P450 inhibitor, such as a CYP3A inhibitor, and with drugs that are substrates of the following CYPs: CYP1A2, 2B6, 2C8, 2C9, 2C19, 2D6, and 3A4.

In some embodiments, the subject is concomitantly receiving, taking, or otherwise being exposed to a CYP2D6

substrate.

CYP 2D6 substrates include but are not limited to imipramine, amitriptyline, fluoxetine, paroxetine, fluvoxamine, venlafaxine, duloxetine, mianserin, mirtazapine, opioids, codeine , morphine, tramadol , O-desmethyltramadol, N,O-didesmethyltramadol, oxycodone, hydrocodone, hydromorphone, tapentadol, haloperidol, risperidone, perphenazine, thioridazine, zuclopenthixol, iloperidone, aripiprazole, chlorpromazine, levomepromazine, remoxipride, minaprine, tamoxifen, hydroxytamoxifen, beta-blockers, metoprolol, timolol, alprenolol, carvedilol, bufuralol, nebivolol, propranolol, debrisoquine, flecainide, propafenone, encainide, mexiletine, lidocaine, sparteine, ondansetron , donepezil, phenformin, tropisetron, amphetamine, methoxyamphetamine, dextromethamphetamine, atomoxetine, chlorphenamine, dexfenfluramine, dextromethorphan, dextrorphan, metoclopramide, perhexiline, phenacetin, promethazine, m-tyramine, warfarin, tolterodine, and p-tyramine.

In some embodiments, the subject is concomitantly receiving, taking, or otherwise being exposed to a P-glycoprotein inhibitor.

CYP3A/P-glycoprotein inhibitors include but are not limited to amiodarone, carvedilol, clarithromycin, dronedarone, itraconazole, lapatinib, lopinavir and ritonavir, propafenone, quinidine, ranolazine, ritonavir, saquinavir and ritonavir, telaprevir, tipranavir and ritonavir, verapamil, curcumin, cyclosporine A, eltrombopag, atazanavir and ritonavir, clarithromycin, cyclosporine, erythromycin, gemfibrozil, lopinavir and ritonavir, rifampin (e.g., single dose), simeprevir, p-aminohippuric acid (PAH)(b), probenecid, teriflunomide, cimetidine, dolutegravir, isavuconazole, ranolazine, trimethoprim, and vandetanib.

In some embodiments, the subject is concomitantly receiving, taking or otherwise being exposed to a muscarinic receptor antagonist

Muscarinic receptor antagonists include but are not limited to scopolamine, atropine, hydroxyzine, ipratropium, tropicamide, pirenzepine, diphenhydramine, doxylamine, dimenhydrinate, dicyclomine, flavoxate, oxybutynin, tiotropium, cyclopentolate, atropine methonitrate, trihexyphenidyl/benzhexol, tolterodine, solifenacin, darifenacin, benztropine, Mebeverine, procyclidine, and aclidinium bromide.

In some embodiments, the subject is administered about 75 mg of vibegron per day and is concomitantly receiving, taking or otherwise being exposed to a muscarinic receptor antagonist.

In some embodiments, the subject is administered about 75 mg of vibegron per day and is concomitantly receiving, taking or otherwise being exposed to a CYP3A inhibitor.

In some embodiments, the subject is administered about 75 mg of vibegron per day and is concomitantly receiving, taking, or otherwise being exposed to a P-glycoprotein inhibitor.

In some embodiments the subject is not concomitantly receiving, taking, or otherwise being exposed to a beta blocker.

In some embodiments the subject is not concomitantly receiving, taking, or otherwise being exposed to a amlodipine.

In some embodiments, vibegron is administered with a meal, within 60 minutes after a meal, or within 2 hours after a meal.

In some embodiments, vibegron is administered without a meal or before a meal. In some embodiments, vibegron is administered more than two hours before a meal. In some embodiments vibegron is administered regardless of whether the subject has or has not had a meal.

In some embodiments, vibegron is administered once per day, twice per day, or three times per day. In some embodiments, vibegron is administered once per day.

Changes from baseline in blood pressure (BP) and heart rate (HR) for the subjects taking vibegron are not substantially different for the subjects taking a placebo. In some embodiments, the subject experiences a mean maximum change of systolic blood pressure (SBP) from baseline over the treatment period (e.g., 8 weeks or 12 weeks), and the mean maximum change is less than 2.0 mm Hg, less than 1.9 mm Hg, less than 1.8 mm Hg, less than 1.7 mm Hg, less than 1.6 mm Hg, less than 1.5 mm Hg, less than 1.4 mm Hg, less than 1.3 mm Hg, less than 1.2 mm Hg, less than 1.1 mm Hg, less than 1.0 mm Hg, less than 0.9 mm Hg, less than 0.8 mm Hg, less than 0.7 mm Hg, less than 0.6 mm Hg, or less than 0.5 mm Hg from that of a subject taking a placebo.

In some embodiments, the subject is administered about 75 mg of vibegron per day, and experiences a mean maximum change of SBP from baseline over the treatment period (e.g., 8 weeks or 12 weeks) of less than 2 mm Hg from that of a subject taking a placebo. In some embodiments, the subject is administered about 75 mg of vibegron per day, and experiences a mean maximum change of SBP from baseline over the treatment period (e.g., 8 weeks or 12 weeks) of less than 1 mm Hg from that of a subject taking a placebo.

In some embodiments, the subject is over the age of 65 and is administered about 75 mg of vibegron per day, and experiences a mean maximum change of SBP from baseline over the treatment period (e.g., 8 weeks or 12 weeks) of less than 2 mm Hg from that of a subject taking a placebo. In some embodiments, the subject is over the age of 65 and is administered about 75 mg of vibegron per day, and experiences a mean maximum change of SBP from baseline over the treatment period (e.g., 8 weeks or 12 weeks) of less than 1 mm Hg from that of a subject taking a placebo.

In some embodiments, the subject is over the age of 45 and is administered about 75 mg of vibegron per day, and experiences a mean maximum change of SBP from baseline over the treatment period (e.g., 8 weeks or 12 weeks) of less than 2 mm Hg from that of a subject taking a placebo. In some embodiments, the subject is over the age of 45 and is administered about 75 mg of vibegron per day, and experiences a mean maximum change of SBP from baseline over the treatment period (e.g., 8 weeks or 12 weeks) of less than 1 mm Hg from that of a subject taking a placebo.

In some embodiments, the subject experiences a mean maximum change of diastolic blood pressure (DBP) from baseline over the treatment period (e.g., 8 weeks or 12 weeks), and the mean maximum change is less than 2.0 mm Hg, less than 1.9 mm Hg, less than 1.8 mm Hg, less than 1.7 mm Hg, less than 1.6 mm Hg, less than 1.5 mm Hg, less than 1.4 mm Hg, less than 1.3 mm Hg, less than 1.2 mm Hg, less than 1.1 mm Hg, less than 1.0 mm Hg, less than 0.9 mm Hg, less than 0.8 mm Hg, less than 0.7 mm Hg, less than 0.6 mm Hg, or less than 0.5 mm Hg from that of a subject taking a placebo.

In some embodiments, the subject is administered about 75 mg of vibegron per day, and experiences a mean maximum change of DBP from baseline over the treatment period (e.g., 8 weeks or 12 weeks) of less than 2 mm Hg from that of a subject taking a placebo. In some embodiments, the subject is administered about 75 mg of vibegron per day, and experiences a mean maximum change of DBP from baseline over the treatment period (e.g., 8 weeks or 12 weeks) of less than 1 mm Hg from that of a subject taking a placebo.

In some embodiments, the subject is over the age of 65 and is administered about 75 mg of vibegron per day, and experiences a mean maximum change of DBP from baseline over the treatment period (e.g., 8 weeks or 12 weeks) of less than 2 mm Hg from that of a subject taking a placebo. In some embodiments, the subject is over the age of 65 and is administered about 75 mg of vibegron per day, and experiences a mean maximum change of DBP from baseline over the treatment period (e.g., 8 weeks or 12 weeks) of less than 1 mm Hg from that of a subject taking a placebo.

In some embodiments, the subject is over the age of 45 and is administered about 75 mg of vibegron per day, and experiences a mean maximum change of DBP from baseline over the treatment period (e.g., 8 weeks or 12 weeks) of less than 2 mm Hg from that of a subject taking a placebo. In some embodiments, the subject is over the age of 45 and is administered about 75 mg of vibegron per day, and experiences a mean maximum change of DBP from baseline over the treatment period (e.g., 8 weeks or 12 weeks) of less than 1 mm Hg from that of a subject taking a placebo.

In some embodiments, the subject is over the age of 45, is administered about 75 mg of vibegron once per day, and experiences a mean maximum change of DBP from baseline over the treatment period (e.g., 8 weeks or 12 weeks) of less than 1 mm Hg from that of a subject taking a placebo, and a mean maximum change of SBP from baseline over the treatment period (e.g., 8 weeks or 12 weeks) of less than 1 mm Hg from that of a subject taking a placebo.

In some embodiments, the subject is over the age of 65, is administered about 75 mg of vibegron once per day, and experiences a mean maximum change of DBP from baseline over the treatment period (e.g., 8 weeks or 12 weeks) of less than 1 mm Hg from that of a subject taking a placebo, and a mean maximum change of SBP from baseline over the treatment period (e.g., 8 weeks or 12 weeks) of less than 1 mm Hg from that of a subject taking a placebo.

In some embodiments, the subject experiences a mean maximum change of systolic blood pressure (SBP) from baseline over the treatment period (e.g., 8 weeks or 12 weeks) of less than 10 mm Hg, less than 9.5 mm Hg, less than 9 mm Hg, less than 8.5 mm Hg, less than 8 mm Hg, less than 7.5 mm Hg, less than 7 mm Hg, less than 6.5 mm Hg, less than 6 mm Hg, less than 5.5 mm Hg, or less than 5 mm Hg.

In some embodiments, the subject is administered about 75 mg of vibegron per day, and experiences a mean maximum change of SBP from baseline over the treatment period (e.g., 8 weeks or 12 weeks) of less than 10 mm Hg.

In some embodiments, the subject is over the age of 65 and is administered about 75 mg of vibegron per day, and experiences a mean maximum change of SBP from baseline over the treatment period (e.g., 8 weeks or 12 weeks) of less than 10 mm Hg.

In some embodiments, the subject is over the age of 45 and is administered about 75 mg of vibegron per day, and experiences a mean maximum change of SBP from baseline over the treatment period (e.g., 8 weeks or 12 weeks) of less than 10 mm Hg.

In some embodiments, the subject experiences a mean maximum change of diastolic blood pressure (DBP) from baseline over the treatment period (e.g., 8 weeks or 12 weeks) of less than 7 mm Hg, less than 6.5 mm Hg, less than 6 mm Hg, less than 5.5 mm Hg, less than 5 mm Hg, less than 4.5 mm Hg, less than 4 mm Hg, less than 3.5 mm Hg, less than 3 mm Hg, less than 2.5 mm Hg, or less than 2 mm Hg.

In some embodiments, the subject is administered about 75 mg of vibegron per day, and experiences a mean maximum change of DBP from baseline over the treatment period (e.g., 8 weeks or 12 weeks) of less than 7 mm Hg.

In some embodiments, the subject is administered about 75 mg of vibegron per day, and experiences a change from baseline over the treatment period (e.g., 8 weeks or 12 weeks) in average number of micturitions per 24 hours, wherein the change is greater than that for a subject taking placebo. The difference from placebo (i.e., the placebo-adjusted change) is from about -0.1 to about -1.5, for example, about 0.1, -0.2, -0.3, -0.4, - 0.5, -0.6, -0.7, -0.8, -0.9, -1.0, -1.1, -1.2, -1.3, -1.4, or -1.5, or a range between any two of the preceding values. In some embodiments, the placebo-adjusted change is from about - 1.0 to about -0.1 or from about -0.8 to about -0.2. In some embodiments, the placebo-adjusted change is about -0.5.

In some embodiments, the subject is administered about 75 mg of vibegron per day, and experiences a change from baseline over the treatment period (e.g., 8 weeks or 12 weeks) in average number of micturitions per 24 hours of from about -1.3 to about - 2.5, for example, about -1.3, -1.4, -1.5, -1.6, -1.7, -1.8, -1.9, -2.0, -2.1, -2.2, -2.3, -2.4, or - 2.5, or a range between any two of the preceding values. In some embodiments, the change from baseline in average number of micturitions per 24 hours is from about -1.3 to about -2.3, from about -1.5 to about -2.1, or from about -1.6 to about -2.0. In some embodiments, the change from baseline in average number of micturitions per 24 hours is about -1.8.

In some embodiments, the subject has an average of ≥ 1 urge urinary incontinence (UUI) episodes per day prior to treatment and is administered about 75 mg of vibegron per day, and experiences a change from baseline over the treatment period (e.g., 8 weeks or 12 weeks) in average number of UUI episodes, wherein the change is greater than that for a subject taking placebo. The difference from placebo (i.e., the placebo-adjusted change) is from about -0.1 to about -1.5, for example, about -0.2, -0.3, -0.4, -0.5, -0.6, - 0.7, -0.8, -0.9, -1.0, -1.1, -1.2, -1.3, -1.4, or -1.5, or a range between any two of the preceding values. In some embodiments, the placebo-adjusted change is from about -1.1 to about -0.1 or from about -0.9 to about -0.3. In some embodiments, the placebo-adjusted change is about -0.6.

In some embodiments, the subject is administered about 75 mg of vibegron per day, and experiences a change from baseline over the treatment period (e.g., 8 weeks or 12 weeks) in average number of UUI episodes of from about -1.3 to about -2.5, for example, about -1.3, -1.4, -1.5, -1.6, -1.7, -1.8, -1.9, -2.0, -2.1, -2.2, -2.3, -2.4, or -2.5, or a range between any two of the preceding values. In some embodiments, the change from baseline in average number of UUI episodes is from about -1.5 to about -2.5, from about - 1.7 to about -2.3, or from about -1.8 to about -2.2. In some embodiments, the change from baseline in average number of UUI episodes is about -2.0.

In some embodiments, the subject is administered about 75 mg of vibegron per day, and experiences a change from baseline over the treatment period (e.g., 8 weeks or 12 weeks) in average number of urgency episodes per 24 hours, wherein the change is greater than that for a subject taking placebo. The difference from placebo (i.e., the placebo-adjusted change) is from about -0.4 to about -1.5, for example, about -0.4, -0.5, - 0.6, -0.7, -0.8, -0.9, -1.0, -1.1, -1.2, -1.3, -1.4, or -1.5, or a range between any two of the preceding values. In some embodiments, the placebo-adjusted change is from about -1.2 to about -0.2 or from about -0.9 to about -0.4. In some embodiments, the placebo-adjusted change is about -0.7.

In some embodiments, the subject is administered about 75 mg of vibegron per day, and experiences a change from baseline over the treatment period (e.g., 8 weeks or 12 weeks) in average number of urgency episodes per 24 hours of from about -2.2 to about -3.2, for example, about -2.2, -2.3, -2.4, -2.5, -2.6, -2.7, -2.8, -2.9, -3.0, -3.1, or -3.2, or a range between any two of the preceding values. In some embodiments, the change from baseline in average number of urgency episodes per 24 hours is from about -2.2 to about -3.2, from about -2.4 to about -3.0, or preferably from about -2.5 to about -2.9. In some embodiments, the change from baseline in average number of urgency episodes per 24 hours is about -2.7.

In some embodiments, the subject is administered about 75 mg of vibegron per day, and experiences a change from baseline over the treatment period (e.g., 8 weeks or 12 weeks) in average number of total incontinence episodes per 24 hours, wherein the change is greater than that for a subject taking placebo. The difference from placebo (i.e., the placebo-adjusted change) is from about -0.4 to about -1.5, for example, about -0.4, - 0.5, -0.6, -0.7, -0.8, -0.9, -1.0, -1.1, -1.2, -1.3, -1.4, or -1.5, or a range between any two of the preceding values. In some embodiments, the placebo-adjusted change is from about - 1.2 to about -0.2 or from about -1.0 to about -0.4. In some embodiments, the placebo-adjusted change is about -0.7.

In some embodiments, the subject is administered about 75 mg of vibegron per day, and experiences a change from baseline over the treatment period (e.g., 8 weeks or 12 weeks) in average number of total incontinence episodes per 24 hours of from about - 1.8 to about -2.8, for example, about -1.8, -1.9, -2.0, -2.1, -2.2, -2.3, -2.4, -2.5, -2.6, -2.7, or -2.8, or a range between any two of the preceding values. In some embodiments, the change from baseline in average number of total incontinence episodes per 24 hours is from about -1.8 to about -2.8, from about -2.0 to about -2.6, or from about -2.1 to about - 2.5. In some embodiments, the change from baseline in average number of total incontinence episodes per 24 hours is about -2.3.

In some embodiments, the subject is administered about 75 mg of vibegron per day, and experiences a change in the volume voided (mL) per micturition, wherein the change is greater than that for a subject taking placebo. The difference from placebo (i.e., the placebo-adjusted change) is from about 20 mL to about 35 mL, for example, about 20 mL, 21 mL, 22 mL, 23 mL, 24 mL, 25 mL, 26 mL, 27 mL, 28 mL, 29 mL, 30 mL, 31 mL, 32 mL, 33 mL, 34 mL, or 30 mL, or a range between any two of the preceding values. In some embodiments, the placebo-adjusted change is from about 15.0 to about 26 from about 18.0 to about 23.0. In some embodiments, the placebo-adjusted change is about 21.2.

In some embodiments, the subject is administered about 75 mg of vibegron per day, and experiences a change from baseline over the treatment period (e.g., 8 weeks or 12 weeks) in the volume voided (mL) per micturition, from from about 18 mL to about 30 mL, from about 20 mL to about 28 mL, or from about 22 mL to about 26 mL. In some embodiments, the change from baseline in the volume voided (mL) per micturition is about 23 or about 24 mL.

In some embodiments, the subject has an average of ≥ 1 urge urinary incontinence (UUI) episodes per day prior to treatment and is administered about 75 mg of vibegron per day, and experiences at least 50%, at least 55%, at least 60%,

at least 65%, at least 70%, at least 75%, at least 80%, or at least 85% reduction in the average number of daily UUI episodes over the treatment period (e.g., 8 weeks or 12 weeks).

In some embodiments, the subject has an average of ≥ 1 urgency episodes per day prior to treatment and is administered about 75 mg of vibegron per day, and experiences at least 30%, at least 40%, at least 45%, at least 50%, at least 55%, at least 60%, at least 65%, at least 70%, or at least 75% reduction in the average number of daily urgency episodes over the treatment period (e.g., 8 weeks or 12 weeks).

In some embodiments, the subject is over the age of 65 and is administered about 75 mg of vibegron per day, and experiences a mean maximum change of DBP from baseline over the treatment period (e.g., 8 weeks or 12 weeks) of less than 7 mm Hg.

In some embodiments, the subject is over the age of 45 and is administered about 75 mg of vibegron per day, and experiences a mean maximum change of DBP from baseline over the treatment period (e.g., 8 weeks or 12 weeks) of less than 7 mm Hg.

In some embodiments, the subject over the age of 45 is administered about 75 mg of vibegron once per day, experiences a mean maximum change of DBP from baseline over the treatment period (e.g., 8 weeks or 12 weeks) of less than 7 mm Hg, and a mean maximum change of SBP from baseline over the treatment period (e.g., 8 weeks or 12 weeks) of less than 10 mm Hg.

In some embodiments, the subject over the age of 65 is administered about 75 mg of vibegron once per day, experiences a mean maximum change of DBP from baseline over the treatment period (e.g., 8 weeks or 12 weeks) of less than 7 mm Hg, and a mean maximum change of SBP from baseline over the treatment period (e.g., 8 weeks or 12 weeks) of less than 10 mm Hg.

In some embodiments, vibegron has onset of action of about 4 weeks. In some embodiments, vibegron has onset of action of about 3 weeks. In some embodiments, vibegron has onset of action of about 2 weeks. "Onset of action," as used herein, refers to the duration of time it takes for a drug's effects to come to prominence upon administration.

The treatment method of vibegron for OAB patients, as disclosed here, is well tolerated, with very few adverse events greater than 2% and greater than placebo, and these adverse events include headache, nasopharyngitis, diarrhea, and nausea.

Vibegron has similar rates for other adverse events as compared to placebo, such as hypertension, blood pressure increased, tachycardia, hypotension, dizziness, urinary tract infection, urinary retention, dry mouth, constipation, and fatigue. In some embodiments, vibegron treatment does not cause any increased risk in hypertension as compared to placebo. In some embodiments, vibegron treatment does not cause any increased risk in blood pressure increase. In some embodiments, vibegron treatment does not cause any increased risk in tachycardia. In some embodiments, vibegron treatment does not cause any increased risk in hypotension. In some embodiments, vibegron treatment does not cause any increased risk in one or more of hypertension, blood pressure, tachycardia, or hypotension.

For systolic blood pressure (SBP) and diastolic blood pressure (DBP), changes from baseline are monitored. Categorical changes in SBP and DBP are measured, for example, changes ≥5 mm Hg, ≥10 mm Hg, ≥15 mm Hg, or ≥20 mm Hg.

## Pharmaceutical Unit Dose Composition

The present disclosure provides pharmaceutical unit dose compositions comprising a dosage of vibegron disclosed herein, wherein the unit dosage composition is suitable for oral administration. Oral dosage forms are recognized by those skilled in the art to include, for example, such forms as liquid formulations, tablets, capsules, and gelcaps. In some embodiments, the unit dose compositions are solid dosage forms, such as tablets and capsules. In some embodiments, the unit dose compositions are tablets.

Pharmaceutically acceptable excipients are excipients generally recognized as safe such as lactose, microcrystalline cellulose, starch, calcium carbonate, magnesium stearate, stearic acid, talc, colloidal silicon dioxide, mannitol, croscarmellose sodium, hydroxypropyl cellulose. In some embodiments, the pharmaceutical unit dose composition disclosed herein comprises a diluent, a disintegrant, a binder, and a lubricant. See generally, Remington's Pharmaceutical Sciences, 20th ed., Mack Publishing, Easton PA (2000), which is incorporated herein by reference in its entirety.

In some embodiments, the pharmaceutical unit dose compositions of the present disclosure can be crushed. In some embodiments, the pharmaceutical unit dose compositions of the present disclosure are crushed before oral administration.

## EXAMPLES

### Example 1

**Vibegron Tablet Formulation**

The composition of vibegron tablets (50 mg, 75 mg, and 100 mg) is shown in Table 1.

**Table 1 Vibegron Tablet Compositions**

| **Components** | **Function** | **Unit Strength** | | |
|---|---|---|---|---|
| | | 50 mg | 75 mg | 100 mg |
| blet | | mg/tablet | mg/tablet | mg/tablet |
| MK4618 | Active | 50.00 | 75.00 | 100.0 |
| Mannitol | Diluent | 20.75 | 31.125 | 41.50 |
| Microcrystalline Cellulose | Diluent | 20.75 | 31.125 | 41.50 |
| Croscarmellose Sodium | Disintegrant | 3.000 | 4.500 | 6.000 |
| Hydroxypropyl Cellulose | Binder | 4.500 | 6.75 | 9.000 |
| Magnesium Stearate | Lubricant | 1.000 | 1.500 | 2.000 |
| Purified Water[1] | Solvent | (35.00-45.00) | (52.5-67.5) | (70.00-90.00) |
| **Total Core Weight** | | **100.0** | **150.0** | **200.00** |
| Film Coating Suspension | | | | |
| Purified Water[1] | Solvent | (45.00) | (67.50 | (90.00) |
| OPADRY II Green (39K110004) | Colorant | 5.000 | 7.500 | 10.00 |
| **Total** | | **105.0** | **157.5** | **210.0** |
| 1-Removed during processing | | | | |

Example 2

**Clinical Efficacy Data**

A randomized, double-blind, placebo- and active-controlled, parallel-group two-part Phase 2b study of vibegron in men and women with OAB (stratified as OAB wet and OAB dry) was completed. Part 1 was a dose-ranging study to assess the safety, tolerability, and efficacy of vibegron and proof of concept study for concomitant dosing of vibegron with tolterodine ER 4 mg. Approximately 980 subjects in Part 1 were equally randomized in a double-blind fashion to one of seven treatment arms: vibegron 3 mg, 15 mg, 50 mg, or 100 mg once daily for 8 weeks; tolterodine ER 4 mg once daily for 8 weeks; placebo once daily for 8 weeks; or vibegron 50 mg with tolterodine ER 4 mg for 4 weeks followed by vibegron 50 mg for 4 weeks. Part 2 was designed to continue to assess the safety and efficacy of concomitant dosing. In Part 2, 408 subjects were randomized in a double-blind fashion to one of four treatment arms in a 2:2:2:1 ratio: vibegron 100 mg, tolterodine ER 4 mg, vibegron 100 mg with tolterodine ER 4 mg, or placebo once daily for 4 weeks. Subjects in both Part 1 and Part 2 had the option of enrolling in a 1-year extension. Participants were required to keep a voiding diary, recording the occurrence of each strong urge, total incontinence, and urge incontinence episode. Efficacy data for Part 1 and Part 2 are summarized herein.

At baseline, subjects must have had an average number of micturitions ≥ 8 per diary day in the Voiding Diary. In addition, subjects in the OAB wet strata must have had an average number of urgency incontinence episodes ≥ 1 per diary day. Subjects in the OAB dry strata must have had an average number of urgency episodes ≥ 3 per diary day and an average of < 1 urgency incontinence episodes per diary day. The total number of urgency incontinence episodes must have exceeded the total number of stress incontinence episodes for all subjects.

The primary objectives of this study were to assess the safety and tolerability of treatment with selected vibegron doses (alone or in combination with tolterodine) and to investigate dose-related reductions in average number of daily micturitions compared with placebo at Week 8.

In Part 1, statistically significant decreases in the average number of daily micturitions were observed in the vibegron 100 mg and 50 mg treatment groups as compared to the placebo group at Week 8. Statistically significant decreases from baseline as compared to placebo were also observed in the vibegron 100 mg and 50 mg treatment groups for secondary endpoints which included urgency incontinence and total incontinence (in subjects with OAB wet), and urgency episodes in all subjects. Statistically significant increases from baseline as compared to placebo were also observed for the secondary endpoint volume voided per micturition in the vibegron 15, 50 and 100 mg treatment groups. (Tables 2 and 3).

**Table 2 Analysis of Change from Baseline In The Volume Voided (ML) Per Micturition at Week 8**

| | | Difference from Placebo | |
|---|---|---|---|
| | | Week 8 | |
| **Treatment** | **N** | **Difference in LS Means** | **P-Value** |
| Vibegron 3 mg | 144 | 15.99 | 0.032 |
| Vibegron 15 mg | 131 | 28.23 | < 0.001 |
| Vibegron 50 mg | 146 | 29.05 | < 0.001 |
| Vibegron 100 mg | 148 | 23.36 | 0.002 |
| Tolterodine ER 4 mg | 133 | 30.77 | < 0.001 |

**Table 3 Analysis of Change from Baseline in Average Daily Number Events at Week 8 - Constrained Longitudinal Data Analysis (cLDA) Model (Full-Analysis-Set Population - Part 1 Base Study)**

| | | Daily Number of Events | | | | | | Change from Baseline | | Difference from Placebo | | Adjusted Differ ence from Placebo | | | |
|---|---|---|---|---|---|---|---|---|---|---|---|---|---|---|---|
| | | Bas eline | | | Week 8 | | | Week 8 | | Week 8 | | Week 8 | | | |
| **Event** | **Treatment** | **N** | **Mean** | **SD** | **N** | **Mean** | **SD** | **Mean** | **SD** | **Difference in LS Means[b]** | **P-Value** | **Difference in LS Means[d]** | **SE** | **95% CI** | **P-Value** |
| **Micturitions** | Placebo | 141 | 10.86 | 2.84 | 131 | 9.77 | 2.51 | -1.09 | 2.17 | n/a | n/a | n/a | n/a | n/a | n/a |
| | Vibegron 3 mg | 144 | 10.93 | 2.35 | 139 | 9.35 | 2.43 | -1.56 | 1.97 | -0.46 | 0.056 | -0.5 | 0.23 | -0.9 to -0.0 | 0.0367 |
| | Vibegron 15 mg | 132 | 11.32 | 3.48 | 125 | 9.53 | 2.85 | -1.71 | 2.22 | -0.45 | 0.064 | -0.5 | 0.23 | -0.9 to -0.0 | 0.0395 |
| | Vibegron 50 mg | 148 | 11.21 | 3.16 | 141 | 9.05 | 2.28 | -1.87 | 1.78 | -0.64 | 0.007 | -0.7 | 0.23 | -1.1 to -0.2 | 0.0028 |
| | Vibegron 100 mg | 148 | 11.15 | 2.32 | 140 | 9.02 | 2.59 | -2.11 | 1.81 | -0.91 | < 0.001 | -0.9 | 0.23 | -1.4 to -0.5 | <0.0001 |
| | Tolterodine ER 4 mg | 134 | 11.00 | 2.17 | 124 | 9.24 | 2.11 | -1.73 | 2.02 | -0.54 | 0.026 | -0.6 | 0.23 | -1.0 to -0.1 | 0.0123 |
| **Urgency Incontinence Episodes[c]** | Placebo | 118 | 3.11 | 2.68 | 112 | 1.71 | 2.50 | -1.34 | 1.77 | n/a | n/a | n/a | n/a | n/a | n/a |
| | Vibegron 3 mg | 113 | 2.70 | 1.94 | 110 | 1.21 | 1.68 | -1.38 | 1.38 | -0.28 | 0.167 | -0.2 | 0.20 | -0.6 to 0.2 | 0.2406 |
| | Vibegron 15 mg | 111 | 2.94 | 2.23 | 107 | 1.12 | 2.06 | -1.81 | 1.60 | -0.57 | 0.005 | -0.6 | 0.20 | -0.9 to -0.2 | 0.0053 |
| | Vibegron 50 mg | 121 | 2.81 | 2.06 | 117 | 0.86 | 1.16 | -1.90 | 1.75 | -0.72 | < 0.001 | -0.7 | 0.19 | -1.1 to -0.3 | 0.0003 |
| | Vibegron 100 mg | 122 | 2.96 | 2.42 | 116 | 0.84 | 1.74 | -2.05 | 1.99 | -0.71 | < 0.001 | -0.7 | 0.19 | -1.1 to -0.3 | 0.0003 |
| | Tolterodine ER 4 mg | 100 | 2.80 | 2.13 | 93 | 1.15 | 2.18 | -1.67 | 1.55 | -0.46 | 0.030 | -0.4 | 0.20 | -0.8 to -0.0 | 0.0359 |

(continued)

| Event | Treatment | Daily Number of Events | | | | | | Change from Baseline | | Difference from Placebo | | Adjusted Differ ence from Placebo | | | |
|---|---|---|---|---|---|---|---|---|---|---|---|---|---|---|---|
| | | Bas eline | | | Week 8 | | | Week 8 | | Week 8 | | Week 8 | | | |
| | | N | Mean | SD | N | Mean | SD | Mean | SD | Difference in LS Means[b] | P-Value | Difference in LS Means[d] | SE | 95% CI | P-Value |
| **Total Incontinence Episodes[c]** | Placebo | 118 | 3.61 | 3.26 | 112 | 1.88 | 2.68 | -1.68 | 2.01 | n/a | n/a | n/a | n/a | n/a | n/a |
| | Vibegron 3 mg | 113 | 3.05 | 2.11 | 110 | 1.38 | 1.75 | -1.56 | 1.55 | -0.18 | 0.401 | -0.1 | 0.21 | -0.5 to 0.3 | 0.6345 |
| | Vibegron 15 mg | 111 | 3.32 | 2.44 | 107 | 1.31 | 2.26 | -1.99 | 1.64 | -0.48 | 0.029 | -0.4 | 0.21 | -0.9 to -0.0 | 0.0371 |
| | Vibegron 50 mg | 121 | 3.10 | 2.26 | 117 | 1.02 | 1.40 | -2.02 | 1.82 | -0.60 | 0.005 | -0.5 | 0.21 | -1.0 to -0.1 | 0.0095 |
| | Vibegron 100 mg | 122 | 3.43 | 2.83 | 116 | 1.12 | 2.08 | -2.26 | 2.41 | -0.58 | 0.007 | -0.6 | 0.21 | -1.0 to -0.2 | 0.0063 |
| | Tolterodine ER 4 mg | 100 | 3.08 | 2.39 | 93 | 1.32 | 2.38 | -1.80 | 1.47 | -0.34 | 0.140 | -0.3 | 0.22 | -0.7 to 0.2 | 0.2210 |
| **Urgency Episodes** | Placebo | 141 | 6.52 | 4.37 | 131 | 4.99 | 3.77 | -1.57 | 3.28 | n/a | n/a | n/a | n/a | n/a | n/a |
| | Vibegron 3 mg | 144 | 6.49 | 3.66 | 139 | 4.68 | 4.16 | -1.69 | 2.65 | -0.18 | 0.598 | -0.2 | 0.32 | -0.8 to 0.4 | 0.5331 |
| | Vibegron 15 mg | 132 | 6.93 | 4.69 | 125 | 4.42 | 4.40 | -2.35 | 2.50 | -0.67 | 0.052 | -0.7 | 0.33 | -1.4 to -0.1 | 0.0319 |
| | Vibegron 50 mg | 148 | 6.43 | 4.22 | 141 | 3.71 | 3.76 | -2.36 | 2.35 | -0.76 | 0.024 | -0.8 | 0.32 | -1.4 to -0.1 | 0.0173 |
| | Vibegron 100 mg | 148 | 7.34 | 4.14 | 140 | 4.22 | 4.36 | -2.98 | 2.84 | -1.24 | < 0.001 | -1.3 | 0.32 | -2.0 to -0.7 | <0.0001 |
| | Tolterodine ER 4 mg | 134 | 6.39 | 3.78 | 124 | 3.91 | 3.65 | -2.52 | 2.73 | -0.94 | 0.007 | -1.0 | 0.33 | -1.6 to -0.3 | 0.0029 |

a. Constrained longitudinal data analysis model includes terms for time, region and interaction of time by treatment.
b. Negative mean treatment differences are in favor of former treatments in comparison.
c. Only in OAB Wet subjects.
d. Mixed effects model for repeated measures includes covariates for study visit, sex, region, baseline number of episodes and treatment by study visit interaction

A double-blind, randomized, placebo controlled, multi-center, Phase 3 study designed to evaluate the safety and efficacy of vibegron in males and females with OAB was completed. Upon completion of the placebo Run-in period, 1,232 patients were randomized to receive blinded study treatment for 12 weeks including: vibegron 50 mg (N=370), vibegron 100 mg (N=369), placebo (N=369), or imidafenacin 0.2 mg (comparator; N=117). The results demonstrate that once daily vibegron produced statistically significant reductions in efficacy parameters including: micturitions, UUI episodes, total incontinence episodes, and urgency episodes (Table 4).

**Table 4 Analysis of Change from Baseline in Average Daily Number Events at Week 12 - Constrained Longitudinal Data Analysis (cLDA) Model**

| Event | 50 mg Dose | 100mg Dose |
|---|---|---|
| **Micturitions** | -0.86 (-1.12, -0.60) p < 0.0001 | -0.81 (-1.07, -0.55) p < 0.0001 |
| **Urge Urinary Incontinence Episodes** | -0.27 (-0.44, -0.10) p = 0.0015 | -0.39 (-0.55, -0.22) p < 0.0001 |
| **Total Incontinence Episodes** | -0.30 (-0.49, -0.12) p = 0.0015 | -0.43 (-0.61, -0.24) p < 0.0001 |
| **Urgency Episodes** | -0.51 (-0.76, -0.25) p = 0.0001 | -0.67 (-0.93, -0.42) p < 0.0001 |
| **Volume Voided (mL)** | 25.76 (20.02, 31.46) p < 0.0001 | 22.16 (16.44, 27.89) p < 0.0001 |
| a. Results presented as least squares mean placebo adjusted change from baseline (95% confidence interval [CI]), P-value. | | |

Example 3

**Safety Data**

*3.1 Phase I Safety Data*

Safety data from 16 Phase 1 studies, which include 15 completed Phase 1 studies and 1 study that was terminated early (this study was terminated for reasons unrelated to efficacy or safety) was collected. In the Phase 1 program, a total of 466 subjects received at least one dose of vibegron; 238 subjects received single doses ranging from 2 to 600 mg and 238 subjects received multiple doses ranging from 25 to 400 mg for up to 28 days. Across the Phase 1 program, vibegron has been generally well tolerated. There were no treatment-emergent serious adverse events (SAEs) or deaths reported, and the majority of adverse events (AEs) were transient and mild or moderate in intensity.

In Phase 1 studies, there were isolated occurrences of orthostatic hypotension (decrease in systolic blood pressure > 20 mmHg and/or decrease in diastolic blood pressure > 10 mmHg), with or without symptoms (e.g., lightheadedness, dizziness, presyncope). The incidence of orthostatic AEs following co-administration of vibegron 100 mg or 150 mg and tolterodine ER 4 mg was similar to the incidence of these AEs following administration of vibegron or tolterodine alone. At doses up to 100 mg in Phase 1 multiple dose studies, AEs such as postural dizziness, dizziness, presyncope, or syncope have not exhibited a clear dose-response relationship. However, postural dizziness appeared to increase at doses of 100 mg and above and the incidence of the AE "orthostatic hypotension with symptoms" has tended to be higher at vibegron doses > 200 mg. There were no occurrences of orthostatic AEs when vibegron 100 mg was coadministered to subjects with essential hypertension who were on a stable regimen of either metoprolol (a representative beta-blocker), or amlodipine (a representative vasodilator).

Review of preliminary Phase 1 safety data suggest no clinically meaningful changes in laboratory safety parameters (chemistry, hematology and urinalyses) or ECG parameters, including PR, QRS and QTc intervals. A thorough QT study has been completed, which found no clinically meaningful effect on QTc or blood pressure.

*3.2 Phase II Safety Data*

Phase 2 safety data from a single Phase 2B study that has completed in which 933 subjects received at least one dose of vibegron was collected. Subjects received vibegron doses ranging from 3 to 100 mg for up to 8 weeks during the main study (alone or in combination with tolterodine). Of those completing the parent study, 605 subjects received doses of vibegron 50 mg (alone) or vibegron 100 mg (alone or in combination with tolterodine 4 mg) for up to 52 weeks during an extension study. A placebo group was included in the main study, and a group that received tolterodine monotherapy was included in the main study and in the extension. There were no deaths reported during the study. Vibegron was generally well tolerated. No meaningful differences in the overall incidence or severity of AEs or drug-related AEs were observed

among the treatment groups compared to placebo.

Adverse events were reported in 607 (43.6%) of the 1393 allocated subjects in the main study. The proportion of subjects with one or more AEs in the vibegron 50 mg and vibegron 100 mg treatment groups was similar to placebo (see Table 14). A higher proportion of subjects reported one or more AEs in the vibegron 15 mg and vibegron 50 mg + tolterodine 4 mg treatment groups compared to placebo. The most frequently reported AEs were dry mouth, headache, urinary tract infections (UTI), and nasopharyngitis. The incidence of dry mouth was higher in groups that received tolterodine (alone or with vibegron) compared to the placebo or vibegron monotherapy groups.

There were 221 subjects with drug-related AEs, with the lowest incidence of drug-related AEs reported in the vibegron 100 mg treatment group. The proportion of subjects with drug-related AEs was similar in the vibegron monotherapy groups compared to placebo and only slightly higher in the concomitant treatment groups compared to placebo or either monotherapy. The proportion of subjects who discontinued due to a drug-related AE was low and similar across all treatment groups.

There were a total of 9 SAEs reported in 8 subjects and occurred across the treatment groups (2 placebo; 1 vibegron 3 mg; 1 vibegron 50 mg; 3 tolterodine 4 mg; 1 vibegron 50 mg + tolterodine 4 mg). The reported SAEs were atrial fibrillation, anaphylactic reaction, lung adenocarcinoma stage IV, chronic obstructive pulmonary disease, hypertension, overdose, foot fracture, and in one subject both gastroesphageal reflux disease and dizziness occurred after a pan endoscopic procedure that prolonged hospitalization. No specific AE term was reported in more than 1 subject. All SAEs were considered unrelated to study drug by the investigator.

During the 52-week extension, no meaningful differences in overall incidences of adverse events or serious adverse events were observed among the treatment groups.

Adverse events were reported in 531 (62.8%) of the 845 subjects. The proportion of subjects with one or more AEs was similar across all treatment groups. The most frequently reported adverse events were UTI, nasopharyngitis, upper respiratory tract infection, and dry mouth. The incidence of dry mouth was higher in the tolterodine ER 4 mg treatment group compared to the other treatment groups. The incidence of constipation was higher in the concomitant treatment group compared to the monotherapy treatment groups.

The proportion of subjects with drug-related AEs was slightly higher for tolterodine ER 4 mg and the concomitant dose arm compared to the vibegron 50 mg and 100 mg treatment arms. The proportion of subjects who discontinued due to an AE or a drug-related AE was higher for tolterodine ER 4 mg compared to the other treatment groups. There were total of 46 SAEs reported in 41 subjects during the extension. An overall higher incidence rate was reported in the tolterodine ER 4 mg and vibegron 50 mg treatment groups compared to the vibegron 100 mg treatment group. There was one drug-related SAE of ileus paralytic reported in the tolterodine ER 4 mg treatment group; the subject was discontinued due to this AE.

Table 5 below summarizes adverse events commonly seen in the vibegron Phase 2 program in patients with overactive bladder.

**Table 5 Adverse Events in ≥2% Subjects in Phase 2 Study (First 12 weeks of Treatment)**

| | Placebo | Vibegron 3 mg | Vibegron 15 mg | Vibegron 50 mg | Vibegron 100 mg | Tolterodine ER 4 mg | Vibegron 100 mg + tolterodine ER 4 mq | Vibegron 50 mg + Tolterodine ER 4 mg/ Vibegron 50 mq | Total |
|---|---|---|---|---|---|---|---|---|---|
| | N = 205 | N = 144 | N = 134 | N = 148 | N = 261 | N = 257 | N = 110 | N = 134 | N = 1,393 |
| | n (%) | n (%) | n (%) | n (%) | n (%) | n (%) | n (%) | n (%) | n (%) |
| ≥ 1 AE | 88 (42.9) | 55 (38.2) | 70 (52.2) | 62 (41.9) | 107 (41.0) | 116 (45.1) | 40 (36.4) | 69 (51.5) | |
| Serious AE | 2 (1.0) | 1 (0.7) | 0 | 1 (0.7) | 0 | 3 (1.2) | 0 | 8 (0.7) | |
| Drug-related AE | 30 (14.6) | 21 (14.6) | 23(17.2) | 23 (15.5) | 31 (11.9) | 42 (16.3) | 21 (19.1) | 30 (14.6) | |
| Discontinuation due to AE | 5 (2.4) | 3 (2.1) | 4 (3.0) | 2 (1.4) | 6 (2.3) | 4 (1.6) | 2 (1.8) | 3 (2.2) | |
| Discontinuation due to drug-related AE | 3 (1.5) | 2 (1.4) | 4 (3.0) | 0 | 3 (1.1) | 0 | 1 (0.9) | 2 (1.5) | |
| **SOC/ Preferred Term** | | | | | | | | | |
| **Eye disorders** | | | | | | | | | |
| Dry eye | 10 (4.9) | 2 (1.4) | 4 (3.0) | 2 (1.4) | 4 (1.5) | 10 (3.9) | 3 (2.7) | 2 (1.5) | 18 (1.3) |
| **Gastrointestinal disorders** | | | | | | | | | |
| Constipation | 5 (2.4) | 5 (3.5) | 6 (4.5) | 6 (4.1) | 2 (0.8) | 5 (1.9) | 4 (3.6) | 6 (4.5) | 39 (2.8) |
| Diarrhea | 5 (2.4) | 4 (2.8) | 2 (1.5) | 1 (0.7) | 5 (1.9) | 9 (3.5) | 1 (0.9) | 6 (4.5) | 33 (2.4) |
| Dry mouth | 6 (2.9) | 5 (3.5) | 6 (4.5) | 7 (4.7) | 4 (1.5) | 22 (8.6) | 13 (11.8) | 11 (8.2) | 74 (5.3) |
| Nausea | 3 (1.5) | 2 (1.4) | 2 (1.5) | 3 (2.0) | 3 (1.1) | 6 (2.3) | 0 (0.0) | 2 (1.5) | 21 (1.5) |
| **General disorders and administration site conditions** | | | | | | | | | |
| Fatigue | 1 (0.5) | 4 (2.8) | 6 (4.5) | 5 (3.4) | 2 (0.8) | 6 (2.3) | 2 (1.8) | 2 (1.5) | 28 (2.0) |

(continued)

| | | | | | | | | | |
|---|---|---|---|---|---|---|---|---|---|
| **Infections and infestations** | | | | | | | | | |
| Nasopharyngitis | 14 (6.8) | 3 (2.1) | 7 (5.2) | 8 (5.4) | 10 (3.8) | 4 (1.6) | 2 (1.8) | 3 (2.2) | 51 (3.7) |
| Sinusitis | 2 (1.0) | 00 | 2 (1.5) | 1 (0.7) | 00 | 1 (0.4) | 0 (0.0) | 4 (3.0) | 10 (0.7) |
| Urinary tract infection | 7 (3.4) | 5 (3.5) | 5 (3.7) | 8 (5.4) | 8 (3.1) | 12 (4.7) | 5 (4.5) | 7 (5.2) | 57 (4.1) |
| **Injury, poisoning and procedural complications** | | | | | | | | | |
| Accidental overdose | 2 (1.0) | 3 (2.1) | 6 (4.5) | 4 (2.7) | 11 (4.2) | 6 (2.3) | 1 (0.9) | 2 (1.5) | 35 (2.5) |
| **Investigations** | | | | | | | | | |
| Alanine aminotransferase increased | 0 (0.0) | 0 (0.0) | 0 (0.0) | 0 (0.0) | 0 (0.0) | 1 (0.4) | 0 (0.0) | 3 (2.2) | 4 (0.3) |
| Aspartate aminotransferase increased | 0 (0.0) | 0 (0.0) | 1 (0.7) | 0 (0.0) | 0 (0.0) | 0 (0.0) | 0 (0.0) | 3 (2.2) | 4 (0.3) |
| **Musculoskeletal and connective tissue** disorders | | | | | | | | | |
| Arthralgia | 2 (1.0) | 00 | 2 (1.5) | 3 (2.0) | 0 (0.0) | 3 (1.2) | 1 (0.9) | 0 (0.0) | 11 (0.8) |
| Osteoarthritis | 1 (0.5) | 2 (1.4) | 1 (0.7) | 4 (2.7) | 1 (0.4) | 0 (0.0) | 0 (0.0) | 0 (0.0) | 9 (0.6) |
| Pain in extremity | 00 | 2 (1.4) | 00 | 2 (1.4) | 1 (0.4) | 1 (0.4) | 3 (2.7) | 2 (1.5) | 11 (0.8) |
| **Nervous system disorders** | | | | | | | | | |
| Dizziness | 5 (2.4) | 1 (0.7) | 6 (4.5) | 3 (2.0) | 7 (2.7) | 5 (1.9) | 3 (2.7) | 1 (0.7) | 31 (2.0) |
| Headache | 9 (4.4) | 3 (2.1) | 6 (4.5) | 6 (4.1) | 12 (4.6) | 9 (3.5) | 7 (6.4) | 6 (4.5) | 58 (4.0) |
| **Renal and urinary disorders** | | | | | | | | | |
| Dysuria | 1 (0.5) | 0 (0.0) | 0 (0.0) | 1 (0.7) | 0 (0.0) | 3 (1.2) | 3 (2.7) | 0 (0.0) | 8 (0.6) |

Serious adverse events observed during the first 12 weeks of treatment with vibegron monotherapy included lung adenocarcinoma stage IV (n=1) and chronic obstructive pulmonary disease (n=1); an SAE of overdose was reported in the vibegron-tolterodine combination arm. During the Phase 2 extension study, SAEs reported by 2 or more subjects receiving monotherapy included cerebrovascular accident (n=2) and osteoarthritis (n=2). The only SAE reported in the vibegron - tolterodine combination arm was borrelia infection. SAEs potentially related to a change in heart rate or blood pressure (at any time during treatment) included: loss of consciousness after 8 weeks of vibegron that did not recur on rechallenge (n=1), and in the tolterodine monotherapy arm atrial fibrillation (n=1) and dizziness (n=1). The frequency of injuries was numerically higher in the tolterodine arm than with vibegron (2.1%, n=5, vs. 0.9%, n=4). Given the low incidence and lack of a pattern for SAEs, no serious event is considered expected for vibegron.

Potential risks that may be associated with vibegron treatment, based on nonclinical data and data available for similar compounds, include orthostatic hypotension and increased exposure (~2-fold) in patients taking concomitant strong P-gp inducers.

*3.3 Cardiovascular Safety*

The cardiovascular safety of vibegron has been evaluated in patients with OAB and healthy volunteers. In a randomized, placebo- and active comparator (tolterodine)-controlled, 2-part efficacy and safety study with 52-week extension, seven orthostatic related AEs (which included the adverse event terms of postural dizziness, presyncope, and orthostatic hypotension) occurred in 6 (0.4%) subjects. The events occurred in one subject each in the placebo group (0.5%), the vibegron 15 mg group (0.3%), and the vibegron 50 mg + tolterodine ER/vibegron 50 mg treatment group (0.8%), and in 3 subjects in the vibegron 100 mg group (1.1%). The events occurred at random times throughout the study and were judged by the investigator to be mild in severity. None led to discontinuation. The overall incidence of orthostatic symptoms was low.

Changes from baseline in BP and HR across treatment groups are shown in Table 6. For systolic blood pressure (SBP) and diastolic blood pressure (DBP), the mean changes at Week 1 and mean maximum changes over 8 weeks for 50 mg and 100 mg were comparable between placebo and vibegron, with differences of < 1 mm Hg. Categorical changes in SBP and DBP also were similar between placebo and vibegron, with a slight increase at 100 mg in percent of vibegron subjects with a change from baseline in DBP > 15 mmHg (1.3% 100 mg vs 0.5% placebo). No dose-dependent pattern was detectable for HR, as the mean maximum changes over 8 weeks were comparable to placebo (< 2 bpm). Small differences in the percent of subjects exceeding categorical heart rate and blood pressure thresholds for vibegron were similar to those in the tolterodine arm.

**Table 6 Vital Sign Changes from Baseline for Vibegron and Tolterodine by Dose**

| HR Change from Baseline | | | | | | | |
|---|---|---|---|---|---|---|---|
| **Treatment** | **n** | **Mean (95% CI) (week 1)** | **n** | **Mean (95% CI) Maximum** | **≥5 bpm n/N (%)** | **≥10 bpm n/N (%)** | **≥15 bpm n/N (%)** |
| Placebo | 186 | 0.12 (-1.04, 1.28) | 200 | 5.08 (4.02, 6.13) | 17/188 (9.0) | 1/188 (0.5) | 0 |
| 3mg | 141 | 0.36 (-0.90, 1.62) | 143 | 5.57 (4.25, 6.90) | 16/140 (11.4) | 5/140 (3.6) | 1/140 (0.7) |
| 15mg | 126 | 0.35 (-1.15, 1.85) | 134 | 6.56 (5.20, 7.92) | 17/132 (12.9) | 4/132 (3.0) | 1/132 (0.8) |
| 50mg | 140 | 0.29 (-0.88, 1.46) | 146 | 5.49 (4.28, 6.69) | 12/144 (8.3) | 4/144 (2.8) | 1/144 (0.7) |
| 100mg | 237 | 0.35 (-0.69, 1.38) | 257 | 6.12 (5.10, 7.15) | 28/237 (11.8) | *7/237* (3.0) | 1/237 (0.4) |
| Tolterodine 4mg | 246 | 0.68 (-0.31, 1.67) | 257 | 5.66 (4.69, 6.63) | 29/242 (12.0) | 11/242 (4.5) | 4/242 (1.7) |
| Mean maximum is from week 1 to 8.<br>Counts based on 3 Consecutive Post-Baseline Visits. | | | | | | | |

(continued)

| SBP Change from Baseline | | | | | | | |
|---|---|---|---|---|---|---|---|
| Treatment | n | Mean (95% CI) (week 1) | n | Mean (95% CI) Maximum | ≥5 mm Hg n/N (%) | ≥10 mm Hg n/N (%) | ≥15 mm Hg n/N (%) |
| Placebo | 186 | -0.21 (-1.85, 1.43) | 200 | 7.84 (6.27, 9.40) | 24/188 (12.8) | 10/188 (5.3) | 3/188 (1.6) |
| 3mg | 141 | -0.35 (-2.34, 1.65) | 143 | 7.14 (5.18, 9.10) | 21/140 (15.0) | 10/140 (7.1) | 4/140 (2.9) |
| 15mg | 126 | -0.34 (-2.46, 1.78) | 134 | 8.93 (7.18, 10.67) | 22/132 (16.7) | 9/132 (6.8) | 1/132 (0.8) |
| 50mg | 140 | -0.79 (-2.65, 1.08) | 146 | 7.01 (5.31, 8.70) | 24/144 (16.7) | 14/144 (9.7) | 3/144 (2.1) |
| 100mg | 237 | -0.77 (-2.22, 0.68) | 257 | 6.51 (5.09, 7.93) | 28/237 (11.8) | 10/237 (4.2) | 3/237 (1.3) |
| Tolterodine 4mg | 246 | 0.04 (-1.36, 1.43) | 257 | 7.29 (6.01, 8.57) | 41/242 (16.9) | 19/242 (7.9) | 7/242 (2.9) |
| Mean maximum is from week 1 to 8.<br>Counts based on 3 Consecutive Post-Baseline Visits. | | | | | | | |
| DBP Change from Baseline | | | | | | | |
| Treatment | n | Mean (95% CI) (week 1) | n | Mean (95% CI) Max | ≥5 mm Hg n/N (%) | ≥10 mm Hg n/N (%) | ≥15 mm n/N Hg (%) |
| Placebo | 186 | 0.11 (-0.94, 1.17) | 200 | 4.89 (3.89, 5.89) | 18/188 (9.6) | 6/188 (3.2) | 1/188 (0.5) |
| 3mg | 141 | -0.37 (-1.69, 0.95) | 143 | 5.03 (3.92, 6.15) | 14/140 (10.0) | 3/140 (2.1) | 1/140 (0.7) |
| 15mg | 126 | 0.03 (-1.52, 1.59) | 134 | 6.37 (5.20, 7.53) | 15/132 (11.4) | 3/132 (2.3) | 1/132 (0.8) |
| 50mg | 140 | -0.70 (-2.07, 0.67) | 146 | 4.19 (3.10, 5.29) | 11/144 (7.6) | 5/144 (3.5) | 1/144 (0.7) |
| 100mg | 237 | -0.69 (-1.72, 0.34) | 257 | 4.80 (3.88, 5.72) | 31/237 (13.1) | 8/237 (3.4) | 3/237 (1.3) |
| Tolterodine 4mg | 246 | -0.12 (-1.10, 0.86) | 257 | 5.19 (4.26, 6.13) | 30/242 (12.4) | 13/242 (5.4) | 3/242 (1.2) |
| Mean maximum is from week 1 to 8.<br>Counts based on 3 Consecutive Post-Baseline Visits. | | | | | | | |

More intensive assessments of heart rate and blood pressure were performed in healthy volunteers in several Phase 1 studies. A 6-part, double-blinded, randomized, placebo-controlled study to assess the safety, tolerability and multiple-dose PK of vibegron in healthy subjects that included specific analyses for heart rate. Doses ranged from 25 to 400 mg once daily for 7 to 28 days depending on the cohort. Least squares mean and 90% confidence intervals of maximum change from baseline in moving average of heart rate over 4 hours postdose (MA4 HR) are presented in Table 7. Effects on heart rate were dose dependent and the 100 mg dose demonstrated a < 1 bpm difference from placebo.

**Table 7 Maximum MA4 HR and Difference between Vibegron and Placebo at Day 14**

| Panel | Dose (mg) | N[a] | Maximum MA4 HR[b] | Difference from Placebo[c] |
|---|---|---|---|---|
| All | Placebo | 14 | 3.07 (0.26, 5.88) | |
| A | 25 | 5 | 1.47 (-3.24, 6.17) | -1.60 (-7.09, 3.88) |
| B | 50 | 6 | 1.50 (-2.79, 5.79) | -1.57 (-6.70, 3.56) |

(continued)

| Panel | Dose (mg) | N[a] | Maximum MA4 HR[b] | Difference from Placebo[c] |
|---|---|---|---|---|
| C | 100 | 6 | 3.28 (-1.02, 7.57) | 0.21 (-4.93, 5.34) |
| D | 150 | 6 | 3.17 (-1.13, 7.46) | 0.10 (-5.04, 5.23) |
| G | 200 | 5 | 5.67 (0.96, 10.37) | 2.60 (-2.89, 8.08) |
| H | 300 | 6 | 9.06 (4.76, 13.35) | 5.98 (0.85, 11.12) |
| I | 400 | 6 | 10.33 (6.04, 14.63) | 7.26 (2.13, 12.39) |

a. One subject each in panels A and G discontinued and had no available data at day 14
b. Least-square mean and corresponding 90% confidence interval
c. Difference of least squares (active - placebo) and corresponding 90% confidence interval calculated from the linear fixed effects model

Cardiovascular safety was also assessed in healthy volunteers in the thorough QT study following single doses of 200 and 400 mg, which approximate vibegron steady-state exposures at 100 mg and 200 mg, respectively. Mean maximum effects on blood pressure and RR interval were reduced with the lower dose as shown in Table 8. Using a log-log regression analysis from multiple-dose vibegron exposures (from three Phase 1 studies), the calculated mean ± standard deviation $C_{max}$ and AUC from a 75 mg dose were 120 ± 74.7 ng/mL and 1140 ± 476 ng·h/mL, respectively. These estimations represent a $C_{max}$ and AUC that are approximately 3.3-fold and 2-fold lower, respectively, than the 200 mg single dose and 9.2-fold and 6-fold lower, respectively, than the 400 mg single dose.

**Table 8 Single-Dose Pharmacokinetic Parameters and Mean Placebo-Corrected Change from Baseline RR interval and Blood Pressure**

| Dose (mg) | Mean±SD $C_{max}$ (ng/mL) | Mean±SD AUC (ng.h/mL) | Maximum Mean Placebo Corrected Change from Baseline RR interval (90% CI) (msec) | Maximum Mean Placebo Corrected Change from Baseline Systolic BP (90% CI) (mmHg) | Maximum Mean Placebo Corrected Change from Baseline Diastolic BP (90% CI) (mmHg) |
|---|---|---|---|---|---|
| Vibegron 400 | 1100 ± 436 | 6800 ± 2300 | -162.45 (-184.24, -140.65) | 3.97 (2.12, 5.81) | 3.99 (2.62, 5.36) |
| Vibegron 200 | 406 ± 180 | 2430 ± 974 | -84.36 (-106.37, -62.35) | 2.20 (0.34, 4.06) | 2.42 (1.04, 3.81) |

Example 4

**Dose Selection**

*4.1 Dose Comparison Efficacy*

The Phase 2 study discussed in Example 2 demonstrated a dose-dependent effect on micturitions as seen in Table 9. Conversely, a dose dependent effect on urge incontinence or total incontinence was not observed. These data reveal a relatively shallow dose-response relationship between 50 and 100 mg once daily. Since vibegron efficacy begins to plateau from 50 to 100 mg, 75 mg captures a majority of the efficacy achieved with 100 mg.

**Table 9 Efficacy of Vibegron 50 mg and 100 mg in Phase 2 Study**

| Parameter | 50mg - Placebo | 100 mg - Placebo | 100mg - 50mg |
|---|---|---|---|
| **Micturitions[a]** | -0.64 | -0.91 | -0.27 |
| **Urgency incontinence[b]** | -0.72 | -0.71 | 0.01 |
| **Total incontinence[b]** | -0.60 | -0.58 | 0.02 |
| **Urgency Episodes[b]** | -0.76 | -1.24 | -0.48 |

(continued)

| Parameter | 50mg - Placebo | 100 mg - Placebo | 100mg - 50mg |
|---|---|---|---|
| **Volume voided[c] (ml)** | 29.1 | 23.4 | -5.69 |
| Data reported as difference in LS means<br>a change from baseline in average number of micturitions at week 8<br>b change from baseline in average number of episodes at week 8<br>c change from baseline in average volume per void over one diary day at week 8 | | | |

Unexpectedly, reductions in UUI episodes and micturitions were not dose-proportional when measured at week 8. Comparing 50 mg week 8 change-from-baseline data from the Phase 2 trial (Table 3) with the 75 mg week 8 change-from-baseline data from the Phase 3 trial with respect to UUI episodes (Table 15), it is unexpected that the 75 mg dose used in the Phase 3 trials provides a lower reduction relative to the 50 mg dose used in Phase 2. Similarly, the decrease in micturitions was lower for the 75 mg dose (Table 16) than the reduction seen with 50 mg (Table 3).

*4.2 Mitigating Side Effects*

Vibegron demonstrates greater than a dose proportional increase in exposures. Unexpectedly, an increase in dose from 50 to 100 mg results in an approximate 3-fold increase in $C_{max}$, the PK parameter considered most closely associated with cardiovascular effects. In order to contextualize PK parameters of a 75 mg dose, dose-$C_{max}$ and dose-AUC models were created using data from Phase 1 studies. Based on simulations, it was found that a vibegron dose of 75 mg avoids approximately 29% of the exposures observed with a 100 mg dose, subsequently reducing the upper range of exposures that would be achieved with a 100 mg dose. This reduction in outlier $C_{max}$ values reduces the potential for clinically relevant cardiovascular effects.

In Phase 1 multiple dose studies, at doses up to 100 mg, adverse events such as postural dizziness, dizziness, presyncope, syncope did not exhibit a clear dose-response relationship. However, postural dizziness appeared to increase at doses ≥ 100 mg and the incidence of the adverse event "orthostatic hypotension with symptoms" was higher at vibegron doses greater than 150 mg. The risk of these dose-related adverse events can be disproportionally reduced by decreasing the dose from 100 mg to 75 mg, as a 25% reduction in dose produces an approximate 40% reduction in $C_{max}$ (120 ng/mL with 75 mg vs. 206 ng/mL with 100 mg). Without wishing to be bound by theory, the greater than dose-proportional increase in bioavailability with increasing dose may be due to saturable P-glycoprotein (P-gp)-mediated efflux in the gut.

A lower exposure with the 75 mg dose compared to a 100 mg dose disproportionally reduces the risk of adverse events in special populations as well. Subjects with moderate renal impairment had a mean increase in AUC of 1.6-fold compared to subjects with normal renal function whereas subjects receiving a potent CYP3A/P-gp inhibitor had an approximate 2-fold higher exposure. Assuming a 2 fold increase in $C_{max}$ of a 75 mg dose, the probability of these special populations achieving a vibegron $C_{max}$ greater than those observed with 100 mg is 15% (see Figure 1). Minimizing exposures of subjects who fall at the extremes is important for elderly and females who demonstrated approximately a 50-70% higher $C_{max}$ than healthy young males.

Example 5

**Pharmacokinetic Data for 75 mg Dose and 100 mg Dose**

A pharmacokinetic study evaluating the drug interaction of vibegron and rifampin was completed. All subjects were healthy adults. A summary of the preliminary results is presented in Table 10. Subjects received a single dose of vibegron 75 mg on day 1, rifampin 600 mg QD on days 10-23, and a single dose of vibegron 75 mg on day 17 concomitantly with the rifampin dose. Administration of vibegron and rifampin were well tolerated in healthy male and female subjects. There were no severe TEAEs, SAEs or deaths reported during the study. Three of 20 subjects (15%) experienced an AE related to study drug, 2 headache and 1 constipation, all mild. No clinically significant changes or findings were observed in vital signs, ECGs or clinical laboratory assessments.

**Table 10. Geometric Mean (%CV): Pharmacokinetic Parameters of Vibegron**

| | Regimens | |
|---|---|---|
| **Pharmacokinetic Parameters** | **Vibegron alone (N = 18)** | **Vibegron + Rifampin (N = 18)** |
| $t_{max}$[a] (h) | 1.0 (0.5, 2.0) | 1.0 (0.5, 2.0) |

(continued)

| | Regimens | |
|---|---|---|
| Pharmacokinetic Parameters | Vibegron alone (N = 18) | Vibegron + Rifampin (N = 18) |
| Cmax (ng/mL) | 82.3 (43.2) | 153.9 (47.1) |
| $AUC_{0-t}$ (ng·h/mL) | 1160 (34.3) | 1210 (30.7) |
| $AUC_{0-\infty}$ (ng·h/mL) | 1310 (36.4) | 1310 (31.5) |
| $t_{1/2}$ (h) | 84.0 (15.0) | 74.2 (25.2) |
| CL/F (L/hr) | 57.2 (39.7) | 57.3 (30.1) |
| V/F (L) | 6930 (48.6) | 6140 (47.0) |
| a.Median (minimum, maximum) | | |

A double blind, double dummy randomized and placebo controlled study evaluating the drug interaction of vibegron and tolterodine, a sensitive CYP2D6 substrate, was completed. All subjects were healthy adults. A summary of the preliminary results is presented in Table 11 and Figure 2. Subjects received either a dose of vibegron 100 mg QD concomitantly with a dose of tolterodine 4mg QD or a dose of vibegron 100 mg QD with a tolterodine placebo on days 1-7. The subjects receiving the tolterodine placebo were dosed with vibegron 100 mg QD and tolterodine 4 mg QD on days 29-35. Those initially receiving the tolterodine 4mg QD were dosed with vibegron 100 mg QD and a tolterodine placebo on days 29-35. Study treatments were well tolerated, with no evidence of increase of adverse events during combination administration with vibegron. As shown in Table 11, co-administration of vibegron did not alter the geometric mean maximum plasma concentration (Cmax) and the area under the curve (AUC) of tolterodine. The elimination half-life of tolterodine was similar alone and in the presence of steady-state vibegron. In addition, plasma concentration over time for tolterodine alone was similar to the profile seen with tolterodine and vibegron (Figure 2). These results demonstrate that vibegron does not inhibit CYP2D6 and has a favorable drug-drug interaction profile for use in patients with OAB.

Table 11. Pharmacokinetic Parameters of Vibegron in Combination with Tolterodine ER

| Pharmacokinetic parameter | Tolterodine ER+ Vibegron (N=12) | Tolterodine ER (n=12) | Ratio: Tolterodine + vibegron/Tolterodine | |
|---|---|---|---|---|
| | GM (%CV) | GM (%CV) | GMR | 90% CI |
| $AUC_{0-24}$ (ng·hr/mL) | 30.7 (102) | 28.4 (104) | 1.08 | (0.97,1.21) |
| $C_{max}$ (ng/mL) | 2.57 (92.5) | 2.28 (83.8) | 1.12 | (1.00, 1.26) |
| $t_{1/2}$ (hr) | 10.0 (46.9) | 10.0 (37.1) | NA | |

Toletrodine ER 4mg; Vibegron 100mg
AUC = area under the concentration time curve from 0 to 24 hours; $C_{max}$ = maximum concentration; $t_{1/2}$ = half-life; GM = geometric mean; CV = coefficient of variation; CI = confidence interval; GMR = geometric mean ratio

A pharmacokinetic study evaluating the drug interaction of vibegron and metoprolol succinate, a moderately sensitive CYP2D6 substrate, was also completed. All subjects were healthy adults. A summary of the preliminary results is presented in Table 12 and Figure 3. Subjects received a single dose of metoprolol succinate 100 mg with warfarin 10 mg on day 1, vibegron 75 mg QD on days 8-16, a dose of vibegron 75 mg concomitantly with a dose of metoprolol succinate 100 mg and warfarin 10 mg on day 17, and vibegron 75 mg QD on days 18-23. Study treatments were well tolerated, with no evidence of increase of adverse events during combination administration with vibegron. Metoprolol geometric mean Cmax and AUC values increased slightly in the presence of vibegron. However, the elimination half-life of metoprolol was similar alone and with vibegron, suggesting CYP2D6 was unlikely inhibited. Plasma concentration over time for metoprolol alone was similar to the profile seen with metoprolol and vibegron (Figure 3).

Table 12. Pharmacokinetic Parameters of Vibegron in Combination with Metoprolol

| Pharmacokinetic parameter | Metoprolol + Vibegron (N=24) | Metoprolol (n=24) | Ratio: Metoprolol + Vibegron/Metoprolol alone | |
|---|---|---|---|---|
| | GM (%CV) | GM (%CV) | Ratio of GLS Mean | 90% CI |
| $AUC_{0-\infty}$ (ng·hr/mL) | 539 (79.4) | 384 (74.7) | 1.40 | (1.30 to 1.52) |

(continued)

| Pharmacokinetic parameter | Metoprolol + Vibegron (N=24) | Metoprolol (n=24) | Ratio: Metoprolol + Vibegron/Metoprolol alone | |
|---|---|---|---|---|
| | GM (%CV) | GM (%CV) | Ratio of GLS Mean | 90% CI |
| $C_{max}$ (ng/mL) | 32.7 (64.7) | 21.9 (67.6) | 1.49 | (1.35 to 1.65) |
| $t_{1/2}$ (hr) | 10.9 (52.8) | 9.5 (45.2) | NA | |
| Metoprolol 100mg; Vibegron 75mg<br>AUC = area under the concentration time curve from 0 to infinity; $C_{max}$ = maximum concentration; $t_{1/2}$ = half-life; GM = geometric mean; CV= coefficient of variation; GLS = geometric least squares; CI = confidence interval | | | | |

Example 6

**A Phase 3, Randomized, Double-Blind, Placebo- and Active (Tolterodine)-Controlled Clinical Study**

An international, Phase 3, randomized, double-blind, placebo-controlled with active control (tolterodine), parallel-group, multicenter study in men and women with overactive bladder, was conducted. Enrollment of patients included individuals with OAB Wet (those with UUI, the involuntary loss of urine accompanied by urgency) and OAB Dry (those without UUI). A total of 1,518 patients were randomized across 215 study sites into one of three groups for a 12-week treatment period.

The study consisted of a Screening Period (1 to 5 weeks), a single-blind Run-in Period (2 weeks), a randomized double-blind Treatment Period (12 weeks), and a Follow-up Period (4 weeks).

The study assessed the safety, tolerability, and efficacy of 75 mg vibegron versus placebo. Tolterodine ER 4 mg was the active control. Patients were randomized 5:5:4 in a double-blind fashion to one of three treatment arms: vibegron 75 mg, placebo, or tolterodine ER 4 mg, all administered once daily for 12 weeks during the Treatment Period. Between the Baseline and Week 12 Visits, patients had study assessments at Week 4 and Week 8.

*6.1. Eligibility Criteria*

To be eligible for participation in this study, a patient must have met all the following Inclusion Criteria, and none of the following Exclusion Criteria, before enrollment.

*6.1.1 Inclusion Criteria*

1. Willing and able to provide written informed consent.
2. Males or females ≥ 18 years of age. Note: Up to 15% of patients can be male.
3. Has a history of OAB (as diagnosed by a physician) for at least 3 months prior to the Screening Visit. Note: OAB is defined as urgency, with or without urge urinary incontinence (UUI), usually associated with frequency and nocturia. Urodynamic evaluation is not required.
4. Meets either the OAB Wet or OAB Dry criteria described below, based on the Patient Voiding Diary returned both at the Run-in Visit and Baseline Visit (all Complete Diary Days must be used in determining eligibility). A minimum of 5 Complete Diary Days [not necessarily consecutive] are required for the diary returned at the Run-in Visit, and 4 Complete Diary Days are required for the diary returned at the Baseline Visit. Averages should not be rounded up to the whole number:

    a. OAB Wet criteria:

        i. An average of ≥ 8.0 micturitions per Diary Day; and
        ii. An average of ≥ 1.0 UUI episodes per Diary Day; and
        iii. If stress urinary incontinence is present, the total number of UUI episodes must be greater than the total number of stress urinary incontinence episodes from the previous visit diary.

    b. OAB Dry criteria:

i. An average of ≥ 8.0 micturitions per Diary Day; and,
ii. An average of ≥ 3.0 urgency episodes per Diary Day; and
iii. An average of < 1.0 UUI episodes per Diary Day; and
iv. If stress urinary incontinence is present, the total number of UUI episodes must be greater than the total number of stress urinary incontinence episodes from the previous visit diary.

5. For females of reproductive potential: Agrees to remain abstinent or use (or have their male partner use) an acceptable method of birth control (as defined in Section 5.2.1) each time the patient has intercourse from the Screening Visit until completion of the Follow-up Visit.
6. For females of reproductive potential: Agrees not to donate ova (eggs) until at least 1 month after the last dose of Study Treatment.
7. Has demonstrated ≥ 80% compliance with self-administration of Study Treatment during the Run-in Period.
8. Is ambulatory and in good general physical and mental health as determined by the Investigator.
9. In the opinion of the Investigator, is able and willing to comply with the requirements of the protocol, including completing electronic versions of questionnaires, the Patient Voiding Diary, and the Urine Volume Diary (will require ability to collect, measure, and record voided volume by herself/himself using a graduated urine collection and measurement container [provided by the Sponsor, if needed]).

*6.1.2. Exclusion Criteria*

Urology Medical History

1. Patient has a history of 24-hour urine volume greater than 3,000 mL in the past 6 months, or a Urine Volume Diary day measurement greater than 3,000 mL during the Run-in Period.
2. Has lower urinary tract pathology that could, in the opinion of the Investigator, be responsible for urgency, frequency, or incontinence; including, but not limited to, urolithiasis, interstitial cystitis, prostate cancer, gastrointestinal (GI) cancer, tuberculosis, stone disease, urothelial tumor, prostatitis, and clinically relevant benign prostatic hypertrophy (BPH) or bladder outlet obstruction, as judged by the Investigator. Note: Male patients with mild to moderate BPH without evidence of bladder obstruction as determined by the Investigator may be included as long as they have been taking a medication for the treatment of BPH for at a least 1-year prior to Screening, with no change in dose of herbal medications, alpha antagonist medications or other symptomatic treatments or medications within 3 months prior to Screening, and no change in dose of 5 alpha reductase inhibitors within 6 months of Screening.
3. Has a history of surgery to correct stress urinary incontinence, pelvic organ prolapse, or procedural treatments for BPH within 6 months of Screening.
4. Has current history or evidence of Stage 2 or greater pelvic organ prolapse (prolapse extends beyond the hymenal ring).
5. Patient is currently using a pessary for the treatment of pelvic organ prolapse.
6. Has a known history of elevated post-void residual volume defined as greater than 150 mL.
7. Has undergone bladder training or electrostimulation within 28 days prior to Screening or plans to initiate either during the study.
8. Has an active or recurrent (> 3 episodes per year) urinary tract infection by clinical symptoms or laboratory criteria (≥ 5 white blood cells [WBC] or a positive urine culture, defined as ≥ 105 colony forming units [CFU]/mL in 1 specimen). Patients diagnosed with a urinary tract infection (UTI) at the Screening Visit may be treated and re-screened once the infection has resolved.
9. Has a requirement for an indwelling catheter or intermittent catheterization.
10. Has received an intradetrusor injection of botulinum toxin within 9 months prior to Screening.

Other Medical History

11. Has uncontrolled hyperglycemia (defined as fasting blood glucose > 150 mg/dL or 8.33 mmol/L and/or non-fasting blood glucose > 200 mg/dL or 11.1 mmol/L) or, if in the opinion of the Investigator, is uncontrolled.
12. Has evidence of diabetes insipidus.
13. Is pregnant, breast-feeding, or is planning to conceive within the projected duration of the study.
14. Has a concurrent malignancy or history of any malignancy within 5 years prior to signing informed consent, except for adequately treated basal cell or squamous cell skin cancer or in situ cervical cancer.
15. Has uncontrolled hypertension (systolic blood pressure of ≥ 180 mmHg and/or diastolic blood pressure of ≥

100 mmHg) or has a resting heart rate (by pulse) > 100 beats per minute.

16. Patients who have systolic blood pressures ≥ 160 mmHg but < 180 mmHg are excluded, unless deemed by the Investigator and/or Medical Monitor as safe to proceed in this study and able to complete the study per protocol; these patients must be on stable hypertension medication for at least 90 days.

17. All patients with signs and symptoms of uncontrolled hypertension, regardless of blood pressure measurement, are excluded from the study. These include, but are not limited to neurological symptoms or findings, hematuria, proteinuria, retinopathy, unstable angina, and acute heart failure.

18. Has narrow angle glaucoma (primary open angle glaucoma is not excluded).

19. Has a history of cerebral vascular accident, transient ischemic attack, unstable angina, myocardial infarction, coronary artery interventions (e.g., coronary artery bypass grafting or percutaneous coronary interventions [e.g., angioplasty, stent insertion]), or neurovascular interventions (e.g., carotid artery stenting) within 6 months prior to the Screening Visit. Patients with these conditions should be on stable medical therapy for at least 3 months prior to the Screening Visit.

20. Has a known history of liver disease.

21. Has a history of injury, surgery, or neurodegenerative diseases (e.g., multiple sclerosis, Parkinson's) that could affect the lower urinary tract or its nerve supply.

*6.2 Study Assessments And Procedures*

The Investigator or qualified designee reviewed prior medication use, including any protocol-specified washout requirement, and recorded prior medication taken by the patient within 28 days prior to beginning completion of the Screening eDiary.

All medications for the treatment of OAB taken within 1 year of the Screening Visit were recorded. Medication history was assessed for male patients with history of mild to moderate BPH to ensure a stable treatment regimen that meets eligibility criteria.

Concomitant medications were reviewed and recorded at each study visit from Screening through Week 12 and at any Unscheduled Visits.

Male patients with mild to moderate BPH without evidence of bladder obstruction as determined by the Investigator could be included as long as they had been taking a medication for the treatment of BPH for at a least 1-year prior to Baseline, with no change in dose of herbal medications, alpha antagonist medications, or other symptomatic treatments or medications within 3 months prior to Baseline. To be eligible for the study, these BPH medication/s must have been stable from Screening until Baseline Visit.

Patients with a history of hypertension must have been on a stable blood pressure treatment regimen for 90 days prior to the Baseline Visit and must have been deemed by the Investigator and/or Medical Monitor as safe to proceed in this study and able to complete the study per protocol.

*6.2.1 Patient Voiding Diary*

The Patient Voiding Diary was used by participants (via the eDiary or paper Diary) to record the frequency of daily OAB symptoms including all micturitions, urgency, incontinence, and main reason for incontinence by selecting the respective box for each symptom occurring during the course of a given day and night.

A "Diary Day" is defined as the time between when the patient gets up for the day each morning (i.e., the time the patient got up for the day yesterday to the time the patient got up for the day today; approximately a 24-hour period).

A "Complete Diary Day" is defined as a Diary Day for which the patient indicates that they have recorded all urinations and any leakages that occurred during that Diary Day. Specifically, on the eDiary the patient will respond Yes to the corresponding item in the Begin Day Questionnaire to indicate that their data are complete for the preceding Diary Day.

*6.2.2 Urine Volume Diary*

Urine volume data were collected separately by patients using the Urine Volume component of the eDiary, or the paper Urine Volume Chart. The Urine Volume Chart is a tool used in clinical practice and clinical investigation to assess voiding functions over a 24-hour period and is regarded as a useful instrument in the investigation of patients with voiding symptoms. Urine volume may be collected during any one (1) of the 7 Diary Days prior to the visit, and it should be recorded for ~24 hours starting from the time the patient gets up for the day and continues until the time the patient gets up for the day on the next day.

*6.2.3 Patient-Reported Outcomes*

Patients completed paper questionnaires at the site at the start of each required study visit to assess patient-perceived symptom relief, symptom bother, and health-related quality of life at the study visits. Recommended guidelines for the use of patient-reported outcome measures can be found in "Guidance for Industry, Patient-Reported Outcome Measures: Use in Medical Product Development to Support Labeling Embodiments," U.S. Department of Health and Human Services, Food and Drug Administration, December 2009, which is incorporated by reference in its entirety. These included the following questionnaires:

1. Global Impression Items include Patient Global Impression of Severity (PGI-Severity), Patient Global Impression of Control (PGI-Control), Patient Global Impression of Frequency (PGI-Frequency), Patient Global Impression of Leakage (PGI-Leakage), and Patient Global Impression of Change (PGI-Change).
2. Overactive Bladder Questionnaire (OAB-q long form [OAB-q LF], 1-week recall) is a 33-item patient-administered, disease-specific questionnaire that includes a Health-Related QoL (HRQL) scale (25 items) and a Symptom Bother Scale (8 items). The HRQL is a multidomain concept that represents the patient's general perception of the effect of illness and treatment on physical, psychological, and social aspects of life. Claiming a statistical and meaningful improvement in HRQL implies: (1) that all HRQL domains that are important to interpreting change in how the clinical trial's population feels or functions as a result of the targeted disease and its treatment were measured; (2) that a general improvement was demonstrated; and (3) that no decrement was demonstrated in any domain. In the present study, the HRQL scale is divided into 4 subscales: Coping, Concern/Worry, Sleep, and Social Interaction. The Coping subscale (also referred to as the coping domain score) was a secondary endpoint, with items scored from 1 (none of the time) to 6 (all the time), with higher scores indicating better QoL. The HRQL total score is calculated by summating the individual HRQL subscale scores. The Symptom Bother Scale items are scored from 1 (not at all) to 6 (a very great deal), with higher Symptom Bother scores indicating greater symptom severity. The instrument was developed and validated in both continent and incontinent OAB patients, including both men and women.
3. Work Productivity and Activity Impairment Questionnaire-Urinary Symptoms (WPAI-US), version 2.0, is a 6-item questionnaire that assesses health-related work productivity loss due to urinary symptoms with a 1-week recall period.
4. The EQ-5D health questionnaire is a standardized instrument for use as a measure of health outcome. It is applicable to a wide range of health conditions and treatments; it provides a simple descriptive profile and a single index value for health status.

*6.2.4 Post-Void Residual Volume*

The risk of acute urinary retention or morbidities related to an increase in Post-Void Residual (PVR) is a concern with antimuscarinic therapy that promotes smooth muscle relaxation by inhibiting acetylcholine-induced smooth muscle contraction. If, during contraction, the bladder cannot generate enough pressure to overcome the outlet resistance in the urethra, either because of poor detrusor contractility or profound obstruction (most commonly from BPH), acute urinary retention or incomplete emptying of the bladder may result.

The volume of urine that remains in the bladder after voiding (PVR) is an objective measurement that may serve as a proxy for impaired ability to void.

PVR were performed via ultrasound at the visits indicated in the Schedule of Activities.

*6.3 Pharmacokinetic Sample Collection*

PK sampling were conducted in a sub-population of patients (approximately 30% of the total study population) at selected sites for assessment of the effect of demographic covariates by population PK analysis.

*6.4 Safety Considerations*

Study assessments of safety included adverse events, physical examinations, vital signs (and weight), and clinical laboratory tests

An adverse event is any untoward medical occurrence in a patient or clinical investigation patient, temporally associated with the use of a medicinal product, whether or not considered related to the medicinal product.

An adverse event can therefore be any unfavorable and unintended sign (including an abnormal laboratory finding), symptom, or disease (new or exacerbated) temporally associated with the use of a medicinal product or protocol-specified procedure, whether or not considered related to the medicinal product or protocol-specified procedure.

Events meeting the definition of an adverse event included:

1. A worsening, excluding minor fluctuations, in the nature, severity, frequency, or duration of a pre-existing condition;
2. A new condition detected or diagnosed after investigational product administration even though it may have been present prior to the start of the study;
3. Injury or accidents: If a medical condition was known to have caused the injury or accident, the medical condition and the accident should be reported as 2 separate medical events (e.g., for a fall secondary to dizziness, both "dizziness" and "fall" should be recorded separately);
4. An investigational abnormality (e.g., laboratory parameter, vital sign, ECG) only if the abnormality was considered clinically significant by the Investigator based on at least one of the following criteria:

   a. Induces clinical signs or symptoms;
   b. Requires active intervention;
   c. Requires interruption or discontinuation of Study Treatment.

5. Signs, symptoms, or the clinical sequelae of a suspected interaction.
6. Signs, symptoms, or the clinical sequelae of a suspected overdose of either investigational product or a concomitant medication.

The Investigator or site staff was responsible for detecting, documenting, and reporting events that meet the definition of an adverse event or serious adverse event.
Adverse Events of Clinical Interest for this study included:

1. Potential Major Adverse Cardiac and Cerebrovascular Events (MACCE), which were adjudicated by an independent external expert clinical adjudication committee (CAC) into the following categories according to the definitions in the CAC Charter:

   Death or any event with fatal outcome
   Myocardial infarction / Heart Attack
   Cerebrovascular Accident / Stroke
   Hospitalization for Unstable Angina / Chest Pain
   Hospitalization for Heart Failure
   Coronary revascularization / Angioplasty / Stent

2. Hypertension:
An adverse event of hypertension was reported and was considered as an AECI as follows:

   For patients without hypertension (average SBP $<140$ mmHg, DBP $<90$ mmHg) at baseline, at two consecutive visits, the average of three systolic blood pressure (SBP) $\geq 140$ mmHg or diastolic blood pressure (DBP) $\geq 90$ mmHg (or both); at 2 consecutive visits in patients who were not hypertensive at baseline; or,
   For patients with hypertension at baseline, an increase compared to baseline at 2 consecutive visits in the average of three SBP by $\geq 20$ mmHg OR DBP by $\geq 10$ mmHg;
   Initiation of, or increase in dose of, medication for treatment of hypertension in any patient.

3. Adverse events consistent with orthostatic hypotension as confirmed by orthostatic vital signs.
4. Adverse events suggestive of cystitis or urinary tract infection.
5. Elevated AST or ALT lab value requiring that study drug be temporarily withheld or permanently discontinued.

*6.5 Statistical Analyses*

Efficacy, safety, and exploratory endpoints that were evaluated for within- and/or between-treatment differences are listed below.
In describing the efficacy variables of interest below, the descriptions were restricted to the primary time point of interest at Week 12 in the study. However, many variables were measured at additional time points, and were analyzed at other time points.
Co-Primary Efficacy Endpoints were:

1. Change from baseline (CFB) at Week 12 in average number of micturitions per 24 hours in all OAB patients;
2. CFB at Week 12 in average number of UUI episodes per 24 hours in OAB Wet patients.

For the purpose of this study, the number of micturitions was defined as the number of times a patient has voided in the toilet as indicated on the Patient Voiding Diary. Average daily micturitions were calculated using the daily entries in the Patient Voiding Diary, which was completed over the 7 days prior to each study visit. Average daily number of micturitions were calculated as the total number of micturitions that occured on a Complete Diary Day divided by the number of Complete Diary Days in the Patient Voiding Diary. A complete diary day required confirmation by the patient in the Patient Voiding Diary that all voids and leakages had been recorded for the diary day. Baseline was defined as the average number of micturitions occurring during the last evaluable diary prior to the Baseline Visit.

The number of UUI episodes was defined as the number of times a patient had checked "urge" as the reason for accidental urine leakage. Average daily UUI episodes at each study visit were calculated in the same manner as described above for the micturition endpoint. The UUI endpoint were analyzed using only OAB Wet patients.

Secondary Efficacy Endpoints were:

1. CFB at Week 12 in average number of urgency episodes (need to urinate immediately) over 24 hours in all OAB patients
2. Percent of all OAB patients with a 50% reduction from baseline in urgency episodes (need to urinate immediately) per 24 hours at Week 12
3. Percent of OAB Wet patients with a 75% reduction from baseline in UUI episodes per 24 hours at Week 12
4. CFB at Week 4 in average number of daily micturitions in all OAB patients
5. CFB at Week 4 in average number of daily UUI episodes in OAB Wet patients
6. CFB at Week 12 in Coping Score from the Overactive Bladder Questionnaire Long Form (OAB-q LF, 1-week recall) in all OAB patients
7. CFB to Week 2 in average number of micturitions per 24 hours in all OAB patients
8. CFB to Week 2 in average number of UUI episodes per 24 hours in OAB Wet patients
9. CFB at Week 12 in average number of total incontinence episodes over 24 hours in OAB Wet patients
10. CFB at Week 12 in average volume voided per micturition in all OAB patients

Additional Secondary Efficacy Endpoints were:

1. CFB at Week 12 in HRQL Total Score from the OAB-q LF (1-week recall) in all OAB patients
2. CFB at Week 12 in Symptom Bother Score from the OAB-q-LF (1-week recall) in all OAB patients
3. Percent of OAB Wet patients with zero UUI episodes at Week 12
4. Percent of all OAB patients with average number of micturitions < 8 per 24 hours at Week 12
5. Percent of OAB Wet patients with a 50% reduction from baseline in total incontinence episodes per 24 hours at Week 12
6. CFB at Week 12 in overall bladder symptoms based on PGI-Severity in all OAB patients
7. CFB at Week 12 in overall control over bladder symptoms based on PGI-Control in all OAB patients

Exploratory Endpoints were:

1. CFB in percent of dry Diary Days (zero UUI episodes) at Week 12 and Week 4 in OAB Wet patients
2. CFB in percent of dry Diary Days (zero total incontinence episodes) at Week 12 in OAB Wet patients
3. CFB at Week 12 and Week 4, in total score of the Work Productivity and Activity Impairment Questionnaire-Urinary Symptoms (WPAI-US) in all OAB patients
4. CFB at Week 12 in scores of the EQ-5D in all OAB patients
5. For all OAB patients with ≥ 1 NVU at baseline, CFB at Week 12 in the average number of NVU per 24 hours
6. CFB at Week 12 in overall symptom frequency based on PGI-Frequency in all OAB patients
7. CFB at Week 12 in overall urgency-related leakage over bladder symptoms based on PGI-Leakage in all OAB Wet patients
8. Overall change of bladder symptoms based on PGI-Change in all OAB patients at Week 12
9. CFB at Week 12 in Concern Score from the OAB-q LF (1-week recall) in all OAB patients
10. CFB at Week 12 in Sleep Score from the OAB-q LF (1-week recall) in all OAB patients
11. CFB at Week 12 in Social Interaction Score from the OAB-q LF (1-week recall) in all OAB patients
12. CFB at week 52 in average number of nighttime voids for all patients
13. CFB at week 52 in average number of nighttime voids for patients with nocturia at baseline

*6.6 Analysis Populations*

The Full Analysis Set (FAS) population served as the primary population for the analysis of efficacy data in this

study. Since the endpoints related to incontinence would only apply to patients who meet the definition of incontinence at study entry, it was necessary to have a separate FAS definition with an additional criterion to define the primary analysis population for incontinence endpoints.

The following FAS populations were defined in the study:

1. Full analysis set (FAS): all randomized OAB patients who took at least one dose of double-blind Study Treatment and have at least one evaluable change from baseline micturition measurement .
2. Full analysis set for incontinence (FAS-I): all randomized OAB Wet patients who took at least one dose of double-blind Study Treatment and have at least one evaluable change from baseline UUI measurement.

The Per-Protocol population (PP) and Per-Protocol population for incontinence (PP-I) would exclude patients due to important deviations from the protocol that may substantially affect the results of the primary efficacy endpoints. A supportive analysis using the Per-Protocol populations would performed for the co-primary and secondary efficacy endpoints.

Patients would be included in the treatment group to which they were randomized for the analysis of efficacy data using the FAS and Per-Protocol populations.

The Safety Set (SAF) were used for the analysis of safety data in this study. The SAF consisted of all patients who received at least one dose of Study Treatment.

The PK population included all subjects in the Safety Set who undergo plasma PK sampling and have evaluable PK assay results.

*6.7 Statistical Methods*

For the analysis of the co-primary endpoints (change from baseline in average number of daily micturitions at Week 12 and change from baseline in average number of daily urge urinary incontinence episodes at Week 12, and placebo adjustment of each), a mixed model for repeated measure (MMRM) with restricted maximum likelihood estimation was used. This model corrects for dropout and accounts for the fact that measurements taken on the same patient over time tend to be correlated by using all available information on patients within the same covariate set to derive an estimate of the treatment effect for a dropout-free population. The analysis model for each efficacy endpoint includes terms for treatment, visit, OAB Type (Wet vs Dry), Sex (Female vs Male), Region (US vs Rest of World), baseline score, and interaction of visit by treatment.

Primary inferences were drawn from treatment differences for the changes from baseline derived from the MMRM models at Week 12. As part of secondary objectives, the treatment differences for each post baseline visit were also derived using the same MMRM model. The estimated treatment difference for at each visit was displayed in the summary of statistical analysis together with the 95% confidence interval and the associated P-value.

An unstructured covariance matrix was used to model the correlation among repeated measurements. The Kenward-Roger adjustment was used with restricted (or residual) maximum likelihood (REML) to make statistical inference. If the unstructured covariance model fails to converge with the default Newton-Raphson algorithm, the Fisher scoring algorithm or other appropriate methods can be used to provide initial values of the covariance parameters. In the rare event that none of the above methods yield convergence, a structured covariance would be used to model the correlation among repeated measurements.

The change from baseline efficacy endpoints was analyzed using the same MMRM model described for co-primary endpoints.

Analysis of the efficacy endpoints of proportion of patients with at least 75% reduction in the average number of daily UUI episodes at Week 12 and proportion of patients with 50% reduction in the average number of daily urgency episodes at Week 12 was analyzed using the Cochran-Mantel-Haenszel risk difference estimate. Missing Week 12 data was analyzed using multiple imputation. The estimated difference in the proportion of responders and 95% confidence interval for the difference was calculated using the Cochran-Mantel-Haenszel risk difference estimate stratified by OAB Type (Wet vs Dry) and Sex (Female vs Male), with weights proposed by Greenland and Robins.

The same statistical methods that were used to analyze the co-primary and secondary efficacy endpoints were used for the exploratory analyses. Exploratory responder analyses were analyzed using the same Cochran-Mantel-Haenszel model as described above for secondary endpoints.

Safety analyses were conducted using the SAF and summarized by treatment group as treated. The treatment-emergent period is defined as the period of time from the first dose date of the double blinded Study Treatment through 28 days after the last dose of Study Treatment, or the date of initiation of another investigational agent or surgical intervention or rollover to the extension study, whichever occurs first. Safety was assessed through summaries of adverse events, the frequency of treatment discontinuations due to adverse events, and clinical laboratory evaluations.

*6.8 Subgroup Analyses and Effect of Baseline Factors*

To determine whether the treatment effect was consistent across various subgroups, the estimate of the between-group treatment effect (with a nominal 95% confidence interval [CI]) for the primary endpoint was estimated and plotted within each category of the following classification variables:

1. Region (US vs. Rest of World)
2. Age category (< 40, ≥ 40 to < 55, ≥ 55 to < 65, ≥ 65 to 75, ≥ 75 years)
3. Age category (< 65, ≥ 65 years)
4. Race (white vs. other)
5. Sex (female vs. male)
6. Prior OAB therapy (naive vs. non-naive)
7. OAB Type (OAB Wet vs. OAB Dry)

For each subgroup, the primary MMRM model was fit including a subgroup by treatment interaction term and model results were presented. The consistency of the treatment effect was assessed descriptively via summary statistics by category for the classification variables listed above.

*6.9 Clinical Trial Data and Results*

The demographics of the patients in this trial are shown in Table 13, and the subject disposition is shown in Table 14.

Table 13. Patient Demographics

| | **Placebo (N=520)** | **Vibegron (N=526)** | **Tolterodine (N=417)** |
|---|---|---|---|
| Mean Age, in years (SD) | 59.9 (13.33) | 60.8 (13.30) | 59.8 (13.19) |
| Subjects ≥ 65 yr, n (%)<br>Subjects ≥75 yr, n (%) | 220 (42.3)<br>57 (11.0) | 242 (46.0)<br>75 (14.3) | 166 (39.8)<br>47 (11.3) |
| Female, n (%)<br>Male, n (%) | 445 (85.6)<br>75 (14.4) | 449 (85.4)<br>77 (14.6) | 352 (84.4)<br>65 (15.6) |
| Race, n (%)<br>White | 406 (78.1%) | 422 (80.2%) | 317 (76.0%) |
| Black/African<br>American<br>Asian<br>American Indian or Alaska Native Other | 79 (15.2%)<br>29 (5.6%)<br>3 (0.6%)<br>3 (0.6%) | 74 (14.1%)<br>27 (5.1%)<br>1 (0.2)<br>2 (0.4%) | 69 (16.5%)<br>26 (6.2%)<br>0<br>5 (1.2%) |
| OAB Wet, n (%) (With Incontinence) | 405 (77.9) | 403 (76.6) | 319 (76.5) |
| OAB Dry, n (%) (Without Incontinence) | 115 (22.1) | 123 (23.4) | 98 (23.5) |
| Region, n (%) | US: 463 (89.0)<br>Non-US: 57 (11.0) | US: 472 (89.7)<br>Non-US: 54 (10.3) | US: 376 (90.2)<br>Non-US: 41 (9.8) |

Table 14. Subject Disposition.

| | **Placebo (N=540)** | **Vibegron (N=547)** | **Tolterodine (N=431)** |
|---|---|---|---|
| Randomized, n (%) | 540 (100.0) | 547 (100.0) | 431 (100.0) |
| Completed 12 Weeks of Treatment, n (%) | 486 (90.0) | 502 (91.8) | 385 (89.3) |
| Study Discontinuation, n (%) Due to AEs | 54 (10.0)<br>6 (1.1) | 45 (8.2)<br>8 (1.5) | 46 (10.7)<br>13 (3.0) |

Vibegron achieved co-primary endpoints demonstrating statistically significant reduction in daily micturitions and daily urge urinary incontinence (UUI), compared to placebo (p<0.001 and P<0.0001, respectively). The co-primary efficacy results are shown in Tables 15 and 16. Figures 4 and 5 show that vibegron achieved statistically significant onset of

action at two weeks for reduction in UUI and micturition, respectively, and the benefit was sustained through week 12.

Table 15. Change from Baseline in Average Daily Number of UUI Episodes (CFB Least Squares Means at Week 12)

| **Urge Urinary Incontinence** | **Placebo** | **Vibegron** | **Tolterodine** |
|---|---|---|---|
| Week 2 | | | |
| Baseline Daily Episodes, Mean (SD, n) | 3.49 (3.1, n=405) | 3.43 (2.9, n=403) | 3.42 (2.6, n=319) |
| Week 2, n Mean (SD) | 391<br>2.69 (2.931) | 390<br>2.03 (2.492) | 310<br>2.24 (2.296) |
| Change from Baseline, n LS Mean 95% CI | 391<br>-0.8<br>-1.0 to -0.5 | 390<br>-1.4<br>-1.6 to -1.2 | 310<br>-1.2<br>-1.5 to -0.9 |
| Active - Placebo LS Mean Difference (95% CI) P-Value | - | -0.6 (-0.9 to -0.4)<br><0.0001 | -0.4 (-0.7 to -0.2)<br><0.001 |
| Week 4 | | | |
| Baseline Daily Episodes, Mean (n) | 3.49 (n=405) | 3.43 (n=403) | 3.42 (n=319) |
| Week 4, n Mean (SD) | 364<br>2.50 (2.925) | 381<br>1.77 (2.494) | 292<br>2.06 (2.287) |
| Change from Baseline, n LS Mean 95% CI | 364<br>-1.0<br>-1.3 to -0.8 | 381<br>-1.7<br>-2.0 to -1.5 | 292<br>-1.4<br>-1.6 to -1.1 |
| Active - Placebo LS Mean Difference (95% CI) P-Value | ---- | -0.7<br>-0.9 to -0.5<br><0.0001 | -0.4<br>-0.6 to -0.1<br>0.0053 |
| Week 8 | | | |
| Baseline Daily Episodes, Mean (SD, n) | 3.49 (3.1, n=405) | 3.43 (2.9, n=403) | 3.42 (2.6, n=319) |
| Week 8, n Mean (SD) | 380<br>2.22 (2.584) | 389<br>1.65 (2.683) | 295<br>1.74 (2.267) |
| Change from Baseline, n LS Mean 95% CI | 380<br>-1.2<br>-1.5 to -1.0 | 389<br>-1.8<br>-2.1 to -1.6 | 295<br>-1.6<br>-1.9 to -1.4 |
| Active - Placebo LS Mean Difference (95% CI) P-Value | ---- | -0.6<br>-0.9 to -0.3<br><0.0001 | -0.4<br>-0.7 to -0.1<br>0.0033 |
| Week 12 | | | |
| Baseline Daily Episodes, Mean (SD, n) | 3.49 (3.1, n=405) | 3.43 (2.9, n=403) | 3.42 (2.6, n=319) |
| Week 12, n Mean (SD) | 372<br>2.01 (2.478) | 383<br>1.46 (2.446) | 286<br>1.55 (2.040) |
| Change from Baseline, n LS Mean 95% CI | 372<br>-1.4<br>-1.7 to -1.2 | 383<br>-2.0<br>-2.3 to -1.8 | 286<br>-1.8<br>-2.1 to -1.5 |
| Active - Placebo LS Mean Difference (95% CI) P-Value | ---- | -0.6 (-0.9 to -0.3)<br><0.0001 | -0.4 (-0.7 to -0.1)<br>0.0123 |

Table 16. Change from Baseline in Average Daily Number of Micturitions (CFB Least Squares Means at Week 12)

| Micturitions | Placebo | Vibegron | Tolterodine |
|---|---|---|---|
| Week 2 | | | |
| Baseline Daily Episodes, Mean (SD, n) | 11.75 (4.0, n=520) | 11.31 (3.4, n=526) | 11.48 (3.2, n=417) |
| Week 2, n Mean (SD) | 499<br>10.99 (3.930) | 508<br>10.18 (3.337) | 406<br>10.40 (3.216) |
| Change from baseline, n LS Mean 95% CI | 499<br>-0.3<br>-0.6 to 0.0 | 508<br>-0.8<br>-1.1 to -0.5 | 406<br>-0.6<br>-1.0 to -0.3 |
| Active - Placebo | ---- | -0.5 (-0.7 to -0.2)<br><0.001 | -0.3 (-0.6 to -0.1)<br>0.0113 |
| LS Mean Difference (95% CI) P-Value | | | |
| Week 4 | | | |
| Baseline Daily Episodes, Mean (SD, n) | 11.75 (4.0, n=520) | 11.31 (3.4, n=526) | 11.48 (3.2, n=417) |
| Week 4, n Mean (SD) | 469<br>10.77 (3.873) | 497<br>9.84 (3.282) | 380<br>10.10 (3.190) |
| Change from baseline, n LS Mean 95% CI | 469<br>-0.5<br>-0.8 to -0.2 | 497<br>-1.1<br>-1.4 to -0.8 | 380<br>-0.9<br>-1.3 to -0.6 |
| Active - Placebo LS Mean Difference (95% CI) P-Value | ---- | -0.6 (-0.8 to -0.3)<br><0.0001 | -0.4 (-0.7 to -0.2)<br>0.0015 |
| Week 8 | | | |
| Baseline Daily Episodes, Mean (SD, n) | 11.75 (4.0, n=520) | 11.31 (3.4, n=526) | 11.48 (3.2, n=417) |
| Week 8, n Mean (SD) | 486<br>10.32 (3.595) | 504<br>9.53 (3.342) | 390<br>9.80 (3.317) |
| Change from baseline, n LS Mean 95% CI | 486<br>-0.9<br>-1.2 to -0.5 | 504<br>-1.4<br>-1.8 to -1.1 | 390<br>-1.2<br>-1.6 to -0.9 |
| Active - Placebo LS Mean Difference (95% CI) P-Value | ---- | -0.6<br>-0.9 to -0.3<br><0.0001 | -0.4<br>-0.7 to -0.1<br>0.0141 |
| Week 12 | | | |
| Baseline Daily Episodes, Mean (SD, n) | 11.75 (4.0, n=520) | 11.31 (3.4, n=526) | 11.48 (3.2, n=417) |
| Week 12, n | 475 | 492 | 378 |

(continued)

| Week 12 | | | |
|---|---|---|---|
| Mean (SD) | 10.05 (3.336) | 9.32 (3.482) | 9.58 (3.298) |
| Change from baseline, n LS Mean 95% CI | 475<br>-1.3<br>-1.6 to -1.0 | 492<br>-1.8<br>-2.1 to -1.5 | 378<br>-1.6<br>-1.9 to -1.3 |
| Active - Placebo LS Mean Difference (95% CI) P-Value | ---- | -0.5 (-0.8 to -0.2)<br><0.001 | -0.3 (-0.6 to 0.1)<br>0.0988 |

Changes in daily urge urinary incontinence (UUI) within several subgroups are shown in Tables 17-21.

Table 17. UUI: Overall and by Subgroup (Week 12 LS Mean Change from Baseline (Placebo Adjusted))

| Subgroup | Vibegron LS Mean (95% CI) | n | Tolterodine LS Mean (95% CI) | n |
|---|---|---|---|---|
| Overall | -0.6 (-0.9 to -0.3) | 383 | -0.4 (-0.7 to -0.1) | 286 |
| Region: US | -0.5 (-0.8 to -0.2) | 330 | -0.3 (-0.7 to 0.0) | 253 |
| Region: Non-US | -1.1 (-1.8 to -0.3) | 53 | -0.6 (-1.5 to 0.2) | 33 |
| Age < 65 years | -0.5 (-0.9 to -0.1) | 200 | -0.2 (-0.6 to 0.2) | 170 |
| Age ≥ 65 years | -0.8 (-1.2 to -0.3) | 183 | -0.6 (-1.1 to -0.1) | 116 |
| Age ≥75 years[1] | -1.6 (-2.4 to -0.8) | 56 | -1.6 (-2.5 to -0.7) | 30 |
| White | -0.8 (-1.1 to -0.4) | 308 | -0.5 (-0.8 to -0.2) | 228 |
| Non-White | 0.0 (-0.6 to 0.7) | 75 | 0.1 (-0.6 to 0.7) | 58 |
| Females | -0.7 (-1.0 to -0.4) | 341 | -0.4 (-0.7 to -0.1) | 253 |
| Males | -0.1 (-0.9 to 0.8) | 42 | -0.5 (-1.4 to 0.4) | 33 |
| [1] Posthoc analysis | | | | |

Unexpectedly, females showed a greater decrease in UUI episodes than males.

Table 18. UUI: Overall and by Subgroup (Week 12 CFB LS Mean (95% CI))

| Subgroup | Placebo Mean (95% CI) | n | Vibegron Mean (95% CI) | n | Tolterodine Mean (95% CI) | n |
|---|---|---|---|---|---|---|
| Overall | -1.4 (-1.7 to -1.2) | 372 | -2.0 (-2.3 to -1.8) | 383 | -1.8 (-2.1 to -1.5) | 286 |
| Region: US | -1.4 (-1.6 to -1.2) | 321 | -1.9 (-2.2 to -1.7) | 330 | -1.7 (-2.0 to -1.5) | 253 |
| Region: Non-US | -1.4 (-1.9 to -0.8) | 51 | -2.4 (-3.0 to -1.9) | 53 | -2.0 (-2.7 to -1.3) | 33 |
| Age < 65 years | -1.5 (-1.9 to -1.2) | 214 | -2.0 (-2.4 to -1.7) | 200 | -1.8 (-2.1 to -1.4) | 170 |
| Age ≥ 65 years | -1.2 (-1.6 to -0.9) | 158 | -2.0 (-2.3 to -1.7) | 183 | -1.8 (-2.2 to -1.4) | 116 |
| Age ≥75 years[1] | -0.4 (-1.0 to 0.2) | 40 | -2.0 (-2.6 to -1.5) | 56 | -2.0 (2.7 to -1.3) | 30 |
| White | -1.4 (-1.7 to -1.1) | 303 | -2.2 (-2.4 to -1.9) | 308 | -1.9 (-2.2 to -1.6) | 228 |
| Non-White | -1.4 (-1.8 to -0.9) | 69 | -1.3 (-1.8 to -0.8) | 75 | -1.3 (-1.8 to -0.8) | 58 |
| Females | -1.4 (-1.6 to -1.2) | 334 | -2.1 (-2.3 to -1.8) | 341 | -1.8 (-2.0 to -1.5) | 253 |

(continued)

| Subgroup | Placebo Mean (95% CI) | n | Vibegron Mean (95% CI) | n | Tolterodine Mean (95% CI) | n |
|---|---|---|---|---|---|---|
| Males | -1.6 (-2.2 to -0.9) | 38 | -1.6 (-2.2 to -1.0) | 42 | -2.0 (-2.7 to -1.3) | 33 |
| For subgroup analysis the covariates in the model include region, study visit, sex, baseline number of UUI Episodes, Treatment by Sex, Treatment by Study Visit, and Treatment by Study Visit by Sex.<br>[1] Posthoc analysis | | | | | | |

Table 19. UUI: Overall and by Subgroup (Descriptive Statistics: Week 12 CFB Mean (Q1, Q3)

| Subgroup | Placebo Mean (Q1,Q3) | n | Vibegron Mean (Q1,Q3) | n | Tolterodine Mean (Q1,Q3) | n |
|---|---|---|---|---|---|---|
| Overall | -1.46 (-2.21 to -0.29) | 372 | -2.01 (-3.00 to -0.60) | 383 | -1.75 (-2.43 to -0.71) | 286 |
| Region: US | -1.41 (-2.14 to -0.29) | 321 | -1.92 (-3.00 to -0.50) | 330 | -1.69 (-2.43 to -0.70) | 253 |
| Region: Non-US | -1.74 (-2.83 to -0.14) | 51 | -2.60 (-3.17 to -1.00) | 53 | -2.26 (-2.81 to -0.88) | 33 |
| Age < 65 years | -1.64 (-2.43 to -0.43) | 214 | -2.08 (-3.06 to -0.71) | 200 | -1.72 (-2.43 to -0.72) | 170 |
| Age ≥ 65 years | -1.20 (-2.00 to -0.14) | 158 | -1.94 (-3.00 to -0.57) | 183 | -1.80 (-2.50 to -0.59) | 116 |
| White | -1.39 (-2.17 to -0.29) | 303 | -2.23 (-3.32 to -0.86) | 308 | -1.92 (-2.64 to -0.88) | 228 |
| Non-White | -1.75 (-2.27 to -0.16) | 69 | -1.14 (-1.71 to 0.14) | 75 | -1.11 (-2.14 to 0.14) | 58 |
| Females | -1.44 (-2.25 to -029) | 334 | -2.10 (-3.00 to -0.71) | 341 | -1.71 (-2.43 to -0.61) | 253 |
| Males | -1.14 (-2.09 to -0.43) | 38 | -1.35 (-1.86 to -0.17) | 42 | -2.08 (-2.43 to -1.00) | 33 |

Table 20. UUI: White vs. Non-White (Descriptive Statistics)

| Subgroup | Visit | Placebo | n | Vibegron | n | Tolterodine | n |
|---|---|---|---|---|---|---|---|
| Overall | Baseline Mean (SD) | 3.49 (3.053) | 405 | 3.43 (2.894) | 403 | 3.42 (2.592) | 319 |
| | Week 12 CFB Mean (SD) | -1.46 (2.379) | 372 | -2.01 (2.495) | 383 | -1.75 (2.304) | 286 |
| | | | | | | | |
| White | Baseline Mean (SD)<br>Median (Q1, Q3)<br>(Min, Max) | 3.36 (2.713)<br>2.43<br>(1.57 to 4.29)<br>(0.1 to 17.4) | 325 | 3.59 (2.972)<br>2.82<br>(1.60 to 4.57)<br>(0.1 to 27.9) | 322 | 3.46 (2.609<br>2.50<br>(1.71 to 4.57)<br>(0.3 to 17.0 | 254 |
| | Week 12 CFB Mean (SD)<br>Median (Q1, Q3)<br>(Min, Max) | -1.39 (2.066)<br>-1.17<br>(-2.17 to - 0.29)<br>(-14.0 to 6.4) | 303 | -2.23 (2.473<br>-1.78<br>(-3.32 to - 0.86)<br>(-14.0 to 5.1) | 308 | -1.92 (2.358)<br>-1.57<br>(-2.64 to - 0.88)<br>(-11.4 to 8.9) | 228 |
| | | | | | | | |
| Non-White | Baseline Mean (SD)<br>Median (Q1, Q3)<br>(Min, Max) | 4.01 (4.141)<br>2.57 (1.69 to 4.86)<br>(0.0 to 23.7) | 80 | 2.81 (2.482)<br>2.00 (1.33, 3.43)<br>(0.0 to 16.1) | 81 | 3.28 (2.539)<br>2.29 (1.57 to 4.00)<br>(0.0, 11.6) | 65 |

(continued)

| Subgroup | Visit | Placebo | n | Vibegron | n | Tolterodine | n |
|---|---|---|---|---|---|---|---|
| | Week 12 CFB Mean (SD) | -1.75 (3.438) | | -1.14 (2.411 ) | | -1.11 (1.968) | |
| | Median (Q1, Q3) | -1.14 (-2.27 to -0.16) | 69 | -1.00 (-1.71 to 0.14) | 75 | -1.00 (-2.14 to 0.14) | 58 |
| | (Min, Max) | (-19.0 to 3.4) | | (-13.1 to 4.7) | | (-7.1 to 4.0) | |

Table 21. UUI: Further Breakdown of Race (Descriptive Statistics)

| Subgroup | Visit | Placebo | n | Vibegron | n | Tolterodine | n |
|---|---|---|---|---|---|---|---|
| White | Baseline Mean (SD) | 3.36 (2.713) | 325 | 3.59 (2.972) | 322 | 3.46 (2.609) | 254 |
| | Median (Q1, Q3) | 2.43 (1.57 to 4.29) | | 2.82 (1.60 to 4.57) | | 2.50 (1.71 to 4.57) | |
| | (Min, Max) | (0.1 to 17.4) | | (0.1 to 27.9) | | (0.3 to 17.0) | |
| | Week 12 CFB Mean (SD) | -1.39 (2.066) | 303 | -2.23 (2.473) | 308 | -1.92 (2.358) | 228 |
| | Median (Q1, Q3) | -1.17 (-2.17 to -0.29) | | -1.78 (-3.32 to -0.86) | | -1.57 (-2.64 to -0.88) | |
| | (Min, Max) | (-14.0 to 6.4) | | (-14.0 to 5.1) | | (-11.4 to 8.9) | |
| African American | Baseline Mean (SD) | 4.46 (4.507) | 59 | 3.40 (2.707) | 56 | 3.61 (2.506) | 48 |
| | Median (Q1, Q3) | 2.75 (1.67 to 5.88) | | 2.71 (1.59 to 3.95) | | 3.05 (1.77 to 4.88) | |
| | (Min, Max) | (0.0 to 23.7) | | (0.7 to 16.1) | | (0.0 to 11.6) | |
| | Week 12 CFB Mean(SD) | -2.42 (3.772) | 48 | -1.61 (2.680) | 52 | -1.42 (2.141) | 43 |
| | Median (Q1, Q3) | -1.48 (-3.00 to -0.57) | | -1.26 (-1.84 to -0.64) | | -1.43 (-2.32 to 0.14) | |
| | (Min, Max) | (-19.0 to 2.5) | | (-13.1 to 4.7) | | (-7.1 to 4.0) | |
| Asian | Baseline Mean (SD) | 2.00 (0.911) | 17 | 1.43 (1.041) | 24 | 2.12 (2.139) | 14 |
| | Median (Q1, Q3) | 2.14 (2.00 to 2.57) | | 1.17 (0.57 to 2.00) | | 1.86 (1.29 to 2.00) | |
| | (Min, Max) | (0.1 to 3.1) | | (0.0 to 4.0) | | (0.0 to 9.1) | |
| | Week 12 CFB Mean | -0.28 (1.567) | 17 | -0.07 (1.081) | 23 | -0.21 (1.015) | 13 |
| | (SD) Median (Q1, Q3) | -0.43 (-1.14 to, 0.86) | | 0.00 (-0.86 to 0.60) | | -0.14 (-0.31 to 0.14) | |
| | (Min, Max) | (-2.9 to 3.4) | | (-2.0 to 2.1) | | (-2.3 to 1.9) | |

Table 22. UUI: Females vs. Males (Descriptive Statistics)

| Subgroup | Visit | Placebo | n | Vibegron | n | Tolterodine | n |
|---|---|---|---|---|---|---|---|
| Overall | Baseline Mean | 3.49 | 405 | 3.43 | 403 | 3.42 | 319 |
| | Week 12 CFB Mean (Q1, Q3) | -1.46 (-2.21 to 0.29) | 372 | -2.01 (-3.00 to -0.60) | 383 | -1.75 (-2.43 to -0.71) | 286 |
| | | | | | | | |
| Females | Baseline Mean (Q1, Q3) | 3.50 (1.57 to 4.50) | 364 | 3.50 (1.67 to 4.43) | 361 | 3.41 (1.71 to 4.49) | 284 |

(continued)

| Subgroup | Visit | Placebo | n | Vibegron | n | Tolterodine | n |
|---|---|---|---|---|---|---|---|
| | Week 12 CFB Mean (Q1, Q3) | -1.44 (-2.25 to -029) | 334 | -2.10 (-3.00 to -0.71) | 341 | -1.71 (-2.43 to -0.61) | 253 |
| | | | | | | | |
| Male | Baseline Mean (Q1, Q3) | 3.38 (1.29 to, 4.38) | 41 | 2.81 (1.14 to 3.71) | 42 | 3.49 (1.86 to 5.29) | 35 |
| | Week 12 CFB Mean (Q1, Q3) | -1.57 (-2.09 to -0.43) | 38 | -1.35 (-1.86 to -0.17) | 42 | -2.08 (-2.43 to -1.00) | 33 |

Changes in daily urge urinary incontinence (UUI) within subjects with/without BPH, subjects with/without prior (ACH) use, and subjects with/without prior beta-3 (B3) agonist use are shown in Table 23. Subjects who had used either ACH or a B3 agonist within the past 12 months had a greater reduction in UUI after taking vibegron than those taking a placebo.

Table 23. UUI: Overall and by Subgroup (Descriptive Statistics: Week 12 CFB Mean (Q1, Q3))

| Subgroup | Placebo Mean (Q1,Q3) | n | Vibegron Mean (Q1,Q3) | n | Tolterodine Mean (Q1,Q3) | n |
|---|---|---|---|---|---|---|
| Overall | -1.46 (-0.21 to - 0.29) | 372 | -2.01 (-3.00 to -0.60) | 383 | -1.75 (-2.43 to -0.71) | 286 |
| Males with BPH | -1.05 (-1.29 to -0.71) | 8 | -1.09 (-1.57 to -0.08) | 16 | -2.15 (-2.71 to -1.36) | 9 |
| Males without BPH | -1.71 (-2.71 to -0.43) | 30 | -1.51 (-2.25 to -0.33) | 26 | -2.05 (-2.29 to -0.93) | 24 |
| Prior ACH Use in last 12 Mo. | -0.83 (-1.77 to -0.08) | 68 | -1.46 (-2.62 to -0.50) | 64 | -0.98 (-1.86 to 0.14) | 39 |
| No Prior ACH Use in last 12 Mo. | -1.60 (-2.29 to -0.41) | 304 | -2.12 (-3.13 to -0.73) | 319 | -1.88 (-2.71 to -0.80) | 247 |
| Prior B3 Agonist Use in last 12 Mo. | -0.31 (-1.00 to 0.86) | 21 | -1.64 (-3.43 to, 0.00) | 14 | -0.65 (-1.43 to 0.31) | 18 |
| No Prior B3 Agonist Use in last 12 Mo. | -1.52 (-2.27 to -0.33) | 351 | -2.03 (-3.00 to -0.71) | 369 | -1.83 (-2.57 to -0.74) | 268 |

As shown in Table 23, the average number of UUI episodes in a 24-hour period for subjects with prior ACH use is decreased about 1.75 times the decrease of that of subjects treated with placebo, while the average number of UUI episodes in a 24-hour period for subjects with prior B3 use is between about 5 and about 6 times the decrease of that of subjects treated with placebo.

Table 24 and Figure 19 show the overall time to first occurrence of 75% reduction in UUI epidoses. Table 25 shows estimates at weeks 2, 4, 8, and 12 of the time to first occurrence of 75% reduction in UUI episodes and Table 26 shows 75% UUI responder analysis through week 12 categorized by baseline UUI severity.

Table 24. Time to First Occurrence of 75% Reduction in UUI Episodes (Overall)

| Statistic | Placebo (N=405) | Vibegron (N=403) | Tolterodine (N=319) |
|---|---|---|---|
| Overall Time to First 75% Decrease in UUI Episodes (weeks) | | | |
| n | 405 | 403 | 319 |
| Events/Censored | 179/226 | 239/164 | 166/153 |
| Median (95% CI) | 12.7 (12.1 to NE) | 8.4 (8.1 to 12.1) | 12.1 (8.6 to 12.3) |
| 25th Percentile, 75th Percentile | 4.1, 14.3 | 3.9, 13.1 | 4.1, 14.2 |

(continued)

| Statistic | Placebo (N=405) | Vibegron (N=403) | Tolterodine (N=319) |
|---|---|---|---|
| Overall Time to First 75% Decrease in UUI Episodes (weeks) | | | |
| Min, Max | 2.0, 16.4+ | 2.0, 15.6+ | 2.0, 16.0 |
| Observed Min, Max | 2.0, 14.3 | 2.0, 14.1 | 2.0, 16.0 |
| | | | |
| Vibegron/Tolterodine Compared to Placebo Using Proportional Hazards [1] | | | |
| Hazard Ratio | | 1.470 | 1.276 |
| 95% CI | | 1.211 to 1.784 | 1.033 to 1.577 |
| P-Value | | <0.0001 | 0.0238 |
| | | | |
| Vibegron/Tolterodine Compared to Placebo Using Stratified Log-Rank [2] | | | |
| P-Value | | <0.0001 | <0.0095 |
| Note: NE= Not Estimable; Subjects are censored at last follow up visit.<br>[1] Cox model includes sex as a covariate. Hazard Ratio Estimates> 1 indicate that more treated subjects attain a 75% reduction than placebo.<br>[2] Stratified Log-Rank includes sex as a covariate. | | | |

Table 25. Time to First Occurrence of 75% Reduction in UUI Episodes (Estimates at Weeks 2, 4, 8, and 12)

| Statistic | Placebo (N=405) | Vibegron (N=403) | Tolterodine (N=319) |
|---|---|---|---|
| Week 2 | | | |
| Events/Censored | 66/339 | 98/305 | 60/259 |
| Estimated Incidence of Subjects with 75% Decrease from Baseline | 16.3 | 24.3 | 18.8 |
| 95% CI | 13.0 to 20.3 | 20.4 to 28.8 | 14.9 to 23.5 |
| Week 4 | | | |
| Events/Censored | 74/331 | 107/296 | 66/253 |
| Estimated Incidence of Subjects with 75% Decrease from Baseline | 18.3 | 26.6 | 20.7 |
| 95% CI | 14.9 to 22.5 | 22.5 to 31.1 | 16.7 to 25.6 |
| Week 8 | | | |
| Events/Censored | 112/293 | 166/237 | 107/212 |
| Estimated Incidence of Subjects with 75% Decrease from Baseline | 28.0 | 41.3 | 34.3 |
| 95% CI | 23.9 to 32.7 | 36.7 to 46.3 | 29.3 to 39.9 |
| Week 12 | | | |
| Events/Censored | 165/240 | 210/193 | 140/179 |
| Estimated Incidence of Subjects with 75% Decrease from Baseline | 42.2 | 52.7 | 45.7 |
| 95% CI | 37.5 to 47.3 | 47.9 to 57.7 | 40.3 to 51.5 |

Table 26. 75% UUI Responder Analysis by Baseline UUI Severity (Through Week 12)

| Statistic | Placebo | Vibegron | Tolterodine |
|---|---|---|---|
| | (N=239) | (N=223) | (N=187) |
| Baseline Number of UUI <3 | | | |
| Patients with at Least 75% Reduction in UUI from Baseline to Week 12 | | | |
| Unadjusted n (%) | 104 (43.5) | 112 (50.2) | 98 (52.4) |
| Adjusted n (%) | 92 (38.4) | 101 (45.5) | 91 (48.4) |
| Active - Placebo [1]<br>CMH Difference (95% CI)<br>P-Value | - | 7.1<br>-2.2 to 16.4<br>0.1359 | 10.1<br>0.4 to 19.7<br>0.0411 |
| | (N=166) | (N=180) | (N=132) |
| Baseline Number of UUI ≧ 3 | | | |
| Patients with at Least 75% Reduction in UUI from Baseline to Week 12 | | | |
| Unadjusted n (%) | 45 (27.1) | 99 (55.0) | 54 (40.9) |
| Adjusted n (%) | 42 (25.0) | 98 (54.4) | 44 (33.7) |
| Active - Placebo [1]<br>CMH Difference (95% CI)<br>P-Value | - | 29.4<br>19.6 to 39.2<br><0.0001 | 8.4<br>-2.5 to 19.3<br>0.1307 |

[1] The difference in proportion and corresponding CI and P-value was calculated using the Cochran-Mantel-Haenszel risk difference estimate stratified by Sex (Female vs Male), with weights proposed by Greenland and Robins. - MI has been used to impute values missing for any reason for the weeks analyzed. - Presented frequencies and the denominator used for percentages are based on patients in the FAS-I, baseline UUI category, and randomized treatment.

Table 27 shows the overall time to first occurrence of 100% reduction in UUI. Table 28 shows the estimated time at weeks 2, 4, 8, and 12 to first occurrence of 100% reduction in UUI episodes and Table 29 shows the week 12 100% UUI responder analysis categorized by baseline UUI severity.

Table 27. Time to First Occurrence of 100% Reduction in UUI Episodes (Overall)

| Statistic | Placebo (N=405) | Vibegron (N=403) | Tolterodine (N=319) |
|---|---|---|---|
| Overall Time to First 100% Decrease in UUI Episodes (weeks) | | | |
| n | 405 | 403 | 319 |
| Events/Censored | 108/297 | 146/257 | 93/226 |
| Median (95% CI) | 14.3 (14.3 to NE) | 13.3 (12.9 to NE) | 13.1 (12.7 to 16.0) |
| $25^{th}$ Percentile, $75^{th}$ Percentile | 12.1, NE | 8.1, NE | 12.1, 16.0 |
| Min, Max | 2.0, 16.4+ | 2.0, 15.6+ | 2.0, 16.0 |
| Observed Min, Max | 2.0, 14.3 | 2.0, 14.1 | 2.0, 16.0 |
| | | | |
| Vibegron/Tolterodine Compared to Placebo Using Proportional Hazards [1] | | | |
| Hazard Ratio | | 1.392 | 1.141 |

(continued)

| Vibegron/Tolterodine Compared to Placebo Using Proportional Hazards [1] | | | |
|---|---|---|---|
| 95% CI | | 1.085 to 1.785 | 0.864 to 1.505 |
| P-Value | | 0.0093 | 0.3521 |
| | | | |
| Vibegron/Tolterodine Compared to Placebo Using Stratified Log-Rank [2] | | | |
| P-Value | | 0.0081 | 0.2531 |

Note: NE= Not Estimable; Subjects are censored at last follow up visit.
[1] Cox model includes sex as a covariate. Hazard Ratio Estimates> 1 indicate that more treated subjects attain a 100% reduction than placebo.
[2] Stratified Log-Rank includes sex as a covariate.

Table 28. Time to First Occurrence of 100% Reduction in UUI Episodes (Estimates at Weeks 2, 4, 8, and 12)

| **Statistic** | **Placebo (N=405)** | **Vibegron (N=403)** | **Tolterodine (N=319)** |
|---|---|---|---|
| Week 2 | | | |
| Events/Censored | 25/380 | 30/373 | 18/301 |
| Estimated Incidence of Subjects with 100% Decrease from Baseline | 6.2 | 7.4 | 5.6 |
| 95% CI | 4.2 to 9.0 | 5.3 to 10.5 | 3.6 to 8.8 |
| Week 4 | | | |
| Events/Censored | 27/378 | 37/366 | 24/295 |
| Estimated Incidence of Subjects with 100% Decrease from Baseline | 6.7 | 9.2 | 7.6 |
| 95% CI | 4.6 to 9.6 | 6.7 to 12.4 | 5.1 to 11.1 |
| Week 8 | | | |
| Events/Censored | 56/349 | 86/317 | 49/270 |
| Estimated Incidence of Subjects with 100% Decrease from Baseline | 14.1 | 21.4 | 15.8 |
| 95% CI | 11.0 to 17.9 | 17.7 to 25.8 | 12.2 to 20.3 |
| Week 12 | | | |
| Events/Censored | 92/313 | 119/284 | 74/245 |
| Estimated Incidence of Subjects with 100% Decrease from Baseline | 23.7 | 30.0 | 24.4 |
| 95% CI | 19.8 to 28.3 | 25.7 to 34.8 | 20.0 to 29.7 |

Table 29. 100% UUI Responder Analysis by Baseline UUI Severity (Through Week 12)

| **Statistic** | **Placebo** | **Vibegron** | **Tolterodine** |
|---|---|---|---|
| | (N=239) | (N=223) | (N=187) |
| Baseline Number of UUI <3 | | | |
| Patients with 100% Reduction in UUI from Baseline to Week 12 | | | |
| Unadjusted n (%) | 78 (32.6) | 79 (35.4) | 62 (33.2) |
| Adjusted n (%) | 66 (27.5) | 68 (30.4) | 54 (29.0) |

(continued)

| | | | |
|---|---|---|---|
| | (N=239) | (N=223) | (N=187) |
| Active - Placebo [1] CMH Difference (95% CI) P-Value | ---- | 2.8<br>-5.7 to 11.3<br>0.5215 | 1.5<br>-7.3 to 10.2<br>0.7443 |
| | (N=166) | (N=180) | (N=132) |
| Baseline Number of UUI ≧ 3 | | | |
| Patients with 100% Reduction in UUI from Baseline to Week 12 | | | |
| Unadjusted n (%) | 13 (7.8) | 37 (20.6) | 23 (17.4) |
| Adjusted n (%) | 11 (6.9) | 34 (19.1) | 13 (9.6) |
| Active - Placebo [1] CMH Difference (95% CI) P-Value | ---- | 12.3<br>5.3 to 19.2<br>0.0005 | 2.6<br>-4.0 to 9.2<br>0.4448 |
| [1] The difference in proportion and corresponding CI and P-value was calculated using the Cochran-Mantel-Haenszel risk difference estimate stratified by Sex (Female vs Male), with weights proposed by Greenland and Robins. - MI has been used to impute values missing for any reason for the weeks analyzed. - Presented frequencies and the denominator used for percentages are based on patients in the FAS-I, baseline UUI category, and randomized treatment. | | | |

Table 30 and Figure 20 show the overall time to first occurrence of 50% reduction in urgency episodes. Table 31 shows the week 2, 4, 8, and 12 estimates of time to first occurrence of 50% reduction in urgency episodes.

Table 30. Time to First Occurrence of 50% Reduction in Urgency Episodes (Overall)

| **Statistic** | | **Placebo (N=520)** | **Vibegron (N=526)** | **Tolterodine (N=417)** |
|---|---|---|---|---|
| Overall Time to First 50% Decrease in Urgency Episodes (weeks) | | | | |
| | n | 520 | 526 | 417 |
| | Events/Censored | 200/320 | 256/270 | 187/230 |
| | Median (95% CI) | 13.1 (12.7 to NE) | 12.3 (12.1 to 12.6) | 12.6 (12.3 to 12.9) |
| | 25th Percentile, 50th Percentile | 8.1, 13.1 | 4.1, 12.3 | 4.3, 12.6 |
| | Min, Max | 2.0, 16.4+ | 2.0, 15.6+ | 2.0, 16.0+ |
| | Observed Min, Max | 2.0, 13.1 | 2.0, 15.1 | 2.0, 15.1 |
| | | | | |
| Vibegron/Tolterodine Compared to Placebo Using Proportional Hazards [1] | | | | |
| Hazard Ratio | | 1.333 | 1.225 | |
| 95% CI | | 1.108 to 1.604 | 1.004 to 1.496 | |
| P-Value | | 0.0023 | 0.0457 | |
| | | | | |
| Vibegron/Tolterodine Compared to Placebo Using Stratified Log-Rank [2] | | | | |

(continued)

| Statistic | | Placebo (N=520) | Vibegron (N=526) | Tolterodine (N=417) |
|---|---|---|---|---|
| P-Value | | 0.0015 | 0.0276 | |
| Note: NE= Not Estimable; Subjects are censored at last follow up visit.<br>[1] Cox model includes sex as a covariate. Hazard Ratio Estimates> 1 indicate that more treated subjects attain a 50% reduction than placebo.<br>[2] Stratified Log-Rank includes sex as a covariate. | | | | |

Table 31. Time to First Occurrence of 50% Reduction in Urgency Episodes (Estimates at Weeks 2, 4, 8, and 12)

| Statistic | Placebo (N=520) | Vibegron (N=526) | Tolterodine (N=417) |
|---|---|---|---|
| Week 2 | | | |
| Events/Censored | 75/445 | 103/423 | 75/342 |
| Estimated Incidence of Subjects with 50% Decrease from Baseline | 14.4 | 19.6 | 18.0 |
| 95% CI | 11.7 to 17.7 | 16.4 to 23.2 | 14.6 to 22.0 |
| Week 4 | | | |
| Events/Censored | 83/437 | 111/415 | 81/336 |
| Estimated Incidence of Subjects with 50% Decrease from Baseline | 16.0 | 21.1 | 19.4 |
| 95% CI | 13.1 to 19.4 | 17.9 to 24.8 | 16.0 to 23.6 |
| Week 8 | | | |
| Events/Censored | 123/397 | 166/360 | 127/290 |
| Estimated Incidence of Subjects with 50% Decrease from Baseline | 23.9 | 31.8 | 30.9 |
| 95% CI | 20.5 to 27.9 | 27.9 to 35.9 | 26.7 to 35.6 |
| Week 12 | | | |
| Events/Censored | 177/343 | 225/301 | 164/253 |
| Estimated Incidence of Subjects with 50% Decrease from Baseline | 35.3 | 43.8 | 40.5 |
| 95% CI | 31.3 to 39.7 | 39.6 to 48.2 | 35.9 to 45.5 |
| Note: NE= Not Estimable; Subjects are censored at last follow up visit. | | | |

Changes in daily micturitions within several subgroups are shown in Tables 32-38. Subjects at or over the age of 65 who were given vibegron showed a statistically significant decrease in micturitions relative to subjects in the same age group who were given tolterodine.

Table 32. Micturitions: Overall and by Subgroup

| Subgroup | Vibegron LS Mean (95% CI) | n | Tolterodine LS Mean (95% CI) | n |
|---|---|---|---|---|
| Overall | -0.5 (-0.8 to -0.2) | 492 | -0.3 (-0.6 to 0.1) | 378 |
| Region: US | -0.5 (-0.8 to -0.2) | 439 | -0.3 (-0.7 to 0.0) | 343 |
| Region: Non-US | -0.4 (-1.4 to 0.5) | 53 | 0.4 (-0.6 to 1.4) | 35 |
| Age < 65 years[1] | -0.2 (-0.6 to 0.2) | 264 | -0.2 (-0.6 to 0.2) | 227 |
| Age ≥ 65 years | -0.9 (-1.3 to -0.4) | 228 | -0.3 (-0.8 to 0.2) | 151 |
| Age ≥75 years[2] | -1.0 (-1.8 to -0.1) | 69 | -0.4 (-1.4 to 0.6) | 41 |
| White | -0.5 (-0.8 to -0.2) | 396 | -0.3 (-0.7 to 0.0) | 288 |
| Non-White | -0.5 (-1.2 to 0.1) | 96 | -0.1 (-0.8 to 0.6) | 90 |

(continued)

| Subgroup | Vibegron LS Mean (95% CI) | n | Tolterodine LS Mean (95% CI) | n |
|---|---|---|---|---|
| Females | -0.5 (-0.8 to -0.2) | 417 | -0.3 (-0.7 to 0.0) | 318 |
| Males | -0.6 (-1.4 to 0.1) | 75 | 0.1 (-0.7 to 0.9) | 60 |
| OAB Type: Wet | -0.5 (-0.8 to -0.1) | 383 | -0.3 (-0.6 to 0.1) | 286 |
| OAB Type: Dry | -0.7 (-1.4 to -0.1) | 109 | -0.3 (-1.0 to 0.3) | 92 |

[1] Age category by treatment interaction term is statistically significant. No other covariates are statistically significant as a main effect or subgroup by treatment interaction. Note: The n represents the number of subjects at week 12 for each treatment group.
[2] Posthoc analysis

Unexpectedly, subjects ≥65 years of age had a greater reduction in micturitions than subjects <65.

Table 33. Micturitions: Overall and by Subgroup (Week 12 CFB LS Mean (95% CI))

| Subgroup | Placebo Mean (95% CI) | n | Vibegron Mean (95% CI) | n | Tolterodine Mean (95% Ci) | n |
|---|---|---|---|---|---|---|
| Overall | -1.3 (-1.6 to -1.0) | 475 | -1.8 (-2.1 to -1.5) | 492 | -1.6 (-1.9 to -1.3) | 378 |
| Region: US | -1.4 (-1.6 to -1.1) | 423 | -1.9 (-2.1 to -1.7) | 439 | -1.7 (-2.0 to -1.4) | 343 |
| Region: Non-US | -1.4 (-2.1 to -0.7) | 52 | -1.8 (-2.5 to -1.2) | 53 | -1.0 (-1.8 to -0.3) | 35 |
| Age < 65 years | -1.5 (-1.9 to -1.2) | 271 | -1.7 (-2.1 to -1.4) | 264 | -1.8 (-2.1 to -1.4) | 227 |
| Age ≥ 65 years | -1.0 (-1.4 to -0.7) | 204 | -1.9 (-2.3 to -1.6) | 228 | -1.3 (-1.7 to -0.9) | 151 |
| Age ≥75 years[1] | -1.2 (-1.8 to -0.5) | 52 | -2.1 (-2.7 to -1.6) | 69 | -1.5 (-2.3 to -0.8) | 412 |
| White | -1.3 (-1.6, to -1.0) | 375 | -1.8 (-2.1 to -1.5) | 396 | -1.6 (-2.0 to -1.3) | 288 |
| Non-White | -1.2 (-1.7 to -0.7) | 100 | -1.7 (-2.2 to -1.2) | 96 | -1.3 (-1.8 to -0.8) | 90 |
| Females | -1.4 (-1.7 to -1.1) | 406 | -1.9 (-2.2 to -1.6) | 417 | -1.7 (-2.0 to -1.5) | 318 |
| Males | -1.1 (-1.7 to -0.5) | 69 | -1.7 (-2.3 to -1.2) | 75 | -1.0 (-1.6 to -0.4) | 60 |

[1] Posthoc analysis

Table 34. Micturitions: Overall and by Subgroup (Descriptive Statistics: Week 12 CFB Mean (Q1, Q3)

| Subgroup | Placebo Mean (Q1,Q3) | n | Vibegron Mean (Q1,Q3) | n | Tolterodine Mean (Q1,Q3) | n |
|---|---|---|---|---|---|---|
| Overall | -1.62 | 475 | -2.04 | 492 | -1.78 | 378 |
| Region: US | -1.61 (-2.89 to 0.14) | 423 | -2.01 (-343 to -0.57) | 439 | -1.83 (-3.29 to -0.29) | 343 |
| Region: Non-US | -1.68 (-2.79 to -0.14) | 52 | -2.26 (-3.48 to -0.73) | 53 | -1.38 (-2.71 to -0.05) | 35 |
| White | -1.62 (-2.86 to 0.14) | 375 | -2.01 (-3.43 to -0.61) | 396 | -1.90 (-3.29 to -0.43) | 288 |
| Non-White | -1.61 (-3.07 to 0.36) | 100 | -2.18 (-4.00 to -0.14) | 96 | -1.42 (-2.86 to 0.29) | 90 |
| Females | -1.65 (-3.04 to 0.13) | 406 | -2.05 (-3.45 to -0.57) | 417 | -1.88 (-3.16 to -0.37) | 318 |
| Males | -1.40 (-2.29 to 0.29) | | -1.97 (-3.43 to -0.38) | 75 | -1.28 (-2.99 to 0.29) | 60 |

Table 35. Micturitions: <65 yrs vs ≥ 65 yrs (Descriptive Statistics)

| Subgroup | Visit | Placebo | n | Vibegron | n | Tolterodin e | n |
|---|---|---|---|---|---|---|---|
| Overall | Baseline Mean | 11.75 | 520 | 11.31 | 526 | 11.48 | 417 |
| | Week 12 CFB Mean (Q1, Q3) | -1.62 | 475 | -2.04 | 492 | -1.78 | 378 |

(continued)

| Subgroup | Visit | Placebo | n | Vibegron | n | Tolterodin e | n |
|---|---|---|---|---|---|---|---|
| | | | | | | | |
| < 65 yrs | Baseline Mean (Q1, Q3) | 11.93 (9.06 to 13.38) | 300 | 11.51 (9.00 to 12.86) | 284 | 11.63 (9.29 to 13.17) | 251 |
| | Week 12 CFB Mean (Q1, Q3) | -1.87 (-3.43 to 0.00) | 271 | -1.99 (-3.46 to - 0.57) | 264 | -1.99 (-3.71 to - 0.57) | 227 |
| | | | | | | | |
| ≥ 65 yrs | Baseline Mean (Q1, Q3) | 11.50 (9.29 to 12.64) | 220 | 11.09 (9.29 to 12.29) | 242 | 11.25 (8.88 to 12.43) | 166 |
| | Week 12 CFB Mean (Q1, Q3) | -1.27 (-2.45 to 0.43) | 204 | -2.10 (-3.43 to - 0.71) | 228 | -1.48 (-2.75 to - 0.14) | 151 |

Table 36. Micturitions: Females vs Males (Descriptive Statistics)

| Subgroup | Visit | Placebo | n | Vibegron | n | Tolterodine | n |
|---|---|---|---|---|---|---|---|
| Overall | Baseline Mean | 11.75 | 520 | 11.31 | 526 | 11.48 | 417 |
| | Week 12 CFB Mean (Q1, Q3) | -1.62 | 475 | -2.04 | 492 | -1.78 | 378 |
| | | | | | | | |
| Females | Baseline Mean (Q1, Q3) | 11.71 (9.00 to 13.1 | 445 | 11.23 (9.00 to 12.57) | 449 | 11.36 (9.00 to 12.71) | 352 |
| | Week 12 CFB Mean (Q1, Q3) | -1.65 (-3.04 to 0.13) | 406 | -2.05 (-3.45 to - 0.57) | 417 | -1.88 (-3.16 to - 0.37) | 318 |
| | | | | | | | |
| Male | Baseline Mean (Q1, Q3) | 11.95 (9.71 to 13.1 14) | 75 | 11.79 (9.14 to 12.86) | 77 | 12.12 (9.14 to 13.43) | 65 |
| | Week 12 CFB Mean (Q1, Q3) | -1.40 (-2.29 to 0.29) | 69 | -1.97 (-3.43 to - 0.38) | 75 | -1.28 (-2.99 to 0.29) | 60 |

Table 37. Micturitions: Overall and by Subgroup (Descriptive Statistics: Week 12 CFB Mean (Q1, Q3)

| Subgroup | Placebo Mean (Q1,Q3) | n | Vibegron Mean (Q1,Q3) | n | Tolterodine Mean (Q1,Q3) | n |
|---|---|---|---|---|---|---|
| Overall | -1.62 (-2.89 to 0.14) | 475 | -2.04 (03.43 to -0.57) | 492 | -1.78 (-3.14 to -0.29) | 378 |
| Males with BPH | -0.68 (-2.29 to 0.43) | 15 | -1.50 (-2.90 to -0.14) | 29 | -1.25 (-2.29 to 0.29) | 21 |
| Males without BPH | -1.60 (-2.29 to 0.29) | 54 | -2.26 (-3.43 to -0.57) | 46 | -1.29 (-3.13 to 0.29) | 39 |
| Prior ACH Use in last 12 Mo. | -1.25 (-2.18 to 0.29) | 79 | -1.97 (-3.33 to -0.83) | 74 | -1.52 (-2.90 to -0.29) | 48 |
| No Prior ACH Use in last 12 Mo. | -1.69 (-3.00 to 0.14) | 396 | -2.05 (-3.45 to -0.57) | 418 | -1.82 (-3.29 to -0.29) | 330 |
| Prior B3 Agonist Use in last 12 Mo. | 0.03 (-0.71 to 1.29) | 25 | -2.83 (-4.00 to -0.50) | 18 | -1.30 (-2.55 to -0.14) | 31 |
| No Prior B3 Agonist Use in last 12 Mo. | -1.71 (-3.00 to 0.13) | 450 | -2.01 (-3.43 to -0.57) | 474 | -1.83 (-3.16 to -0.29) | 347 |

Table 38. Micturitions: Males With and Without BPH (Descriptive Statistics)

| Subgroup | Visit | Placebo | n | Vibegron | n | Tolterodine | n |
|---|---|---|---|---|---|---|---|
| Overall for Males | Baseline Mean (Q1, Q3) | 11.95 (9.71 to 13.14) | 75 | 11.79 (9.14 to 12.86) | 77 | 12.12 (9.14 to 13.43) | 65 |
| | Week 12 CFB Mean (Q1, Q3) | -1.40 -2.29 to 0.29) | 69 | -1.97 (-3.43 to -0.38) | 75 | -1.28 (-2.99 to 0.29) | 60 |
| | | | | | | | |
| Males with BPH | Baseline Mean (Q1, Q3) | 11.45 (9.79 to 12.21) | 15 | 11.22 (8.83 to 12.14) | 29 | 12.65 (10.00 to 14.29) | 21 |
| | Week 12 CFB Mean (Q1, Q3) | -0.68 (-2.29 to 0.43) | 16 | -1.50 (-2.90 to -0.14) | 16 | -1.25 (-2.29 to 0.29) | 9 |
| | | | | | | | |
| Males without BPH | Baseline Mean (Q1, Q3) | 12.09 (9.67 to 13.29) | 59 | 12.14 (9.36 to 14.39) | 48 | 11.84 (9.14 to 13.29) | 43 |
| | Week 12 CFB Mean (Q1, Q3) | -1.60 (-2.29 to 0.29) | 54 | -2.26 (-3.43 to -0.57) | 46 | -1.29 (-3.13 to 0.29) | 39 |

Results for certain subgroups are shown in Table 39. Greater reductions in micturitions and UUI episodes were seen in the subpopulations that were dosed with vibegron relative to the groups that were dosed with tolterodine or a placebo.

Table 39. Vibregon Efficacy in Some Subpopulations at Week 12

| Subpopulation | Placebo | Vibegron | Tolterodine |
|---|---|---|---|
| **Age ≥ 65 years (Micturitions)** | -1.27 (2.67)<br>n=204 | **-2.10** (2.83)<br>n=228 | -1.48 (2.15)<br>n=151 |
| **Age ≥ 65 years (UUI Episodes)** | -1.20 (2.70)<br>n=158 | **-1.94** (2.50)<br>n=183 | -1.80 (2.41)<br>n=116 |
| **Age ≥ 65 years (Daily Urgency)[c]** | -1.7<br>n=204 | **-2.7**<br>**n=228** | -2.1<br>n=151 |
| **Prior anticholinergic use[a] (Micturitions[b])**<br>**n at baseline**<br>**Baseline value**<br>**n at 12 weeks**<br>**12-week change** | <br>85<br>11.1 (3.0)<br>79<br>-1.25 (2.33) | <br>77<br>11.7 (3.3)<br>74<br>**-1.97** (2.40) | <br>51<br>11.2 (2.6)<br>48<br>-1.52 (1.88) |
| **No prior anticholinergic use[a] (Micturitions[b])**<br>**n at baseline**<br>**Baseline value**<br>**n at 12 weeks**<br>**12-week change** | <br>435<br>11.9 (4.2)<br>396<br>-1.7 (2.8) | <br>449<br>11.3 (3.4)<br>418<br>-2.1 (2.6) | <br>366<br>11.5 (3.2)<br>330<br>-1.8 (2.7) |
| | | | |
| **Prior anticholinergic use[a] (UUI Episodes[b])**<br>**n at baseline**<br>**Baseline value**<br>**n at 12 weeks**<br>**12-week change** | <br>74<br>3.2 (2.3)<br>68<br>-0.83 (2.18) | <br>64<br>2.9 (2.4)<br>64<br>**-1.46** (2.16) | <br>42<br>3.4 (2.2)<br>39<br>-0.98 (1.80) |
| **No prior anticholinergic use[a] (UUI Episodes[b])**<br>**n at baseline**<br>**Baseline value** | <br>331<br>3.6 (3.2) | <br>339<br>3.5 (3.0) | <br>277<br>3.4 (2.7) |

(continued)

| Subpopulation | Placebo | Vibegron | Tolterodine |
|---|---|---|---|
| **n at 12 weeks** | 304 | 319 | 247 |
| **12-week change** | -1.6 (2.4) | -2.1 (2.5) | -1.9 (2.4) |
| **Prior mirabegron use[a] (Micturitions[b])** | | | |
| **n at baseline** | 27 | 21 | 32 |
| **Baseline value** | 10.4 (2.5) | 11.2 (2.8) | 11.0 (3.2) |
| **n at 12 weeks** | 25 | 18 | 31 |
| **12-week change** | 0.03 (2.22) | **-2.83** (3.83) | -1.30 (1.99) |
| **No prior mirabegron use[a] (Micturitions[b])** | | | |
| **n at baseline** | 493 | *505* | 385 |
| **Baseline value n at 12 weeks** | 11.8 (4.1) 450 | 11.3 (3.4) 474 | 11.5 (3.2) 347 |
| **12-week change** | -1.7 (2.7) | -2.0 (2.5) | -1.8 (2.7) |
| **Prior mirabegron use[a] (UUI Episodes[b])** | | | |
| **n at baseline** | 23 | 16 | 19 |
| **Baseline value** | 2.6 (1.7) | 2.6 (1.9) | 2.3 (1.1) |
| **n at 12 weeks** | 21 | 14 | 18 |
| **12-week change** | -0.31 (1.62) | **-1.64** (2.59) | -0.65 (1.42) |
| **No prior mirabegron use[a] (UUI Episodes[b])** | | | |
| **n at baseline** | 382 | 387 | 300 |
| **Baseline value** | 3.6 (3.1) | 3.5 (2.9) | 3.5 (2.6) |
| **n at 12 weeks** | 351 | 369 | 268 |
| **12-week change** | -1.5 (2.4) | -2.0 (2.5) | -1.8 (2.3) |

[a] During the prior 12 months
[b] Calculated as the total number of such events on complete diary days, divided by the number of complete days
[c] Posthoc analysis

As shown in Table 39, the decrease in the average number of micturitions in a 24-hour period in subjects with prior ACH use is about 0.7 more than the decrease of micturitions in subjects treated with placebo, while the decrease in the average number of micturitions in a 24-hour period for subjects with prior B3 use is about 2.9 more than the decrease in micturitions in subjects treated with placebo.

Unexpectedly, treatment-experienced subjects (subjects previously treated with an ACH or B3 agonist), showed improved reductions in micturitions and/or UUI episodes compared to treatment naive subjects. This presents an unexpected benefit for patients who have sought other treatments, have not been successfully treated, and therefore have an unmet need.

Also unexpectedly, subjects ≥65 years old treated with vibegron showed an improvement in reductions in micturitions, urgency episodes, and UUI episodes relative to subjects treated with placebo. This is significant as the number of patients experiencing OAB symptoms is higher in this demographic.

Vibegron achieved statistical significance for all seven secondary endpoints, compared to placebo: (1) reduction of daily urgency episodes; (2) 75% and 100% reduction for UUI; (3) 50% reduction for daily urgency; (4) reduction in daily total incontinence; (5) OAB-q coping score; and (5) average volume voided per micturition. For example, the data on reduction in urgency episodes are shown in Table 40, and Figures 6 and 7.

Table 40. Change from Baseline in Average Daily Number of Urgency Episodes (CFB Least Squares Means at Week 12)

| Urgency | Placebo | Vibegron | Tolterodine |
|---|---|---|---|
| Week 2 | | | |
| Baseline Daily Episodes, n Mean (SD) | 520<br>8.13 (4.668) | 526<br>8.11 (4.400) | 417<br>7.92 (3.883) |
| Week 2, n Mean (SD) | 499<br>7.04 (4.755) | 508<br>6.47 (4.458) | 406<br>6.30 (3.989) |

(continued)

| Urgency | Placebo | Vibegron | Tolterodine |
|---|---|---|---|
| Week 2 | | | |
| Change from baseline, n LS Mean 95% CI | 499<br>-0.9<br>-1.2 to -0.6 | 508<br>-1.5<br>-1.8 to -1.2 | 406<br>-1.5<br>-1.8 to -1.1 |
| Active - Placebo LS Mean Difference (95% CI) P-Value | --- | -0.6 (-0.9 to -0.3)<br><0.001 | -0.6 (-0.9 to -0.2)<br><0.001 |
| Week 4 | | | |
| Baseline Daily Episodes, n Mean (SD) | 520<br>8.13 (4.668) | 526<br>8.11 (4.400) | 417<br>7.92 (3.883) |
| Week 4, n Mean (SD) | 469<br>6.88 (4.893) | 497<br>6.02 (4.458) | 380<br>6.00 (4.097) |
| Change from baseline, n LS Mean 95% CI | 469<br>-1.1<br>-1.4 to -0.8 | 497<br>-1.9<br>-2.2 to -1.5 | 380<br>-1.8<br>-2.2 to -1.5 |
| Active - Placebo LS Mean Difference (95% CI) P-Value | --- | -0.8 (-1.1 to -0.5)<br><0.0001 | -0.7 (-1.1 to -0.4)<br><0.0001 |
| Week 8 | | | |
| Baseline Daily Episodes, n Mean (SD) | 520<br>8.13 (4.668) | 526<br>8.11 (4.400) | 417<br>7.92 (3.883) |
| Week 8, n Mean (SD) | 486<br>6.27 (4.633) | 504<br>5.58 (4.399) | 390<br>5.60 (4.266) |
| Change from baseline, n LS Mean 95% CI | 486<br>-1.6<br>-1.9 to -1.2 | 504<br>-2.4<br>-2.7 to -2.0 | 390<br>-2.2<br>-2.6 to -1.8 |
| Active - Placebo LS Mean Difference (95% CI) P-Value | ---- | -0.8<br>-1.2 to -0.4<br><0.001 | -0.6<br>-1.1 to -0.2<br>0.0027 |
| Week 12 | | | |
| Baseline Daily Episodes, n Mean (SD) | 520<br>8.13 (4.668) | 526<br>8.11 (4.400) | 417<br>7.92 (3.883) |
| Week 12, n Mean (SD) | 475<br>5.76 (4.473) | 492<br>5.29 (4.500) | 378<br>5.36 (4.425) |
| Change from baseline, n LS Mean 95% CI | 475<br>-2.0<br>-2.4 to -1.7 | 492<br>-2.7<br>-3.1 to -2.3 | 378<br>-2.5<br>-2.9 to -2.0 |
| Active - Placebo LS Mean Difference (95% CI) P-Value | ---- | -0.7 (-1.1 to -0.2)<br>0.0020 | -0.4 (-0.9 to 0.0)<br>0.0648 |

Data on reduction in urgency episodes in the dry population are shown in Tables 41 and 42 and in Figure 21.

Table 41. Change from Baseline in Average Daily Number of Urgency Episodes of Dry Population (CFB Least Squares Means at Week 12)

| Urgency | Placebo (N=115) | Vibegron (N=123) | Tolterodine (N=98) |
|---|---|---|---|
| Week 2 | | | |
| Baseline Daily Episodes, n Mean (SD) | 115<br>8.60 (5.027) | 123<br>8.58 (4.421) | 98<br>8.41 (3.892) |
| Week 2, n Mean (SD) | 108<br>7.85 (5.167) | 118<br>6.98 (4.732) | 96<br>6.87 (4.260) |
| Change from Baseline, n LS Mean 95% CI | 108<br>-0.8 (0.25)<br>-1.3 to -0.3 | 118<br>-1.5 (0.24)<br>-2.0 to -1.1 | 96<br>-1.4 (0.27)<br>-1.9 to -0.8 |
| Active - Placebo LS Mean Difference (95% CI) P-Value | ---- | -0.8 (0.35)<br>-1.5 to -0.1<br>0.0271 | -0.6 (0.37)<br>-1.3 to 0.1<br>0.1036 |
| Week 4 | | | |
| Baseline Daily Episodes, n Mean (SD) | 115<br>8.60 (5.027) | 123<br>8.58 (4.421) | 98<br>8.41 (3.892) |
| Week 4, n Mean (SD) | 105<br>7.83 (5.342) | 116<br>6.80 (4.667) | 88<br>7.03 (4.865) |
| Change from baseline, n LS Mean 95% CI | 105<br>-0.9 (0.27)<br>-1.4 to -0.4 | 116<br>-1.8 (0.26) -2.3 to -1.3 | 88<br>-1.6 (0.29) -2.2 to -1.0 |
| Active - Placebo LS Mean Difference (95% CI) P-Value | ---- | -0.9 (0.37)<br>-1.7 to -0.2<br>0.0147 | -0.7 (0.40)<br>-1.5 to 0.1<br>0.0862 |
| Week 8 | | | |
| Baseline Daily Episodes, n Mean (SD) | 115<br>8.60 (5.027) | 123<br>8.58 (4.421) | 98<br>8.41 (3.892) |
| Week 8, n Mean (SD) | 106<br>7.39 (5.261) | 115<br>6.39 (4.627) | 95<br>6.25 (4.957) |
| Change from baseline, n | 106 | 115 | 95 |
| LS Mean 95% CI | -1.2 (0.31)<br>-1.8 to -0.6 | -2.2 (0.30)<br>-2.8 to -1.6 | -2.2 (0.34)<br>-2.9 to -1.5 |
| Active - Placebo LS Mean Difference (95% CI) P-Value | ---- | -1.0 (0.44)<br>-1.9 to -0.2<br>0.0214 | -1.0 (0.46)<br>-1.9 to -0.1<br>0.0261 |

(continued)

| Week 12 | | | |
|---|---|---|---|
| Baseline Daily Episodes, n Mean (SD) | 115<br>8.60 (5.027) | 123<br>8.58 (4.421) | 98<br>8.41 (3.892) |
| Week 12, n Mean (SD) | 103<br>6.75 (4.888) | 109<br>6.17 (4.958) | 92<br>6.56 (5.480) |
| Change from baseline, n LS Mean 95% CI | 103<br>-1.6 (0.34)<br>-2.2 to -0.9 | 109<br>-2.6 (0.33)<br>-3.2 to -2.0 | 92<br>-2.0 (0.36)<br>-2.7 to -1.3 |
| Active - Placebo LS Mean Difference (95% CI) P-Value | ---- | -1.0 (0.47)<br>-2.0 to -0.1<br>0.0282 | -0.4 (0.49)<br>-1.4 to 0.5<br>0.3873 |
| Covariates included in the mixed model for repeated measures are study visit, baseline number of urgency episodes and treatment by study visit interaction.<br>Hypothesis testing was performed for Vibegron - Placebo.<br>Comparisons between Tolterodine ER and Placebo are considered descriptive. | | | |

Reduction in urgency in dry population categorized by age group is shown in Table 42.

Table 42. Reduction in Urgency in Dry Population by Age Group (Change from Baseline to Week 12)

| **Urgency for Age <65** | **Placebo** | **Vibegron** | **Tolterodine** |
|---|---|---|---|
| Change from Baseline LS Mean (n) | -1.5 (63) | -2.9 (64) | -2.3 (57) |
| LS Mean Difference (95% CI) P-Value | - | Vibegron - Placebo<br>-1.4 (-2.6, -0.2)<br>0.0252 | Tolterodine - Placebo<br>-0.8 (-2.1, 0.4)<br>0.2010 |
| | | | |
| **Urgency for Age ≥65** | **Placebo** | **Vibegron** | **Tolterodine** |
| Change from Baseline LS Mean (n) | -1.7 (46) | -2.2 (45) | -1.5 (35) |
| LS Mean Difference (95% CI) P-Value | | Vibegron - Placebo<br>-0.5 (-1.9, 0.9)<br>0.4696 | Tolterodine - Placebo<br>0.2 (-1.3, 1.7)<br>0.8207 |
| Note: Covariates included in the mixed model for repeated measures are age category, study visit, baseline number of urgency episodes and the interaction terms. | | | |

Urgency 50% responder data in OAB dry patients is shown in Table 43.

Table 43. Urgency 50% Responder Analysis in Dry Population

| **Statistic** | **Placebo (N=115)** | **Vibegron (N=123)** | **Tolterodine (N=98)** |
|---|---|---|---|
| Patients with at Least 50% Reduction in Urgency Episodes from Baseline to Week 2 | | | |
| Unadjusted n (%) | 19 (16.5) | 23 (18.7) | 16 (16.3) |
| Adjusted n (%) | 16 (13.5) | 22 (18.1) | 16 (16.3) |

(continued)

| Statistic | Placebo (N=115) | Vibegron (N=123) | Tolterodine (N=98) |
|---|---|---|---|
| Active - Placebo [1] CMH Difference (95% CI) P-Value | ---- | 4.7<br>-4.6 to 14.0<br>0.3209 | 2.8<br>-6.9 to 12.4<br>0.5714 |
| | | | |
| Patients with at Least 50% Reduction in Urgency Episodes from Baseline to Week 4 | | | |
| Unadjusted n (%) | 23 (20.0) | 31 (25.2) | 25 (25.5) |
| Adjusted n (%) | 20 (17.1) | 28 (22.4) | 20 (20.6) |
| Active - Placebo [1] CMH Difference (95% CI) P-Value | ---- | 5.4<br>-4.9 to 15.7<br>0.3068 | 3.5<br>-7.2 to 14.3<br>0.5217 |
| | | | |
| Patients with at Least 50% Reduction in Urgency Episodes from Baseline to Week 8 | | | |
| Unadjusted n (%) | 27 (23.5) | 43 (35.0) | 30 (30.6) |
| Adjusted n (%) | 23 (19.7) | 39 (31.9) | 28 (28.5) |
| Active - Placebo [1] CMH Difference (95% CI) P-Value | ---- | 12.4<br>1.4 to 23.3<br>0.0269 | 8.7<br>-2.8 to 20.2<br>0.1375 |
| | | | |
| Patients with at Least 50% Reduction in Urgency Episodes from Baseline to Week 12 | | | |
| Unadjusted n (%) | 31 (27.0) | 53 (43.1) | 32 (32.7) |
| Adjusted n (%) | 27 (23.1) | 45 (36.9) | 28 (29.0) |
| Active - Placebo [1] CMH Difference (95% CI) P-Value | ---- | 13.9<br>1.9 to 25.9<br>0.0227 | 5.8<br>-6.2 to 17.9<br>0.3434 |
| [1] The difference in proportion and corresponding CI and p-value was calculated using the Cochran-Mantel-Haenszel risk difference estimate stratified by Sex (Female vs Male), with weights proposed by Greenland and Robins. - MI has been used to impute values missing for any reason for the weeks analyzed. - Presented frequencies and the denominator used for percentages are based on patients in the FAS and randomized treatment. | | | |

Data on reduction in urgency epiosodes in 75% UUI Responders is shown in Table 44.

Table 44. Change from Baseline in Average Daily Number of Urgency Episodes in 75% UUI Responders

| Urgency | Placebo (N=149) | Vibegron (N=211) | Tolterodine (N=152) |
|---|---|---|---|
| Week 2 | | | |
| Baseline Daily Episodes, n Mean (SD) | 149<br>7.13 (4.257) | 211<br>7.46 (3.886) | 152<br>7.72 (3.708) |
| Week 2, n Mean (SD) | 142<br>5.41 (4.295) | 203<br>5.40 (3.762) | 146<br>5.37 (3.577) |
| Change from baseline, n LS Mean 95% CI | 142<br>-1.8 (0.21)<br>-2.2 to -1.4 | 203<br>-2.2 (0.17)<br>-2.5 to -1.8 | 146<br>-2.3 (0.21)<br>-2.7 to -1.9 |

(continued)

| | | | |
|---|---|---|---|
| Week 4 | | | |
| Baseline Daily Episodes, n Mean (SD) | 149<br>7.13 (4.257) | 211<br>7.46 (3.886) | 152<br>7.72 (3.708) |
| Week 4, n Mean (SD) | 134<br>5.15 (4.446) | 202<br>4.72 (3.838) | 139<br>4.93 (3.675) |
| Change from baseline, n LS Mean 95% CI | 134<br>-2.0 (0.23)<br>-2.4 to -1.5 | 202<br>-2.7 (0.19)<br>-3.1 to -2.3 | 139<br>-2.8 (0.23)<br>-3.2 to -2.3 |
| Week 8 | | | |
| Baseline Daily Episodes, n Mean (SD) | 149<br>7.13 (4.257) | 211<br>7.46 (3.886) | 152<br>7.72 (3.708) |
| Week 8, n Mean (SD) | 135<br>3.95 (3.922) | 200<br>3.95 (3.742) | 135<br>4.42 (3.677) |
| Change from baseline, n LS Mean 95% CI | 135<br>-3.1 (0.25)<br>-3.6 to -2.6 | 200<br>-3.5 (0.21)<br>-3.9 to -3.1 | 135<br>-3.3 (0.25)<br>-3.8 to -2.8 |
| Week 12 | | | |
| Baseline Daily Episodes, n Mean (SD) | 149<br>7.13 (4.257) | 211<br>7.46 (3.886) | 152<br>7.72 (3.708) |
| Week 12, n Mean (SD) | 126<br>3.02 (3.653) | 192<br>3.31 (3.433) | 126<br>3.67 (3.542) |
| Change from baseline, n LS Mean 95% CI | 126<br>-3.9 (0.27)<br>-4.5 to -3.4 | 192<br>-4.2 (0.23)<br>-4.6 to -3.7 | 126<br>-3.9 (0.27)<br>-4.4 to -3.3 |
| Only subjects that are considered 75% UUI Responders at Week 12 based on unadjusted imputed values are included in these analyses.<br>Covariates included in the mixed model for repeated measures are study visit, baseline number of urgency episodes and treatment by study visit interaction. | | | |

Changes in average volume voided in 75% UUI responders from baseline to week 12 is shown in Table 45.

Table 45. Change from Baseline in Average Volume Voided per Micturition in 75% UUI Responders (Change from Baseline to Week 12)

| Statistic | Placebo (N-149) | Vibegron (N=211) | Tolterodine (N=152) |
|---|---|---|---|
| Week 2 | | | |
| Baseline Daily Episodes, n Mean (SD) | 148<br>151.6 (66.83) | 210<br>160.6 (68.05) | 152<br>157.0 (66.40) |
| n Mean (SD) | 142<br>148.3 (68.20) | 199<br>174.3 (77.29) | 141<br>170.6 (77.20) |
| Change from baseline, n LS Mean 95% CI | 142<br>-2.3 (4.88)<br>-11.9 to 7.3 | 199<br>17.7 (4.28)<br>9.3 to 26.1 | 141<br>17.5 (4.96)<br>7.8 to 27.3 |
| Week 4 | | | |
| Baseline Daily Episodes, n Mean (SD) | 148<br>151.6 (66.83) | 210<br>160.6 (68.05) | 152<br>157.0 (66.40) |

(continued)

| | | | |
|---|---|---|---|
| Week 4 | | | |
| n Mean (SD) | 110<br>153.8 (73.08) | 168<br>178.1 (82.51) | 118<br>174.8 (73.04) |
| Change from baseline, n LS Mean 95% CI | 110<br>2.0 (5.28)<br>-8.4 to 12.3 | 168<br>20.7 (4.52)<br>11.9 to 29.6 | 118<br>16.2 (5.27)<br>5.8 to 26.5 |
| Week 8 | | | |
| Baseline Daily Episodes, n Mean (SD) | 148<br>151.6 (66.83) | 210<br>160.6 (68.05) | 152<br>157.0 (66.40) |
| n Mean (SD) | 138<br>151.5 (67.32) | 199<br>184.2 (87.70) | 135<br>182.1 (77.27) |
| Change from baseline, n LS Mean 95% CI | 138<br>0.1 (5.41)<br>-10.6 to 10.7 | 199<br>27.2 (4.67)<br>18.0 to 36.4 | 135<br>26.1 (5.51)<br>15.3 to 37.0 |
| Week 12 | | | |
| Baseline Daily Episodes, n Mean (SD) | 148<br>151.6 (66.83) | 210<br>160.6 (68.05) | 152<br>157.0 (66.40) |
| n Mean (SD) | 131<br>151.4 (78.67) | 192<br>185.0 (87.89) | 125<br>177.3 (77.70) |
| Change from baseline, n LS Mean 95% CI | 131<br>-0.1 (5.69)<br>-11.3 to 11.0 | 192<br>26.9 (4.87)<br>17.4 to 36.5 | 125<br>19.1 (5.83)<br>7.6 to 30.5 |
| Only subjects that are considered 75% UUI Responders at Week 12 based on unadjusted imputed values are included in these analyses.<br>Covariates included in the mixed model for repeated measures are study visit, region, baseline average volume voided and treatment by study visit interaction. | | | |

Changes in PGI-Control in 75% UUI responders is shown in Table 46 while changes in OAB-q coping in 75% UUI responders at week 12 are shown in Table 47. Changes in OAB-q symptom bother in 75% UUI responders at week 12 are shown in Table 48.

Table 46. Change from Baseline in PGI-Control in 75% UUI Responders

| Statistic | Placebo (N=149) | Vibegron (N=211) | Tolterodine (N=152) |
|---|---|---|---|
| Week 4 | | | |
| Baseline Daily Episodes, n Mean (SD) | 149<br>3.06 (0.995) | 210<br>3.27 (0.920) | 152<br>3.20 (0.864) |
| n Mean (SD) | 146<br>2.49 (0.772) | 208<br>2.42 (0.887) | 148<br>2.51 (0.907) |
| Change from baseline, n LS Mean 95% CI | 146<br>-0.7 (0.07)<br>-0.8 to -0.5 | 208<br>-0.8 (0.06)<br>-0.9 to -0.7 | 148<br>-0.7 (0.07)<br>-0.8 to -0.5 |
| Week 8 | | | |
| Baseline Daily Episodes, n Mean (SD) | 149<br>3.06 (0.995) | 210<br>3.27 (0.920) | 152<br>3.20 (0.864) |
| n Mean (SD) | 139<br>2.21 (0.697) | 202<br>2.25 (0.863) | 143<br>2.18 (0.861) |

(continued)

| | | | |
|---|---|---|---|
| Week 8 | | | |
| Change from baseline, n LS Mean 95% CI | 139<br>-0.9 (0.07)<br>-1.1 to -0.8 | 202<br>-1.0 (0.06)<br>-1.1 to -0.8 | 143<br>-1.0 (0.07)<br>-1.1 to -0.9 |
| Week 12 | | | |
| Baseline Daily Episodes, n Mean (SD) | 149<br>3.06 (0.995) | 210<br>3.27 (0.920) | 152<br>3.20 (0.864) |
| n Mean (SD) | 128<br>2.14 (0.820) | 193<br>2.02 (0.784) | 127<br>2.05 (0.862) |
| Change from baseline, n LS Mean 95% CI | 128<br>-1.0 (0.07)<br>-1.1 to -0.9 | 193<br>-1.2 (0.06)<br>-1.3 to -1.1 | 127<br>-1.1 (0.07)<br>-1.2 to -1.0 |
| Only subjects that are considered 75% UUI Responders at Week 12 based on unadjusted imputed values are included in these analyses.<br>Covariates included in the mixed model for repeated measures are study visit, baseline PGI-Control score and treatment by study visit. interaction. | | | |

Table 47. Changes in OAB-q Coping in 75% UUI Responders (Change from Baseline to Week 12)

| Statistic | Placebo (N=149) | Vibegron (N=211) | Tolterodine (N=152) |
|---|---|---|---|
| Coping Subscore | | | |
| Baseline n Mean (SD) | 149<br>65.21 (22.693) | 209<br>59.53 (25.849) | 152<br>60.13 (25.953) |
| Week 12 n Mean (SD) | 143<br>83.25 (20.167) | 202<br>83.99 (19.232) | 141<br>83.05 (20.409) |
| Change from Baseline at Week 12 n LS Means (SE) 95% CI | 143<br>17.4 (1.69)<br>14.1 to 20.7 | 202<br>19.7 (1.48)<br>16.7 to 22.6 | 141<br>18.4 (1.73)<br>15.0 to 21.8 |
| Only subjects that are considered 75% UUI Responders at Week 12 based on unadjusted imputed values are included in these analyses.<br>Covariates included in the ANCOVA model are region and baseline score. | | | |

Table 48. Changes in OAB-q Symptom Bother in 75% UUI Responders (Change from Baseline to Week 12)

| Statistic | Placebo (N=149) | Vibegron (N=211) | Tolterodine (N=152) |
|---|---|---|---|
| Symptom Bother Subscore | | | |
| Baseline n Mean (SD) | 149<br>46.07 (19.210) | 209<br>49.33 (20.187) | 152<br>47.46 (20.451) |
| Week 12 n Mean (SD) | 143<br>23.67 (19.212) | 202<br>20.13 (15.862) | 141<br>22.59 (17.919) |

(continued)

| Statistic | Placebo (N=149) | Vibegron (N=211) | Tolterodine (N=152) |
|---|---|---|---|
| Symptom Bother Subscore | | | |
| Change from Baseline at Week 12 n LS Means (SE) 95% CI | 143<br>-21.5 (1.57)<br>-24.5 to -18.4 | 202<br>-25.8 (1.38)<br>-28.5 to -23.1 | 141<br>-22.7 (1.60)<br>-25.8 to -19.5 |
| Only subjects that are considered 75% UUI Responders at Week 12 based on unadjusted imputed values are included in these analyses.<br>Covariates included in the ANCOVA model are region and baseline score. | | | |

Data on reduction in urgency episodes are shown for certain subpopulations in Tables 49 and 50.

Table 49. Urgency Episodes: Overall and by Subgroup (Descriptive Statistics: Week 12 CFB Mean (Q1, Q3)

| Subgroup | Placebo Mean (Q1,Q3) | n | Vibegron Mean (Q1,Q3) | n | Tolterodine Mean (Q1,Q3) | n |
|---|---|---|---|---|---|---|
| Overall | -2.26 (-4.00 to -0.29) | 475 | -2.93 (-4.71 to - 0.65) | 492 | -2.57 (-4.17 to -0.57) | 378 |
| Males with BPH | -2.43 (-4.29 to -0.0) | 15 | -1.90 (-4.71 to 0.10) | 29 | -3.29 (-5.14 to 0.31) | 21 |
| Males without BPH | -2.38 (-3.84 to -0.40) | 54 | -2.56 (-3.71 to 0.24) | 46 | -2.32 (-4.29 to -0.04) | 39 |
| Prior ACH Use in last 12 Mo. | -1.39 (-3.14 to 0.86) | 79 | -2.50 (-4.14 to - 0.90) | 74 | -2.23 (-3.31 to, -0.64) | 48 |
| No Prior ACH Use in last 12 Mo. | -2.44 (-4.14 to, -0.30) | 396 | -3.00 (-4.86 to - 0.57) | 418 | -2.62 (-4.43 to, -0.57) | 330 |
| Prior B3 Agonist Use in last 12 Mo. | -0.91 (-2.29 to 1.02) | 25 | -2.56 (-4.71 to - 0.57) | 18 | -2.72 (-4.14 to 0.00) | 31 |
| No Prior B3 Agonist Use in last 12 Mo. | -2.34 (-4.00 to -0.30) | 450 | -2.94 (-4.71 to - 0.67) | 474 | -2.56 (-4.29 to -0.63) | 347 |

Table 50. Daily Urgency Episodes: Reduction from Baseline at Week 12 (LS Mean, (SE))

| Age Group | Placebo | Vibegron 75 mg | Vibegron - Placebo LS Means Difference (SE) 95% CI | Tolterodine ER 4 mg |
|---|---|---|---|---|
| All patients | -2.0 (0.19)<br>n=475 | -2.7 (0.19)<br>n=492 | -0.7 (0.22)<br>-1.1 to -0.2 | -2.5 (0.21)<br>n=378 |
| Age ≥ 65 years | -1.7 (0.26)<br>n=204 | -2.7 (0.25)<br>n=228 | -1.0 (0.32)<br>-1.6 to -0.4 | -2.1 (0.30)<br>n=151 |
| Age ≥75 years | -0.9 (0.46)<br>n=52 | -2.6 (0.41)<br>n=69 | -1.7 (0.61)<br>-2.9 to -0.5 | -2.4 (0.52)<br>n=41 |

Total incontinence data is shown in Table 51 and Figure 9. The mean change from baseline for total daily incontinence was reduced by 2.3 episodes in the vibegron 75 mg group after 12 weeks of treatment. The placebo-adjusted mean total daily incontinence episodes was significantly reduced at week 12 in the vibegron 75 mg group by -0.7 episodes (SE, 0.16; P<0.0001 vs placebo), which was numerically greater than the reduction in the tolterodine arm, -0.5 (SE, 0.17; P=0.0074 vs placebo) (Table 35). Rapid and statistically significant improvement in total daily incontinence was observed at week 2 (-0.7, placebo adjusted) in the vibegron group and maintained statistical significance through all timepoints, including week 12.

For average volume (mL) voided, the placebo-adjusted mean change from baseline was significantly improved (increased) at week 12 in the vibegron 75 mg group by 21.2 mL (SD, 3.52; P<0.0001 vs placebo), which was also

numerically greater than the tolterodine group, 13.3 mL (SD, 3.76; P<0.001 vs placebo) (Table 36).

Table 51. Change from Baseline in Average Daily Number of Total Incontinence (CFB Least squares means at weeks 2, 4, 8, and 12)

| Total Incontinence | Placebo | Vibegron | Tolterodine |
|---|---|---|---|
| Week 2 | | | |
| Baseline Daily Episodes, n Mean (SD) | 405<br>4.17 (3.823) | 403<br>4.14 (3.631) | 319<br>4.06 (3.071) |
| Week 2, n Mean (SD) | 391<br>3.26 (3.701) | 390<br>2.53 (3.118) | 310<br>2.75 (2.743) |
| Change from baseline, n LS Mean (SE) 95% CI | 391<br>-0.9 (0.14)<br>-1.1 to -0.6 | 390<br>-1.6 (0.14)<br>-1.8 to -1.3 | 310<br>-1.3 (0.15)<br>-1.6 to -1.1 |
| Active - Placebo LS Mean Difference (SE) 95% CI P-Value | | -0.7 (0.14)<br>-1.0 to -0.4<br><0.0001 | -0.5 (0.15)<br>-0.8 to -0.2<br>0.0012 |
| Week 4 | | | |
| Baseline Daily Episodes, n Mean (SD) | 405<br>4.17 (3.823) | 403<br>4.14 (3.631) | 319<br>4.06 (3.071) |
| Week 4, n Mean (SD) | 364<br>3.06 (3.641) | 381<br>2.28 (3.239) | 292<br>2.54 (2.784) |
| Change from baseline, n LS Mean (SE) 95% CI | 364<br>-1.1 (0.14)<br>-1.4 to -0.8 | 381<br>-1.9 (0.14)<br>-2.2 to -1.6 | 292<br>-1.6 (0.15)<br>-1.9 to -1.3 |
| Active - Placebo LS Mean Difference (SE) 95% CI P-Value | | -0.8 (0.14)<br>-1.1 to -0.5<br><0.0001 | -0.5 (0.15)<br>-0.8 to -0.2<br>0.0014 |
| Week 8 | | | |
| Baseline Daily Episodes, n Mean (SD) | 405<br>4.17 (3.823) | 403<br>4.14 (3.631) | 319<br>4.06 (3.071) |
| Week 8, n Mean (SD) | 380<br>2.71 (3.210) | 389<br>2.05 (3.245) | 295<br>2.20 (2.673) |
| Change from baseline, n LS Mean (SE) 95% CI | 380<br>-1.4 (0.15)<br>-1.7 to -1.1 | 389<br>-2.1 (0.15)<br>-2.4 to -1.8 | 295<br>-1.8 (0.16)<br>-2.1 to -1.5 |
| Active - Placebo LS Mean Difference (SE) 95% CI P-Value | | -0.7 (0.15)<br>-1.0 to -0.4<br><0.0001 | -0.5 (0.17)<br>-0.8 to -0.1<br>0.0054 |
| Week 12 | | | |
| Baseline Daily Episodes, n Mean (SD) | 405<br>4.17 (3.823) | 403<br>4.14 (3.631) | 319<br>4.06 (3.071) |
| Week 12, n Mean (SD) | 372<br>2.50 (3.807) | 383<br>1.89 (3.120) | 286<br>1.89 (2.353) |
| Change from baseline, n LS Mean (SE) 95% CI | 372<br>-1.6 (0.15)<br>-1.9 to -1.3 | 383<br>-2.3 (0.15)<br>-2.6 to -2.0 | 286<br>-2.0 (0.16)<br>-2.4 to -1.7 |

(continued)

| Week 12 | | | |
|---|---|---|---|
| Active - Placebo LS Mean Difference (SE) 95% CI P-Value | ------ | -0.7 (0.16)<br>-1.0 to -0.4<br><0.0001 | -0.5 (0.17)<br>-0.8 to -0.1<br>0.0074 |

Tables 52 shows the analysis of total incontinence 75% responders while Tables 54-57 show data for 50% responders.

Table 52. Total Incontinence 75% Responder Analysis

| **Statistic** | **Placebo (N=405)** | **Vibegron (N=403)** | **Tolterodine (N=319)** |
|---|---|---|---|
| Patients with at Least 75% Reduction in Total Incontinence from Baseline to Week 2 | | | |
| Unadjusted n (%) | 56 (13.8) | 95 (23.6) | 58 (18.2) |
| Adjusted n (%) | 52 (12.9) | 89 (22.1) | 53 (16.5) |
| Active - Placebo [1] CMH Difference (95% CI) p-Value | ----- | 9.3<br>4.0 to 14.5<br>0.0005 | 3.6<br>-1.6 to 8.9<br>0.1747 |
| | | | |
| Patients with at Least 75% Reduction in Total Incontinence from Baseline to Week 4 | | | |
| Unadjusted n (%) | 95 (23.5) | 139 (34.5) | 90 (28.2) |
| Adjusted n (%) | 80 (19.8) | 127 (31.5) | 75 (23.5) |
| Active - Placebo [1] CMH Difference (95% CI) p-Value | ----- | 11.8<br>5.7 to 17.8<br>0.0001 | 3.7<br>-2.5 to 10.0<br>0.2420 |
| | | | |
| Patients with at Least 75% Reduction in Total Incontinence | | | |
| from Baseline to Week 8 | | | |
| Unadjusted n (%) | 123 (30.4) | 176 (43.7) | 116 (36.4) |
| Adjusted n (%) | 111 (27.4) | 169 (42.0) | 104 (32.5) |
| Active - Placebo [1] CMH Difference (95% CI) p-Value | | 14.7<br>8.1 to 21.2<br><0.0001 | 5.1<br>-1.7 to 11.9<br>0.1389 |
| | | | |
| Patients with at Least 75% Reduction in Total Incontinence from Baseline to Week 12 | | | |
| Unadjusted n (%) | 141 (34.8) | 202 (50.1) | 144 (45.1) |
| Adjusted n (%) | 127 (31.4) | 191 (47.3) | 127 (39.9) |

(continued)

| Statistic | Placebo (N=405) | Vibegron (N=403) | Tolterodine (N=319) |
|---|---|---|---|
| Active - Placebo [1] CMH Difference (95% CI) p-Value | ---- | 15.8<br>9.1 to 22.6<br><0.0001 | 8.4<br>1.2 to 15.6<br>0.0222 |
| [1] The difference in proportion and corresponding CI and p-value was calculated using the Cochran-Mantel-Haenszel risk difference estimate stratified by Sex (Female vs Male), with weights proposed by Greenland and Robins.<br>MI has been used to impute values missing for any reason for the weeks analyzed.<br>Presented frequencies and the denominator used for percentages are based on patients in the FAS-I and randomized treatment. | | | |

Table 53. Change from Baseline in Average Daily Number of Urgency Episodes in 50% Total Incontinence Responders

| Total Incontinence | Placebo (N=218) | Vibegron (N=258) | Tolterodine (N=212) |
|---|---|---|---|
| Week 2 | | | |
| Baseline Daily Episodes, n Mean (SD) | 218<br>7.47 (4.176) | 258<br>7.65 (3.973) | 212<br>7.75 (3.839) |
| Week 2, n Mean (SD) | 212<br>5.80 (4.176) | 249<br>5.62 (3.792) | 205<br>5.68 (3.690) |
| Change from baseline, n LS Mean (SE) 95% CI | 212<br>-1.7 (0.16)<br>-2.0 to -1.4 | 249<br>-2.1 (0.15)<br>-2.4 to -1.8 | 205<br>-2.1 (0.17)<br>-2.4 to -1.8 |
| Week 4 | | | |
| Baseline Daily Episodes, n Mean (SD) | 218 7.47 (4.176) | 258 7.65 (3.973) | 212 7.75 (3.839) |
| Week 4, n Mean (SD) | 195<br>5.55 (4.243) | 248<br>5.01 (3.889) | 192<br>5.18 (3.663) |
| Change from baseline, n LS Mean (SE) 95% CI | 195<br>-1.9 (0.18)<br>-2.3 to -1.5 | 248<br>-2.6 (0.16)<br>-3.0 to -2.3 | 192<br>-2.6 (0.18)<br>-2.9 to -2.2 |
| Week 8 | | | |
| Baseline Daily Episodes, n Mean (SD) | 218<br>7.47 (4.176) | 258<br>7.65 (3.973) | 212<br>7.75 (3.839) |
| Week 8, n Mean (SD) | 200<br>4.55 (3.870) | 247<br>4.25 (3.833) | 191<br>4.68 (3.707) |
| Change from baseline, n LS Mean (SE) 95% CI | 200<br>-2.9 (0.21)<br>-3.3 to -2.5 | 247<br>-3.4 (0.19)<br>-3.8 to -3.0 | 191<br>-3.1 (0.21)<br>-3.5 to -2.7 |
| Week 12 | | | |
| Baseline Daily Episodes, n Mean (SD) | 218<br>7.47 (4.176) | 258<br>7.65 (3.973) | 212<br>7.75 (3.839) |
| Week 12, n Mean (SD) | 192<br>3.79 (3.736) | 239<br>3.67 (3.481) | 183<br>4.02 (3.539) |

(continued)

| | | | |
|---|---|---|---|
| Week 12 | | | |
| Change from baseline, n LS Mean (SE) 95% CI | 192<br>-3.6 (0.22)<br>-4.0 to -3.2 | 239<br>-4.0 (0.20)<br>-4.4 to -3.6 | 183<br>-3.6 (0.22)<br>-4.1 to -3.2 |
| Notes: - Only subjects that are considered 50% Total Incontinence Responders at Week 12 based on unadjusted imputed values are included in these analyses. - Covariates included in the mixed model for repeated measures are study visit, baseline number of urgency episodes and treatment by study visit interaction. | | | |

Table 54. Change from Baseline in OAB-q Coping Score for in 50% Total Incontinence Responders (Change from Baseline to Week 12)

| Statistic | Placebo N=218 | Vibegron N=258 | Tolterodine N=212 |
|---|---|---|---|
| Coping Subscore | | | |
| Baseline<br>n<br>Mean (SD) | 217<br>61.23 (25.913) | 256<br>59.97 (25.389) | 212<br>60.84 (25.204) |
| Week 12<br>n<br>Mean | 210<br>79.90 (22.559) | 249<br>83.08 (18.924) | 199<br>81.66 (20.882) |
| Change from baseline at week 12<br>n<br>LS Means (SE) 95% CI | 210<br>17.0 (1.45)<br>14.1 to 19.8 | 249<br>20.3 (1.35)<br>17.7 to 23.0 | 199<br>18.5 (1.49)<br>15.6 to 21.5 |
| Notes: Only subjects that are considered 50% Total Incontinence Responders at Week 12 based on unadjusted imputed values are included in these analyses.<br>Covariates included in the ANCOVA model are region and baseline score. | | | |

Table 55 Change from Baseline in OAB-q Symptom Bother Score for in 50% Total Incontinence Responders (Change from Baseline to Week 12)

| Statistic | Placebo N=218 | Vibegron N=258 | Tolterodine N=212 |
|---|---|---|---|
| Symptom Bother Subscore | | | |
| Baseline n Mean (SD) | 217<br>48.49 (19.100) | 256<br>48.81 (19.153) | 212<br>48.20 (19.529) |
| Week 12 n Mean | 210<br>27.58 (19.304) | 249<br>21.18 (15.324) | 199<br>24.70 (18.107) |
| Change from baseline at week 12 n LS Means (SE) 95% CI | 210<br>-19.1 (1.33)<br>-21.7 to -16.4 | 249<br>-25.5 (1.24)<br>-27.9 to -23.0 | 199<br>-21.7 (1.37)<br>-24.4 to -19.0 |
| Notes: Only subjects that are considered 50% Total Incontinence Responders at Week 12 based on unadjusted imputed values are included in these analyses.<br>Covariates included in the ANCOVA model are region and baseline score. | | | |

Table 56. Change from Baseline in Average Volume Voided per Micturition in 50% Total Incontinence Responders (Change from Baseline to Week 12)

| Statistic | Placebo | Vibegron | Tolterodine |
|---|---|---|---|
| Week 2 | | | |
| Baseline Daily Episodes, n Mean (SD) | 216<br>149.1 (63.76) | 256<br>161.5 (66.00) | 212<br>153.4 (64.23) |
| n Mean (SD) | 210<br>149.5 (68.40) | 244<br>173.8 (76.89) | 199<br>167.1 (75.27) |
| Change from baseline, n LS Mean 95% CI | 210<br>0.9 (4.01)<br>-7.0 to 8.7 | 244<br>16.1 (3.79)<br>8.7 to 23.6 | 199<br>16.9 (4.12)<br>8.8 to 25.0 |
| Week 4 | | | |
| Baseline Daily Episodes, n Mean (SD) | 216<br>149.1 (63.76) | 256<br>161.5 (66.00) | 212<br>153.4 (64.23) |
| n Mean (SD) | 161<br>151.3 (74.85) | 207<br>176.5 (81.31) | 164<br>171.3 (73.88) |
| Change from baseline, n LS Mean 95% CI | 161<br>4.2 (4.33)<br>-4.3 to 12.7 | 207<br>18.0 (3.99)<br>10.1 to 25.8 | 164<br>16.3 (4.38)<br>7.7 to 24.9 |
| Week 8 | | | |
| Baseline Daily Episodes, n Mean (SD) | 216<br>149.1 (63.76) | 256<br>161.5 (66.00) | 212<br>153.4 (64.23) |
| n Mean (SD) | 202<br>147.8 (70.61) | 245<br>182.0 (84.53) | 192<br>177.7 (76.78) |
| Change from baseline, n LS Mean 95% CI | 202<br>-0.4 (4.43)<br>-9.1 to 8.3 | 245<br>24.2 (4.12)<br>16.1 to 32.3 | 192<br>24.8 (4.56)<br>15.9 to 33.8 |
| Week 12 | | | |
| Baseline Daily Episodes, n Mean (SD) | 216<br>149.1 (63.76) | 256<br>161.5 (66.00) | 212<br>153.4 (64.23) |
| n Mean (SD) | 198<br>149.3 (74.75) | 238<br>(186.2 (84.24) | 181<br>175.4 (77.58) |
| Change from baseline, n LS Mean 95% CI | 198<br>0.6 (4.56)<br>-8.4 to 9.5 | 238<br>27.6 (4.24)<br>19.3 to 35.9 | 181<br>21.1 (4.74)<br>11.8 to 30.4 |
| Notes: Only subjects that are considered 50% Total Incontinence Responders at Week 12 based on unadjusted imputed values are included in these analyses.<br>Covariates included in the mixed model for repeated measures are study visit, region, baseline average volume voided and treatment by study visit interaction. | | | |

Change in dry diary days in 50% total incontinence responders is shown in Table 57.

Table 57. Change from Percent Dry Diary Days in 50% Total Incontinence Responders (Change from Baseline to Week 12)

| Statistic | Placebo (N=218) | Vibegron (N=258) | Tolterodine (N=212) |
|---|---|---|---|
| Week 2 | | | |
| Baseline Daily Episodes, n Mean (SD) | 218<br>6.41 (15.700) | 258<br>5.56 (14.069) | 212<br>4.02 (11.220) |
| n Mean (SD) | 217<br>29.79 (35.720) | 255<br>34.64 (36.322) | 208<br>28.44 (35.758) |
| Change from baseline, n LS Mean 95% CI | 217<br>23.5 (2.29)<br>19.0 to 28.0 | 255<br>29.0 (2.11)<br>24.9 to 33.1 | 208<br>23.9 (2.33)<br>19.4 to 28.5 |
| Week 4 | | | |
| Baseline Daily Episodes, n Mean (SD) | 218<br>6.41 (15.700) | 258<br>5.56 (14.069) | 212<br>4.02 (11.220) |
| n Mean (SD) | 204<br>37.50 (38.901) | 257<br>43.31 (41.468) | 202<br>36.82 (40.267) |
| Change from baseline, n LS Mean 95% CI | 204<br>31.0 (2.67)<br>25.8 to 36.2 | 257<br>37.8 (2.41)<br>33.0 to 42.5 | 202<br>32.6 (2.69)<br>27.4 to 37.9 |
| Week 8 | | | |
| Baseline Daily Episodes, n Mean (SD) | 218<br>6.41 (15.700) | 258<br>5.56 (14.069) | 212<br>4.02 (11.220) |
| n Mean (SD) | 200<br>45.13 (14.008) | 247<br>53.01 (39.898) | 191<br>44.86 (40.529) |
| Change from baseline, n LS Mean 95% CI | 200<br>38.4 (2.71)<br>33.1 to 43.7 | 247<br>47.4 (2.45)<br>42.6 to 52.2 | 191<br>40.0 (2.76)<br>34.5 to 45.4 |
| Week 12 | | | |
| Baseline Daily Episodes, n Mean (SD) | 218<br>6.41 (15.700) | 258<br>5.56 (14.069) | 212<br>4.02 (11.220) |
| n Mean (SD) | 193<br>55.03 (40.822) | 239<br>61.0 (37.857) | 183<br>55.43 (37.734) |
| Change from baseline, n LS Mean 95% CI | 193<br>48.7 (2.62)<br>43.6 to 53.9 | 239<br>55.5 (2.37)<br>50.8 to 60.1 | 183<br>49.7 (2.68)<br>44.4 to 55.0 |
| Notes: Only subjects that are considered 50% Total Incontinence Responders at Week 12 based on unadjusted imputed values are included in these analyses.<br>Covariates included in the mixed model for repeated measures are study visit, baseline percent of dry diary days and treatment by study visit interaction. | | | |

Table 58 and Figure 10 show changes to average volume voided per micturition.

Table 58. Change from Baseline in Average Volume Voided per Micturition (mL) (CFB Least squares means at weeks 2, 4, 8, and 12)

| Volume Voided | Placebo | Vibegron | Tolterodine |
|---|---|---|---|
| Week 2 | | | |
| Baseline, n Mean (SD) | 514<br>148.3 (60.67) | 524<br>155.4 (63.07) | 415<br>147.0 (60.79) |
| Week 2, n Mean (SD) | 492<br>148.2 (63.83) | 497<br>165.6 (74.26) | 398<br>156.7 (71.23) |
| Change from baseline, n LS Mean (SE) 95% CI | 492<br>2.3 (3.02)<br>-3.6 to 8.2 | 497<br>14.2 (3.02)<br>8.3 to 20.1 | 398<br>12.5 (3.20)<br>6.3 to 18.8 |
| Active - Placebo LS Mean Difference (SE) 95% CI P-Value | | 11.9 (3.05)<br>5.9 to 17.9<br><0.001 | 10.2 (3.24)<br>3.9 to 16.6<br>0.0016 |
| Week 4 | | | |
| Baseline, n Mean (SD) | 514<br>148.3 (60.67) | 524<br>155.4 (63.07) | 415<br>147.0 (60.79) |
| Week 4, n Mean (SD) | 382<br>149.2 (68.47) | 399<br>172.6 (76.57) | 304<br>160.2 (72.76) |
| Change from baseline, n LS Mean (SE) 95% CI | 382<br>4.4 (3.18)<br>-1.8 to 10.6 | 399<br>18.9 (3.15)<br>12.7 to 25.1 | 304<br>13.3 (3.39)<br>6.7 to 20.0 |
| Active - Placebo LS Mean Difference (SE) 95% CI P-Value | | 14.5 (3.32)<br>8.0 to 21.0<br><0.0001 | 8.9 (3.55)<br>2.0 to 15.9<br>0.0118 |
| Week 8 | | | |
| Baseline, n Mean (SD) | 514<br>148.3 (60.67) | 524<br>155.4 (63.07) | 415<br>147.0 (60.79) |
| Week 8, n Mean (SD) | 489<br>149.9 (69.12) | 503<br>173.4 (81.95) | 390<br>162.9 (74.46) |
| Change from baseline, n LS Mean (SE) 95% CI | 489<br>3.1 (3.22)<br>-3.2 to 9.4 | 503<br>21.4 (3.20)<br>15.1 to 27.7 | 390<br>17.5 (3.44)<br>10.7 to 24.2 |
| Active - Placebo LS Mean Difference (SE) 95% CI P-Value | | 18.3 (3.41)<br>11.6 to 25.0<br><0.0001 | 14.4 (3.64)<br>7.2 to 21.5<br><0.0001 |
| Week 12 | | | |
| Baseline, n Mean (SD) | 514<br>148.3 (60.67) | 524<br>155.4 (63.07) | 415<br>147.0 (60.79) |
| Week 12, n Mean (SD) | 478<br>149.01 (69.42) | 490<br>175.3 (81.78) | 375<br>162.1 (72.96) |
| Change from baseline, n LS Mean (SE) 95% CI | 478<br>2.2 (3.28)<br>-4.2 to 8.7 | 490<br>23.5 (3.26)<br>17.1 to 29.9 | 375<br>15.5 (3.52)<br>8.6 to 22.4 |

(continued)

| Week 12 | | | |
|---|---|---|---|
| Active - Placebo LS Mean Difference (SE) 95% CI P-Value | - | 21.2 (3.52)<br>14.3 to 28.1<br><0.0001 | 13.3 (3.76)<br>5.9 to 20.7<br><0.001 |

The patients treated with vibegron also showed improvement in OAB-q coping score (see Table 59). Vibegron demonstrated statistically significant improvements on the Bother (p<0.0001), Concern (p<0.0001), Sleep (p<0.001), and Coping (p=0.0038) scales and the total HRQL score (p<0.001) compared to placebo at week 12 (see Table 60). The LS mean difference between vibegron and placebo was numerically improved for the HRQL Social Interaction subscale but did not reach statistical significance (*P*=0.1116). Vibegron results were numerically better than tolterodine results for all OAB-q scales measured. These patients reported QoL improvements observed after treatment with vibegron that paralleled the significant improvements seen in the co-primary efficacy endpoints of change from baseline in average number of micturitions and UUI episodes compared to placebo.

Table 59. Change from Baseline in OAB-q Coping Score (CFB Least squares means at week 12)

| OAB-q Coping Score | Placebo N=520 | Vibegron N=526 | Tolterodine N=417 |
|---|---|---|---|
| Baseline, n Mean (SD) | 518<br>58.77 (27.169) | 524<br>57.60 (28.054) | 417<br>59.88 (26.470) |
| Coping Subscore, n Week 12, Mean (SD) | 504<br>74.06 (24.416) | 512<br>77.26 (23.242) | 401<br>77.77 (23.439) |
| Change from baseline, n LS Means (SE) 95% CI | 504<br>12.9 (1.32)<br>10.3 to 15.5 | 512<br>16.5 (1.31)<br>13.9 to 19.1 | 401<br>15.9 (1.39)<br>13.2 to 18.7 |
| Active - Placebo LS Means Difference (SE) 95% CI P-Value | - | 3.6 (1.24)<br>1.2 to 6.0<br>0.0038 | 3.1 (1.32)<br>0.5 to 5.6<br>0.0212 |
| Higher scores correspond to a higher quality of life on the OAB-q Coping Score.<br>Covariates included in the mixed model for repeated measures are study visit, OAB type, sex, region, baseline OAB-q coping score and treatment by study visit interaction. | | | |

Table 60. Change from Baseline in OAB-q Scores (CFB Least squares means at week 12)

| OAB-q Scale | Placebo N=520 | Vibegron N=526 | Tolterodine N=417 |
|---|---|---|---|
| Concern Subscore Baseline, n Mean (SD) | 518<br>63.54 (25.890) | 524<br>61.89 (27.427) | 417<br>63.80 (26.063) |
| Concern Week 12, n Mean (SD) | 504<br>76.10 (23.464) | 512<br>80.58 (21.867) | 401<br>80.93 (21.467) |
| Change from baseline, n Concern LS Means (SE) 95% CI | 504<br>10.3 (1.25)<br>7.8 to 12.7 | 512<br>15.3 (1.24)<br>12.8 to 17.7 | 401<br>14.7 (1.32)<br>12.1 to 17.3 |

(continued)

| OAB-q Scale | Placebo N=520 | Vibegron N=526 | Tolterodine N=417 |
|---|---|---|---|
| Active - Placebo Concern LS Means Difference (SE) 95% CI P-Value | - - - - - | 5.0 (1.18)<br>2.7 to 7.3<br><0.0001 | 4.4 (1.25)<br>2.0 to 6.9<br><0.001 |
| Sleep Subscore Baseline, n Mean (SD) | 518<br>57.15 (26.429) | 524<br>58.05 (27.917) | 417<br>59.68 (25.236) |
| Sleep Week 12, n Mean (SD) | 504<br>70.35 (25.019) | 512<br>75.59 (23.883) | 401<br>74.25 (23.140) |
| Change from baseline, n | 504 | 512 | 401 |
| Sleep LS Means (SE) 95% CI | 10.3 (1.35)<br>7.7 to 13.0 | 14.9 (1.34)<br>12.2 to 17.5 | 12.9 (1.42)<br>10.1 to 15.7 |
| Active - Placebo Sleep LS Means Difference (SE) 95% CI P-Value | - | 4.5 (1.27)<br>2.0 to 7.0<br><0.001 | 2.6 (1.36)<br>-0.1 to 5.2<br>0.0596 |
| Social Interaction Subscore Baseline, n Mean (SD) | 518<br>78.78 (25.049) | 524<br>76.82 (26.026) | 417<br>78.34 (24.721) |
| Social Interaction Week 12, n Mean (SD) | 504<br>87.74 (18.191) | 512<br>88.57 (18.011) | 401<br>88.35 (18.214) |
| Change from baseline, n Social Interaction LS Means (SE) 95% CI | 504<br>8.7 (0.97)<br>6.8 to 10.6 | 512<br>10.2 (0.97)<br>8.3 to 12.1 | 401<br>9.4 (1.03)<br>7.3 to 11.4 |
| Active - Placebo Social Interaction LS Means Difference (SE) 95% CI P-Value | - | 1.5 (0.92)<br>-0.3 to 3.3<br>0.1116 | 0.6 (0.98)<br>-1.3 to 2.5<br>0.5237 |
| Symptom Bother Subscore Baseline, n Mean (SD) | 518<br>50.07 (20.642) | 524<br>49.63 (21.912) | 416<br>47.97 (20.552) |
| Symptom Bother Week 12, n Mean (SD) | 504<br>34.92 (22.126) | 512<br>27.78 (19.816) | 400<br>29.33 (19.568) |
| Change from baseline, n Symptom Bother LS Means (SE) 95% CI | 504<br>-12.7 (1.25)<br>-15.2 to -10.3 | 512<br>-19.6 (1.24)<br>-22.1 to -17.2 | 400<br>-17.4 (1.31)<br>-19.9 to -14.8 |
| Active - Placebo Symptom Bother LS Means Difference (SE) 95% CI P-Value | - | -6.9 (1.17)<br>-9.2 to -4.6<br><0.0001 | -4.6 (1.25)<br>-7.1 to -2.2<br><0.001 |

(continued)

| OAB-q Scale | Placebo N=520 | Vibegron N=526 | Tolterodine N=417 |
|---|---|---|---|
| Total HRQL Score Baseline, n Mean (SD) | 518<br>63.78 (23.509) | 524<br>62.73 (24.910) | 417<br>64.63 (22.947) |
| Total HRQL Score Week 12, n Mean (SD) | 504<br>76.63 (21.065) | 512<br>80.12 (20.175) | 401<br>80.07 (19.826) |
| Change from baseline, n HRQL Total Score LS Means (SE) 95% CI | 504<br>10.8 (1.13)<br>8.6 to 13.0 | 512<br>14.6 (1.12)<br>12.4 to 16.8 | 401<br>13.6 (1.19)<br>11.3 to 16.0 |
| Active - Placebo HRQL Total Score LS Means Difference (SE) 95% CI P-Value | - | 3.8 (1.06)<br>1.7 to 5.9<br><0.001 | 2.9 (1.13)<br>0.6 to 5.1<br>0.0117 |
| Covariates included in the mixed model for repeated measures are study visit, OAB type, sex, region, baseline OAB-q coping score and treatment by study visit interaction.<br>For Symptom Bother, higher scores correspond to the symptoms having a larger bother. For other scores, higher values correspond to higher quality of life, and lower scores represent a lower amount of bother due to symptoms. | | | |

Table 61 shows the OAB-q proportion of responders at week 12. With the exception of the symptom bother score, a responder was defined as having a change from baseline ≥10. For symptom bother score, a responder was defined as having a change from baseline ≤10. A higher proportion of patients represented a favorable response.

As shown in the table, odds ratios were statistically significant in the comparison of vibegron to placebo for symptom bother and coping subscale.

Table 61. OAB-q Proportion of Responders at Week 12 (Full Analysis Set)

| Scale | Statistic | Placebo (N=520) | Vibegron (N=526) | Tolterodine (N=417) |
|---|---|---|---|---|
| | n | 504 | 512 | 400 |
| Total HRQL | Week 12, n (%) | 245 (48.6) | 277 (54.1) | 207 (51.8) |
| | Difference from Placebo (95% CI) [1] | | 5.5 (-0.6, 11.6) | 3.1 (-3.4, 9.7) |
| | Odds Ratio (95% CI) [2]<br>P-Value | | 1.3 (1.0, 1.7)<br>0.0548 | 1.3 (0.9, 1.7)<br>0.1318 |
| | | | | |
| Symptom Bother | Week 12, n (%) | 294 (58.3) | 359 (70.1) | 266 (66.5) |
| | Difference from Placebo (95% CI) [2] | | 11.8 (5.9, 17.6) | 8.2 (1.8, 14.5) |
| | Odds Ratio (95% CI)<br>P-Value | | 1.9 (1.4, 2.5)<br><0.0001 | 1.6 (1.2, 2.2)<br>0.0009 |
| | | | | |
| Concern | Week 12, n (%) | 251 (49.8) | 285 (55.7) | 228 (57.0) |
| | Difference from Placebo (95% CI) [1] | | 5.9 (-0.3, 12.0) | 7.2 (0.7, 13.7) |
| | Odds Ratio (95% CI) [2]<br>P-Value | | 1.3 (1.0, 1.8)<br>0.0507 | 1.6 (1.1, 2.1)<br>0.0051 |
| | | | | |
| Coping | Week 12, n (%) | 274 (54.4) | 317 (61.9) | 245 (61.3) |

(continued)

| Scale | Statistic | Placebo (N=520) | Vibegron (N=526) | Tolterodine (N=417) |
|---|---|---|---|---|
| | Difference from Placebo (95% CI) [1] | | 7.5 (1.5, 13.6) | 6.9 (0.4, 13.3) |
| | Odds Ratio (95% CI) [2]<br>P-Value | | 1.4 (1.1, 1.9)<br>0.0105 | 1.5 (1.1, 2.0)<br>0.0092 |
| | | | | |
| Sleep | Week 12, n (%) | 261 (51.8) | 276 (53.9) | 205 (51.3) |
| | Difference from Placebo (95% CI) [1] | | 2.1 (-4.0, 8.3) | -0.5 (-7.1, 6.0) |
| | Odds Ratio (95% CI) [2]<br>P-Value | | 1.2 (0.9, 1.6)<br>0.1933 | 1.1 (0.8, 1.5)<br>0.5334 |
| | | | | |
| Social Interaction | Week 12, n (%) | 177 (35.2) | 208 (40.6) | 150 (37.4) |
| | Difference from Placebo (95% CI) [1] | | 5.4 (-0.5, 11.4) | 2.2 (-4.1, 8.5) |
| | Odds Ratio (95% CI) [2]<br>P-Value | | 1.3 (0.9, 1.9)<br>0.1034 | 1.2 (0.8, 1.7)<br>0.3072 |
| [1] 95% 2-Side CIs for the difference in proportion of responders are based on normal approximation.<br>[2] Odds Ratios, corresponding 95% CIs, and P-values are derived from a proc logistic regression model including treatment, sex, OAB Type, and baseline values.<br>Note: post-hoc analysis using Full Analysis Set; percentages are based on the number of subjects with non-missing baseline and week 12 values. | | | | |

The comparison of the percent of responders with 75% and 100% reductions in UUI episodes and 50% reduction in Urgency episodes is shown in Figure 8. The proportion of patients (UUI responders) that achieved a clinically meaningful (75% or more reduction) in daily UUI episodes was highest in the vibegron 75 mg group (49.3%) compared to placebo (32.8%) (p<0.001), and tolterodine (42.2%) at week 12. Vibegron demonstrated rapid onset of effect, with statistically significant more UUI responders by week 2 vs placebo (p<0.01).

Vibegron was well tolerated with very few adverse events >2% and greater than placebo. A summary of treatment emergent adverse events is shown in Table 62. The most common adverse events greater than 2% and greater than placebo were headache, nasopharyngitis, diarrhea, nausea (see Table 63). The data regarding other adverse events are listed in Table 64. Adverse events in age ≥75 subjects are shown in Table 65.

Table 62. Summary of Treatment Emergent Adverse Events

| Safety population n (%) | Placebo N= 540 | Vibegron N= 545 | Tolterodine N= 430 |
|---|---|---|---|
| Patients with at least one treatment emergent adverse events | 180 (33.3) | 211 (38.7) | 166 (38.6) |
| Any AE of clinical interest | 40 (7.4%) | 36 (6.6%) | 38 (8.8%) |
| Patients with at least one treatment emergent serious AEs | 6 (1.1) | 8 (1.5) | 10 (2.3) |
| - SAEs resulting in death | 0 | 0 | 1 (0.2) |
| - Hospitalization | 6 (1.1) | 7 (1.3) | 8 (1.9) |
| - other serious criteria | 0 | 2 (0.4) | 2 (0.5) |
| SAEs considered treatment- related by the investigator | 0 | 2 (0.4) | 0 |

Table 63. Comparison of Most Common Adverse Events (≥2% in Vibegron and > Placebo)

| AE term n (%) | Placebo N = 540 | Vibegron N= 545 | Tolterodine N= 430 |
|---|---|---|---|
| Headache | 13 (2.4) | 22 (4.0) | 11 (2.6) |
| Nasopharyngitis | 9 (1.7) | 15 (2.8) | 11 (2.6) |

(continued)

| AE term n (%) | Placebo N = 540 | Vibegron N= 545 | Tolterodine N= 430 |
|---|---|---|---|
| Diarrhea | 6 (1.1) | 12 (2.2) | 9(2.1) |
| Nausea | 6 (1.1) | 12 (2.2) | 5 (1.2) |

Table 64. Comparison of Other Adverse Events (≥2% in Vibegron and > Placebo Plus All AEs of Clinical Interest)

| AE term n (%) | Placebo N = 540 | Vibegron N= 545 | Tolterodine N= 430 |
|---|---|---|---|
| Urinary tract infection[b] | 33 (6.1) | 27 (5.0) | 25 (5.8) |
| Escherichia urinary-tract infection[b] | 0 | 0 | 1 (0.2%) |
| Urinary retention[b] | 2 (0.4) | 3 (0.6) | 3 (0.7) |
| Hypertension[b] | 9 (1.7) | 9 (1.7) | 11 (2.6) |
| Tachycardia | 0 | 0 | 1 (0.2) |
| Blood pressure increased[b] | 5 (0.9) | 4 (0.7) | 8 (1.9) |
| Blood pressure diastolic increased[b] | 0 | 0 | 1 (0.2%) |
| Hypotension | 1 (0.2) | 1 (0.2) | 1 (0.2) |
| Dizziness[b] | 6 (1.1) | 5 (0.9) | 4 (0.9) |
| Pollakiuria[b] | 1 (0.2%) | 0 | 0 |
| Syncope[b] | 2 (0.4) | 0 | 1 (0.2) |
| Vertigo | 0 | 1 (0.2) | 1 (0.2) |
| Positional vertigo | 1 (0.2) | 0 | 1 (0.2) |
| Atrial fibrillation | 1 (0.2) | 1 (0.2) | 1 (0.2) |
| Cerebrovascular accident | 0 | 1 (0.2) | 1 (0.2) |
| Chest discomfort | 1 (0.2) | 4 (0.7) | 0 |
| Chest pain | 3 (0.6) | 0 | 0 |
| Cardiac failure congestive[b] | 0 | 1 (0.2%) | 0 |
| Fatigue[b] | 5 (0.9) | 2 (0.4) | 6 (1.4) |
| Constipation[b] | 7 (1.3) | 9 (1.7) | 6 (1.4) |
| Dry mouth[b] | 5 (0.9) | 9 (1.7) | 28 (6.5) |
| Somnolence | 2 (0.4) | 2 (0.4) | 2 (0.5) |
| Hot flush | 2 (0.4) | 4 (0.7) | 1 (0.2) |
| Drug hypersensitivity | 1 (0.2) | 2 (0.4) | 0 |
| Hyperglycemia | 2 (0.4) | 3 (0.6) | 2 (0.5) |
| Type 2 diabetes mellitus | 0 | 2 (0.4) | 0 |
| Dysuria | 3 (0.6) | 0 | 0 |
| Cystitis | 1 (0.2) | 1 (0.2) | 1 (0.2) |
| Residual urine volume increased | 2 (0.4) | 4 (0.7) | 6 (1.4) |
| Chronic kidney disease | 1 (0.2) | 1 (0.2) | 0 |
| Blood creatinine increased | 1 (0.2) | 1 (0.2) | 1 (0.2) |
| Glomerular filtration rate decreased | 2 (0.4) | 1 (0.2) | 2 (0.5) |
| ALT increase[b] | 2 (0.4) | 1 (0.2) | 1 (0.2) |
| AST increase[b] | 1 (0.2) | 1 (0.2) | 1 (0.2) |

(continued)

| AE term n (%) | Placebo N = 540 | Vibegron N= 545 | Tolterodine N= 430 |
|---|---|---|---|
| Transaminases increased | 0 | 1 (0.2) | 0 |
| Bilirubin increased | 0 | 0 | 1 (0.2) |
| Hepatic enzyme increased | 1 (0.2) | 0 | 1 (0.2) |
| [b] AE of clinical interest | | | |

Table 65. Summary of Treatment Emergent Adverse Events in Patients ≥75 Years

| Safety population (%) | Placebo | Vibegron | Tolterodine |
|---|---|---|---|
| Overall AE rate | 40.0% | 49.3% | 50.0% |
| Most common AEs (>2% and >placebo) | | | |
| Back pain | 3.3% | 2.7% | 2.1% |
| Nausea | 3.3% | 2.7% | 0% |
| Urinary tract infection | 6.7% | 8.0% | 8.3% |
| Upper respiratory tract infection | 1.7% | 4.0% | 0% |
| Diarrhea | 3.3% | 4.0% | 4.2% |
| Dyspnea | 0% | 4.0% | 0% |
| Urinary Retention | 0% | 2.7% | 4.2% |
| Rash | 1.7% | 2.7% | 0% |
| Somnolence 0% | 2.7% | 2.1% | |

No differences from placebo in adverse events for hypertension, increases in blood pressure, and urinary tract infection were observed. Drop-out rates due to AEs were 1.1% Placebo, 1.5% Vibegron, and 3.0% Tolterodine. Patients with at least one Serious AE were Placebo (1.1%), Vibegron (1.5%), and Tolterodine (2.3%). Tachycardia rate of 0.2% was only reported in the Tolterodine treatment group.

The data on the vital signs of the subjects including heart rates and blood pressures are shown in Tables 66-74.

Table 66. Heart Rate Change from Baseline at Week 12

| Heart Rate (beats/minute) | Placebo N= 540 | Vibegron N= 545 | Tolterodine N= 430 |
|---|---|---|---|
| Mean Baseline (SD) | 71.6 (9.13) | 72.2 (9.08) | 72.5 (9.15) |
| Min, Max | 45, 102 | 45, 100 | 49, 100 |
| Mean Change from Baseline at Week 12 (SD) | -0.1 (8.16) | 0.5 (8.27) | 0.3 (8.69) |
| Min, Max | -28, 30 | -28, 29 | -31, 30 |

Table 67. Subpopulation Heart Rate Change from Baseline at Week 12

| Heart Rate (beats/minute) | Placebo | Vibegron | Tolterodine |
|---|---|---|---|
| Hypertensive at baseline[a] n at baseline | 56 | 44 | 47 |
| n at 12 weeks<br>12-week change (95%CI) | 54<br>+1.7 (-0.7, +4.0) | 40<br>+2.2 (-0.4, +4.8) | 47<br>+1.5 (-1.4, +4.4) |
| Normotensive at baseline[a] n at baseline | 484 | 501 | 383 |
| n at 12 weeks<br>12-week change (95%CI) | 439<br>-0.3 (-1.1, +0.4) | 463<br>+0.4 (-0.4, +1.1) | 346<br>+0.2 (-0.7, +1.1) |
| [a]Defined as average systolic BP ≥140 mmHg and/or diastolic BP ≥90 mmHg at 2 consecutive visits. | | | |

Table 68. Heart Rate Categorical Changes from Baseline at Week 12

| Heart Rate Categorical Changes from Baseline | Placebo N= 540 | Vibegron N= 545 | Tolterodine N= 430 |
|---|---|---|---|
| ≥ 5 bpm | 7.2% | 8.6% | 6.5% |
| ≥ 10 bpm | 1.5% | 1.8% | 2.1% |
| ≥ 15 bpm | 0.4% | 0.4% | 0.5% |
| bpm= beats/minute<br>Categorical changes are based on 3 consecutive post-baseline visits and patients are counted in all applicable categories (e.g., if a patient has a 12 unit increase then that patient is counted in both ≥ 5 and ≥ 10 unit columns). | | | |

Table 69. Systolic Blood Pressure Change from Baseline at Week 12

| Systolic BP (mm Hg) | Placebo N= 539 | Vibegron N= 545 | Tolterodine N= 430 |
|---|---|---|---|
| Baseline Mean (SD) | 123.3 (12.35) | 124.2 (11.93) | 124.0 (12.14) |
| Min, Max | 89, 164 | 94, 171 | 87, 157 |
| n at 12 weeks Mean Change from Baseline at Week 12 (SD) | 493<br>-0.9 (10.56) | 503<br>0.2 (11.89) | 388<br>0.1 (10.74) |
| Min, Max | -45, 36 | -52, 48 | -36, 62 |
| Blood Pressure assessments are taken in triplicate and averaged at each visit. | | | |

Table 70. Subpopulation Systolic Blood Pressure Change from Baseline at Week 12

| Systolic BP (mm Hg) | Placebo | Vibegron | Tolterodine |
|---|---|---|---|
| Hypertensive at baseline[a] n at baseline | 56 | 44 | 47 |
| n at 12 weeks<br>12-week change (95% CI) | 54<br>-12.6 (-15.8, -9.3) | 40<br>-9.1 (-13.8, -4.4) | 42<br>-7.6 (-10.9, -4.3) |
| Normotensive at baseline n at baseline | 484 | 501 | 383 |
| n at 12 weeks<br>12-week change (95% CI) | 439<br>+0.5 (-0.3, +1.4) | 463<br>+1.0 (0, +2.0) | 346<br>+1.0 (-0.1, +2.1) |
| Blood Pressure assessments are taken in triplicate and averaged at each visit.<br>[a]Defined as average systolic BP≥ 140 mmHg and/or diastolic BP ≥90 mmHg at 2 consecutive visits | | | |

Table 71. Systolic Blood Pressure Categorical Changes from Baseline at Week 12

| Systolic Blood Pressure Categorical Changes from Baseline | Placebo N= 540 | Vibegron N= 545 | Tolterodine N= 430 |
|---|---|---|---|
| ≥ 5 mm Hg | 8.0% | 11.2% | 9.3% |
| ≥ 10 mm Hg | 3.0% | 4.2% | 4.7% |
| ≥ 15 mm Hg | 0.7% | 1.3% | 2.1% |
| Blood Pressure assessments were taken in triplicate and averaged at each visit.<br>Categorical changes are based on 3 consecutive post-baseline visits and patients are counted in all applicable categories (e.g., if a patient has a 12 unit increase then that patient is counted in both ≥ 5 and ≥ 10 unit columns). | | | |

Table 72. Diastolic Blood Pressure Change from Baseline at Week 12

| Diastolic BP (mm Hg) | Placebo N= 540 | Vibegron N= 545 | Tolterodine N= 430 |
|---|---|---|---|
| Baseline Mean (SD) | 75.7 (8.15) | 76.0 (7.81) | 76.2 (7.89) |

(continued)

| Diastolic BP (mm Hg) | Placebo N= 540 | Vibegron N= 545 | Tolterodine N= 430 |
|---|---|---|---|
| Min, Max | 53, 98 | 53, 104 | 41, 98 |
| n at 12 weeks Mean Change from Baseline at Week 12 (SD) | 493 -0.6 (7.47) | 503 0.2 (7.43) | 388 0.3 (7.11) |
| Min, Max | -23, 30 | -22, 26 | -22, 24 |
| Blood Pressure assessments were taken in triplicate and averaged at each visit. | | | |

Table 73. Subpopulation Diastolic Blood Pressure Change from Baseline at Week 12

| Diastolic BP (mm Hg) | Placebo | Vibegron | Tolterodine |
|---|---|---|---|
| Hypertensive at baseline[a] | 56 | 44 | 47 |
| n at baseline | | | |
| n at 12 weeks<br>12-week change (95% CI) | 54<br>-6.1 (-8.3, -4.0) | 40<br>-3.2 (-6.3, -0.1) | 42<br>-1.3 (-3.8, +1.3) |
| Normotensive at baseline n at baseline | 484 | 501 | 383 |
| n at 12 weeks<br>12-week change (95% CI) | 439<br>+0.1 (-0.6, +0.8) | 463<br>+0.5 (-0.2, +1.1) | 346<br>+0.5 (-0.2, +1.3) |
| Blood Pressure assessments are taken in triplicate and averaged at each visit.<br>[a]Defined as average systolic BP≥ 140 mmHg and/or diastolic BP ≥90 mmHg at 2 consecutive visits | | | |

Table 74. Diastolic Blood Pressure Categorical Changes from Baseline at Week 12

| Diastolic Blood Pressure Categorical Changes from Baseline | Placebo N= 540 | Vibegron N= 545 | Tolterodine N= 430 |
|---|---|---|---|
| ≥ 5 mm Hg | 5.2% | 7.0% | 7.4% |
| ≥ 10 mm Hg | 1.1% | 1.3% | 2.1% |
| ≥ 15 mm Hg | 0 | 0.2% | 0.7% |
| Blood Pressure assessments were taken in triplicate and averaged at each visit.<br>Categorical changes are based on 3 consecutive post-baseline visits and patients are counted in all applicable categories (e.g., if a patient has a 12 unit increase then that patient is counted in both ≥ 5 and ≥ 10 unit columns). | | | |

The data on the Post Void Residual Urine Volume (PVR) in all subjects and subpopulations are shown in Tables 75-77.

Table 75. Post Void Residual Urine Volume (PVR) Change from Baseline to Week 12

| PVR (mL) | Placebo N= 540 | Vibegron N= 545 | Tolterodine N= 430 |
|---|---|---|---|
| Mean Baseline (SD)* | 27.1 (31.05) | 28.8 (32.49) | 27.9 (37.94) |
| Mean Change from Baseline at Week 12 (SD) | 2.1 (37.25) | 0.4 (38.27) | 3.1 (40.93) |
| PVR Categories at Week 12 | | | |
| < 100 mL | 89.1% | 89.9% | 86.5% |
| ≥ 100 mL to < 200 mL | 3.7% | 3.5% | 5.8% |
| ≥ 200 mL to < 350 mL | 0.6% | 0.6% | 0.7% |

(continued)

| PVR Categories at Week 12 | | | |
|---|---|---|---|
| ≥ 350 mL | 0 | 0 | 0 |
| Note that the protocol required the baseline PVR to be below 150 mL<br>* n's for each treatment group at baseline are 539, 544 and 430 patients for placebo, vibegron and tolterodine, respectively | | | |

Table 76. Change in Post Void Residual Urine Volume (PVR) in Females from Baseline to Week 12

| PVR (mL) in Females | Placebo N= 459 | Vibegron N= 463 | Tolterodine N= 364 |
|---|---|---|---|
| Mean Baseline (SD)* | 27.0 (31.35) | 27.0 (31.29) | 27.2 (38.77) |
| Mean Change from Baseline at Week 12 (SD) | 0.2 (35.75) | 0.1 (36.19) | 1.9 (40.24) |
| PVR Categories at Week 12 | | | |
| < 100 mL | 96.0% | 96.8% | 94.4% |
| ≥ 100 mL to < 200 mL | 3.5% | 3.0% | 5.3% |
| ≥ 200 mL to < 350 mL | 0.5% | 0.2% | 0.3% |
| ≥ 350 mL | 0 | 0 | 0 |
| Note that the protocol required the baseline PVR to be below 150 mL<br>* n's for each treatment group at baseline are 459, 462 and 364 patients for placebo, vibegron and tolterodine, respectively | | | |

Table 77. Change in Post Void Residual Urine Volume (PVR) in Males from Baseline to Week 12

| PVR (mL) in Males | Placebo N= 81 | Vibegron N= 82 | Tolterodine N= 66 |
|---|---|---|---|
| Mean Baseline (SD)* | 27.8 (29.47) | 38.8 (37.20) | 31.5 (33.00) |
| Mean Change from Baseline at Week 12 (SD) | 13.1 (43.63) | 2.2 (48.45) | 10.2 (44.24) |
| PVR Categories at Week 12 | | | |
| < 100 mL | 92.0% | 89.7% | 85.2% |
| ≥ 100 mL to < 200 mL | 6.7% | 7.7% | 11.5% |
| ≥ 200 mL to < 350 mL | 1.3% | 2.6% | 3.3% |
| ≥ 350 mL | 0 | 0 | 0 |
| Note that the protocol required the baseline PVR to be below 150 mL<br>* n's for each treatment group at baseline are 80, 82 and 66 patients for placebo, vibegron and tolterodine, respectively | | | |

Table 78. Change in Post Void Residual Urine Volume (PVR) in Males with Medical History of BPH from Baseline to Week 12

| PVR (mL) in Males with BPH | Placebo N= 17 | Vibegron N= 29 | Tolterodine N= 22 |
|---|---|---|---|
| Mean Baseline (SD) | 27.3 (26.73) | 35.3 (33.57) | 41.0 (43.17) |
| Mean Change from Baseline at Week 12 (SD) | 28.6 (62.36) | -3.4 (42.45) | 3.7 (50.64) |
| PVR Categories at Week 12 | | | |
| < 100 mL | 82.4% | 96.6% | 71.4% |
| ≥ 100 mL to < 200 mL | 11.8% | 0 | 28.6% |
| ≥ 200 mL to < 350 mL | 5.9% | 3.4% | 0 |
| ≥ 350 mL | 0 | 0 | 0 |
| Note that the protocol required the baseline PVR to be below 150 mL | | | |

Overall, vibegron demonstrated strong efficacy across all OAB endpoints. (See Table 79)

Table 79. Week 12 LS Mean Change from Baseline (Placebo-Adjusted)

| Endpoint | Vibegron | n | P-Value | Tolterodine | n | P-Value |
|---|---|---|---|---|---|---|
| **UUI Episodes[1]** | **-0.6** | **383** | **<0.0001** | **-0.4** | **286** | **0.0123** |
| **Micturitions[1]** | **-0.5** | **492** | **<0.001** | **-0.3** | **378** | **0.0988** |
| Urgency Episodes[2] | -0.7 | 492 | 0.0020 | -0.4 | 378 | 0.0648 |
| Total Incontinence Episodes[2] | -0.7 | 383 | <0.0001 | -0.5 | 286 | 0.0074 |
| Volume Voided (ml)[2] | 21.2 | 490 | <0.0001 | 13.3 | 375 | <0.001 |
| OAB-q Coping Score[2] | 3.6 | 512 | 0.0038 | 3.1 | 401 | 0.0212 |
| 1. Co-primary endpoint; 2. Secondary Endpoint; LS=Least Squares. | | | | | | |

Once daily vibegron 75 mg demonstrated significantly better efficacy compared with placebo across three secondary endpoints (as well as two co-primary endpoints), indicating further benefit of vibegron for patients with UUI. Vibegron 75 mg demonstrated statistically significantly reductions in total incontinence episodes by week 2 and maintained this significance throughout the 12-week treatment period. Vibegron 75 mg demonstrated a clear increase in volume voided per micturition, a relatively objective measure increasing bladder capacity. Total incontinence episodes were reduced by half in the vibegron group vs baseline, and almost half of vibegron-treated patients with UUI at baseline had at least 75% reductions in UUI episodes after 12 weeks of vibegron therapy. Vibegron tolerability was favorable, with very few adverse events >2% and greater than placebo.

In addition, once daily vibegron 75mg demonstrated statistically significant improvements in the OAB-q Coping, Concern, Sleep and Symptom Bother scales and the total HRQL score compared to placebo at week 12. The results shown above demonstrate that 75 mg of vibegron can objectively improve the symptoms of OAB (frequency, urgency, UUI) and increase the QoL of patients suffering from OAB.

Example 7

**A Double-Blind, Active-Controlled Multicenter, 40-Week Extension Study from the 12-Week Parent Study to Evaluate Safety, Tolerability, and Efficacy of Vibegron 75mg in Men and Women with Overactive Bladder (OAB)**

A Phase 3, double-blind, active-controlled multicenter study in men and women with overactive bladder was conducted as an extension of the study described in Example 6 ("parent study"). Approximately 500 men and women with overactive bladder who completed 12 weeks in the parent study were permitted to enroll, at approximately 110 study sites in the U.S.

All subjects who were randomized in the parent study to either vibegron or tolterodine ER 4 mg continued their same treatment. Subjects who were randomized to placebo in the parent study were randomized 1:1 to vibegron or tolterodine, stratified by sex and baseline OAB type.

The primary endpoint was to evaluate safety and tolerability of vibegron for up to 52 weeks in patients with symptoms of OAB. Secondary endpoints were evaluation of CFB at Week 52 in average number of micturitions, Urge Urinary Incontinence (UUI) episodes, urgency episodes, and total urinary incontinence episodes. All CFB calculations used the parent study Baseline value.

Table 80 shows the overall group demographics while Table 81 shows the demographics for the vibegron and tolterodine arms.

Table 80. Demographics for Overall Groups

| | Overall Vibegron N=273 | Overall Tolterodine N=232 | Overall N=505 |
|---|---|---|---|
| Mean Age, in years (SD) | 61.0 (12.91) | 61.2 (12.65) | 61.1 (12.78) |
| Subjects ≥ 65 yr, n (%)<br>Subjects ≥75 yr, n (%) | 129 (47.3)<br>31 (11.4) | 106 (45.7)<br>29 (12.5) | 235 (46.5)<br>60 (11.9) |
| Female, n (%)<br>Male, n (%) | 213 (78.0)<br>60 (22.0) | 182 (78.4)<br>50 (21.6) | 395 (78.2)<br>110 (21.8) |

(continued)

| | Overall Vibegron N=273 | Overall Tolterodine N=232 | Overall N=505 |
|---|---|---|---|
| OAB Wet, n (%) (with incontinence) | 217 (79.5) | 178 (76.7) | 395 (78.2) |
| OAB Dry, n (%) (without incontinence) | 56 (20.5) | 54 (23.3) | 110 (21.8) |
| **Baseline** Hypertension n (%) | 20 (7.3) | 23 (9.9) | 43 (8.5) |
| Pre-existing Hypertension n (%) | 137 (50.2) | 103 (44.4) | 240 (47.5) |
| Safety Set Extension | | | |

Table 81. Demographics for Vibegron and Tolterodine Arms

| | 40-Weeks Vibegron N=92 | 52-Weeks Vibegron N=181 | 40-Weeks Tolterodine N=91 | 52-Weeks Tolterodine N=141 |
|---|---|---|---|---|
| Mean Age, in years (SD) | 58.8 (13.69) | 62.1 (12.39) | 62.1 (12.14) | 60.6 (12.98) |
| Subjects ≥ 65 yr, n (%)<br>Subjects ≥75 yr, n (%) | 36 (39.1)<br>8 (8.7) | 93 (51.4)<br>23 (12.7) | 43 (47.3)<br>13 (14.3) | 63 (44.7)<br>16 (11.3) |
| Female, n (%)<br>Male, n (%) | 73 (79.3)<br>19 (20.7) | 140 (77.3)<br>41 (22.7) | 70 (76.9)<br>21 (23.1) | 112 (79.4)<br>29 (20.6) |
| OAB Wet, n (%) (with incontinence) | 71 (77.2) | 146 (80.7) | 70 (76.9) | 108 (76.6) |
| OAB Dry, n (%) (without incontinence) | 21 (22.8) | 35 (19.3) | 21 (23.1) | 33 (23.4) |
| Baseline Hypertension n (%) | 9 (9.8) | 11 (6.1) | 8 (8.8) | 15 (10.6) |
| Pre-existing Hypertension n (%) | 40 (43.5) | 97 (53.6) | 31 (34.1) | 72 (51.1) |
| Safety Set Extension; Baseline hypertension is based on baseline vitals. Pre-existing hypertension is based on baseline vitals and prior medical history | | | | |

Table 82 shows the overall group subject dispositions while Table 83 shows the subject disposition for the vibegron and tolterodine arms.

Table 82. Subject Disposition for Overall Groups

| | Overall Vibegron n (%) | Overall Tolterodine n (%) | Overall n (%) |
|---|---|---|---|
| Randomized (N) | 274 | 232 | 506 |
| Took at least one dose (SAF-Ext) | 273 (99.6) | 232 (100) | 505 (99.8) |
| Completed the study | 235 (85.8) | 195 (84.1) | 430 (85.0) |
| Discontinued the study | 39 (14.2) | 37 (15.9) | 76 (15.0) |
| Withdrew Consent | 17 (6.2) | 15 (6.5) | 32 (6.3) |
| Adverse Event | 4 (1.5) | 8 (3.4) | 12 (2.4) |
| Death | 1 (0.4) | 0 | 1 (0.2) |
| Lost to Follow-up | 10 (3.6) | 5 (2.2) | 15 (3.0) |
| Other | 4 (1.5) | 5 (2.2) | 9 (1.8) |
| | | | |
| Full Analysis Set (FAS-Ext) | 266 (97.1) | 219 (94.4) | 485 (95.8) |
| Randomized Set Extension; some reasons for discontinuation (e.g. Patient with withdrawal due to PI or Sponsor, and Protocol Deviation, Lack of Efficacy) are not shown | | | |

Table 83. Subject Disposition for Overall Groups

| | 40-Weeks Vibegron n (%) | 52-Weeks Vibegron n (%) | 40-Weeks Tolterodine n (%) | 52-Weeks Tolterodine n (%) |
|---|---|---|---|---|
| Randomized (N) | 92 | 182 | 91 | 141 |
| Took at least one dose (SAF-Ext) | 92 (100) | 182 (100) | 91 (100) | 141 (100) |
| Completed the study | 79 (85.9) | 156 (85.7) | 72 (79.1) | 123 (87.2) |
| Discontinued the study | 13 (14.1) | 26 (14.3) | 19 (20.9) | 18 (12.8) |
| Withdrew Consent | 6 (6.5) | 11 (6.0) | 7 (7.7) | 8 (5.7) |
| Adverse Event | 1 (1.1) | 3 (1.6) | 4 (4.4) | 4 (2.8) |
| Death | 1 (1.1) | 0 | 0 | 0 |
| Lost to Follow-up | 4 (4.3) | 6 (3.3) | 3 (3.3) | 2 (1.4) |
| Other | 1 (1.1) | 3 (1.6) | 4 (4.4) | 1 (0.7) |
| | | | | |
| Full Analysis Set (FAS-Ext) | 90 (97.8) | 176 (96.7) | 83 (91.2) | 136 (96.5) |
| Randomized Set Extension; some reasons for discontinuation (e.g. Patient with withdrawal due to PI or Sponsor, and Protocol Deviation, Lack of Efficacy) are not shown | | | | |

Tables 84 through 87 show the adverse events which occurred during the study. As shown in these tables, there were no relevant difference between vibegron and tolterodine with respect to hypertension.

Table 84. Summary of Treatment Emergent Adverse Events

| Safety population n (%) | Overall Vibegron N=273 | Overall Tolterodine N=232 |
|---|---|---|
| Patients with at least one treatment-emergent adverse event | 171 (62.6) | 126 (54.3) |
| Patients with at least one treatment-emergent serious AE | 9 (3.3) | 10 (4.3) |
| SAEs resulting in death (not considered related by Investigator or Sponsor) | 1 (0.4) | 0 |
| Hospitalization | 7 (2.6) | 9 (3.9) |
| Other serious criteria | 1 (0.4) | 2 (0.9) |
| SAEs considered treatment-related by the investigator (Vibegron: Collagenous colitis Tolterodine: Syncope and Cardiac failure) | 1 (0.4) | 2 (0.9) |
| Safety Analysis Set Extension<br>SAE= Serious Adverse Event<br>TEAE= Treatment Emergent Adverse Event | | |

Table 85. Most Common Adverse Events (Vibegron >2% Arm)

| AE term n (%) | Overall Vibegron N=273 | Overall Tolterodine N=232 |
|---|---|---|
| Hypertension | 24 (8.8) | 20 (8.6) |
| Urinary tract infection | 18 (6.6) | 17 (7.3) |
| Headache | 15 (5.5) | 9 (3.9) |
| Nasopharyngitis | 13 (4.8) | 12 (5.2) |
| Diarrhea | 13 (4.8) | 4 (1.7) |

(continued)

| AE term n (%) | Overall Vibegron N=273 | Overall Tolterodine N=232 |
|---|---|---|
| Nausea | 10 (3.7) | 7 (3.0) |
| Constipation | 10 (3.7) | 6 (2.6) |
| Upper respiratory tract infection | 10 (3.7) | 1 (0.4) |
| Bronchitis | 8 (2.9) | 3 (1.3) |
| Residual urine volume increased | 7 (2.6) | 3 (1.3) |
| Hyperglycemia | 7 (2.6) | 2 (0.9) |
| Anaemia | 7 (2.6) | 2 (0.9) |
| Back pain | 6 (2.2) | 3 (1.3) |
| Musculoskeletal pain | 6 (2.2) | 1 (0.4) |
| Safety Analysis Set Extension; Represents number of patients | | |

Table 86. Select Adverse Events

| AE term n (%) | Overall Vibegron N=273 | Overall Tolterodine N=232 |
|---|---|---|
| Hypertension | 24 (8.8) | 20 (8.6) |
| Blood pressure increased | 2 (0.7) | 4 (1.7) |
| Tachycardia (Sinus Tachycardia) | 1 (0.4) | 1 (0.4) |
| Atrial Fibrillation | 0 | 1 (0.4) |
| Hypotension | 0 | 0 |
| Dizziness | 4 (1.5) | 2 (0.9) |
| Syncope | 0 | 1 (0.4) |
| Urinary tract infection | 18 (6.6) | 17 (7.3) |
| Urinary retention | 3 (1.1) | 1 (0.4) |
| Dry mouth | 5 (1.8) | 12 (5.2) |
| Constipation | 10 (3.7) | 6 (2.6) |
| Fatigue | 3 (1.1) | 0 |
| Analysis Set Extension; Represents number of patients | | |

Table 87. Adverse Events Leading to Discontinuation of Study Drug

| AE term n (%) | Overall Vibegron N=273 | Overall Tolterodine N=232 |
|---|---|---|
| Any | 4 (1.5) | 8 (3.4) |
| Amnesia | 1 (0.4) | 0 |
| Constipation | 1 (0.4) | 0 |
| Diarrhea | 1 (0.4) | 0 |
| Blood creatinine increased | 1 (0.4) | 0 |
| Blood urea increased | 1 (0.4) | 0 |
| Acute prerenal failure | 0 | 1 (0.4) |
| Aortic stenosis | 0 | 1 (0.4) |
| Atrial fibrillation | 0 | 1 (0.4) |
| Cardiac failure | 0 | 1 (0.4) |

(continued)

| AE term n (%) | Overall Vibegron N=273 | Overall Tolterodine N=232 |
|---|---|---|
| Cardiomyopathy | 0 | 1 (0.4) |
| Chronic kidney disease | 0 | 1 (0.4) |
| Dizziness | 0 | 1 (0.4) |
| Dry eye | 0 | 1 (0.4) |
| Dyspnoea | 0 | 1 (0.4) |
| Haematuria | 0 | 1 (0.4) |
| Headache | 0 | 1 (0.4) |
| Intervertebral disc degeneration | 0 | 1 (0.4) |
| Mitral valve incompetence | 0 | 1 (0.4) |
| Osteoarthritis | 0 | 1 (0.4) |
| Pulmonary embolism | 0 | 1 (0.4) |
| Sinus tachycardia | 0 | 1 (0.4) |
| Tricuspid valve incompetence | 0 | 1 (0.4) |
| Safety Analysis Set Extension; Represents number of patients | | |

As shown in Figures 15 - 18 and in Tables 88 through 95, baseline adjusted changes were maintained over the 52 weeks in all four efficacy endpoints (micturitions, UUI, urgency, and total incontinence). In addition, there was a numerically better effect for vibegron relative to tolterodine in all four categories.

Table 88. Week 52 Micturitions

| | | 40-Week Vibegron N=90 | 52-Week Vibegron N=176 | 40-Week Tolterodine N=83 | 52-Week Tolterodine N=136 |
|---|---|---|---|---|---|
| Baseline | n<br>Mean (SD) | 90<br>12.2 (3.80) | 176<br>11.3 (3.41) | 83<br>11.3 (3.07) | 136<br>11.3 (3.22) |
| Week 52 | n<br>Mean (SD) | 79<br>9.3 (3.64) | 152<br>8.7 (3.04) | 69<br>8.8 (2.59) | 120<br>9.0 (3.90) |
| Change from Baseline | n<br>Mean (SD) | 79<br>-3.1 (3.67) | 152<br>-2.5 (2.71) | 69<br>-2.6 (3.01) | 120<br>-2.3 (3.29) |
| Full Analysis Set ExtensionSD=standard deviation | | | | | |

Table 89. Reduction in Micturitions at Week 52

| Micturitions | 52-Weeks Vibegron N=176 | 52-Weeks Tolterodine N=136 |
|---|---|---|
| n Baseline Mean (SD) | 176<br>11.32 (3.415) | 136<br>11.33 (3.218) |
| n Change from Baseline LS Mean (SE) | 152<br>-2.4 (0.24) | 120<br>-2.0 (0.26) |
| 95% CI | -2.9 to -2.0 | -2.5 to -1.5 |
| Full Analysis Set Extension, CFB Least squares mean at Week 52. Covariates included in the mixed model for repeated measures are study visit, treatment, treatment by study visit, baseline, OAB type and sex | | |

Table 90. Week 52 UUI Episodes

| | | 40-Week Vibegron N=69 | 52-Week Vibegron N=143 | 40-Week Tolterodine N=64 | 52-Week Tolterodine N=106 |
|---|---|---|---|---|---|
| Baseline | n<br>Mean (SD) | 69<br>3.5 (2.92) | 143<br>3.2 (2.84) | 64<br>2.8 (2.40) | 106<br>3.0 (2.04) |
| Week 52 | n<br>Mean (SD) | 61<br>1.2 (2.68) | 125<br>1.0 (1.55) | 55<br>1.1 (1.85) | 91<br>1.4 (2.17) |
| Change from Baseline | n<br>Mean (SD) | 61<br>-2.3 (3.25) | 125<br>-2.2 (2.40) | 55<br>-1.5 (2.33) | 91<br>-1.7 (2.09) |
| Full Analysis Set Extension<br>SD=standard deviation | | | | | |

Table 91. Reduction in UUI Episodes at Week 52

| UUI Episodes | 52-Weeks Vibegron N=143 | 52-Weeks Tolterodine N=106 |
|---|---|---|
| n Baseline Mean (SD) | 143<br>3.18 (2.837) | 106<br>3.00 (2.038) |
| n Change from Baseline LS Mean (SE) | 125<br>-2.2 (0.15) | 91<br>-1.7 (0.17) |
| 95% CI | -2.5 to -1.9 | -2.0 to -1.3 |
| Full Analysis Set Extension for Incontinence, CFB Least squares mean at Week 52. Covariates included in the mixed model for repeated measures are study visit, treatment, treatment by study visit interaction, baseline, and sex | | |

Table 92. Week 52 Urgency Episodes

| | | 40-Week Vibegron N=90 | 52-Week Vibegron N=176 | 40-Week Tolterodine N=83 | 52-Week Tolterodine N=136 |
|---|---|---|---|---|---|
| Baseline | n<br>Mean (SD) | 90<br>8.6 (4.93) | 176<br>8.0 (4.59) | 83<br>7.6 (3.82) | 136<br>8.0 (3.71) |
| Week 52 | n<br>Mean (SD) | 79<br>4.7 (5.04) | 152<br>4.5 (4.38) | 69<br>3.9 (3.74) | 120<br>4.7 (4.74) |
| Change from Baseline | n<br>Mean (SD) | 79<br>-4.0 (4.13) | 152<br>-3.4 (4.37) | 69<br>-3.5 (4.07) | 120<br>-3.5 (4.30) |
| Full Analysis Set Extension.<br>SD=standard deviation | | | | | |

Table 93. Reduction in Urgency Episodes at Week 52

| Urgency | 52-Weeks Vibegron N=176 | 52-Weeks Tolterodine N=136 |
|---|---|---|
| n Baseline Mean (SD) | 176<br>8.00 (4.586) | 136<br>8.03 (3.706) |
| n Change from Baseline LS Mean (SE) | 152<br>-3.4 (0.34) | 120<br>-3.2 (0.37) |

(continued)

| Urgency | 52-Weeks Vibegron N=176 | 52-Weeks Tolterodine N=136 |
|---|---|---|
| 95% CI | -4.0 to -2.7 | -4.0 to -2.5 |
| Full Analysis Set Extension , CFB Least squares mean at Week 52. Covariates included in the mixed model for repeated measures are study visit, treatment, treatment by study visit interaction, baseline, OAB type and sex | | |

Table 94: Week 52 Total Urinary Incontinence Episodes

| | | 40-Week Vibegron N=69 | 52-Week Vibegron N=143 | 40-Week Tolterodine N=64 | 52-Week Tolterodine N=106 |
|---|---|---|---|---|---|
| Baseline | n<br>Mean (SD) | 69<br>4.2 (3.79) | 143<br>3.7 (3.23) | 64<br>3.3 (2.96) | 106<br>3.6 (2.52) |
| Week 52 | n<br>Mean (SD) | 61<br>1.8 (3.34) | 125<br>1.2 (1.77) | 55<br>1.2 (1.86) | 91<br>1.7 (2.40) |
| Change from Baseline | n<br>Mean (SD) | 61<br>-2.5 (3.76) | 125<br>-2.5 (2.72) | 55<br>-1.9 (2.73) | 91<br>-2.0 (2.31) |
| Full Analysis Set Extension. SD=standard deviation | | | | | |

Table 95. Reduction in Total Urinary Incontinence Episodes at Week 52

| Total Urinary Incontinence | 52-Weeks Vibegron N=143 | 52-Weeks Tolterodine N=106 |
|---|---|---|
| n Baseline Mean (SD) | 143<br>3.17 (3.225) | 106<br>3.56 (2.524) |
| n Change from Baseline LS Mean (SE) | 125<br>-2.5 (0.17) | 91<br>-1.9 (0.19) |
| 95% CI | -2.8 to -2.2 | -2.3 to -1.6 |
| Full Analysis Set Extension for Incontinence , CFB Least squares mean at Week 52. Covariates included in the mixed model for repeated measures are study visit, treatment, treatment by study visit interaction, baseline and sex | | |

Table 96 shows the Week 52 urgency and UUI responder endpoints.

Table 96. Week 52 Urgency and UUI Responder Endpoints

| Endpoint | | 40-Week Vibegron | 52-Week Vibegron | 40-Week Tolterodine | 52-Week Tolterodine |
|---|---|---|---|---|---|
| Urgency Episodes 50% Responders | N Percent 95% CI | 90<br>51.4<br>40.6 to 62.1 | 176<br>49.4<br>41.8 to 57.0 | 83<br>50.1<br>39.0 to 61.1 | 136<br>51.0<br>42.2 to 59.9 |
| | | | | | |
| UUI Episodes 75% Responders | N Percent 95% CI | 69<br>58.7<br>46.7 to 70.8 | 143<br>61.0<br>52.6 to 69.4 | 64<br>54.0<br>41.2 to 66.7 | 106<br>54.4<br>44.5 to 64.3 |
| | | | | | |

(continued)

| Endpoint | | 40-Week Vibegron | 52-Week Vibegron | 40-Week Tolterodine | 52-Week Tolterodine |
|---|---|---|---|---|---|
| UUI Episodes 100% Responders | N Percent 95% CI | 69<br>42.1<br>30.2 to 54.0 | 143<br>40.8<br>32.4 to 49.2 | 64<br>37.8<br>25.6 to 50.0 | 106<br>34.2<br>24.7 to 43.8 |
| Full Analysis Set-Extension , Full Analysis Set Extension for Incontinence, Multiple Imputation of missing values | | | | | |

As shown in Table 97, vibegron demonstrated strong efficacy against all OAB endpoints at Week 52.

Table 97. Week 52 OAB Endpoints

| Endpoint | 52-week Vibegron | n | 95% CI | 52-week Tolterodine | n | 95% CI |
|---|---|---|---|---|---|---|
| **Least Squares Means CFB** | | | | | | |
| UUI Episodes | **-2.2** | 125 | -2.5, -1.9 | **-1.7** | 91 | -2.0, -1.3 |
| Micturitions | **-2.4** | 152 | -2.9, -2.0 | **-2.0** | 120 | -2.5, -1.5 |
| Urgency Episodes (UE) | **-3.4** | 152 | -4.0, -2.7 | **-3.2** | 120 | -4.0, -2.5 |
| Total Incontinence Episodes | -2.5 | 125 | -2.8, -2.2 | **-1.9** | 91 | -2.3, -1.6 |
| Volume Voided (ml)[l] | **26 ml** | 145 | -23, 71 | **9 ml** | 114 | -26, 39 |
| **% Responders** | | | | | | |
| 50% Reduction in UE | **49%** | 176 | 42%, 57% | **51%** | 136 | 42%, 60% |
| 75% Reduction in UUI | **61%** | 143 | 53%, 69% | **54%** | 106 | 45%, 64% |
| 100% Reduction in UUI | **41%** | 143 | 32%, 49% | **34%** | 106 | 25%, 44% |
| 50% Reduction in Total Inc | **71%** | 143 | 63%, 79% | **62%** | 106 | 52%, 72% |
| 1. Q1, Q3 presented for 95% CI<br>Endpoints with UUI are based on Full Analysis Set Extension for Incontinence, other endpoints are based on the Full Analysis Set<br>LS mean change from baseline shown for UUI, Micturitions Urgency, and Total Incontinence, Covariates included in the mixed model for repeated measures are study visit, treatment, treatment by study visit interaction, baseline, OAB type (except UUI) and sex. Mean change from baseline shown for Volume Voided | | | | | | |

Table 98 shows the Week 52 coping subscore from the Quality-of-Life Long Form (OABq-LF).

Table 98. Week 52 OAB-q LF Coping Subscore

| | | 40-Week Vibegron N=90 | 52-Week Vibegron N=176 | 40-Week Tolterodine N=83 | 52-Week Tolterodine N=136 |
|---|---|---|---|---|---|
| Baseline | n<br>Mean (SD) | 89<br>58.5 (29.59) | 174<br>56.4 (30.86) | 82<br>58.0 (28.06) | 136<br>59.7 (27.41) |
| Week 52 | n<br>Mean (SD) | 83<br>86.3 (18.76) | 166<br>83.6 (21.25) | 75<br>82.5 (18.60) | 134<br>81.8 (22.16) |
| Change from Baseline | n<br>Mean (SD) | 83<br>28.3 (27.52) | 164<br>27.1 (26.56) | 74<br>24.7 (27.20) | 134<br>22.1 (28.24) |
| Full Analysis Set Extension; Baseline value is from parent study<br>SD=standard deviation | | | | | |

Example 8

**A Phase 1, Double-Blind, Placebo-Controlled Study to Assess the Effect of Vibegron on Blood Pressure and Heart Rate in Subjects with Overactive Bladder (OAB)**

A Phase 1, double-blind, placebo-controlled multicenter study in men and women with overactive bladder was conducted. A total of 214 patients were randomized across approximately 10 study sites into one of two study groups (108 receiving placebo, and 106 receiving 75 mg of vibegron) for a 28-day treatment period.

The study assessed the effect of vibegron on blood pressure and heart rate. The objectives and endpoints were as described below. "Mean daytime" ABPM endpoints are calculated as the mean of all valid ABPM readings while the subject was awake during the 24-hour monitoring session. "Mean 24hr" ABPM endpoints are calculated as the mean of all valid ABPM readings during the 24hr monitoring session. "Maximum mean" ABPM endpoints are calculated as the maximum of the hourly means during the Tmax window (0.5 to 6.5 hours post cuff-fitting at baseline and 0.5 to 6.5 hours post-Day 28).

| Objectives | Endpoints |
|---|---|
| **Primary** | |
| To determine and compare changes from baseline to Day 29 in mean daytime ambulatory systolic blood pressure (BP) | Change from baseline to Day 29 in mean daytime ambulatory systolic BP |
| **Secondary** | |
| To explore the relationship between vibegron and measurements of BP and heart rate | Change from baseline to Day 29 in mean daytime ambulatory:<br>• diastolic BP<br>• heart rate<br>Change from baseline to Day 29 in mean 24-hour ambulatory:<br>• systolic BP<br>• diastolic BP<br>• heart rate<br>**Exploratory**<br>Change from baseline to Day 29 in maximum mean ambulatory:<br>• systolic BP at 0.5 to 6.5 hours post-dose Day 28<br>• diastolic BP at 0.5 to 6.5 hours post-dose Day 28<br>• heart rate at 0.5 to 6.5 hours post - dose Day 28 [Note: the $t_{max}$ window of vibegron is 0.5 to 6.5 hours] |
| **Other** | |
| To determine the pharmacokinetics, safety and tolerability of repeat-dose of vibegron 75 mg | Vibegron pharmacokinetics at Day 15<br>**Safety**<br>Safety and tolerability parameters including adverse events (AEs), change in concurrent medications, clinical laboratory and vital sign assessments collected at office visits. |

*8.1 Eligibility Criteria*

To be eligible for participation in this study, a patient must have met all of the following Inclusion Criteria, and none of the following Exclusion Criteria, before enrollment.

*8.1.1 Inclusion Criteria*

1. Capable of giving written informed consent, which includes compliance with the requirements and restrictions listed in the consent form.
2. Male or female between 40 and 75 years of age inclusive at Screening (Visit 1). NOTE: Up to 30% of subjects can be male.
3. Subjects with a history of OAB. Note: OAB is defined as urgency, with or without urge urinary incontinence (UUI), usually associated with frequency and nocturia.
4. The postvoid residual urine (PVR) needs to be at or below 100mL at Screening (Visit 1) based on bladder scan or ultrasound.
5. A female subject is eligible to participate if she is of:

- Non-childbearing potential defined as pre-menopausal females with a documented bilateral tubal ligation, bilateral oophorectomy (removal of theovaries) or hysterectomy; hysteroscopic sterilization, or postmenopausal defined as 12 months of spontaneous amenorrhea. Documented verbal history from the subject is acceptable.
- Child-bearing potential and agrees to use one of the contraception methods listed in Section 5.6.1 for an appropriate period of time (as determined by the product label or Investigator) prior to the start of dosing to sufficiently minimize the risk of pregnancy at that point. Female subjects must agree to use contraception until the follow-up visit.

6. For females of child-bearing potential: Agrees not to donate ova (eggs) until at least 1 month after the last dose of study treatment.
7. Body weight ≥ 50 kg for men and ≥ 45 kg for women and Body Mass Index within the range 18.5-35.0 kg/$m^2$ (inclusive) at Screening (Visit 1).
8. Mid-arm circumference must be ≤ 45 cm to accommodate the ABPM cuff maximal size.

*8.1.2 Exclusion Criteria*

1. The subject has a positive drug screen at Screening (Visit 1), except if the drug has been prescribed to the subject and is not a prohibited medication.
2. Has ALT (alanine aminotransferase) or AST (aspartate aminotransferase) > 2.0 times the upper limit of normal (ULN), or bilirubin (total bilirubin) > 1.5 x ULN (or > 2.0 x ULN if secondary to Gilbert syndrome or pattern consistent with Gilbert syndrome) at Screening (Visit 1).
3. Has an estimated glomerular filtration rate (eGFR) < 30 mL/min/1.73 $m^2$ using the Modification of Diet in Renal Disease Study (MDRD) equation at Screening (Visit 1).
4. History of regular alcohol consumption within 6 months of the Screening (Visit 1) defined as:

- An average weekly intake of > 14 drinks/week for men or >7 drinks/week for women. One drink is equivalent to (12 g alcohol) = 5 ounces (150 ml) of wine or 12 ounces (360 ml) of beer or 1.5 ounces (45 ml) of 80 proof distilled spirits.

5. The subject has received an investigational product or device (including placebo) within the following time period prior to the first dosing day in the current study: 30 days, 5 half-lives or twice the duration of the biological effect of the investigational product (whichever is longer).
6. Is currently participating in or has participated in a study with vibegron.
7. Use of any prohibited medications as follows (suitable washout periods from these medications are also provided): anticholinergics; smooth muscle relaxants; beta-2 adrenergic agonists for the treatment of stress urinary incontinence; beta-2 adrenergic agonists; synthetic antidiuretic hormones; beta-3 adrenergic agonists; intradetrusor botulinum toxins; CNS stimulants; cannabis products; alpha-1-agonists (oral); NSAIDs; herbal supplements.
8. Had changed the dose of certain medications (e.g., tricyclic antidepressants or combinations; serotonin and/or norepinephrine reuptake inhibitors; inhaled anticholinergics; antihypertensive not listed as prohibited; alpha-1-antagonists; 5-alpha reductase inhibitors; phosphodiesterase type 5 inhibitors) within 4 weeks prior to the Screening (Visit 1) or

plans to initiate or change the dosing of any of these medications during the study.
9. History of sensitivity to any of the study treatments or excipients, or components thereof or a history of drug or other allergy that, in the opinion of the Investigator, contraindicates their participation.
10. If heparin is used during PK sampling (for those subjects consenting to PK sampling), subjects with a history of sensitivity to heparin or heparin-induced thrombocytopenia should not be enrolled.
11. Pregnant females as determined by positive serum (Screening Visit 1) or urine (Day -1) human chorionic gonadotropin test at screening or prior to dosing.
12. Lactating females who are actively breastfeeding.
13. Subjects with uncontrolled hypertension (systolic blood pressure of > 160 mmHg and/or diastolic blood pressure of > 95 mmHg) or has a resting heart rate (by pulse) >100 beats per minute at Screening (Visit 1).
14. Subjects with a pre-existing condition interfering with normal gastrointestinal anatomy, function, or motility (including gastric bypass), hepatic and/or renal function that could interfere with the absorption, metabolism, and/or excretion of the study treatments.
15. Subjects with an active urinary tract infection at Day -1 (Visit 2)
16. History of significant psychiatric or neurologic diseases.
17. History of asthma or chronic obstructive pulmonary disease requiring use of inhaled beta-2 agonists.
18. History of uncontrolled diabetes mellitus (HbA1c > 9%) at screening (Visit 1).
19. History of cerebrovascular accident, transient ischemic attack, unstable angina, myocardial infarction, coronary artery interventions (e.g., coronary artery bypass grafting or percutaneous coronary interventions [e.g., angioplasty, stent insertion]), or neurovascular interventions (e.g., carotid artery stenting) within 6 months prior to the Screening Visit. Subjects with these conditions should be on stable medical therapy for at least 3 months prior to the Screening Visit.
20. Subjects whose occupation or recreational activities involve heavy lifting and are not willing to forego participation in these activities on study days where ABPM measurements are collected.
21. Subjects whose occupation (i.e., night shift workers) or recreational activities involve sleeping for at least 8 continuous hours between 0700 and 2300 on a consistent basis.
22. Exclusion criteria for screening ECG (a single repeat is allowed for eligibility determination):

| | |
|---|---|
| QTcF | >450 msec for males<br>>470 msec for females |

- Wolff Parkinson White syndrome [unless curative ablation treatment]) oratrial fibrillation
- Sinus pauses > 3 seconds

23. Postvoid residual urine (PVR) > 100 mL at Screening (Visit 1)
24. Subjects who have failed the ABPM device testing based on the pass/fail criteria located in the study reference manual
25. Has a history of chronic pain or chronic use of non-steroidal antiinflammatory drugs (NSAIDs) to treat pain; except for low-dose aspirin therapy, which is allowed. Chronic pain is defined as daily pain that impairs daily living and requires specific daily treatment (e.g., daily pain medications, or regular acupuncture, electrostimulation treatment etc.) for the pain.
26. Any other condition, therapy, laboratory abnormality or other circumstances that might, in the opinion of the investigator, interfere with the subject's ability to comply with the study procedures or make the participation in the study not in the subject's best interest.

*8.2 Study Assessments And Procedures*

*8.2.1. Investigational Product and Other Study Treatment*

The term 'study treatment' is used throughout the protocol to describe a single dose of vibegron or placebo.

| | *Study* | |
|---|---|---|
| *Product name:* | Vibegron | Placebo |
| *Physical description:* | Light green oval film- coated tablet with 901 embossed on one side | Light green oval film- coated tablet with 901 embossed on one side |

(continued)

| | | |
|---|---|---|
| *Dosage form:* | Tablet | Tablet |
| *Unit dose strength(s)/Dosage level(s):* | 75 mg / 1 tablet per dose | placebo tablet / 1 tablet per dose |
| *Route of Administration/-Duration* | Oral / 28 days | Oral / 28 days |
| *Dosing instructions:*[a] | Administered with 240 mL of water in the morning without regard to meals. | Administered with 240 mL of water in the morning without regard to meals. |
| a. Applicable only to witness dose in clinic | | |

*8.2.2. Randomization / Treatment Assignment*

Subjects were randomized to receive one of the two treatments in a 1:1 ratio:

- Vibegron 75 mg
- Placebo to match vibegron 75 mg

Randomization was stratified based on age (≤ 55 or > 55 years), sex (Male or Female), and pre-existing hypertension (Yes or No).

Enrollment was capped using the following limits:

- Up to 30% of the subjects enrolled may be male
- May be capped at 55% of the subjects with a pre-existing medical history of hypertension and/or hypertension at baseline

*8.2.3. Time and Events Table*

Figure 11 shows the Time and Events Table for the Study.

*8.4 Safety Considerations*

Safety was evaluated by assessment of clinical laboratory tests, physical examinations, and vital signs measurements from office visits at various time points during the study, and by the documentation of AEs.

*8.5 Statistical Analyses*

The primary hypothesis was to test if a change from baseline (CFB) to Day 29 in mean daytime ambulatory systolic BP for the vibegron arm is less than 3.5 mmHg CFB to Day 29 for the placebo arm. The null and alternative statistical hypotheses are:

$$H_0 : \mu_v - \mu_p \geq 3.5$$

$$: \mu_v - \mu_p < 3.5$$

where $\mu_v$ = mean daytime CFB to Day 29 for the vibegron arm, and $\mu_p$ = mean daytime CFB to Day 29 for the placebo arm.

*8.6 Analysis Populations*

*Safety Set (SAF):* All subjects who received at least one dose of double-blind study treatment were included in the SAF. Subjects were included in the treatment group corresponding to the Study Treatment that was actually received. This

was the population used to summarize safety analyses collected during clinic visits and correspondence with site staff; summarization of baseline/demographic characteristics also presented for the SAF.

*Full Analysis Set (FAS):* All subjects who were randomized, took at least one dose of double-blind Study Treatment, and had a valid baseline ambulatory blood pressure monitoring (ABPM) measurement and valid Day 29 ABPM measurement be included in the FAS. In accordance with the intent-to-treat principle, subjects were included in the treatment group to which they were randomized, regardless of which treatment was actually received. This population was used for all analyses of ABPM measurements. If the FAS differed from the SAF, then baseline/demographic characteristics was also presented for the FAS.

*Pharmacokinetic Population:* The PK Population includes all subjects who underwent plasma PK sampling and had evaluable concentration-time data for analysis.

*8.7 Final Analysis*

Final analysis was performed after the completion of the study and the final datasets authorization.

Data was listed and summarized. Treatment was assigned based on the dosing schedule and was included in the data listings. Listings were sorted by subject, day, and time; summaries were presented by treatment, day, and time.

Version 9.2 or higher of the SAS system was used to analyze the data as well as to generate tables, figures, and listings.

*8.71 ABPM Analyses*

For the analysis of the ABPM endpoints (CFB to Day 29 of systolic and diastolic blood pressure, and heart rate), a generalized linear model (GLM) was used. The FAS was used to analyze these endpoints; by definition, the FAS does not have any missing data, thus no imputation of missing data was required. The analysis model for each efficacy endpoint included terms for treatment, age group (≤ 55 vs > 55 years), sex (Male vs Female), and pre-existing hypertension (Yes vs No), and baseline score.

Primary inferences were drawn from treatment differences for the CFB at Day 29 from the GLM. The estimated treatment difference was displayed in the summary of statistical analysis together with the two-sided 90% confidence interval and the associated p-value. To test the primary hypothesis in Section 85, the upper limit of the two-sided 90% confidence interval was compared to 3.5, and the null hypothesis was rejected if the upper limit was strictly less than 3.5.

The 24-mean ambulatory baseline for systolic and diastolic blood pressure, and heart rate was the arithmetic mean of all valid measurements between the time of cuff-fitting on Day -1 to cuff-removal on Day 1. The 24-hour mean at Day 29 was arithmetic mean of all valid measurements between the time of cuff- fitting on Day 28 to cuff removal on Day 29.

The maximum SBP, DBP and HR in the tmax window at baseline was the maximum of the hourly means of valid measurements from 0.5 to 6.5 hours after the time of cuff fitting on Day -1. On Day 28, the maximum SBP, DBP and HR in the tmax window was the maximum of the hourly means of valid measurements from 0.5 to 6.5 hours after dose.

The mean daytime ambulatory baseline for systolic and diastolic blood pressure, and heart rate was the arithmetic mean of all measurements taken during the time interval in which the subject was awake. The same definition was applied to Day 29 measurements.

*8.8 Clinical Trial Data and Results*

The demographics of the patients in this trial are shown in Table 99, and the subject disposition is shown in Table 100.

Table 99. Patient Demographics

| | **Statistic** | **Placebo (N= 101)** | **Vibegron (N=96)** |
|---|---|---|---|
| Female | n (%) | 74 (73.3) | 73 (76.0) |
| Male | n (%) | 27 (26.7) | 23 (24.0) |
| Age (yrs) | mean (sd) | 59.2 (8.21) | 59.4 (8.56) |
| Age > 55 yrs | n (%) | 66 (65.3) | 66 (68.8) |
| Age > 65 yrs | n (%) | 33 (32.7) | 31 (32.3) |
| Pre-existing hypertension[1] | n (%) | 26 (25.7) | 31 (32.3) |
| SBP (mmHg) | mean (sd) | 121.5 (13.45) | 119.8 (15.45) |

(continued)

| | Statistic | Placebo (N= 101) | Vibegron (N=96) |
|---|---|---|---|
| DBP (mmHg) | mean (sd) | 76.3 (8.20) | 75.0 (8.67) |
| HR (bpm) | mean (sd) | 70.3 (8.94) | 71.7 (9.73) |
| [1] From Medical History<br>Population: FAS | | | |
| SBP: systolic blood pressure, DBP: diastolic blood pressure HR: heart rate | | | |

Table 100. Subject Disposition

| Patient Disposition | Placebo n (%) | Vibegron n (%) |
|---|---|---|
| Randomized (N) | 108 | 106 |
| Took at least one dose (SAF) | 108 (100) | 106 (100) |
| Completed the study (4wks) | 105 (97.2) | 103 (97.2) |
| Discontinued the study | 3 (2.8) | 3 (2.8) |
| Adverse Event* | 0 | 2 (1.9) |
| Lost to Follow up | 1 (0.9) | 1 (0.9) |
| Non-Compliance with Study Drug | 1 (0.9) | 0 |
| Withdrawal by Subject | 1 (0.9) | 0 |
| | | |
| Full Analysis Set (FAS) | 101 (93.5) | 96 (90.6) |
| Population: Randomized | | |

Vibegron achieved the primary endpoint: the mean change of daytime ambulatory systolic blood pressure from baseline over a treatment period from that of subjects taking a placebo was less than about 1 mm Hg with an upper bound 90% confidence interval less of than 3.5 mm Hg. The results are shown in Tables 101 (Full Analysis Set) and 102 (Per Protocol Set).

Table 101. Mean Daytime ABPM SBP (mm Hg) Change from Baseline to Day 28-FAS

| Placebo (N=101) | Vibegron (N=96) | Treatment Difference | |
|---|---|---|---|
| LSMean | LSMean | Vibegron-Placebo | 90% CI |
| 0.01 | 0.82 | 0.81 | -0.88, 2.49 |
| ABPM = Ambulatory Blood Pressure Monitoring via Spacelabs 90207, SBP = Systolic Blood Pressure, LSMean = Least Squares Mean, CI = Confidence Interval, FAS = Full Analysis Set | | | |

Table 102. Mean Daytime ABPM SBP (mm Hg) Change from Baseline to Day 28-PPS

| Placebo (N=98) | Vibegron (N=93) | Treatment Difference | |
|---|---|---|---|
| LSMean | LSMean | Vibegron-Placebo | 90% CI |
| -0.22 | 0.19 | 0.41 | -1.18, 2.00 |
| ABPM = Ambulatory Blood Pressure Monitoring via Spacelabs 90207, SBP = Systolic Blood Pressure, LSMean = Least Squares Mean, CI = Confidence Interval, PPS = Per-Protocol Set | | | |

Secondary endpoints for the Full Analysis Set and Per Protocol Set, describing changes to blood pressure and heart rate are shown in Tables 103 and 104. Figure 12 shows the 90% confidence interval for treatment difference in Day 28 change from baseline for ABPM systolic blood pressure. Figure 13 shows the 90% confidence interval for treatment difference in Day 28 change from baseline for ABPM diastolic blood pressure. Figure 14 shows the 90% confidence

interval for treatment difference in Day 28 change from baseline for ABPM heart rate.

Table 103. ABPM Secondary Endpoints: Change from Baseline to Day 28

| | Placebo (N=101) | Vibegron (N=96 ) | Treatment Difference | |
|---|---|---|---|---|
| | LSMean | LSMean | Vibegron-Placebo | 90% CI |
| Mean Daytime DBP (mmHg) | 0.55 | 0.50 | -0.04 | -1.20, 1.11 |
| Mean Daytime HR (bpm) | 0.19 | 1.08 | 0.88 | -0.26, 2.03 |
| | | | | |
| Mean 24H SBP (mmHg) | 0.03 | 0.60 | 0.57 | -0.97, 2.10 |
| Mean 24H DBP (mmHg) | 0.70 | 0.51 | -0.19 | -1.25, 0.87 |
| Mean 24H HR (bpm) | -0.15 | 0.80 | 0.96 | -0.10, 2.01 |
| | | | | |
| Max (0.5-6.5h) SBP (mmHg) | 0.29 | 1.96 | 1.67 | -0.83, 4.18 |
| Max (0.5-6.5h) DBP (mmHg) | -0.53 | 0.63 | 1.16 | -0.43, 2.75 |
| Max (0.5-6.5h) HR (bpm) | 0.28 | 1.76 | 1.48 | -1.07, 4.04 |
| Population: FAS | | | | |
| ABPM = Ambulatory Blood Pressure Monitoring, DBP = Diastolic Blood Pressure, HR = Heart Rate, SBP = Systolic Blood Pressure, LS Mean = Least Squares Mean, CI = Confidence Interval, FAS = Full Analysis Set | | | | |

Table 104. Change from Baseline to Day 28 for Maximum (0.5 to 6.5hrs) ABPM SBP (mm Hg)- PPS

| Placebo (N=95) | Vibegron (N=90) | Treatment Difference | |
|---|---|---|---|
| LSMean | LSMean | Vibegron-Placebo | 90% CI |
| -0.04 | 1.0 | 1.05 | -1.30, 3.40 |
| ABPM = Ambulatory Blood Pressure Monitoring via Spacelabs 90207, SBP = Systolic Blood Pressure, LSMean = Least Squares Mean, CI = Confidence Interval, PPS = Per-Protocol Set | | | |

A categorical summary of day 28 change from baseline for the study's endpoints are shown in Table 105.

Table 105. ABPM Endpoints: Categorical Summary of Day 28 Change from Baseline

| Parameter | Criteria | Endpoint | Placebo (N= 101) n (%) | Vibegron (N=96) n (%) |
|---|---|---|---|---|
| SBP (mmHg) | ≥ 15 mmHg | Mean Daytime | 2 (2.0) | 2 (2.1) |
| | | Mean 24-hour | 0 | 2 (2.1) |
| | | Maximum (0.5-6.5h) | 4 (4.2) | 6 (6.5) |
| | | | | |
| DBP (mmHg) | ≥ 10 mmHg | Mean Daytime | 2 (2.0) | 2 (2.1) |
| | | Mean 24-hour | 0 | 1 (1.0) |
| | | Maximum (0.5-6.5h) | 5 (5.2) | 9 (9.8) |
| | | | | |
| HR (bpm) | ≥ 10 bpm | Mean Daytime | 4 (4.0) | 3 (3.1) |
| | | Mean 24-hour | 3 (3.0) | 3 (3.1) |

(continued)

| Parameter | Criteria | Endpoint | Placebo (N= 101) n (%) | Vibegron (N=96) n (%) |
|---|---|---|---|---|
| | | Maximum (0.5-6.5h) | 17 (17.7) | 20 (21.7) |
| Population: FAS | | | | |
| ABPM = Ambulatory Blood Pressure Monitoring, SBP = Systolic Blood Pressure, DBP = Diastolic Blood Pressure, HR = Heart Rate, FAS = Full Analysis Set | | | | |

Tables 106, 107, and 108 show the changes to in-clinic measurements of systolic blood pressure, diastolic blood pressure, and heart rate over the treatment period.

Table 106. In-Clinic Vital Signs: Categorical Summary of Change from Baseline to Day 28

| Parameter | Treatment | N | n* | ≥ 5 n (%) | ≥ 10 n (%) | ≥ 15 n (%) |
|---|---|---|---|---|---|---|
| SBP (mmHg) | Placebo | 108 | 105 | 27 (25.7) | 18 (17.1) | 11 (10.5) |
| | Vibegron | 106 | 103 | 33 (32.0) | 22 (21.4) | 10 (9.7) |
| DBP (mmHg) | Placebo | 108 | 105 | 21 (20.0) | 7 (6.7) | 3 (2.9) |
| | Vibegron | 106 | 103 | 24 (23.3) | 9 (8.7) | 2 (1.9) |
| HR (bpm) | Placebo | 108 | 105 | 21 (20.0) | 7 (6.7) | 3 (2.9) |
| | Vibegron | 106 | 103 | 24 (23.3) | 8 (7.8) | 3 (2.9) |
| Population: SAF | | | | | | |
| * n=number of subjects with baseline and day 28 vitals SBP = Systolic Blood Pressure DBP = Diastolic Blood Pressure, HR = Heart Rate, CFB = Change from Baseline, sd=standard deviation, SAF = Safety Set | | | | | | |

Table 107. In-Clinic Vital Signs: Categorical Summary of Change from Baseline to End of Treatment

| Parameter | Treatment | N | n* | ≥ 5 n (%) | ≥ 10 n (%) | ≥ 15 n (%) |
|---|---|---|---|---|---|---|
| SBP (mmHg) | Placebo | 108 | 107 | 38 (35.5) | 14 (13.1) | 10 (9.3) |
| | Vibegron | 106 | 105 | 36 (34.3) | 22 (21.0) | 13 (12.4) |
| DBP (mmHg) | Placebo | 108 | 107 | 21 (19.6) | 7 (6.5) | 1 (0.9) |
| | Vibegron | 106 | 105 | 31 (29.5) | 12 (11.4) | 7 (6.7) |
| HR (bpm) | Placebo | 108 | 107 | 22 (20.6) | 10 (9.3) | 5 (4.7) |
| | Vibegron | 106 | 105 | 37 (35.2) | 12 (11.4) | 4 (3.8) |
| Population: SAF | | | | | | |
| * n=number of subjects with baseline and end of treatment assessments, SBP = Systolic Blood Pressure DBP = Diastolic Blood Pressure, HR = Heart Rate, CFB = Change from Baseline, sd=standard deviation, SAF = Safety Set | | | | | | |

Table 108. In-Clinic Vital Signs: Categorical Summary of Change from Baseline Over 28 Days on 3 Consecutive Visits

| Parameter | Treatment | N | n* | ≥ 5 n (%) | ≥ 10 n (%) | ≥ 15 n (%) |
|---|---|---|---|---|---|---|
| SBP (mmHg) | Placebo | 108 | 103 | 10 (9.7) | 5 (4.9) | 2 (1.9) |
| | Vibegron | 106 | 98 | 14 (14.3) | 6 (6.1) | 0 |
| DBP (mmHg) | Placebo | 108 | 103 | 6 (5.8) | 3 (2.9) | 0 |
| | Vibegron | 106 | 98 | 5 (5.1) | 3 (3.1) | 0 |
| HR (bpm) | Placebo | 108 | 103 | 9 (8.7) | 2 (1.9) | 2 (1.9) |

(continued)

| Parameter | Treatment | N | n* | ≥ 5 n (%) | ≥ 10 n (%) | ≥ 15 n (%) |
|---|---|---|---|---|---|---|
| | Vibegron | 106 | 98 | 6 (6.1) | 3 (3.1) | 0 |
| Population: SAF | | | | | | |
| * n=number of subjects with baseline and end of treatment assessments, SBP = Systolic Blood Pressure DBP = Diastolic Blood Pressure, HR = Heart Rate, CFB = Change from Baseline, sd=standard deviation, SAF = Safety Set | | | | | | |

Changes to in-clinic vital signs of subgroups are shown in Tables 109 through 114. For subjects with a body weight of at least about 65.4 kg, there was no difference between treatment and placebo in the number of subjects with a ≥5, ≥10, or ≥15 mm Hg increase in systolic blood pressure. The same was true for subjects over the age of 66.

Table 109 In-Clinic Vital Signs: Categorical Summary of Change from Baseline to Day 28 SBP by Subgroup

| Subgroup | Treatment | N | n* | ≥ 5 n (%) | ≥ 10 n (%) | ≥ 15 n (%) |
|---|---|---|---|---|---|---|
| Body Weight <= 25th Percentile | Placebo | 24 | 23 | 3 (13.0) | 3 (13.0) | 2 (8.7) |
| | Vibegron | 31 | 28 | 9 (32.1) | 6 (21.4) | 2 (7.1) |
| | | | | | | |
| Body Weight > 25th Percentile | Placebo | 84 | 82 | 24 (29.3) | 15 (18.3) | 9 (11.0) |
| | Vibegron | 75 | 75 | 24 (32.0) | 16 (21.3) | 8 (10.7) |
| | | | | | | |
| **eGFR** <= 25th Percentile | Placebo | 26 | 26 | 7 (26.9) | 5 (19.2) | 3 (11.5) |
| | Vibegron | 30 | 30 | 7 (23.3) | 4 (13.3) | 1 ( 3.3) |
| | | | | | | |
| eGFR > 25th Percentile | Placebo | 82 | 79 | 20 (25.3) | 13 (16.5) | 8 (10.1) |
| | Vibegron | 76 | 73 | 26 (35.6) | 18 (24.7) | 9 (12.3) |
| Population: SAF | | | | | | |
| n=number of subjects with baseline and day 28 vitals, SBP = Systolic Blood Pressure, SAF = Safety Set, eGFR $25^{th}$ percentile= 72 mL/min/1.73m$^2$, $25^{th}$ percentile BW= 65.4 kg | | | | | | |

Table 110. In-Clinic Vital Signs: Categorical Summary of Change from Baseline to Day 28 SBP by Subgroup

| Subgroup | Treatment | N | n* | ≥ 5 n (%) | ≥ 10 n (%) | ≥ 15 n (%) |
|---|---|---|---|---|---|---|
| Age <= $75^{th}$ percentile | Placebo | 81 | 78 | 16 (20.5) | 11 (14.1) | 7 (9.0) |
| | Vibegron | 84 | 81 | 28 (34.6) | 18 (22.2) | 9 (11.1) |
| | | | | | | |
| Age > $75^{th}$ percentile | Placebo | 27 | 27 | 11 (40.7) | 7 (25.9) | 4 (14.8) |
| | Vibegron | 22 | 22 | 5 (22.7) | 4 (18.2) | 1 (4.5) |
| | | | | | | |
| Pre-existing hypertension: YES | Placebo | 36 | 35 | 9 (25.7) | 8 (22.9) | 4 (11.4) |
| | Vibegron | 39 | 39 | 13 (33.3) | 9 (23.1) | 4 (10.3) |
| | | | | | | |
| Pre-existing hypertension: NO | Placebo | 72 | 70 | 18 (25.7) | 10 (14.3) | 7 (10.0) |

(continued)

| Subgroup | Treatment | N | n* | ≥ 5 n (%) | ≥ 10 n (%) | ≥ 15 n (%) |
|---|---|---|---|---|---|---|
| | Vibegron | 67 | 64 | 20 (31.3) | 13 (20.3) | 6 (9.4) |
| Population: SAF | | | | | | |
| n=number of subjects with baseline and day 28 vitals, SBP = Systolic Blood Pressure, SAF = Safety Set, 75th percentile age 66 years old | | | | | | |

Table 111. In-Clinic Vital Signs: Categorical Summary of Change from Baseline Over 28 Days on 3 Consecutive Visits

| Subgroup | Treatment | N | n* | ≥ 5 n (%) | ≥ 10 n (%) | ≥ 15 n (%) |
|---|---|---|---|---|---|---|
| Body Weight <= 25th Percentile | Placebo | 24 | 22 | 2 (9.1) | 0 | 0 |
| | Vibegron | 31 | 26 | 6 (23.1) | 2 (7.7) | 0 |
| | | | | | | |
| Body Weight > 25th Percentile | Placebo | 84 | 81 | 8 (9.9) | 5 (6.2) | 2 (2.5) |
| | Vibegron | 75 | 72 | 8 (11.1) | 4 (5.6) | 0 |
| | | | | | | |
| eGFR <= 25th Percentile | Placebo | 26 | 25 | 3 (12.0) | 3 (12.0) | 2 (8.0) |
| | Vibegron | 30 | 28 | 3 (10.7) | 1 (3.6) | 0 |
| | | | | | | |
| eGFR > 25th Percentile | Placebo | 82 | 78 | 7 ( 9.0) | 2 (2.6) | 0 |
| | Vibegron | 76 | 70 | 11 (15.7) | 5 (7.1) | 0 |
| Population: SAF | | | | | | |
| n=number of subjects with baseline and 3 post-baseline visits, SAF = Safety Set, eGFR $25^{th}$ percentile= 72 mL/min/1.73m$^2$, $25^{th}$ percentile BW= 65.4 kg | | | | | | |

Table 112. In-Clinic Vital Signs: Categorical Summary of Change from Baseline Over 28 Days on 3 Consecutive Visits

| Subgroup | Treatment | N | n* | ≥ 5 n (%) | ≥ 10 n (%) | ≥ 15 n (%) |
|---|---|---|---|---|---|---|
| Age <= $75^{th}$ percentile | Placebo | 81 | 77 | 6 (7.8) | 3 (3.9) | 2 (2.6) |
| | Vibegron | 84 | 78 | 13 (16.7) | 5 (6.4) | 0 |
| | | | | | | |
| Age > $75^{th}$ percentile | Placebo | 27 | 26 | 4 (15.4) | 2 (7.7) | 0 |
| | Vibegron | 22 | 20 | 1 (5.0) | 1 (5.0) | 0 |
| | | | | | | |
| Pre-existing hypertension: YES | Placebo | 36 | 34 | 5 (14.7) | 5 (14.7) | 2 (5.9) |
| | Vibegron | 39 | 37 | 6 (16.2) | 3 (8.1) | 0 |
| | | | | | | |
| Pre-existing hypertension: NO | Placebo | 72 | 69 | 5 (7.2) | 0 | 0 |
| | Vibegron | 67 | 61 | 8 (13.1) | 3 (4.9) | 0 |
| Population: SAF | | | | | | |
| n=number of subjects with baseline and 3 post-baseline visits, SAF = Safety Set, 75th percentile age 66 years old | | | | | | |

Table 113. In-Clinic Vital Signs (SBP): Categorical Summary of Change from Baseline to End of Treatment Shifts

| Parameter | Treatment | N | n* | ≥ 5 n (%) | ≥ 10 n (%) | ≥ 15 n (%) |
|---|---|---|---|---|---|---|
| | | | | | | |
| <= 25th percentile BW | Placebo | 24 | 24 | 8 (33.3) | 3 (12.5) | 2 (8.3) |
| | Vibegron | 31 | 30 | 13 (43.3) | 9 (30.0) | 5 (16.7) |
| | | | | | | |
| >25th percentile BW | Placebo | 84 | 83 | 30 (36.1) | 11 (13.3) | 8 (9.6) |
| | Vibegron | 75 | 75 | 23 (30.7) | 13 (17.3) | 8 (10.7) |
| | | | | | | |
| eGFR <= 25th percentile | Placebo | 26 | 26 | 10 (38.5) | 7 (26.9) | 5 (19.2) |
| | Vibegron | 30 | 30 | 13 (43.3) | 9 (30.0) | 6 (20.0) |
| | | | | | | |
| eGFR >25th percentile | Placebo | 82 | 81 | 28 (34.6) | 7 (8.6) | 5 (6.2) |
| | Vibegron | 76 | 75 | 23 (30.7) | 13 (17.3) | 7 (9.3) |
| Population: SAF | | | | | | |
| *n=number of subjects with baseline and any post-baseline visit, SAF = Safety Set SBP= Systolic Blood Pressure eGFR, 25th percentile=72 mL/min/1.73m$^2$, 25th percentile BW=65.4 kg | | | | | | |

Table 114. In-Clinic Vital Signs (SBP) Categorical Summary of Change from Baseline to End of Treatment Shifts

| Parameter | Treatment | N | n* | ≥ 5 n (%) | ≥ 10 n (%) | ≥ 15 n (%) |
|---|---|---|---|---|---|---|
| Age <= 75th percentile | Placebo | 81 | 80 | 28 (35.0) | 9 (11.3) | 6 (7.5) |
| | Vibegron | 84 | 83 | 29 (34.9) | 18 (21.7) | 10 (12.0) |
| | | | | | | |
| Age > 75th percentile | Placebo | 27 | 27 | 10 (37.0) | 5 (18.5) | 4 (14.8) |
| | Vibegron | 22 | 22 | 7 (31.8) | 4 (18.2) | 3 (13.6) |
| | | | | | | |
| Pre-existing hypertension: YES | Placebo | 36 | 36 | 12 (33.3) | 7 (19.4) | 6 (16.7) |
| | Vibegron | 39 | 39 | 14 (35.9) | 8 (20.5) | 4 (10.3) |
| | | | | | | |
| Pre-existing hypertension: NO | Placebo | 72 | 71 | 26 (36.6) | 7 (9.9) | 4 (5.6) |
| | Vibegron | 67 | 66 | 22 (33.3) | 14 (21.2) | 9 (13.6) |
| Population: SAF | | | | | | |
| *n=number of subjects with baseline and 3 post-baseline visits, SAF = Safety Set SBP= Systolic th Blood Pressure , 75 percentile age 66 years old | | | | | | |

Summaries of adverse events are shown in Tables 115, 116, and 117.

Table 115. Overall Treatment Emergent Adverse Events

| | Placebo N=108 n (%) | Vibegron N=106 n (%) |
|---|---|---|
| Adverse event (AE) | 27 (25.0) | 49 (46.2) |
| Serious adverse event (SAE) | 1 (0.9) | 1 (0.9) |
| Fatal adverse event | 0 | 0 |
| Treatment-related SAE by the investigator | 0 | 0 |

(continued)

| | Placebo N=108 n (%) | Vibegron N=106 n (%) |
|---|---|---|
| AEs Leading to Treatment Discontinuation[1] | 1 (0.9) | 3 (2.8) |
| AECIs | 4 (3.7) | 8 (7.5) |
| Treatment-related AECIs | 3 (2.8) | 3 (2.8) |
| Population: Safety | | |
| [1] Placebo: blood pressure increase, vibegron: 1.) nausea, vomiting dizziness 2.) back pain 3.) feeling hot & anxiety | | |

Table 116. Most Common Adverse Events: Vibegron >2% and >Placebo

| Preferred Term | Placebo N=108 n (%) | Vibegron N=106 n (%) |
|---|---|---|
| Hypertension | 4 (3.7) | 5 (4.7) |
| Upper respiratory tract infection | 1 (0.9) | 5 (4.7) |
| Headache | 2 (1.9) | 4 (3.8) |
| Diarrhea | 1 (0.9) | 3 (2.8) |
| Dry mouth | 1 (0.9) | 3 (2.8) |
| Fatigue | 0 | 3 (2.8) |
| Haematuria | 0 | 3 (2.8) |
| Nasopharyngitis | 3 (2.8) | 3 (2.8) |
| Nausea | 1 (0.9) | 3 (2.8) |
| Urinary tract infection | 1 (0.9) | 3 (2.8) |
| Population: Safety | | |

Table 117. Selected Adverse Events

| Preferred Term | Placebo N=108 n (%) | Vibegron N=106 n (%) |
|---|---|---|
| Hypertension | 4 (3.7) | 5 (4.7) |
| Blood pressure increased | 1 (0.9) | 0 |
| Tachycardia | 0 | 0 |
| Hypotension | 0 | 1 (0.9) |
| Urinary tract infection | 1 (0.9) | 3 (2.8) |
| Increased residual urine | 0 | 0 |
| Urinary retention | 0 | 1 (0.9) |
| Constipation | 1 (0.9) | 2 (1.9) |
| Diarrhea | 1 (0.9) | 3 (2.8) |
| Population: Safety | | |

Table 118 shows a QTc study comparison between mirabegron and vibegron.

Table 118. QTc Study Comparison - Mirabegron and Vibegron: BP and HR Data in Healthy Subjects

| | Vibegron (From QT Study) | Mirabegron (From QT Study/Label 4/2018) |
|---|---|---|
| **Doses** | 200, 400 mg (single-dose) | 50, 100, 200 mg (repeat-dose) |

(continued)

| | Vibegron (From QT Study) | Mirabegron (From QT Study/Label 4/2018) |
|---|---|---|
| SBP | At Cmax, +2.2, +3.97 mmHg (200, 400 mg)*<br>24-hour average: +0.7, 1.3 mmHg (200, 400 mg)<br>75mg estimate<br>At Cmax: 0.4 mmHg<br>24-hour average: 0.1 mmHg | Maximum mean +4.0 mmHg (50 mg)<br>24-hour average: +3.0, +5.5, +9.7 mmHg (50, 100, 200 mg) |
| HR | At Cmax, 4.1 and 11.2 BPM (200, 400 mg) | Maximum mean 6.7, 11, 17 bpm |
| * Vibegron 200 and 400 mg dose = 3.3x and 9x exposures of 75 mg<br>All data presented as placebo adjusted | | |

The safety profile was in line with the Phase 3 study described in Example 6 with 1 AE more of hypertension in the vibegron group over placebo. This study overall confirms the safety profile of vibegron with no clinically relevant effects on blood pressure.

The following are examples of certain embodiments of the invention:

Embodiment 1: A method of treating overactive bladder, the method comprising orally administering to a subject in need thereof an amount of 75 mg of vibegron per day, wherein the treatment achieves at least one of changes (1) to (5) from baseline over a treatment period:

(1) a change in average number of micturitions per 24 hours of from about -1.3 to about -2.5;
(2) a change in average number of UUI episodes per 24 hours of from about -1.5 to about -2.5 when the subject is an OAB Wet patient;
(3) a change in average number of urgency episodes per 24 hours of from about -2.2 to about -3.5;
(4) a change in average number of total incontinence episodes per 24 hours of from about -1.7 to about -2.7; and
(5) a change in average volume voided per micturition of from about 18 mL to about 30 mL.

Embodiment 2: The method of Embodiment 1, wherein the treatment achieves at least two of changes (1) to (5) from baseline over the treatment period.
Embodiment 3: The method of Embodiment 2, wherein the treatment achieves changes (1) and (2) from baseline over the treatment period.
Embodiment 4: The method of any one of Embodiments 1 to 3, wherein the change in average number of micturitions per 24 hours is from about -1.5 to about -2.1, or preferably from about -1.6 to about -2.0.
Embodiment 5: The method of any one of Embodiments1 to 4, wherein the change in average number of UUI episodes per 24 hours is from about -1.7 to about -2.3, or preferably from about -1.8 to about -2.2.
Embodiment 6: The method of any one of Embodiments 1 to 5, wherein the change in average number of urgency episodes per 24 hours is from about -2.4 to about - 3.0, or preferably from about -2.5 to -2.9.
Embodiment 7: The method of any one of Embodiments 1 to 6, wherein the change in average number of total incontinence episodes per 24 hours is from about -1.9 to about -2.5, or preferably from about -2.0 to -2.4.
Embodiment 8: The method of any one of Embodiments 1 to 7, wherein the change in average volume voided per micturition is from about 20 mL to about 28 mL, or preferably from about 22 mL to about 26 mL.
Embodiment 9: The method of any one of Embodiments 1 to 8, wherein the subject has a symptom selected from the group consisting of urgency urinary incontinence, urinary urgency, urinary frequency, or a combination thereof.
Embodiment 10: The method of Embodiment 9, wherein the subject has the symptoms of urgency urinary incontinence, urinary urgency, and urinary frequency.
Embodiment 11: A method of reducing the average number of micturitions per 24 hours in a subject suffering from overactive bladder, the method comprising orally administering to the subject in need thereof an amount of 75 mg of vibegron per day over a treatment period.
Embodiment 12: A method of reducing the average number of UUI episodes per 24 hours in a subject suffering from overactive bladder, the method comprising orally administering to the subject in need thereof an amount of 75 mg of vibegron per day over a treatment period.
Embodiment 13: A method of reducing the average number of urgency episodes per 24 hours in a subject suffering from overactive bladder, the method comprising orally administering to the subject in need thereof an amount of 75 mg of vibegron per day over a treatment period.
Embodiment 14: A method of reducing the average number of total incontinence episodes per 24 hours in a subject

suffering from overactive bladder, the method comprising orally administering to the subject in need thereof an amount of 75 mg of vibegron per day over a treatment period.

Embodiment 15: A method of increasing the average volume voided per micturition in a subject suffering from overactive bladder, the method comprising orally administering to the subject in need thereof an amount of 75 mg of vibegron per day over a treatment period.

Embodiment 16: The method of any one of Embodiments 1 to 15, wherein the subject is a human.

Embodiment 17: The method of Embodiment 16, wherein the human is a female.

Embodiment 18: The method of Embodiment 16, wherein the human is a male.

Embodiment 19: The method of any one of Embodiments 16 to 18, wherein the human is at or over the age of 65 years.

Embodiment 20: The method of any one of Embodiments 16 to 18, wherein the human is over the age of 75 years.

Embodiment 21: The method of any one of Embodiments 1 to 20, wherein the subject suffers from severe renal impairment.

Embodiment 22: The method of any one of Embodiments 1 to 20, wherein the subject suffers from moderate renal impairment.

Embodiment 23: The method of any one of Embodiments 1 to 22, wherein the subject is concomitantly receiving a CYP3A/P-glycoprotein inhibitor.

Embodiment 24: The method of any one of Embodiments 1 to 23, wherein the subject is concomitantly receiving a CYP2D6 substrate.

Embodiment 25: The method of any one of Embodiments 1 to 24 wherein the subject received an anticholinergic up to 12 months before vibegron administration.

Embodiment 26: The method of any one of Embodiments 1 to 25 wherein the subject received a beta-3 agonist other than vibegron up to 12 months before vibegron administration.

Embodiment 27: The method of any one of Embodiments 1 to 26, wherein vibegron is administered once per day.

Embodiment 28: The method of any one of Embodiments 1 to 27, wherein vibegron is administered as a free base.

Embodiment 29: The method of any one of Embodiments 1 to 28, wherein vibegron is administered as a pharmaceutically acceptable salt thereof.

Embodiment 30: The method of any one of Embodiments 1 to 29, wherein the subject experiences a mean maximum change of systolic blood pressure from baseline over a treatment period, wherein the mean maximum change is less than 1 mm Hg from that of a subject taking a placebo.

Embodiment 31: The method of any one of Embodiments 1 to 29, wherein the subject experiences a mean maximum change of systolic blood pressure from baseline over a treatment period of less than 8 mm Hg.

Embodiment 32: The method of any one of Embodiments 1 to 29, wherein the subject experiences a mean maximum change of diastolic blood pressure from baseline over a treatment period, wherein the mean maximum change is less than 1 mm Hg from that of a subject taking a placebo.

Embodiment 33: The method of any one of Embodiments 1 to 29, wherein the subject experiences a mean maximum change of diastolic blood pressure from baseline over a treatment period of less than 5 mm Hg.

Embodiment 34: The method of any one of Embodiments 1 to 33, wherein the treatment period is selected from the group consisting of 2, 4, 6, 8, 10, 12, 14, 16, 18, 20, 22, 24, 26, 28, 30, 32, 34, 36, 38, 40, 42, 44, 46, 48, 50, and 52 weeks.

Embodiment 35: The method of any one of Embodiments 1 to 33, wherein vibegron achieves onset of action at about 4 weeks, about 3 weeks, or about 2 weeks.

Embodiment 36: A method of treating overactive bladder, the method comprising orally administering to a subject in need thereof an amount of 75 mg of vibegron per day, wherein the treatment achieves at least one of changes (1) to (5) over a 12-week treatment period:

(1) a reduction in average number of micturitions per 24 hours that is greater than or equivalent to that achieved with tolterodine extended release (ER) 4mg;

(2) a reduction in average number of UUI episodes per 24 hours that is greater than or equivalent to that achieved with tolterodine ER 4mg, when the subject is an OAB Wet patient;

(3) a reduction in average number of urgency episodes per 24 hours that is greater than or equivalent to that achieved with tolterodine ER 4mg;

(4) a reduction in average number of total incontinence episodes per 24 hours that is greater than or equivalent to that achieved with tolterodine ER 4mg; and

(5) an increase in average volume voided per micturition that is greater than or equivalent to that achieved with tolterodine ER 4mg.

Embodiment 37: The method of Embodiment 36, wherein the treatment achieves a change that is greater than that achieved with tolterodine ER 4mg.

Embodiment 38: The method of Embodiment 36, wherein the treatment achieves at least two of changes (1) to (5) from baseline over the treatment period.

Embodiment 39: The method of Embodiment 37, wherein the treatment achieves changes (2) and (4) from baseline over the treatment period.

Embodiment 40: The method of any one of Embodiments 36 to 39, wherein the subject has a symptom selected from the group consisting of urgency urinary incontinence, urinary urgency, urinary frequency, or a combination thereof.

Embodiment 41: The method of Embodiment 40, wherein the subject has the symptoms of urgency urinary incontinence, urinary urgency, and urinary frequency.

Embodiment 42: A method of reducing the average number of micturitions per 24 hours in a subject suffering from overactive bladder, the method comprising orally administering to the subject in need thereof an amount of 75 mg of vibegron per day over a 12-week treatment period, wherein the reduction is greater than or equivalent to that achieved with tolterodine extended release (ER) 4mg.

Embodiemnt 43: The method of Embodiment 42, wherein the subject is a human.

Embodiment 44: The method of Embodiment 43, wherein the human is at or over the age of 65 years.

Embodiment 45: The method of Embodiment 44, wherein the reduction in average number of micturitions in a 24-hour period is between about 1.5 and about 2.0 times greater than that achieved with a placebo.

Embodiment 46: The method of Embodiment 44, wherein the reduction in average number of micturitions in a 24-hour period is between about 1.0 and about 1.5 times greater than that achieved with tolterodine extended release (ER) 4mg.

Embodiment 47: The method of Embodiment 43, wherein the subject received an anticholinergic up to 12 months before vibegron administration.

Embodiment 48: The method of Embodiment 47, wherein the decrease in average number of micturitions in a 24-hour period is between about 1.5 and about 2.0 times greater than that achieved with a placebo.

Embodiment 49: The method of Embodiment 47, wherein the decrease in average number of micturitions in a 24-hour period is between about 1.0 and about 1.5 times greater than that achieved with tolterodine extended release (ER) 4mg.

Embodiment 50: The method of Embodiment 43,wherein the subject received a beta-3 agonist other than vibegron up to 12 months before vibegron administration.

Embodiment 51: The method of Embodiment 50, wherein the average number of micturitions in a 24-hour period is between about 1 and about 4 fewer than average number of micturitions in a subject who receives a placebo.

Embodiment 52: The method of Embodiment 50, wherein the decrease in average number of micturitions in a 24-hour period is between about 2.0 and about 2.5 times greater than that achieved with tolterodine extended release (ER) 4mg.

Embodiment 53: A method of reducing the average number of UUI episodes per 24 hours in a subject suffering from overactive bladder, the method comprising orally administering to the subject in need thereof an amount of 75 mg of vibegron per day over a 12-week treatment period, wherein the reduction is greater than or equivalent to that achieved with tolterodine extended release (ER) 4mg.

Embodiment 54: The method of Embodiment 53, wherein the subject is a human.

Embodiment 55: The method of Embodiment54, wherein the human is at or over the age of 65 years.

Embodiment 56: The method of Embodiment 55, wherein the reduction in average number of UUI episodes in a 24-hour period is between about 1.25 and about 1.75 times greater than that achieved with a placebo.

Embodiment 57: The method of Embodiment 55, wherein the subject received an anticholinergic up to 12 months before vibegron administration.

Embodiment 58: The method of Embodiment 57, wherein the reduction in average number of UUI episodes in a 24-hour period is between about 1.50 and about 2.0 times greater than that achieved with a placebo.

Embodiment 59: The method of Embodiment 57, wherein the reduction in average number of UUI episodes in a 24-hour period is between about 1.25 and about 1.75 times greater than that achieved with tolterodine extended release (ER) 4mg.

Embodiment 60: The method of Embodiment 55, wherein the subject received a beta-3 agonist other than vibegron up to 12 months before vibegron administration.

Embodiment 61: The method of Embodiment 60, wherein the decrease in average number of UUI episodes in a 24-hour period is between about 4 and about 6 times greater than that achieved with a placebo.

Embodiment 62: The method of Embodiment 60, wherein the decrease in average number of UUI episodes in a 24-hour period is between about 1.5 and about 3 times greater than that achieved with tolterodine extended release (ER) 4mg.

Embodiment 63: A method of reducing the average number of urgency episodes per 24 hours in a subject suffering from overactive bladder, the method comprising orally administering to the subject in need thereof an amount of 75 mg of vibegron per day over a 12-week treatment period, wherein the reduction is greater than or equivalent to that

achieved with tolterodine extended release (ER) 4mg.

Embodiment 64: A method of reducing the average number of total incontinence episodes per 24 hours in a subject suffering from overactive bladder, the method comprising orally administering to the subject in need thereof an amount of 75 mg of vibegron per day over a 12-week treatment period, wherein the reduction is greater than or equivalent to that achieved with tolterodine extended release (ER) 4mg.

Embodiment 65: A method of increasing the average volume voided per micturition in a subject suffering from overactive bladder, the method comprising orally administering to the subject in need thereof an amount of 75 mg of vibegron per day over a 12-week treatment period, wherein the increase is greater than or equivalent to that achieved with tolterodine extended release (ER) 4mg.

Embodiment 66: The method of any one of Embodiments 63 to 65, wherein the subject is a human.

Embodiment 67: The method of Embodiment 66, wherein the human is a female.

Embodiment 68: The method of Embodiment 66, wherein the human is a male.

Embodiment 69: The method of any one of Embodiments 63 to 68, wherein the human is at or over the age of 65 years.

Embodiment 70: The method of any one of Embodiments 63 to 68, wherein the human is over the age of 75 years.

Embodiment 71: The method of any one of Embodiments 36 to 70, wherein the subject suffers from severe renal impairment.

Embodiment 72: The method of any one of Embodiments 36 to 70, wherein the subject suffers from moderate renal impairment.

Embodiment 73: The method of any one of Embodiments 36 to 72, wherein the subject is concomitantly receiving a CYP3A/P-glycoprotein inhibitor.

Embodiment 74: The method of any one of Embodiments 36 to 73, wherein the subject is concomitantly receiving a CYP2D6 substrate.

Embodiment 75: The method of any one of Embodiments 36 to 74, wherein vibegron is administered once per day.

Embodiment 76: The method of any one of Embodiments 36 to 75, wherein vibegron is administered as a free base.

Embodiment 77: The method of any one of Embodiments 36 to 76, wherein vibegron is administered as a pharmaceutically acceptable salt thereof.

Embodiment 78: The method of any one of Embodiments 36 to 77, wherein the subject experiences a mean maximum change of systolic blood pressure from baseline over the treatment period, wherein the mean maximum change is less than 1 mm Hg from that of a subject taking a placebo.

Embodiment 79: The method of any one of Embodiments 36 to 77, wherein the subject experiences a mean maximum change of systolic blood pressure from baseline over the treatment period of less than 8 mm Hg.

Embodiment 80: The method of any one of Embodiments 36 to 77, wherein the subject experiences a mean maximum change of diastolic blood pressure from baseline over the treatment period, wherein the mean maximum change is less than 1 mm Hg from that of a subject taking a placebo.

Embodiment 81: The method of any one of 36 to 77, wherein the subject experiences a mean maximum change of diastolic blood pressure from baseline over the treatment period of less than 5 mm Hg.

Embodiment 82: The method of any one of Embodiments 1 to 81, wherein vibegron achieves onset of action at about 4 weeks, about 3 weeks, or about 2 weeks.

Embodiment 83: A method of improving from baseline coping in a subject suffering from overactive bladder, the method comprising orally administering to the subject in need thereof an amount of 75 mg of vibegron per day over a treatment period.

Embodiment 84: The method of Embodiment 83, wherein the improvement in coping is greater than that achieved with tolterodine extended release (ER) 4mg.

Embodiment 85: A method of improving from baseline sleep in a subject suffering from overactive bladder, the method comprising orally administering to the subject in need thereof an amount of 75 mg of vibegron per day over a treatment period.

Embodiment 86: The method of Embodiment 85, wherein the improvement in sleep is greater than that achieved with tolterodine extended release (ER) 4mg.

Embodiment 87: A method of improving from baseline Health-related Quality of Life (HRQL) in a subject suffering from overactive bladder, the method comprising orally administering to the subject in need thereof an amount of 75 mg of vibegron per day over a treatment period.

Embodiment 88: The method of Embodiment 87, wherein the improvement in HRQL is greater than that achieved with tolterodine extended release (ER) 4mg.

Embodiment 89: The method of Embodiment 87 or 88, wherein the HRQL includes one or more subscales selected from coping, concern, sleep, or social interaction.

Embodiment 90: A method of decreasing from baseline symptom bother in a subject suffering from overactive bladder, the method comprising orally administering to the subject in need thereof an amount of 75 mg of vibegron per day over a treatment period.

Embodiment 91: The method of Embodiment 90, wherein the decrease in symptom bother is greater than that achieved with tolterodine extended release (ER) 4mg.

Embodiment 92: A method of treating overactive bladder, the method comprising orally administering to a subject in need thereof an amount of 75 mg of vibegron per day, wherein the subject experiences a mean change of daytime ambulatory blood pressure from baseline over a treatment period of less than about 2.0 mm Hg.

Embodiment 93: The method of Embodiment 92, wherein the subject experiences a mean change of daytime ambulatory systolic blood pressure from baseline over a treatment period and wherein the mean change from that of a subject taking a placebo has an upper bound of a 90% confidence interval less than about 3.5 mm Hg.

Embodiment 94: The method of Embodiment 93, wherein the upper bound of a 90% confidence interval is less than about 2.5 mm Hg.

Embodiment 95: The method of embodiment 93, wherein the upper bound of a 90% confidence interval is about 2.0 mm Hg.

Embodiment 96: The method of Embodiment 92, wherein the subject experiences a mean change of daytime ambulatory systolic blood pressure from baseline over a treatment period and wherein the mean change is less than about 1.0 mm Hg from that of a subject taking a placebo.

Embodiment 97: The method of Embodiment 96, wherein the mean change is less than about 0.5 mm Hg from that of a subject taking a placebo.

Embodiment 98: The method of Embodiment 92, wherein the subject experiences a mean change of daytime ambulatory systolic blood pressure from baseline over a treatment period of less than about 1.0 mm Hg.

Embodiment 99: The method of Embodiment 98, wherein the mean change is less than about 0.25 mm Hg.

Embodiment 100: The method of Embodiment 92, wherein the subject does not experience a mean change of daytime ambulatory diastolic blood pressure from baseline over a treatment period greater than that of a subject taking a placebo.

Embodiment 101: The method of Embodiment 100, wherein the subject experiences a mean change of daytime ambulatory diastolic blood pressure from baseline over a treatment period of less than about 0.75 mm Hg.

Embodiment 102: A method of treating overactive bladder, the method comprising orally administering to a subject in need thereof an amount of 75 mg of vibegron per day, wherein the subject experiences a mean change of daytime ambulatory heart rate from baseline over a treatment period of less than about 1.25 bpm.

Embodiment 103: The method of Embodiment 102, wherein the subject experiences a mean change of daytime ambulatory heart rate from baseline over a treatment period and wherein the mean change from that of a subject taking a placebo is less than about 1.0 bpm.

Embodiment 104: A method of treating overactive bladder, the method comprising orally administering to a subject in need thereof an amount of 75 mg of vibegron per day, wherein the subject experiences a mean change of 24-hour ambulatory blood pressure from baseline over a treatment period of less than about 2.0 mm Hg.

Embodiment 105: The method of Embodiment 104, wherein the subject experiences a mean change of 24-hour ambulatory systolic blood pressure from baseline over a treatment period and wherein the mean change from that of a subject taking a placebo is less than about 0.75 mm Hg.

Embodiment 106: The method of Embodiment 104, wherein the subject experiences a mean change of 24-hour ambulatory systolic blood pressure from baseline over a treatment period of less than about 0.75 mm Hg.

Embodiment 107: The method of Embodiment 104, wherein the subject does not experience a mean change of 24-hour ambulatory diastolic blood pressure from baseline over a treatment period greater than that of a subject taking a placebo.

Embodiment 108: The method of Embodiment 104, wherein the subject experiences a mean change of 24-hour ambulatory diastolic blood pressure from baseline over a treatment period of less than 0.75 mm Hg.

Embodiment 109: A method of treating overactive bladder, the method comprising orally administering to a subject in need thereof an amount of 75 mg of vibegron per day, wherein the subject experiences a mean change of 24-hour ambulatory heart rate from baseline over a treatment period of less than about 1.0 bpm.

Embodiment 110: The method of Embodiment 109, wherein the mean change from that of a subject taking a placebo is less than about 1.0 bpm.

Embodiment 111: A method of treating overactive bladder, the method comprising orally administering to a subject in need thereof an amount of 75 mg of vibegron per day, wherein the subject experiences a mean change of systolic blood pressure at $C_{max}$ of less than about 0.50 mm Hg.

Embodiment 112: A method of treating overactive bladder, the method comprising orally administering to a subject in need thereof an amount of 75 mg of vibegron per day, wherein the subject experiences a mean change of 24-hour systolic blood pressure of less than about 0.50 mm Hg.

Embodiment 113: A method of treating overactive bladder, the method comprising orally administering to a subject in need thereof an amount of 75 mg of vibegron per day, wherein the subject experiences a maximum mean change of blood pressure from baseline over 0.5 hours to 6.5 hours post-dose of less than about 2.0 mm Hg.

Embodiment 114: The method of Embodiment 113, wherein the subject experiences a maximum mean change of systolic blood pressure from baseline over 0.5 hours to 6.5 hours post-dose and wherein the maximum mean change is less than about 1.75 mm Hg from that of a subject taking a placebo.
Embodiment 115: The method of Embodiment 113, wherein the subject experiences a maximum mean change of systolic blood pressure from baseline over 0.5 hours to 6.5 hours post-dose of less than about 2.0 mm Hg.
Embodiment 116: The method of Embodiment 113, wherein the subject experiences a maximum mean change of diastolic blood pressure from baseline over 0.5 hours to 6.5 hours post-dose and wherein the maximum mean change is less than about 1.25 mm Hg from that of a subject taking a placebo.
Embodiment 117: The method of Embodiment 113, wherein the subject experiences a maximum mean change of diastolic blood pressure from baseline over 0.5 hours to 6.5 hours post-dose of less than 0.75 mm Hg.
Embodiment 118: A method of treating overactive bladder, the method comprising orally administering to a subject in need thereof an amount of 75 mg of vibegron per day, wherein the subject experiences a maximum mean change of heart rate from baseline over 0.5 hours to 6.5 hours post-dose of less than about 2.0 bpm.
Embodiment 119: The method of Embodiment 118, wherein the maximum mean change is less than about 1.50 bpm from that of a subject taking a placebo.
Embodiment 120: A method of treating overactive bladder, the method comprising orally administering to a subject in need thereof an amount of from about 75 mg to about 400 mg of vibegron per day, wherein the subject experiences a smaller maximum mean change in systolic blood pressure than a subject receiving a therapeutically effective amount of mirabegron.
Embodiment 121: The method of Embodiment 120, wherein the subject receiving vibegron experiences an increase in maximum mean change in systolic blood pressure of from about 1.5 mm Hg to about 4.0 mm Hg smaller than a subject receiving a therapeutically effective amount of mirabegron.
Embodiment 122: A method of treating overactive bladder, the method comprising orally administering to a subject in need thereof an amount of from about 75 mg to about 400 mg of vibegron per day, wherein the subject experiences a smaller mean 24-hour change in systolic blood pressure than a subject receiving a therapeutically effective amount of mirabegron.
Embodiment 123: The method of Embodiment 122, wherein the subject receiving vibegron experiences an increase in mean 24-hour change in systolic blood pressure of from about 1.0 mm Hg to about 10.0 mm Hg smaller than a subject receiving a therapeutically effective amount of mirabegron.
Embodiment 124: A method of treating overactive bladder, the method comprising orally administering to a subject in need thereof an amount of from about 75 mg to about 400 mg of vibegron per day, wherein the subject experiences a smaller maximum mean increase in heart rate than a subject receiving a therapeutically effective amount of mirabegron.
Embodiment 125: The method of Embodiment 124, wherein the subject receiving vibegron experiences an increase in maximum mean increase in heart rate that is from about 2 bpm to about 14 bpm smaller than a subject receiving a therapeutically effective amount of mirabegron.
Embodiment 126: The method of any one of Embodiments 120 to 125, wherein the therapeutically effective amount of mirabegron is from about 50 mg to about 200 mg.
Embodiment 127: The method of any one of Embodiments 92 to 126, wherein the subject is a human.
Embodiment 128: The method of Embodiment 127, wherein the human is a male.
Embodiment 129: The method of Embodiment 127, wherein the human is a female.
Embodiment 130: The method of any one of Embodiments 127 to 129, wherein the human has a body weight greater than about 65 kg.
Embodiment 131: The method of any one of Embodiments 127 to 129, wherein the human has a body weight less than about 65 kg.
Embodiment 132: The method of any one of Embodiments 127 to 129, wherein the human is at or over the age of about 67 years.
Embodiment 133: The method of Embodiment 132 wherein the human is from the age of about 67 years to about 75 years.
Embodiment 134: The method of any one of Embodiments 127 to 129, wherein the human is under the age of about 67 years.
Embodiment 135: The method of any one of Embodiments 127 to 129, wherein the human is hypertensive or is at risk of hypertension.
Embodiment 136: The method of any one of Embodiments 127 to 129, wherein the human has chronic kidney disease.
Embodiment 137: The method of Embodiment 136, wherein the chronic kidney disease is Stage 1, Stage 2, Stage 3a, or Stage 3b.
Embodiment 138: The method of any one of Embodiments 127 to 129, wherein human has an estimated glomerular filtration rate (eGFR) of greater than about 30 mL/min/1.73m$^2$.

Embodiment 139: The method of Embodiment 138, wherein the eGFR is selected from about 30 to about 44 mL/min/1.73m$^2$, about 45 to about 59 mL/min/1.73m$^2$, about 60 to about 89 mL/min/1.73m$^2$, or about 90 or higher mL/min/1.73m$^2$.
Embodiment 140: The method of Embodiment 138, wherein the eGFR is greater than about 72 mL/min/1.73m$^2$.
Embodiment 141: The method of Embodiment 138, wherein the eGFR is less than about 72 mL/min/1.73m$^2$.
Embodiment 142: A method of treating overactive bladder, the method comprising orally administering to a subject in need thereof an amount of 75 mg of vibegron per day, wherein the subject has a body weight of greater than about 65 kg and wherein the subject experiences a similar mean change of systolic blood pressure from baseline over a treatment period compared to a subject taking placebo.
Embodiment 143: A method of treating overactive bladder, the method comprising orally administering to a subject in need thereof an amount of 75 mg of vibegron per day, wherein the subject is at or over the age of about 67 years and wherein the subject experiences a similar mean change of systolic blood pressure from baseline over a treatment period compared to a subject taking placebo.
Embodiment 144: The method of any one of Embodiments 36 to 91 wherein the reduction in average number of micturitions per 24 hours, reduction in average number of UUI episodes per 24 hours, reduction in average number of urgency episodes per 24 hours, reduction in average number of total incontinence episodes per 24 hours and/or increase in average volume voided per micturition at Week 52 is from about 100% to about 140% of those at Week 12.
Embodiment 145: Vibegron for use in a method of treating overactive bladder in a treatment-experienced subject, the method comprising orally administering to the subject about 75 mg of vibegron per day.
Embodiment 146: Vibegron for use according to Embodiment 145, wherein following administration of vibegron to the subject over a treatment period:

a. the decrease in the average number of micturitions in a 24-hour period in the subject is about 1.5 to about 10 more than the decrease in the average number of micturitions in a subject who receives a placebo; or

b. the average number of urge urinary incontinence (UUI) episodes per 24 hours by the subject decreases by about 1.7 to about 6 times the decrease as that of a subject treated with placebo.

Embodiment 147: Vibegron for use according to Embodiment 146, wherein the decrease in the average number of micturitions in a 24-hour period in the subject is about 2 to about 4 more than the decrease in the average number of micturitions in a subject who receives a placebo.
Embodiment 148: Vibegron for use according to Embodiment 146 or 147, wherein following administration of vibegron to the subject over a treatment period the average number of micturitions per 24 hours by the subject decreases from about -1.3 to about -2.5 and the average number of UUI episodes per 24 hours by the subject decreases from about -1.5 to about -2.5.
Embodiment 149: Vibegron for use according to any one of Embodiments 146-148, wherein following administration of vibegron to the subject over a treatment period the average number of micturitions in a 24-hour period by the subject decreases between about 1.5 and about 10 compared to the average number of micturitions in a subject who receives a placebo and the average number of UUI episodes per 24 hours by the subject decreases from about -1.5 to about -2.5.
Embodiment 150: Vibegron for use according to any one of Embodiments 145-149, wherein the treatment-experienced subject has been previously treated with an acetylcholinergic.
Embodiment 151: Vibegron for use according to Embodiment 150, wherein the treatment-experienced subject has been treated with an acetylcholinergic within 12 months prior to treatment with vibegron.
Embodiment 152: Vibegron for use according to Embodiment 150, wherein the treatment-experienced subject has been treated with an acetylcholinergic more than 12 months prior to treatment with vibegron.
Embodiment 153: Vibegron for use according to Embodiment 150, wherein the treatment-experienced subject is concomitantly being treated with an acetylcholinergic.
Embodiment 154: Vibegron for use according to any one of Embodiments 145-149, wherein the treatment-experienced subject has been previously treated with a beta-3 agonist other than vibegron.
Embodiment 155: Vibegron for use according to Embodiment 154, wherein the beta-3 agonist is mirabegron.
Embodiment 156: Vibegron for use according to Embodiment 154 or 155, wherein the treatment-experienced subject has been treated with a beta-3 agonist other than vibegron within 12 months prior to treatment with vibegron.
Embodiment 157: Vibegron for use according to Embodiment 154 or 155, wherein the treatment-experienced subject has been treated with a beta-3 agonist other than vibegron more than 12 months prior to treatment with vibegron.
Embodiment 158: Vibegron for use according to Embodiment 154 or 155, wherein the treatment-experienced subject is concomitantly being treated with a beta-3 agonist other than vibegron.
Embodiment 159: Vibegron for use in a method of improving Health-related Quality of Life (HRQL) in a subject

suffering from overactive bladder symptoms, the method comprising orally administering to the subject about 75 mg of vibegron per day over a treatment period and wherein the improvement is compared to the HRQL of a subject receiving placebo.

Embodiment 160: Vibegron for use according to Embodiment 159, wherein the HRQL includes one or more subscales selected from coping, concern, sleep, or social interaction.

Embodiment 161: Vibegron for use according to Embodiment 159 or 160, wherein the improvement in HRQL is greater than with tolterodine extended release (ER) 4mg.

Embodiment 162: Vibegron for use according to any one of Embodiments 159-161, wherein the HRQL of the subject receiving vibegron is improved by a total score of at least about 3.8 compared to a subject receiving placebo.

Embodiment 163: Vibegron for use in a method of reducing coping behaviors in a subject suffering from overactive bladder symptoms, the method comprising orally administering to the subject about 75 mg of vibegron per day over a treatment period and wherein the coping behaviors in the subject are reduced compared to a subject receiving placebo.

Embodiment 164: Vibegron for use in a method of improving coping domain score in a subject suffering from overactive bladder symptoms, the method comprising orally administering to the subject about 75 mg of vibegron per day over a treatment period and wherein the improvement is compared to the coping domain score of a subject receiving placebo.

Embodiment 165: Vibegron for use according to Embodiment 164, wherein the improvement in coping domain score is greater than with tolterodine extended release (ER) 4mg.

Embodiment 166: Vibegron for use according to Embodiments 164 or 165, wherein the coping domain score of the subject receiving vibegron is improved by a score of at least about 3.2 compared to a subject receiving placebo.

Embodiment 167: Vibegron for use in a method of improving sleep in a subject suffering from overactive bladder symptoms, the method comprising orally administering to the subject about 75 mg of vibegron per day over a treatment period and wherein the improvement is compared to the sleep of a subject receiving placebo.

Embodiment 168: Vibegron for use according to Embodiment 167, wherein the improvement in sleep is greater than with tolterodine extended release (ER) 4mg.

Embodiment 169: Vibegron for use according to Embodiments 167 or 168, wherein the sleep of the subject receiving vibegron is improved compared to a subject receiving placebo by a score of at least about 2.6.

Embodiment 170: Vibegron for use in a method of decreasing symptom bother in a subject suffering from overactive bladder symptoms, the method comprising orally administering to the subject about 75 mg of vibegron per day over a treatment period and wherein the decrease is compared to the symptom bother of a subject receiving placebo.

Embodiment 171: Vibegron for use according to Embodiment 170, wherein the decrease in symptom bother is greater than with tolterodine extended release (ER) 4mg.

Embodiment 172: Vibegron for use of any one of Embodiments 170 or 171, wherein the symptom bother of the subject receiving vibegron is decreased compared to a subject receiving placebo by a score of at least about -5.0.

Embodiment 173: Vibegron for use in a method of maintaining daytime ambulatory blood pressure while treating overactive bladder in a subject in need thereof, the method comprising orally administering to the subject about 75 mg, of vibegron per day over a treatment period and wherein the subject experiences a mean change of daytime ambulatory blood pressure over a treatment period of less than about 2.0 mm Hg.

Embodiment 174: Vibegron for use according to Embodiment 173, wherein the subject experiences a mean change of daytime ambulatory systolic blood pressure over a treatment period and wherein the mean change from that of a subject taking a placebo has an upper bound of a 90% confidence interval less than about 3.5 mm Hg.

Embodiment 175: Vibegron for use according to Embodiment 174, wherein the upper bound of a 90% confidence interval is less than about 2.5 mm Hg.

Embodiment 176: Vibegron for use according to Embodiment 175, wherein the upper bound of a 90% confidence interval is about 2.0 mm Hg.

Embodiment 177: Vibegron for use according to any one of Embodiments 173-176, wherein the subject experiences a mean change of daytime ambulatory systolic blood pressure over a treatment period and wherein the mean change is less than about 1.0 mm Hg from that of a subject taking a placebo.

Embodiment 178: Vibegron for use according to Embodiment 177, wherein the mean change is less than about 0.5 mm Hg from that of a subject taking a placebo.

Embodiment 179: Vibegron for use according to any one of Embodiments 173-178, wherein the subject experiences a mean change of daytime ambulatory systolic blood pressure over a treatment period of less than about 1.0 mm Hg.

Embodiment 180: Vibegron for use according to Embodiment 179, wherein the mean change is less than about 0.25 mm Hg.

Embodiment 181: Vibegron for use according to any one of Embodiments 173-180, wherein the subject does not experience a mean change of daytime ambulatory diastolic blood pressure over a treatment period greater than that of a subject taking a placebo.

Embodiment 182: Vibegron for use according to any one of Embodiments 173-181, wherein the subject experiences a mean change of daytime ambulatory diastolic blood pressure over a treatment period of less than about 0.75 mm Hg.

Embodiment 183: Vibegron for use in a method of maintaining daytime ambulatory heart rate while treating overactive bladder in a subject in need thereof, the method comprising orally administering to the subject about 75 mg of vibegron per day, and wherein the subject experiences a mean change of daytime ambulatory heart rate over a treatment period of less than about 1.25 bpm.

Embodiment 184: Vibegron for use according to Embodiment 183, wherein the subject experiences a mean change of daytime ambulatory heart rate over a treatment period and wherein the mean change from that of a subject taking a placebo is less than about 1.0 bpm.

Embodiment 185: Vibegron for use in a method of maintaining 24-hour ambulatory blood pressure while treating overactive bladder in a subject in need thereof, the method comprising orally administering to the subject about 75 mg of vibegron per day, and wherein the subject experiences a mean change of 24-hour ambulatory blood pressure over a treatment period of less than about 2.0 mm Hg.

Embodiment 186: Vibegron for use according to Embodiment 185, wherein the subject experiences a mean change of 24-hour ambulatory systolic blood pressure over a treatment period and wherein the mean change from that of a subject taking a placebo is less than about 0.75 mm Hg.

Embodiment 187: Vibegron for use according to Embodiment 186, wherein the subject experiences a mean change of 24-hour ambulatory systolic blood pressure over a treatment period of less than about 0.75 mm Hg.

Embodiment 188: Vibegron for use according to any one of Embodiments 185-187, wherein the subject does not experience a mean change of 24-hour ambulatory diastolic blood pressure over a treatment period greater than that of a subject taking a placebo.

Embodiment 189: Vibegron for use according to any one of Embodiments 185-188, wherein the subject experiences a mean change of 24-hour ambulatory diastolic blood pressure over a treatment period of less than 0.75 mm Hg.

Embodiment 190: Vibegron for use in a method of maintaining 24-hour ambulatory heart rate while treating overactive bladder in a subject in need thereof, the method comprising orally administering to the subject about 75 mg of vibegron per day, and wherein the subject experiences a mean change of 24-hour ambulatory heart rate over a treatment period of less than about 1.0 bpm.

Embodiment 191: Vibegron for use according to Embodiment 190, wherein the mean change from that of a subject taking a placebo is less than about 1.0 bpm.

Embodiment 192: Vibegron for use according to any one of Embodiments 173-191, wherein the subject has hypertension.

Embodiment 193: Vibegron for use according to any one of Embodiments 145-192, wherein vibegron is administered once per day.

Embodiment 194: Vibegron for use according to any one of Embodiments 145-193, wherein vibegron is administered as a free base.

Embodiment 195: Vibegron for use according to any one of Embodiments 145-193, wherein vibegron is administered as a pharmaceutically acceptable salt thereof.

Embodiment 196: Vibegron for use according to any one of Embodiments 145-195, wherein the treatment period is selected from the group consisting of about 2, 4, 6, 8, 10, 12, 14, 16, 18, 20, 22, 24, 26, 28, 30, 32, 34, 36, 38, 40, 42, 44, 46, 48, 50, and 52 weeks.

Embodiment 197: Vibegron for use according to any one of Embodiments 145-195, wherein the treatment period is selected from about 2, 4, 8, 12, and 52 weeks.

Embodiment 198: Vibegron for use according to any one of Embodiments 145-195, wherein the treatment period is about 12 weeks or wherein the treatment period is about 52 weeks.

Having now fully described this invention, it will be understood by those of ordinary skill in the art that the same can be performed within a wide and equivalent range of conditions, formulations and other parameters without affecting the scope of the invention or any embodiment thereof.

Other embodiments of the invention will be apparent to those skilled in the art from consideration of the specification and practice of the invention disclosed herein. It is intended that the specification and examples be considered as exemplary only, with a true scope and spirit of the invention being indicated by the following embodiments.

All patents, patent applications, and other publications cited herein are fully incorporated by reference herein in their entirety.

Further embodiments:

1. A method of treating overactive bladder in a treatment-experienced subject in need thereof, the method comprising orally administering to the subject a therapeutically effective amount of vibegron per day, wherein the therapeutically effective amount is about 75 mg, and wherein following administration of vibegron to the subject over a treatment period:

a. the decrease in the average number of micturitions in a 24-hour period in the subject is about 1.5 to about 10 more than the decrease in the average number of micturitions in a subject who receives a placebo; or

b. the average number of urge urinary incontinence (UUI) episodes per 24 hours by the subject decreases by about 1.7 to about 6 times the decrease as that of a subject treated with placebo.

2. The method of embodiment 1, wherein the decrease in the average number of micturitions in a 24-hour period in the subject is about 2 to about 4 more than the decrease in the average number of micturitions in a subject who receives a placebo.

3. The method of embodiment 1 or 2, wherein following administration of vibegron to the subject over a treatment period the average number of micturitions per 24 hours by the subject decreases from about -1.3 to about -2.5 and the average number of UUI episodes per 24 hours by the subject decreases from about -1.5 to about -2.5.

4. The method of any one of embodiments 1-3, wherein following administration of vibegron to the subject over a treatment period the average number of micturitions in a 24-hour period by the subject decreases between about 1.5 and about 10 compared to the average number of micturitions in a subject who receives a placebo and the average number of UUI episodes per 24 hours by the subject decreases from about -1.5 to about - 2.5.

5. The method of any one of embodiments 1 to 4, wherein following administration of vibegron to the subject over a treatment period the average number of urgency episodes per 24 hours decreases by about -2.2 to about -3.5.

6. The method of any one of embodiments 1 to 5, wherein following administration of vibegron to the subject over a treatment period the average number of total incontinence episodes decreases by about -1.7 to about -2.7.

7. The method of any one of embodiments 1 to 6, wherein following administration of vibegron to the subject over a treatment period the average amount of volume voided per micturition increases by about 18 mL to about 30 mL.

8. The method of any one of embodiments 1-7, wherein the treatment-experienced subject has been previously treated with an anticholinergic.

9. The method of embodiment 8, wherein the treatment-experienced subject has been treated with an anticholinergic within 12 months prior to treatment with vibegron.

10. The method of embodiment 8, wherein the treatment-experienced subject has been treated with an anticholinergic more than 12 months prior to treatment with vibegron.

11. The method of embodiment 8, wherein the treatment-experienced subject is concomitantly receiving an anticholinergic.

12. The method of any one of embodiments 1-8, wherein the treatment-experienced subject has been previously treated with a beta-3 agonist other than vibegron.

13. The method of embodiment 12, wherein the beta-3 agonist is mirabegron.

14. The method of embodiment 12 or 13, wherein the treatment-experienced subject has been treated with a beta-3 agonist other than vibegron within 12 months prior to treatment with vibegron.

15. The method of embodiment 12 or 13, wherein the treatment-experienced subject has been treated with a beta-3 agonist other than vibegron more than 12 months prior to treatment with vibegron.

16. The method of embodiment 12 or 13, wherein the treatment-experienced subject is concomitantly receiving a beta-3 agonist other than vibegron.

17. A method of treating overactive bladder in a subject in need thereof while improving Health-related Quality of Life (HRQL) , the method comprising orally administering to the subject in need thereof a therapeutically effective amount of vibegron per day over a treatment period, wherein the therapeutically effective amount is about 75 mg and wherein the improvement is compared to the HRQL of a subject receiving placebo.

18. The method of embodiment 17, wherein the HRQL includes one or more subscales selected from coping, concern, sleep, or social interaction.

19. The method of embodiment 17 or 18, wherein the improvement in HRQL is greater than with tolterodine extended release (ER) 4mg.

20. The method of any one of embodiments 17 to 19, wherein the HRQL of the subject receiving vibegron is improved by a total score of at least about 3.8 compared to a subject receiving placebo.

21. A method of reducing coping behaviors in a subject suffering from overactive bladder symptoms, the method comprising orally administering to the subject in need thereof a therapeutically effective amount of vibegron per day over a treatment period, wherein the therapeutically effective amount is about 75 mg and wherein the coping behaviors in the subject are reduced compared to a subject receiving placebo.

22. A method of improving coping domain score in a subject suffering from overactive bladder symptoms, the method comprising orally administering to the subject in need thereof a therapeutically effective amount of vibegron per day over a treatment period, wherein the therapeutically effective amount is about 75 mg and wherein the improvement is compared to the coping domain score of a subject receiving placebo.

23. The method of embodiment 22, wherein the improvement in coping domain score is greater than with tolterodine extended release (ER) 4mg.

24. The method of embodiment 22 or 23, wherein the coping domain score of the subject receiving vibegron is improved by a score of at least about 3.2 compared to a subject receiving placebo.

25. A method of improving sleep in a subject suffering from overactive bladder symptoms, the method comprising orally administering to the subject in need thereof a therapeutically effective amount of vibegron per day over a treatment period, wherein the therapeutically effective amount is about 75 mg and wherein the improvement is compared to the sleep of a subject receiving placebo.

26. The method of embodiment 25, wherein the improvement in sleep is greater than with tolterodine extended release (ER) 4mg.

27. The method of embodiment 25 or 26, wherein the sleep of the subject receiving vibegron is improved compared to a subject receiving placebo by a score of at least about 2.6.

28. A method of decreasing symptom bother in a subject suffering from overactive bladder symptoms, the method comprising orally administering to the subject in need thereof a therapeutically effective amount of vibegron per day over a treatment period, wherein the therapeutically effective amount is about 75 mg and wherein the decrease is compared to the symptom bother of a subject receiving placebo.

29. The method of embodiment 28, wherein the decrease in symptom bother is greater than with tolterodine extended release (ER) 4mg.

30. The method of embodiment 28 or 29, wherein the symptom bother of the subject receiving vibegron is decreased compared to a subject receiving placebo by a score of at least about -5.0.

31. A method of maintaining daytime ambulatory blood pressure while treating overactive bladder in a subject in need thereof, the method comprising orally administering to the subject a therapeutically effective amount of vibegron per day, wherein the therapeutically effective amount is about 75 mg, and wherein the subject experiences a mean change of daytime ambulatory blood pressure over a treatment period of less than about 2.0 mm Hg.

32. The method of embodiment 31, wherein the subject experiences a mean change of daytime ambulatory systolic

blood pressure over a treatment period and wherein the mean change from that of a subject taking a placebo has an upper bound of a 90% confidence interval less than about 3.5 mm Hg.

33. The method of embodiment 32, wherein the upper bound of a 90% confidence interval is less than about 2.5 mm Hg.

34. The method of embodiment 33, wherein the upper bound of a 90% confidence interval is about 2.0 mm Hg.

35. The method of any one of embodiments 31 to 34, wherein the subject experiences a mean change of daytime ambulatory systolic blood pressure over a treatment period and wherein the mean change is less than about 1.0 mm Hg from that of a subject taking a placebo.

36. The method of embodiment 35, wherein the mean change is less than about 0.5 mm Hg from that of a subject taking a placebo.

37. The method of any one of embodiments 31 to 36, wherein the subject experiences a mean change of daytime ambulatory systolic blood pressure over a treatment period of less than about 1.0 mm Hg.

38. The method of embodiment 37, wherein the mean change is less than about 0.25 mm Hg.

39. The method of any one of embodiments 31 to 38, wherein the subject does not experience a mean change of daytime ambulatory diastolic blood pressure over a treatment period greater than that of a subject taking a placebo.

40. The method of any one of embodiments 31 to 39, wherein the subject experiences a mean change of daytime ambulatory diastolic blood pressure over a treatment period of less than about 0.75 mm Hg.

41. A method of maintaining daytime ambulatory heart rate while treating overactive bladder in a subject in need thereof, the method comprising orally administering to the subject a therapeutically effective amount of vibegron per day, wherein the therapeutically effective amount is about 75 mg, and wherein the subject experiences a mean change of daytime ambulatory heart rate over a treatment period of less than about 1.25 bpm.

42. The method of embodiment 41, wherein the subject experiences a mean change of daytime ambulatory heart rate over a treatment period and wherein the mean change from that of a subject taking a placebo is less than about 1.0 bpm.

43. A method of maintaining 24-hour ambulatory blood pressure while treating overactive bladder in a subject in need thereof, the method comprising orally administering to the subject a therapeutically effective amount of vibegron per day, wherein the therapeutically effective amount is about 75 mg, and wherein the subject experiences a mean change of 24-hour ambulatory blood pressure over a treatment period of less than about 2.0 mm Hg.

44. The method of embodiment 43, wherein the subject experiences a mean change of 24-hour ambulatory systolic blood pressure over a treatment period and wherein the mean change from that of a subject taking a placebo is less than about 0.75 mm Hg.

45. The method of embodiment 43, wherein the subject experiences a mean change of 24-hour ambulatory systolic blood pressure over a treatment period of less than about 0.75 mm Hg.

46. The method of any one of embodiments to 43 to 45, wherein the subject does not experience a mean change of 24-hour ambulatory diastolic blood pressure over a treatment period greater than that of a subject taking a placebo.

47. The method of any one of embodiments 43 to 46, wherein the subject experiences a mean change of 24-hour ambulatory diastolic blood pressure over a treatment period of less than 0.75 mm Hg.

48. A method of maintaining 24-hour ambulatory heart rate while treating overactive bladder in a subject in need thereof, the method comprising orally administering to the subject a therapeutically effective amount of vibegron per day, wherein the therapeutically effective amount is about 75 mg, and wherein the subject experiences a mean change of 24-hour ambulatory heart rate over a treatment period of less than about 1.0 bpm.

49. The method of embodiment 48, wherein the mean change from that of a subject taking a placebo is less than about 1.0 bpm.

50. The method of any one of embodiments 31 to 49, wherein the subject being administered vibegron is hypertensive.

51. The method of any one of embodiments 1-50, wherein vibegron is administered once per day.

52. The method of any one of embodiments 1-51, wherein vibegron is administered as a free base.

53. The method of any one of embodiments 1-51, wherein vibegron is administered as a pharmaceutically acceptable salt thereof.

54. The method of any one of embodiments 1-53, wherein the treatment period is selected from the group consisting of about 2, 4, 6, 8, 10, 12, 14, 16, 18, 20, 22, 24, 26, 28, 30, 32, 34, 36, 38, 40, 42, 44, 46, 48, 50, and 52 weeks.

55. The method of any one of embodiments 1-53, wherein the treatment period is selected from about 2, 4, 8, 12, and 52 weeks.

56. The method of embodiment 55, wherein the treatment period is about 12 weeks.

57. The method of embodiment 55, wherein the treatment period is about 52 weeks.

## Claims

**1.** Use of a therapeutically effective amount of vibegron in the manufacture of a medicament for treating overactive bladder in a subject with bladder outlet obstruction (BOO), wherein vibegron is to be administered orally to the subject in need thereof, and wherein the therapeutically effective amount is about 75 mg of vibegron per day.

**2.** The use of claim 1, further comprising monitoring the subject for signs and/or symptoms of urinary retention.

**3.** The use of claim 1, further comprising monitoring the subject for signs and symptoms of urinary retention.

**4.** The use of claim 1, wherein the subject does not experience urinary retention.

**5.** The use of claim 1, wherein the subject experiences urinary retention.

**6.** The use of claim 1, wherein treating overactive bladder comprises treating one or more symptoms selected from the group consisting of urge urinary incontinence (UUI), urinary urgency, urinary frequency, nocturia, and a combination thereof.

**7.** The use of claim 1, wherein vibegron is to be orally administered as a crushed pharmaceutical unit dose comprising vibegron.

**8.** The use of claim 1, wherein vibegron is to be administered with a meal.

**9.** The use of claim 1, wherein vibegron is to be administered more than two hours before a meal.

**10.** The use of claim 1, wherein vibegron is to be administered once per day.

**11.** The use of claim 1, wherein the subject is human.

**12.** The use of claim 1, wherein the subject is male.

**13.** The use of claim 1, wherein vibegron is to be administered as a pharmaceutically acceptable salt thereof.

**14.** The use of claim 1, wherein vibegron is to be administered as a free base.

**15.** The use of claim 15, wherein vibegron is to be administered as a tablet.

FIGURE 1

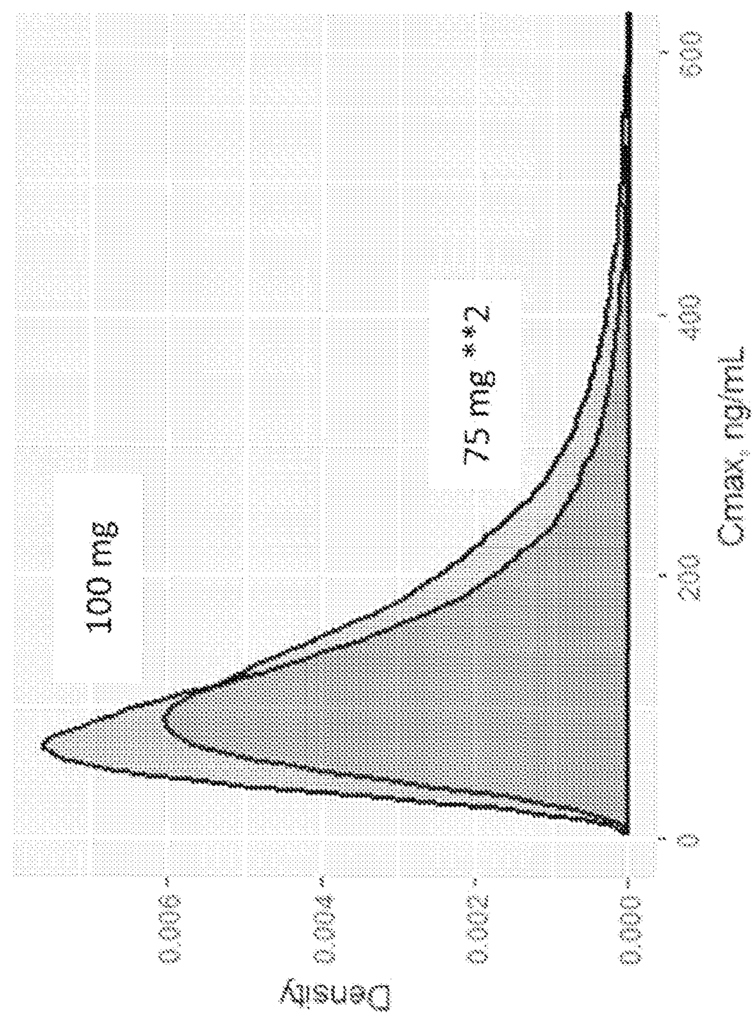
100 mg
75 mg **2
Density
0.000
0.002
0.004
0.006
0
200
400
600
Cmax, ng/mL

FIGURE 2

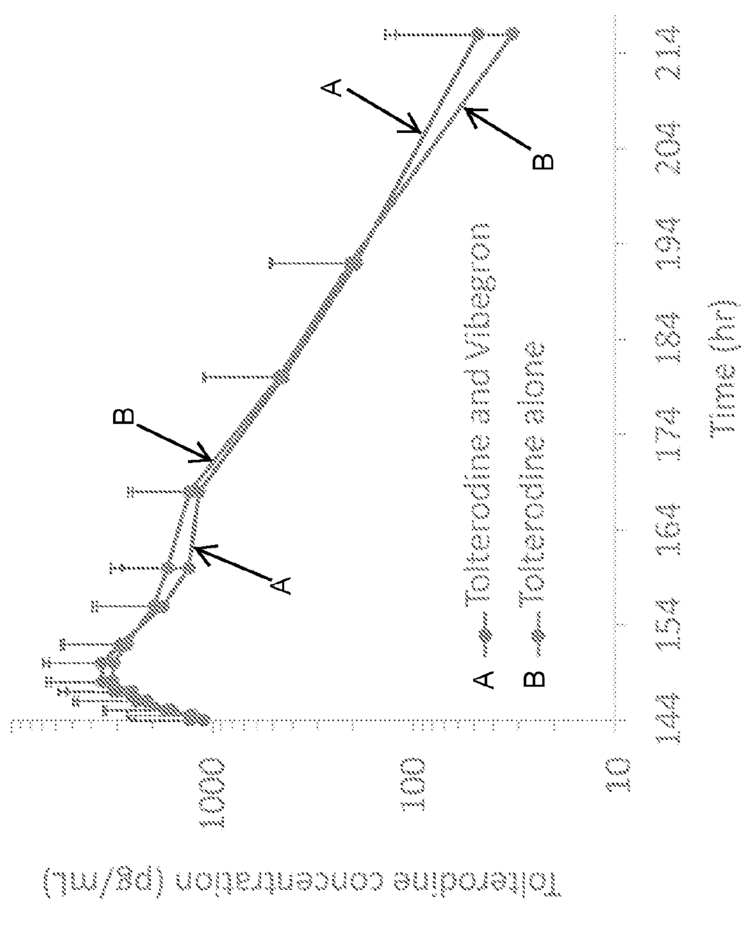
Tolterodine concentration (pg/mL)
1000
100
10
144
154
164
174
184
194
204
214
Time (hr)
A —◆— Tolterodine and Vibegron
B —◆— Tolterodine alone
A
B
A
B

FIGURE 3

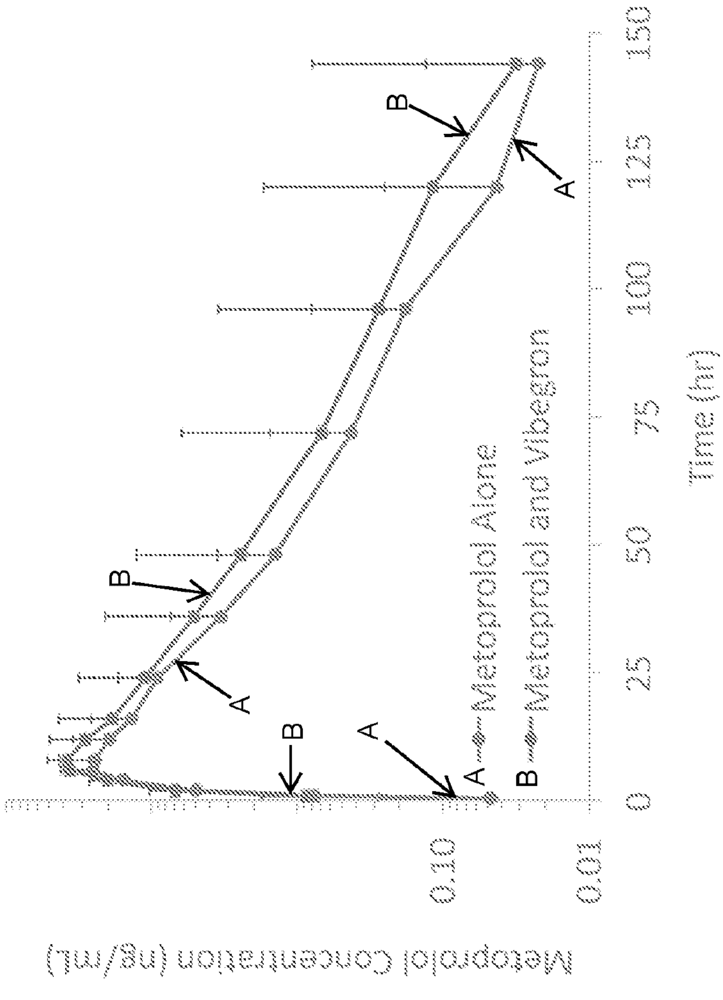
Metoprolol Concentration (ng/mL)
0.10
0.01
0
25
50
75
100
125
150
Time (hr)
A Metoprolol Alone
B Metoprolol and Vibegron
A
B

FIGURE 4

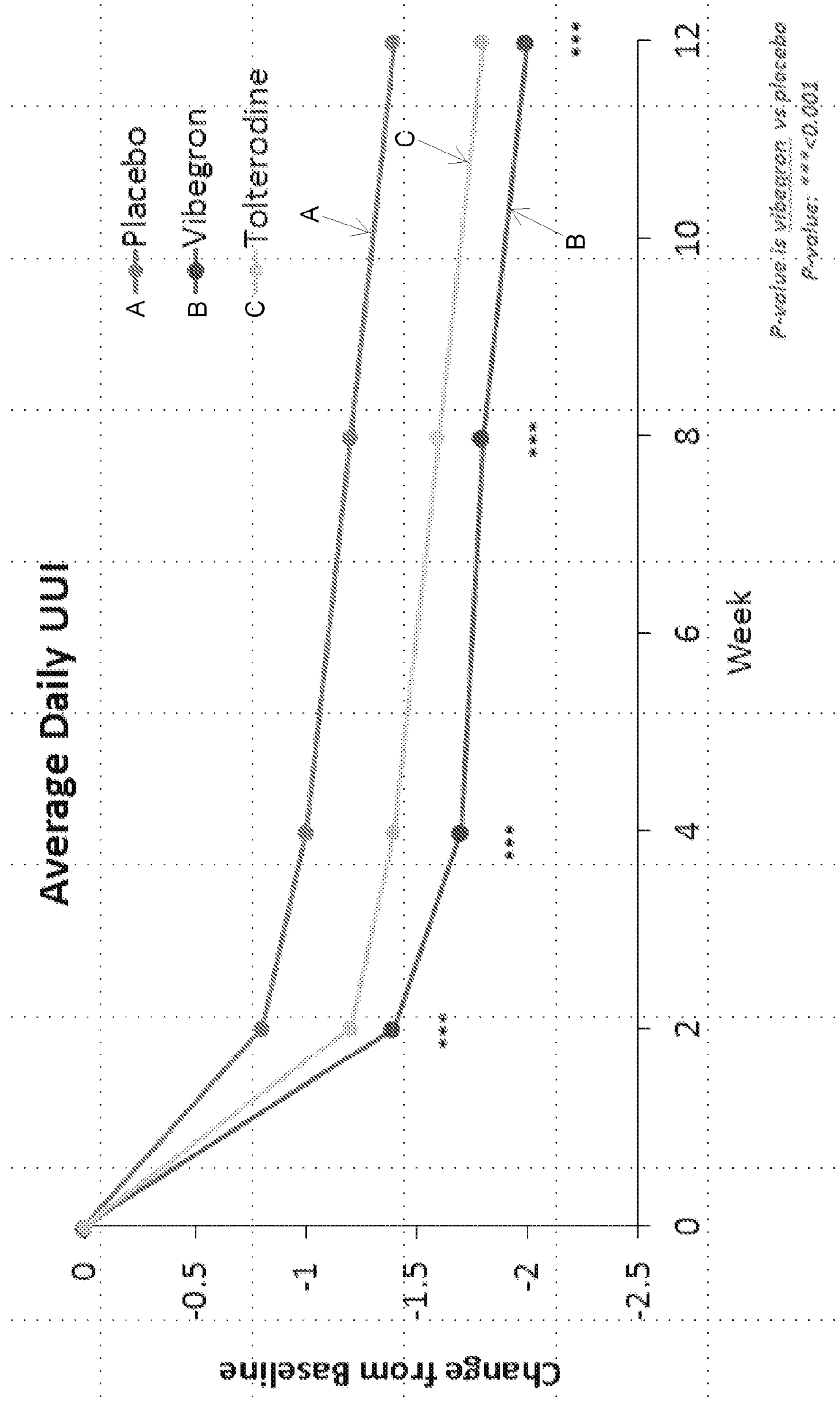
Average Daily UUI
A Placebo
B Vibegron
C Tolterodine
Change from Baseline
0
-0.5
-1
-1.5
-2
-2.5
Week
0
2
4
6
8
10
12
A
B
C
***
***
***
***
P-value is vibegron vs placebo
P-value: *** <0.001

FIGURE 5

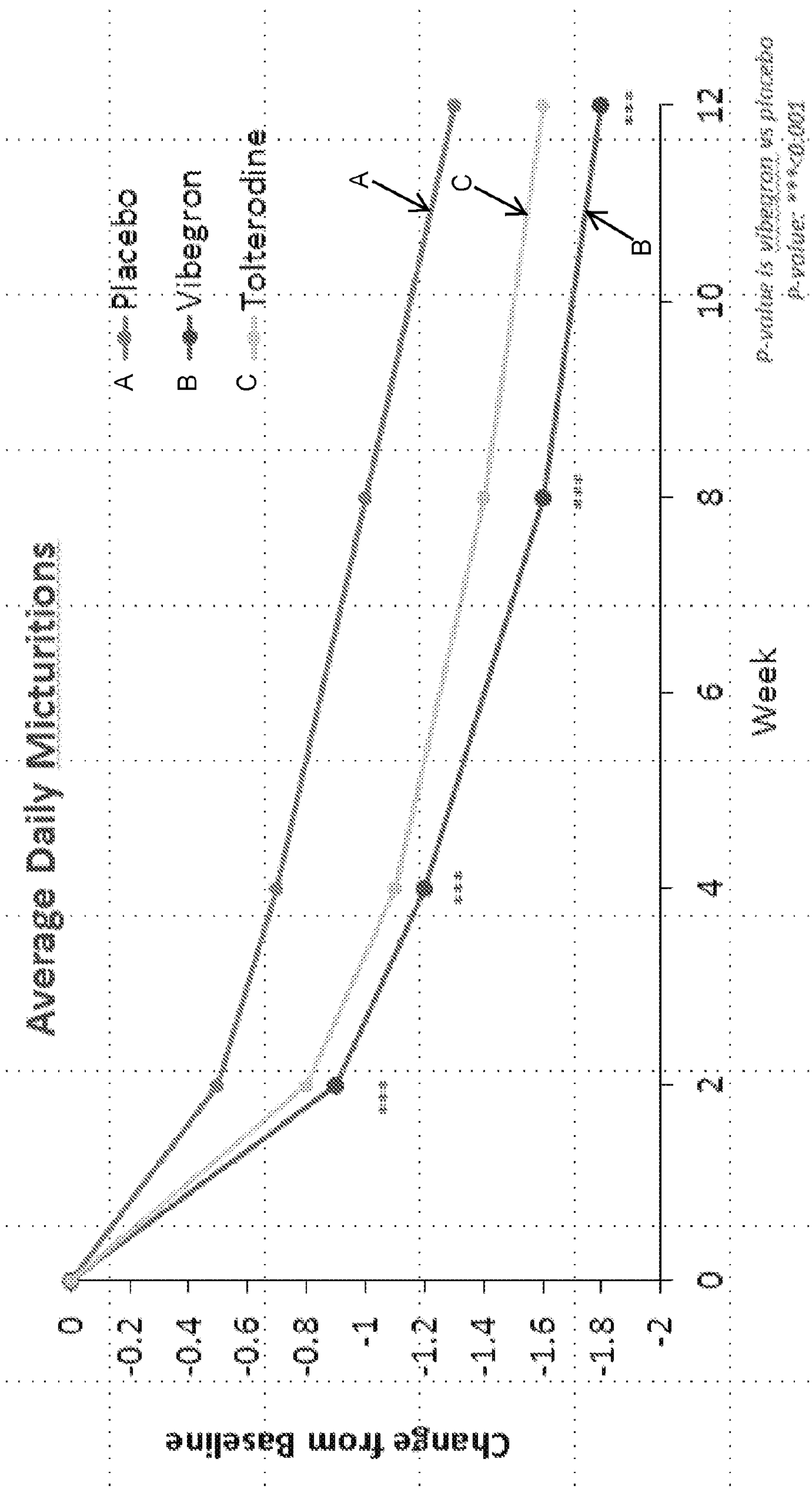
Average Daily Micturitions
Change from Baseline
0
-0.2
-0.4
-0.6
-0.8
-1
-1.2
-1.4
-1.6
-1.8
-2
0
2
4
6
8
10
12
Week
A Placebo
B Vibegron
C Tolterodine
A
B
C
***
p-value is vibegron vs placebo
p-value: ***<0.001

FIGURE 6

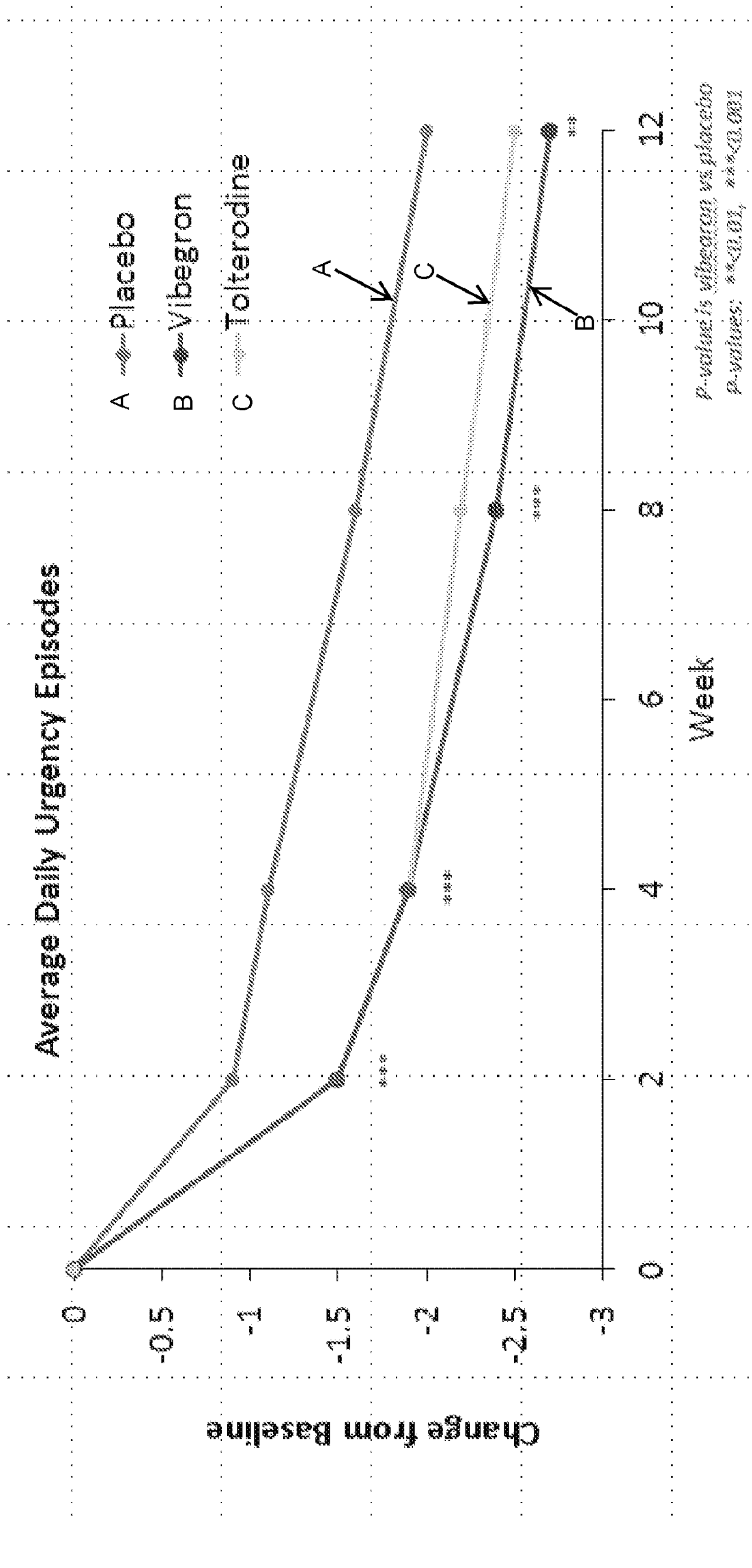
Average Daily Urgency Episodes
Change from Baseline
0
-0.5
-1
-1.5
-2
-2.5
-3
Week
0
2
4
6
8
10
12
A Placebo
B Vibegron
C Tolterodine
A
B
C
***
***
***
**
p-value is vibegron vs placebo
p-values: **<0.01, ***<0.001

FIGURE 7

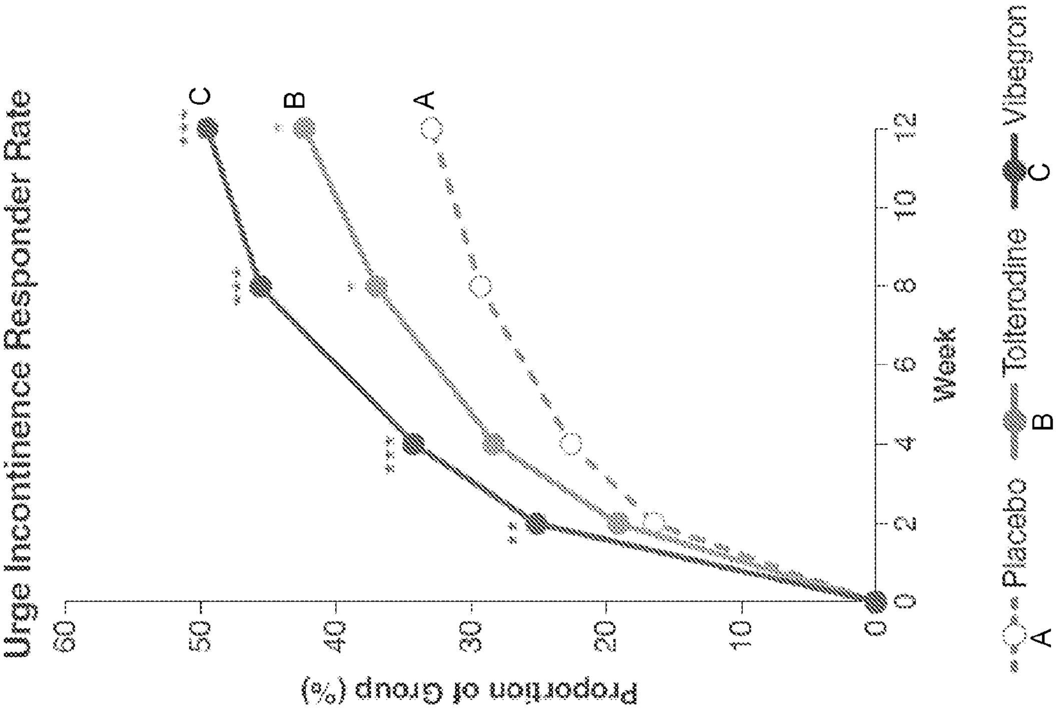
Urge Incontinence Responder Rate
Proportion of Group (%)
Week
Placebo
Tolterodine
Vibegron
A
B
C

FIGURE 8

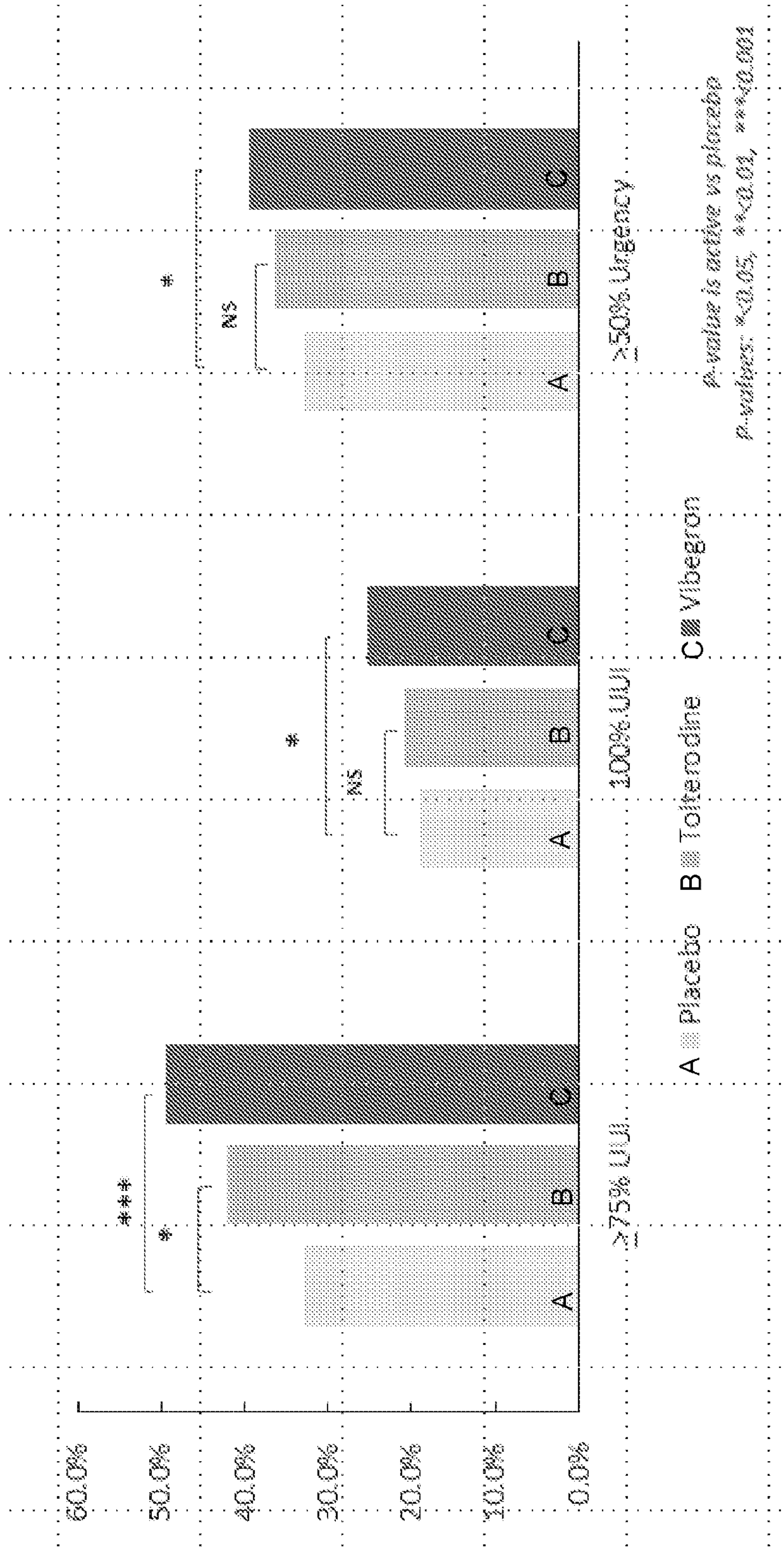
60.0%
50.0%
40.0%
30.0%
20.0%
10.0%
0.0%
***
*
NS
A
B
C
≥75% UUI
100% UUI
≥50% Urgency
A Placebo
B Tolterodine
C Vibegron
p-value is active vs placebo
p-values: *<0.05, **<0.01, ***<0.001

FIGURE 9

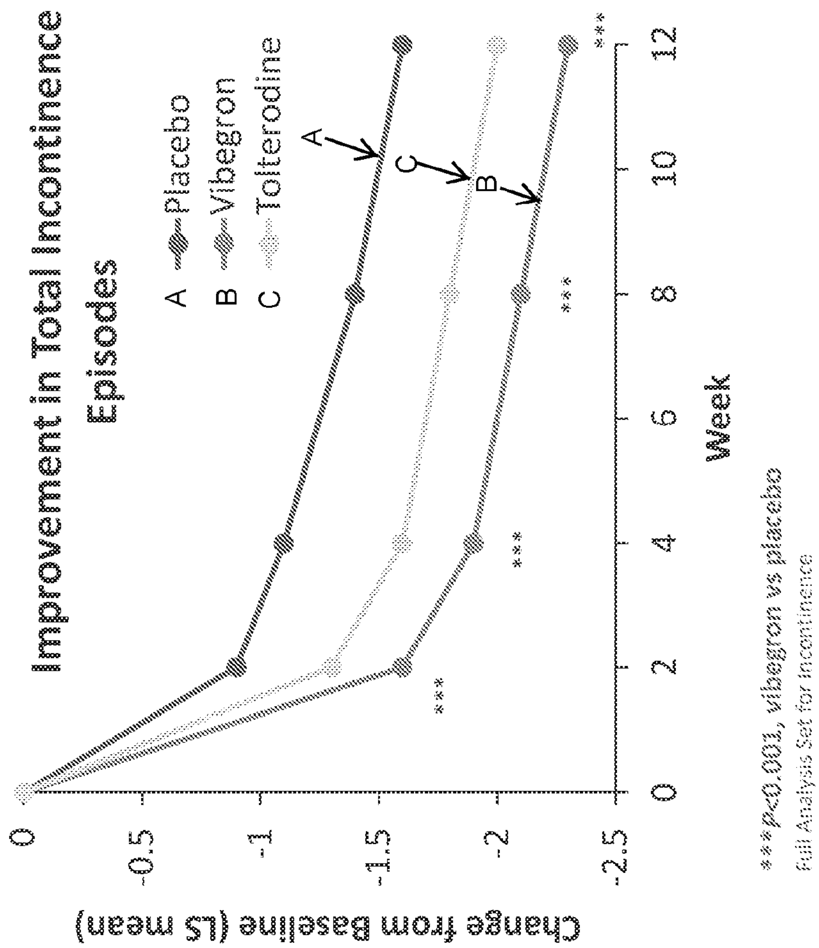
Improvement in Total Incontinence Episodes
A Placebo
B Vibegron
C Tolterodine
A
B
C
Change from Baseline (LS mean)
0
-0.5
-1
-1.5
-2
-2.5
0
2
4
6
8
10
12
Week
***
***
***
***
***p<0.001, vibegron vs placebo
Full Analysis Set for Incontinence

FIGURE 10

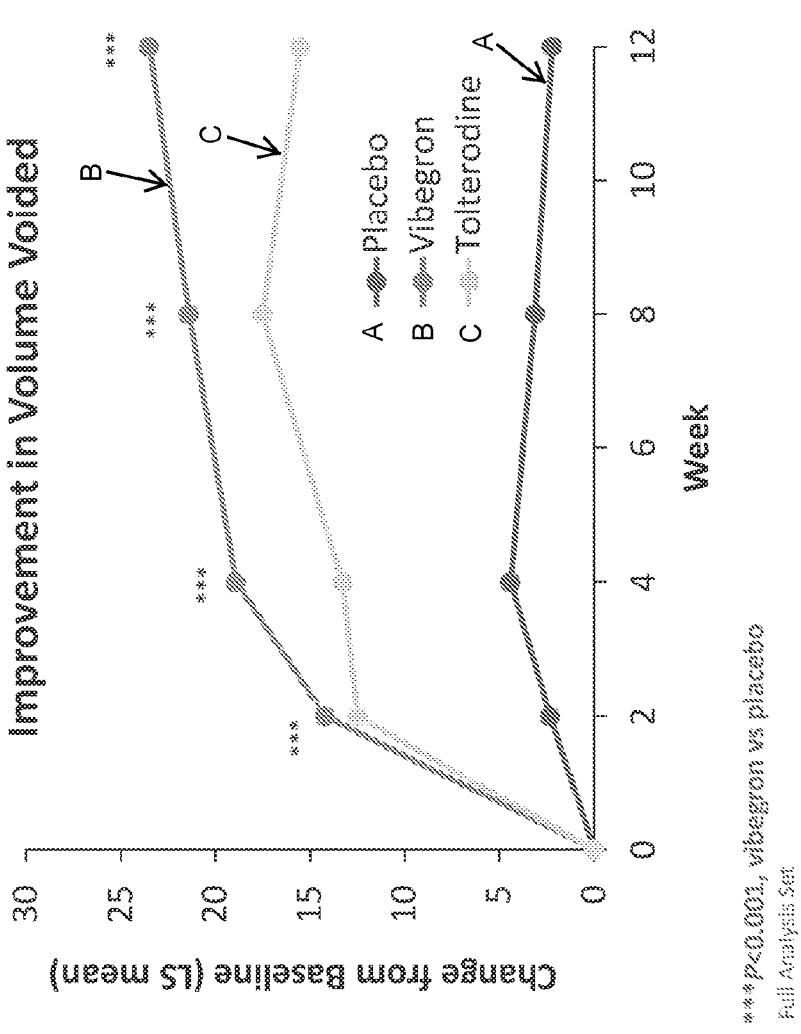
Improvement in Volume Voided
Change from Baseline (LS mean)
30
25
20
15
10
5
0
0
2
4
6
8
10
12
Week
***
***
***
***
A Placebo
B Vibegron
C Tolterodine
A
B
C
***p<0.001, vibegron vs placebo
Full Analysis Set

# FIGURE 11

| Procedure | Screening (up to 45 days prior to Day 1) | Treatment Period (Days | | | | | | | Early Termination | Follow up (7-10 days post last dose) telephone call |
|---|---|---|---|---|---|---|---|---|---|---|
| | | -1 | 1 | 2-14 | 15 | 16-27 | 28 | 29 | | |
| Visit | Visit 1 | Visit 2 | Visit 3 | | Visit 4 | | Visit 5 | Visit 6 | | |
| Window Visit | | -2 days | | | ± 2 days | | + 4 days | +4 days | | |
| Informed consent | X | | | | | | | | | |
| Office Visit | X | X | X | | X | | X | X | X | |
| Telephone Call[1] | | | | X | | X | | | | |
| Inclusion and Exclusion criteria | X | X | | | | | | | | |
| Demography | X | | | | | | | | | |
| Physical exam | X | | | | | | | X | | |
| Medical history | X | | | | | | | | | |
| Pregnancy test (serum or urine)[2] | X | X | | | | | | | X | |
| Urine Drug Screen | X | | | | | | | | | |
| Laboratory assessments[3] | X | X | | | X | | | X | X | |
| Review screening lab assessments | | X | | | | | | | | |
| Bladder scan[4] | X | | | | | | | | | |
| 12-lead ECG | X | | | | | | | | | |
| Vital signs[5] | X | X | X | | X | | X | X | X | |
| ABP Monitoring[6] | | X | X | | | | X | X | | |
| Witnessed Dose | | | X | | X | | X | | | |
| At Home Dose | | | | X | | X | | | | |
| PK samples (PK Subset Only)[7] | | | | | X | | | | | |
| AE/SAE Collection | X | X | X | X | X | X | X | X | X | X |
| Concomitant Medication Review | X | X | X | | X | | X | X | X | X |

1. Phone call on day prior to Day 15 and Day 28 to remind subject of office visit
2. Serum pregnancy test at screening and urine test at other visits for women of childbearing potential only
3. All lab assessments will include liver chemistries and INR.
4. Vital signs will be performed on Day -1 prior to fitting the ABPM device, Day 15 prior to PK sampling, and Day 28 prior to fitting the ABPM device at 1 min intervals at least 15 minutes after arrival to the clinic but before blood draws and prior to dosing (Day 15 and Day 28).
5. Optional second visit during screening to perform bladder scan
6. Subjects will return on Day 1 and Day 29 to assess the validity of the data.
7. PK samples will be collected on Day 15 (± 2 days) at pre-dose and at 0.5, 1, 2, 3, and 4.5 hours post dose for vibegron concentrations (for subjects who have consented to PK sampling)

FIGURE 12

**90% CI for Treatment Difference in Day 28 Change from Baseline ABPM Systolic Blood Pressure**

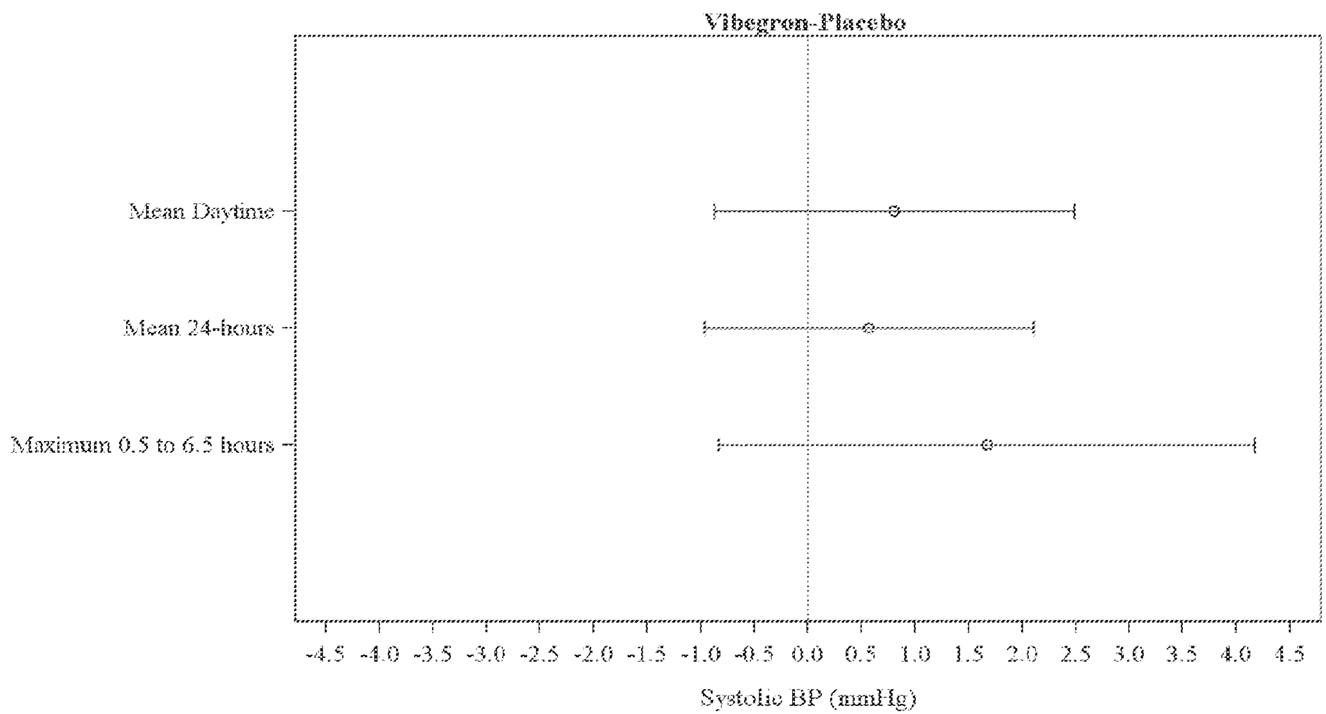


ABPM = Ambulatory Blood Pressure Monitoring, SBP = Systolic Blood Pressure, CI = Confidence Interval

FIGURE 13

**90% CI for Treatment Difference in Day 28 Change from Baseline ABPM Diastolic Blood Pressure**

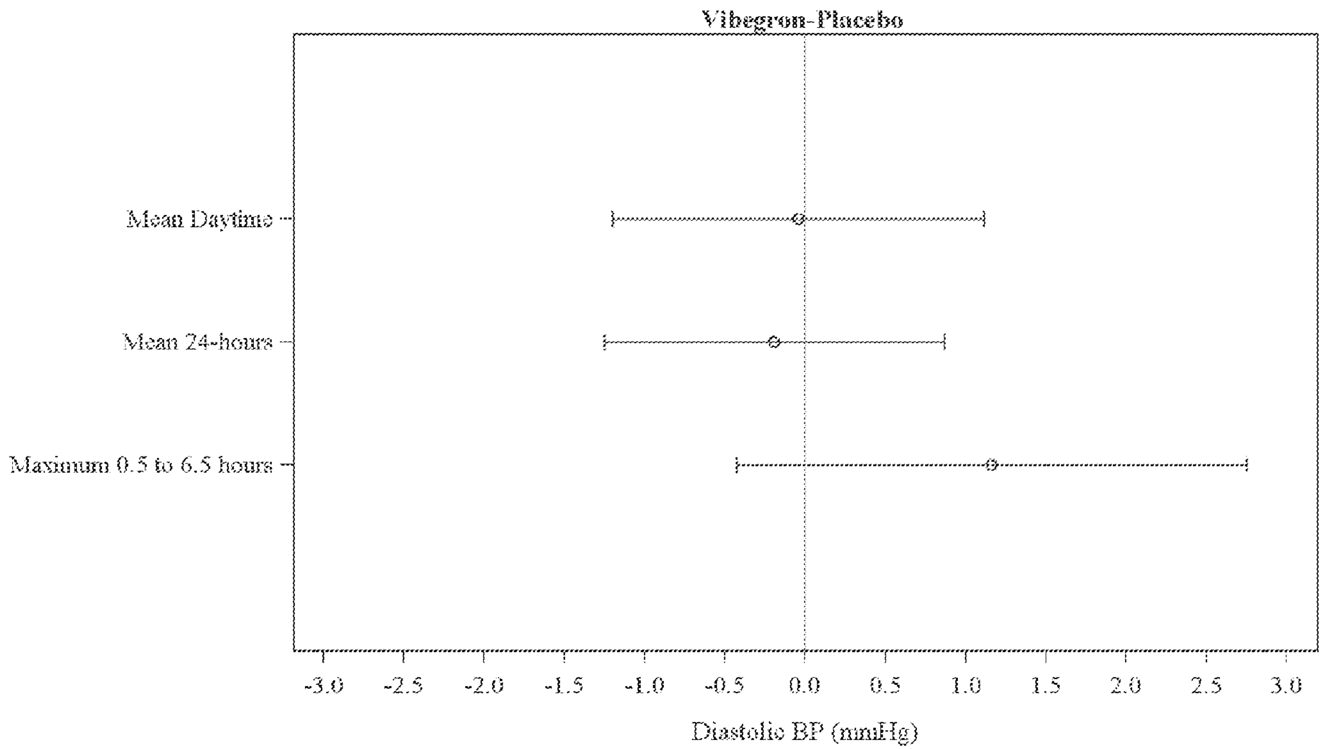


ABPM = Ambulatory Blood Pressure Monitoring, DBP = Diastolic Blood Pressure, CI = Confidence Interval

FIGURE 14

90% CI for Treatment Difference in Day 28 Change from Baseline
ABPM Heart Rate

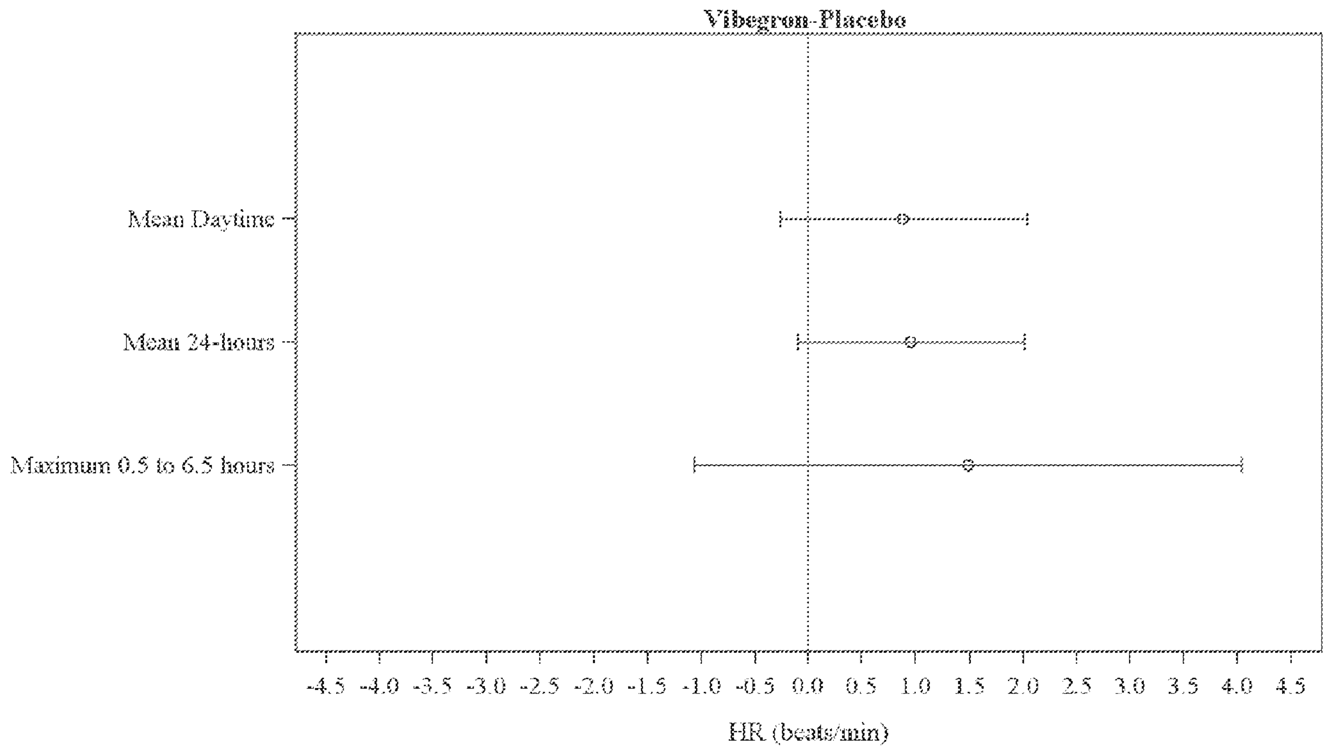


ABPM = Ambulatory Blood Pressure Monitoring, HR = Heart Rate, CI = Confidence Interval

**FIGURE 15**

## Reduction in Micturitions Over Time

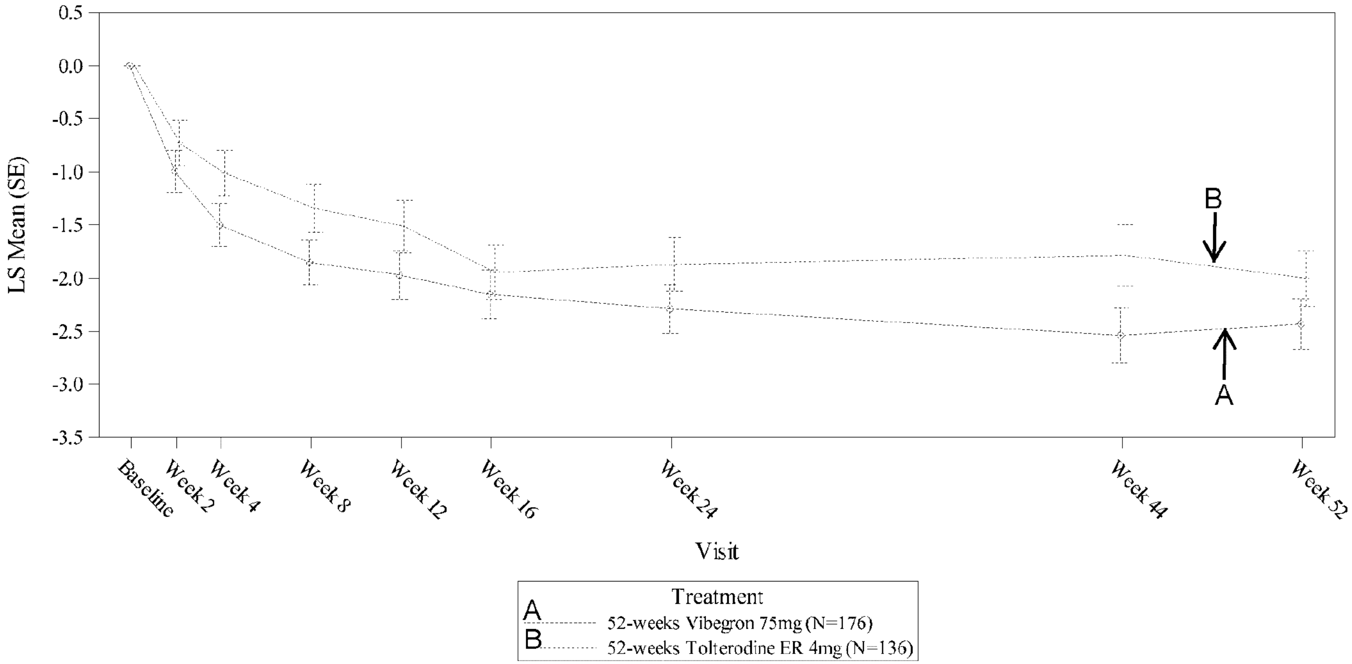


Full Analysis Set Extension, CFB Least squares mean; 52-week groups only. Covariates included in the mixed model for repeated measures are study visit, treatment, treatment by study visit interaction, baseline, OAB type and sex

Baseline value is computed using the Run-in diary of parent study.

# FIGURE 16

## Reduction in Urinary Urge Incontinence (UUI) Episodes Over Time

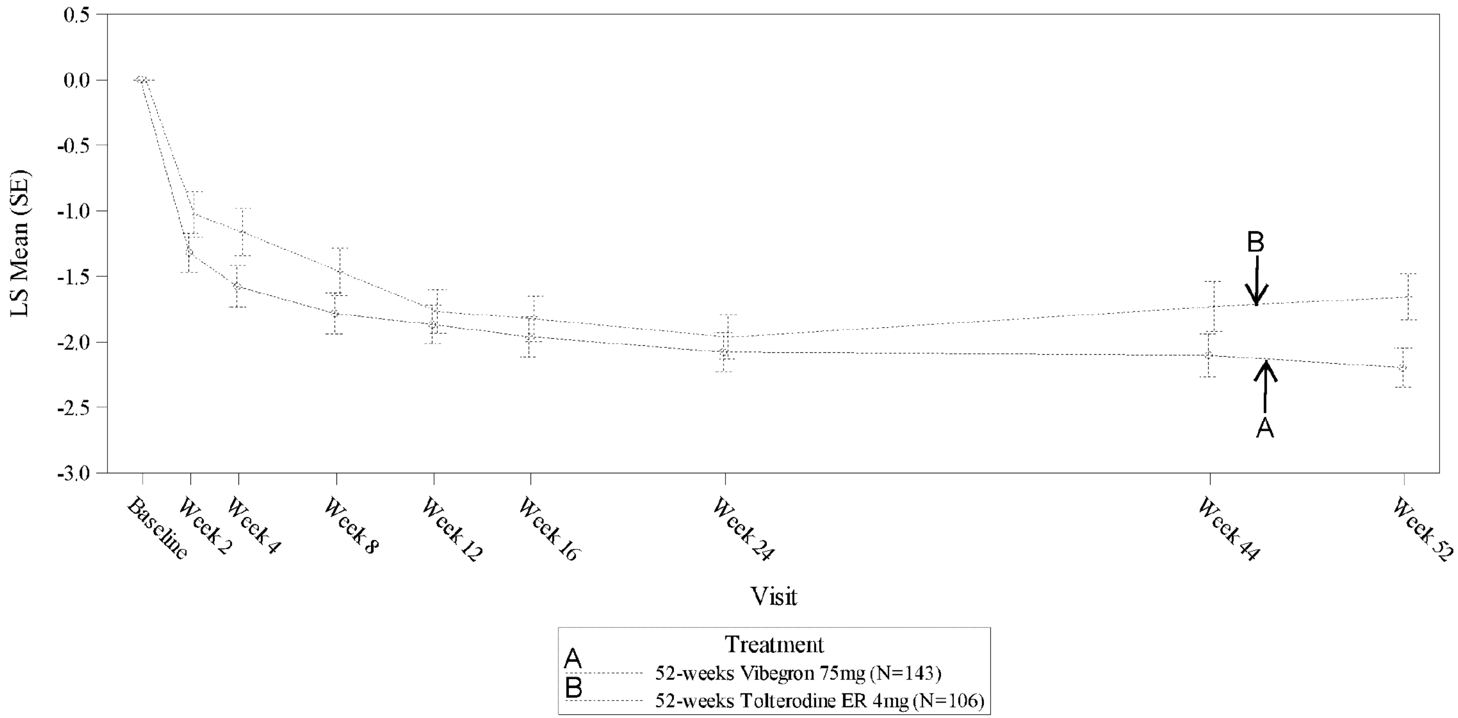


Full Analysis Set Extension for Incontinence, CFB Least squares mean; 52-week groups only. Covariates included in the mixed model for repeated measures are study visit, treatment, treatment by study visit interaction, baseline and sex

Baseline value is computed using the Run-in diary of parent study.

# FIGURE 17

## Reduction in Urgency Episodes Over Time

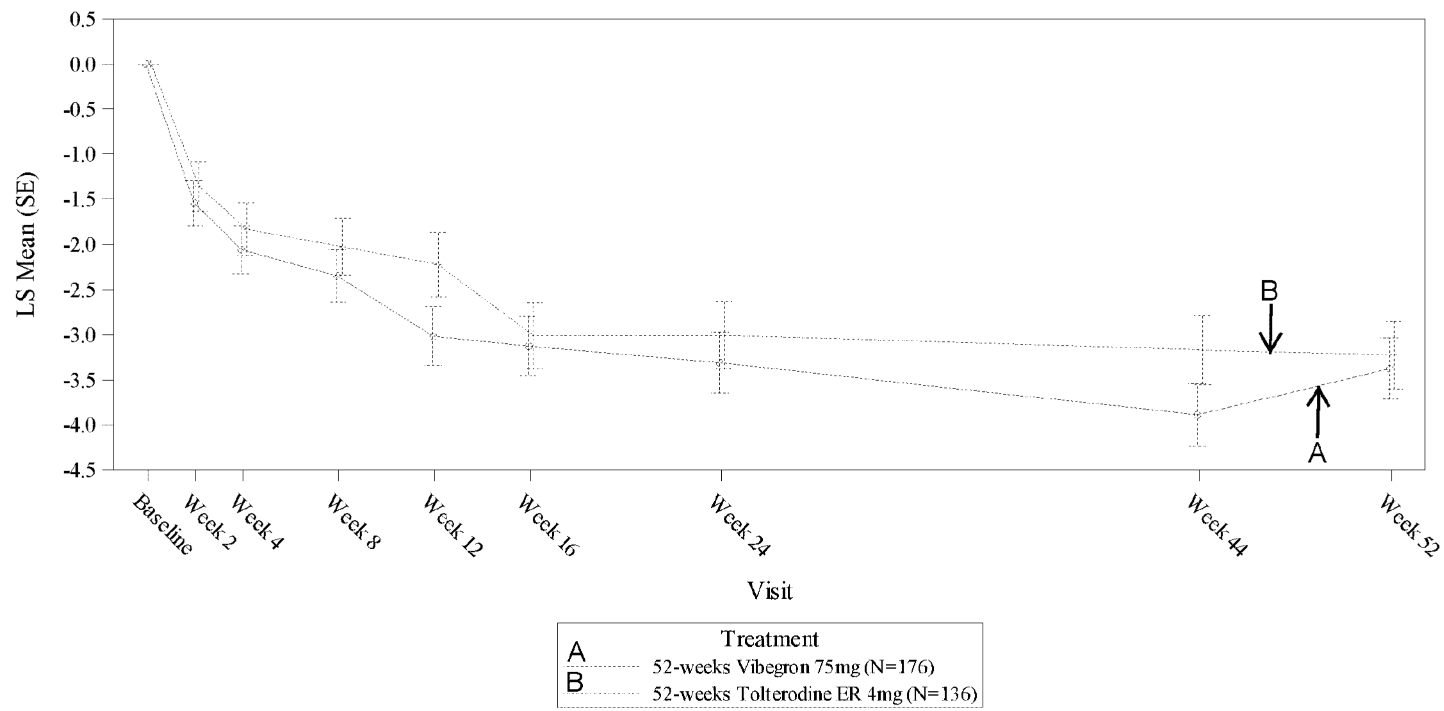


Full Analysis Set, CFB Least squares mean; 52-week groups only. Covariates included in the mixed model for repeated measures are study visit, treatment, treatment by study visit interaction, baseline, OAB type and sex

Baseline value is computed using the Run-in diary of parent study.

# FIGURE 18

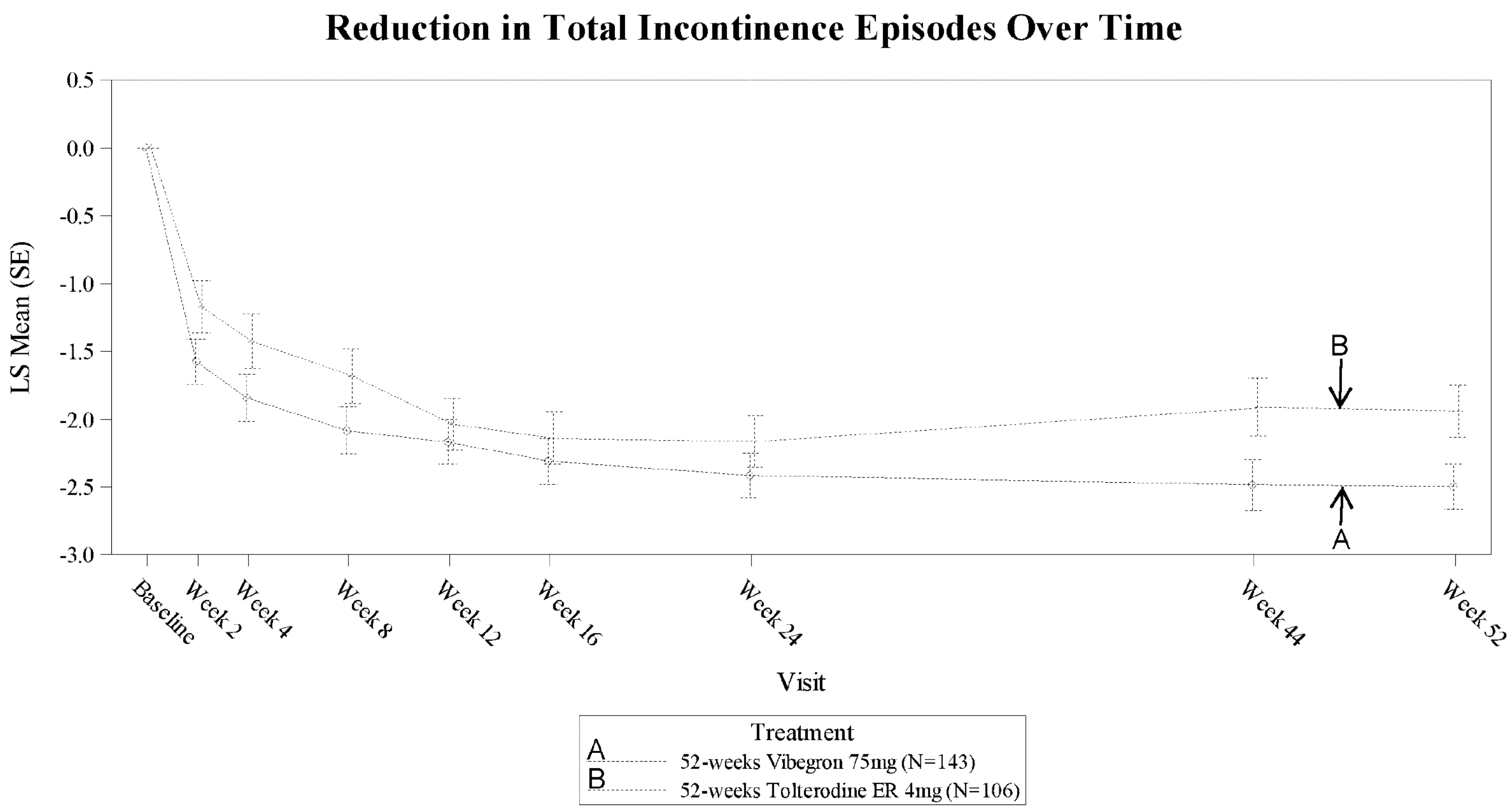


Full Analysis Set Extension for Incontinence, CFB Least squares mean; 52-week groups only. Covariates included in the mixed model for repeated measures are study visit, treatment, treatment by study visit interaction, baseline and sex

Baseline value is computed using the Run-in diary of parent study.

# FIGURE 19

## Kaplan Meier Plot of Time to 75% Reduction in Urinary Urge Incontinence (UUI)

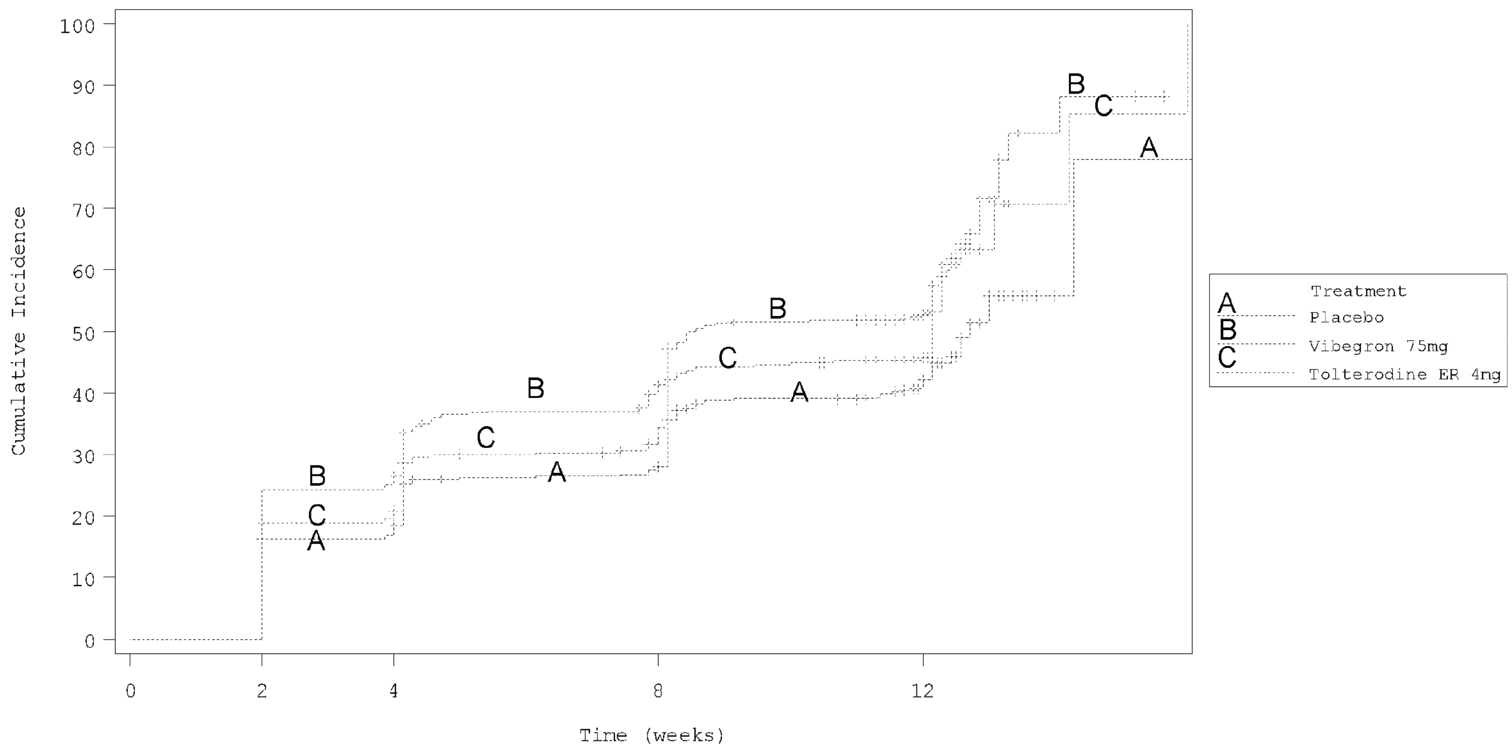


Note: Planned assessments are at Weeks 2, 4, 8 and 12

# FIGURE 20

## Kaplan Meier Plot of Time to 50% Reduction in Urgency

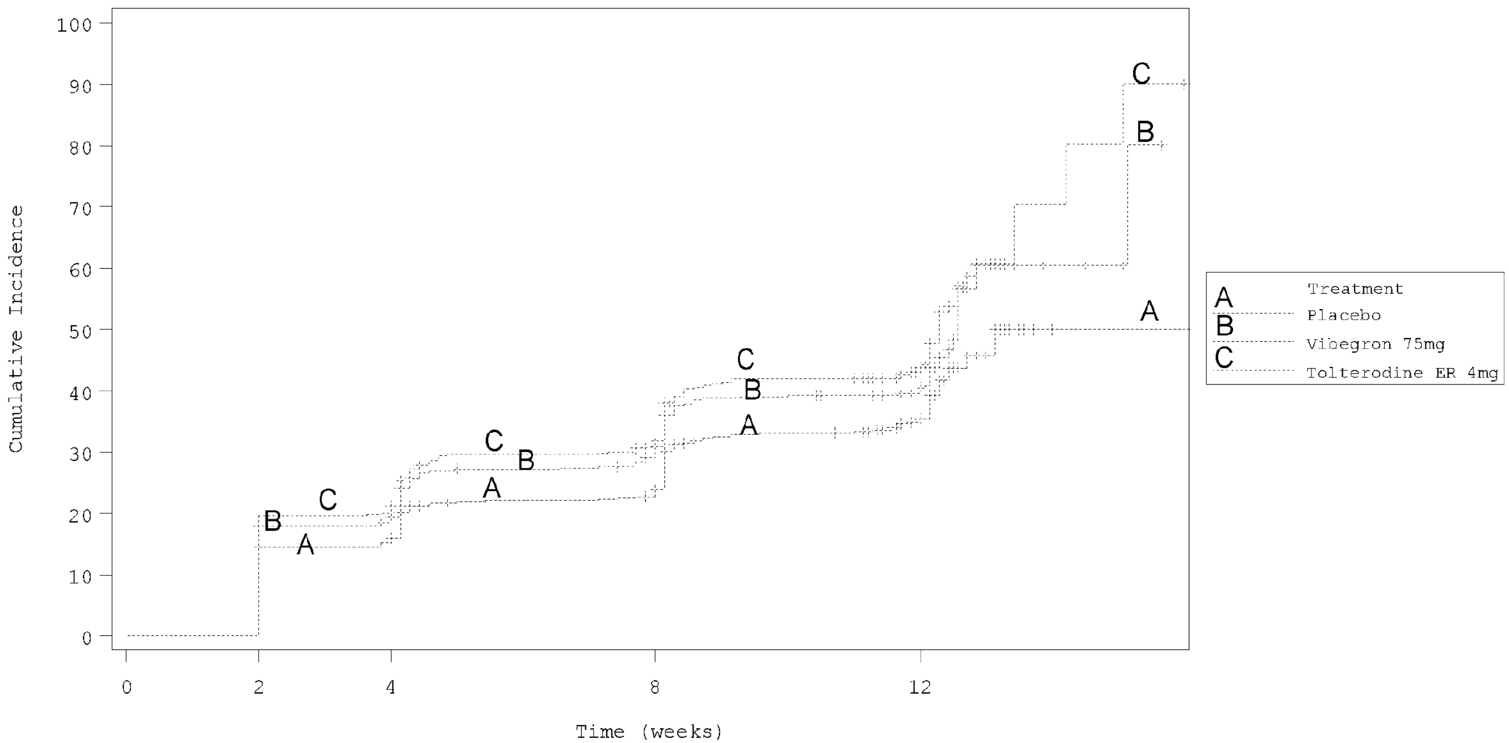


Note: Planned assessments are at Weeks 2, 4, 8 and 12. K-M estimates are unadjusted for covariates.

# FIGURE 21

## Plot of LS Mean (SE) of Change from Baseline Urgency in Dry Population

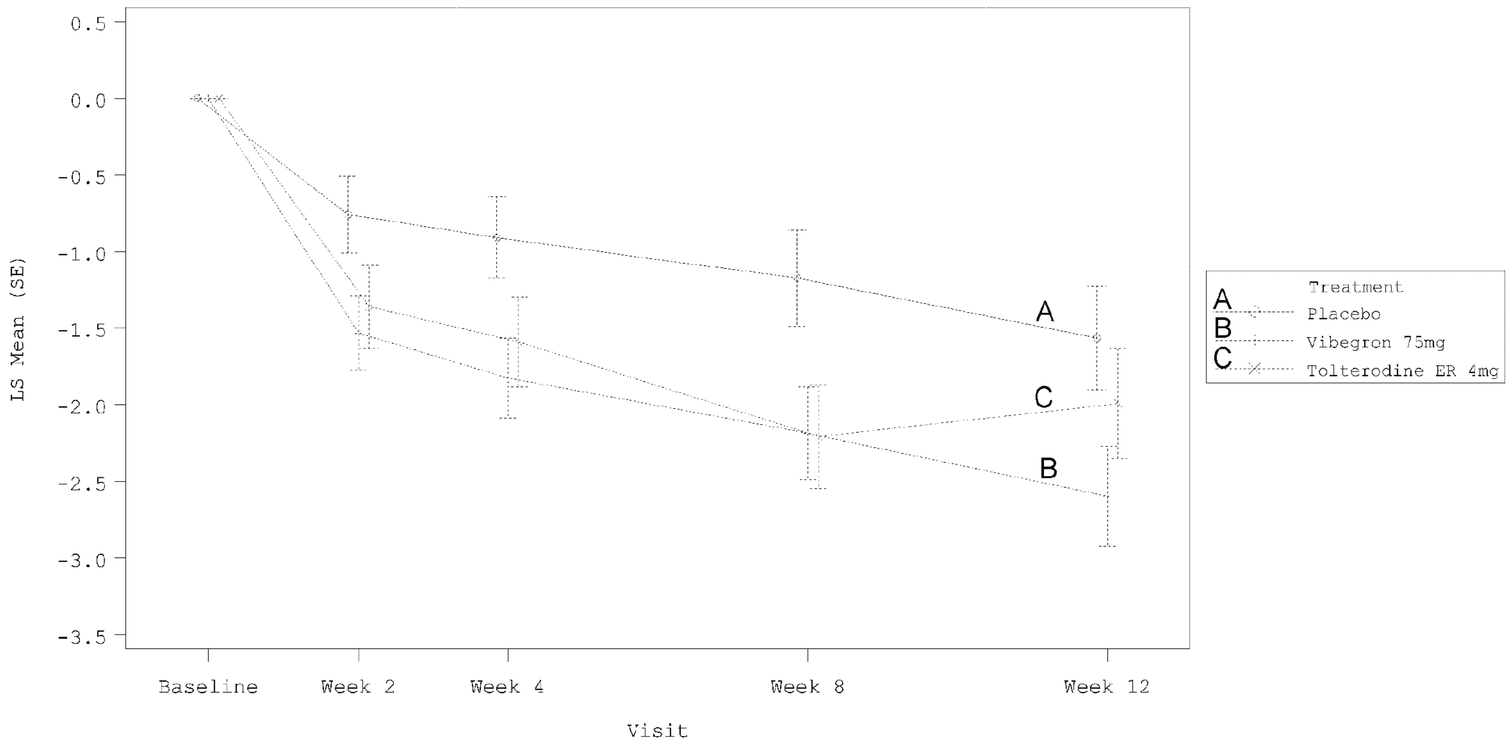

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8399480 B
- US 8247415 B
- WO 2018224989 A
- US 20170145014 A
- US 20150087832 A
- US 20160176884 A
- US 20140242645 A


### Non-patent literature cited in the description


- **GRAY SL et al.** *JAMA Intern Med*, 2015, vol. 175 (3), 401-407
- **EDMONDSON et al.** *J. Med. Chem.*, 2016, vol. 59, 609-623
- **ABRAMS P et al.** *Urology*, 2003, vol. 61 (1), 37-49
- **ABRAMS P et al.** *Urology*, 2003, vol. 62 (5B), 28-37, 40-42
- *Journal of Pharmaceutical Science*, 1977, vol. 66, 2
- **CHAPPLE et al.** Terminology report from the International Continence Society (ICS) Working Group on Underactive Bladder (UAB). *Neurology and Urodynamics*, 2018, vol. 37, 2928-2931
- **COYNE et al.** The responsiveness of the Overactive Bladder Questionnaire (OAB-q). *Quality of Life Research*, 2005, vol. 14, 849-855
- **COYNE et al.** Psychometric validation of an overactive bladder symptom and health-related quality of life questionnaire: The OAB-q. *Quality of Life Research*, 2002, vol. 11, 563-574
- Remington's Pharmaceutical Sciences. Mack Publishing, 2000
- Guidance for Industry, Patient-Reported Outcome Measures: Use in Medical Product Development to Support Labeling Embodiments. U.S. Department of Health and Human Services, Food and Drug Administration, December 2009